(19) Europäisches Patentamt
European Patent Office
Office européen des brevets

(11) EP 4 596 561 A1

(12) EUROPEAN PATENT APPLICATION
published in accordance with Art. 153(4) EPC

(43) Date of publication:
**06.08.2025 Bulletin 2025/32**

(21) Application number: **23881973.4**

(22) Date of filing: **27.10.2023**

(51) International Patent Classification (IPC):
***C07F 9/572*** *(2006.01)* ***C07F 9/6506*** *(2006.01)*
***C07F 9/6584*** *(2006.01)* ***A61K 31/66*** *(2006.01)*
***A61K 31/505*** *(2006.01)* ***A61P 35/00*** *(2006.01)*

(52) Cooperative Patent Classification (CPC):
**A61K 31/505; A61K 31/66; A61K 31/675; A61P 35/00; C07F 9/53; C07F 9/572; C07F 9/58; C07F 9/6506; C07F 9/6558; C07F 9/6584**

(86) International application number:
**PCT/CN2023/127202**

(87) International publication number:
**WO 2024/088398 (02.05.2024 Gazette 2024/18)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **27.10.2022 CN 202211328612**
**30.03.2023 CN 202310331481**
**20.10.2023 CN 202311368432**
**20.10.2023 CN 202311368477**
**20.10.2023 CN 202311368532**

(71) Applicant: **Suzhou Genhouse Bio Co., Ltd.**
**Suzhou,Jiangsu 215123 (CN)**

(72) Inventors:
- **ZHANG, Guiping**
  **Suzhou, Jiangsu 215123 (CN)**
- **LI, Jiapeng**
  **Suzhou, Jiangsu 215123 (CN)**
- **WANG, Kuifeng**
  **Suzhou, Jiangsu 215123 (CN)**
- **DUAN, Shuangshuang**
  **Suzhou, Jiangsu 215123 (CN)**
- **ZHENG, Jiyue**
  **Suzhou, Jiangsu 215123 (CN)**
- **TONG, Chenhua**
  **Suzhou, Jiangsu 215123 (CN)**
- **WANG, Xiang**
  **Suzhou, Jiangsu 215123 (CN)**
- **XIE, Shengwu**
  **Suzhou, Jiangsu 215123 (CN)**
- **LIU, Tao**
  **Suzhou, Jiangsu 215123 (CN)**

(74) Representative: **Murgitroyd & Company**
**165-169 Scotland Street**
**Glasgow G5 8PL (GB)**

Remarks:
The complete document including Reference Table(s) and the Sequence Listing(s) can be downloaded from the EPO website

# (54) PHOSPHORUS-CONTAINING COMPOUND, PHARMACEUTICAL COMPOSITION AND USE THEREOF

(57) Provided in the present invention are a phosphorus-containing compound, a pharmaceutical composition and the use thereof. Specifically, provided are a compound as represented by formula I, a pharmaceutically acceptable salt, an ester, a stereoisomer, a tautomer, a polymorph, a solvate, a metabolite, an isotopic derivative or a prodrug thereof. The phosphorus-containing compound of the present invention can effectively treat diseases and conditions mediated by TEAD, such as cancer.

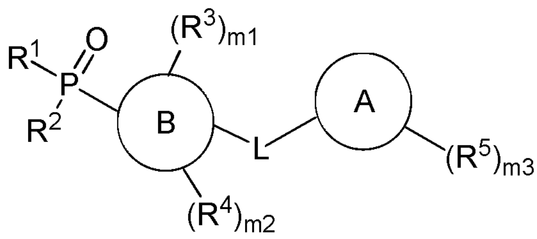

I



Processed by Luminess, 75001 PARIS (FR)

## Description

The present application claims priority to Chinese Patent Application No. 202211328612.4, filed on October 27, 2022, which is incorporated herein by reference in its entirety.
The present application claims priority to Chinese Patent Application No. 202310331481.3, filed on March 30, 2023, which is incorporated herein by reference in its entirety.
The present application claims priority to Chinese Patent Application No. 202311368477.0, filed on October 20, 2023, which is incorporated herein by reference in its entirety.
The present application claims priority to Chinese Patent Application No. 202311368432.3, filed on October 20, 2023, which is incorporated herein by reference in its entirety.
The present application claims priority to Chinese Patent Application No. 202311368532.6, filed on October 20, 2023, which is incorporated herein by reference in its entirety.

## TECHNICAL FIELD

The present disclosure relates to a phosphorus-containing compound, a pharmaceutical composition, and use thereof.

## BACKGROUND

The Hippo signaling pathway is a highly conserved cell signaling pathway from Drosophila to mammals. The main functions of this pathway include regulating the normal development of the organism, determining the size ratio of tissues and organs, and maintaining the balance between cell proliferation and apoptosis, as well as the stemness of stem cells. The core components of the signaling pathway mainly consist of (1) upstream signaling factors; (2) the core kinase cascade; and (3) downstream effector molecules. Once activated by upstream stimulating factors (such as cell contact inhibition), MST1/2 kinases and SAV1 interact with upstream regulatory factors and become activated through phosphorylation. This in turn phosphorylates MOB1A/B and LATS1/2, leading to the full activation of the LATS1/2 kinase complex. The complex further phosphorylates the transcriptional coactivators YAP/TAZ. The phosphorylated YAP/TAZ are blocked in the cytoplasm by 14-3-3 proteins and are polyubiquitinated by the E3 ligase β-TrCP, thereby being recognized and degraded by proteasomes. When the Hippo pathway is inhibited (e.g., by serum factor stimulation), unphosphorylated YAP/TAZ enter the nucleus and mainly form transcription complexes with the TEAD protein family of transcription factors. This activates the transcription of downstream target genes (such as CTGF, FGF1, AMOTL2, and CYR61), promoting cell survival and proliferation. At the same time, the pathway can also regulate the self-renewal and differentiation of stem cells, thereby participating in tissue regeneration and wound healing. Therefore, under some conditions, the YAP/TAZ-TEAD transcription complex acts as an oncogene, while the Hippo pathway functions as a tumor suppressor.

Currently, in various tumors, including but not limited to liver cancer, lung cancer, ovarian cancer, brain cancer, malignant mesothelioma, breast cancer, head and neck cancer, colorectal cancer, prostate cancer, and leukemia, the inactivation or loss of upstream inhibitory factors in the Hippo signaling pathway, such as NF2, MST1/2, and LATS1/2, has been found. Meanwhile, abnormal amplification of YAP/TAZ and TEAD family proteins has been discovered in many tumors, and a large number of reports have identified TEAD family proteins as key mediators of the oncogenic ability of YAP/TAZ. Clinical studies suggest that the amplification of YAP/TAZ can serve as a prognostic and diagnostic biomarker for various tumors.

It is generally believed that the overactivation of YAP and TAZ can regulate cell proliferation, migration, and apoptosis, participate in the self-renewal and EMT (epithelial-mesenchymal transition) characteristics related to tumor stem cells, and promote the maintenance of the immunosuppressive effects on the tumor microenvironment, thereby mediating the resistance of tumor cells to conventional chemotherapy, targeted therapy, and immunotherapy. Therefore, targeting the YAP/TAZ-TEAD protein complex will be a highly promising method for treating various cancers with alterations in the functions of this pathway.

The transcription complex formed by YAP/TAZ and TEAD is mainly maintained by three protein-protein interaction interfaces, among which interface II and interface III are both crucial for the formation of this protein complex. However, both of these interfaces lack the geometrically characterized, electrostatically enriched pockets required for small molecules to bind to proteins. In recent years, researchers have found that the YAP-binding domain of the TEAD protein contains a pocket that binds to palmitic acid/myristic acid, and palmitic acid molecules mediate reversible palmitoylation of the TEAD protein at this site. The palmitoylation of the TEAD protein is crucial for its protein stability, as well as for the formation and transcriptional activity of the YAP/TAZ-TEAD transcription complex. Moreover, the inhibition of TEAD family proteins has no significant impact on the homeostasis of adult tissues and organs. Therefore, directly inhibiting the palmitoylation of TEAD family proteins can effectively hinder the oncogenic activity of YAP/TAZ and is particularly applicable for treating various tumors with inactivation of upstream Hippo genes.

**SUMMARY**

The present disclosure provides inhibitors related to one or more members of the Hippo pathway network, especially new compounds that inhibit the transcriptional activity of the YAP/TAZ-TEAD protein complex. The compounds of the present disclosure also have superior properties, including good physicochemical properties (such as solubility, physical and/or chemical stability), improved pharmacokinetic properties (such as improved bioavailability, improved metabolic stability, appropriate half-life, and duration of action), and enhanced safety (low toxicity (such as reduced cardiotoxicity) and/or few side effects).

The present disclosure provides a compound represented by formula I, a pharmaceutically acceptable salt thereof, an ester thereof, a stereoisomer thereof, a tautomer thereof, a polymorph thereof, a solvate thereof, a metabolite thereof, an isotopic derivative thereof, or a prodrug thereof,

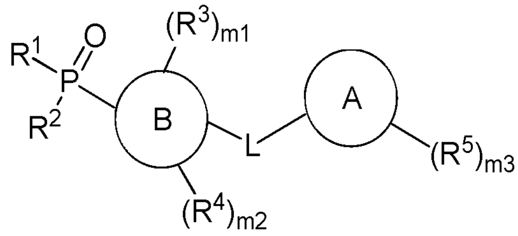

**I**

wherein

$R^1$ and $R^2$ are independently $C_1$-$C_6$ alkyl, $C_3$-$C_6$ cycloalkyl, or $C_6$-$C_{10}$ aryl;
or $R^1$ and $R^2$, together with the atom to which they are attached, form 3- to 12-membered heterocycloalkyl or 3- to 12-membered heterocycloalkyl substituted with one or more $R^{1-1}$, wherein the 3-to 12-membered heterocycloalkyl contains, in addition to the attached P atom, 0, 1, 2, or 3 heteroatoms selected from 1, 2, or 3 of N, O, and S;
$R^{1-1}$ is independently $C_1$-$C_6$ alkyl;
ring B is a $C_3$-$C_{12}$ saturated or unsaturated carbocyclic ring or a "5- to 16-membered saturated or unsaturated heterocyclic ring with 1, 2, 3, 4, or 5 heteroatoms selected from 1, 2, or 3 of N, O, and S";
ring B is monocyclic or polycyclic; when ring B is monocyclic, ring B is an aromatic ring; when ring B is polycyclic, the ring connected to

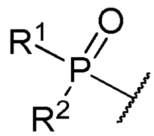

is an aromatic ring, with the remaining ring(s) being saturated or unsaturated ring(s);
m1 is 0 or 1;
$R^3$ is "5- to 12-membered saturated or unsaturated heterocyclyl with 1, 2, or 3 heteroatoms selected from 1, 2, or 3 of N, O, and S", "5- to 12-membered saturated or unsaturated heterocyclyl with 1, 2, or 3 heteroatoms selected from 1, 2, or 3 of N, O, and S" substituted with one or more $R^{3-1}$, $C_6$-$C_{10}$ aryl, or $C_6$-$C_{10}$ aryl substituted with one or more $R^{3-5}$;
$R^{3-1}$ and $R^{3-5}$ are independently H, deuterium, halogen, $C_1$-$C_6$ alkyl, $C_1$-$C_6$ alkoxy, CN, $C_1$-$C_6$ alkyl substituted with one or more $R^{3-2}$, $C_1$-$C_6$ alkoxy substituted with one or more $R^{3-3}$, "3- to 12-membered heterocycloalkyl with 1, 2, or 3 heteroatoms selected from 1, 2, or 3 of N, O, and S", "5- to 12-membered heteroaryl with 1, 2, or 3 heteroatoms selected from 1, 2, or 3 of N, O, and S", C(=O)$NR^cR^d$, $C_3$-$C_6$ cycloalkyl, or $C_3$-$C_6$ cycloalkyl substituted with one or more $R^{3-4}$;
$R^{3-2}$ and $R^{3-3}$ are independently deuterium, halogen, OH, CN, $C_1$-$C_6$ alkoxy, "3- to 12-membered heterocycloalkyl with 1, 2, or 3 heteroatoms selected from 1, 2, or 3 of N, O, and S", C(=O)$NR^cR^d$, or $NR^b$C(=O)$R^a$;
$R^{3-4}$ is independently CN, C(=O)$NR^cR^d$, or C(=O)$OR^a$;
m2 is 0, 1, 2, or 3;
$R^4$ is independently oxo (=O), CN, halogen, $C_1$-$C_6$ alkyl, $C_1$-$C_6$ haloalkyl, $C_1$-$C_6$ alkoxy, or $C_1$-$C_6$ haloalkoxy;
L is a single bond, $-CR^{L1}R^{L2}-$, -O-, -S-, $-NR^{L3}-$, $-NR^{L3}CR^{L1}R^{L2}-$, $-CR^{L4}=CR^{L5}-$,

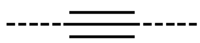,

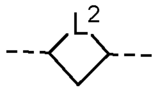 , 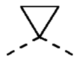 , or 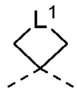 ;

$R^{L1}$, $R^{L2}$, and $R^{L3}$ are independently H, $C_1$-$C_6$ alkyl, or $C_3$-$C_6$ cycloalkyl;
$R^{L4}$ and $R^{L5}$ are independently H or $C_1$-$C_6$ alkyl;
$L^1$ and $L^2$ are independently $-CH_2-$, -O-, -S-, or -NH-;
ring A is a $C_3$-$C_{12}$ saturated or unsaturated carbocyclic ring or a "5- to 12-membered saturated or unsaturated heterocyclic ring with 1, 2, or 3 heteroatoms selected from 1, 2, or 3 of N, O, and S";
m3 is 0, 1, 2, or 3;
$R^5$ is independently $SF_5$, halogen, $C_1$-$C_6$ alkyl, $C_1$-$C_6$ alkyl substituted with one or more $R^{5-1}$, $SR^{5-2}$, $OR^{5-3}$, CN, $S(=O)_2R^{5-4}$, $C(=O)R^{5-5}$, $S(=O)_2NR^dR^{5-6}$, $NR^dR^{5-6}$, $C_3$-$C_6$ cycloalkyl, or $C_3$-$C_6$ cycloalkyl substituted with one or more $R^{5-7}$;
$R^{5-1}$ is independently hydroxy, CN, or halogen;
$R^{5-2}$, $R^{5-3}$, $R^{5-4}$, and $R^{5-6}$ are independently H, $C_1$-$C_6$ haloalkyl, $C_1$-$C_6$ alkyl, $C_3$-$C_6$ cycloalkyl, or $C_3$-$C_6$ cycloalkyl substituted with one or more $R^{5-8}$;
$R^{5-5}$ is independently $C_1$-$C_6$ haloalkyl, H, $C_1$-$C_6$ alkyl, $C_1$-$C_6$ alkoxy, $C_1$-$C_6$ haloalkoxy, $C_3$-$C_6$ cycloalkyl or $C_3$-$C_6$ cycloalkyl substituted with one or more $R^{5-8}$, or $NR^dR^{5-6}$;
$R^{5-7}$ and $R^{5-8}$ are independently halogen or $C_1$-$C_6$ haloalkyl;
$R^a$, $R^b$, $R^c$, and $R^d$ are independently H or $C_1$-$C_6$ alkyl.

In certain preferred embodiments of the present disclosure, certain groups in the compound represented by formula I, or the pharmaceutically acceptable salt thereof, the ester thereof, the stereoisomer thereof, the tautomer thereof, the polymorph thereof, the solvate thereof, the metabolite thereof, the isotopic derivative thereof, or the prodrug thereof are defined as follows, and the groups not mentioned are as described in any one of the embodiments of the present disclosure (referred to as "in some embodiments"); $R^5$ is independently $C_3$-$C_6$ cycloalkyl.
In some embodiments, $R^1$ and $R^2$ are independently $C_1$-$C_6$ alkyl, $C_3$-$C_6$ cycloalkyl, or $C_6$-$C_{10}$ aryl;

or $R^1$ and $R^2$, together with the atom to which they are attached, form 3- to 12-membered heterocycloalkyl or 3- to 12-membered heterocycloalkyl substituted with one or more $R^{1-1}$, wherein the 3-to 12-membered heterocycloalkyl contains, in addition to the attached P atom, 0, 1, 2, or 3 heteroatoms selected from 1, 2, or 3 of N, O, and S;
$R^{1-1}$ is independently $C_1$-$C_6$ alkyl;
ring B is a $C_3$-$C_{12}$ saturated or unsaturated carbocyclic ring or a "5- to 16-membered saturated or unsaturated heterocyclic ring with 1, 2, 3, 4, or 5 heteroatoms selected from 1, 2, or 3 of N, O, and S";
ring B is monocyclic or polycyclic; when ring B is monocyclic, ring B is an aromatic ring; when ring B is polycyclic, the ring connected to

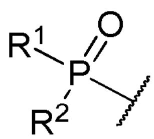

is an aromatic ring, with the remaining ring(s) being saturated or unsaturated ring(s);
m1 is 0 or 1;
$R^3$ is "5- to 12-membered saturated or unsaturated heterocyclyl with 1, 2, or 3 heteroatoms selected from 1, 2, or 3 of N, O, and S", "5- to 12-membered saturated or unsaturated heterocyclyl with 1, 2, or 3 heteroatoms selected from 1, 2, or 3 of N, O, and S" substituted with one or more $R^{3-1}$, $C_6$-$C_{10}$ aryl, or $C_6$-$C_{10}$ aryl substituted with one or more $R^{3-5}$;
$R^{3-1}$ and $R^{3-5}$ are independently H, deuterium, halogen, $C_1$-$C_6$ alkyl, $C_1$-$C_6$ alkoxy, CN, $C_1$-$C_6$ alkyl substituted with one or more $R^{3-2}$, $C_1$-$C_6$ alkoxy substituted with one or more $R^{3-3}$, "3- to 12-membered heterocycloalkyl with 1, 2, or 3 heteroatoms selected from 1, 2, or 3 of N, O, and S", "5- to 12-membered heteroaryl with 1, 2, or 3 heteroatoms selected from 1, 2, or 3 of N, O, and S", $C(=O)NR^cR^d$, $C_3$-$C_6$ cycloalkyl, or $C_3$-$C_6$ cycloalkyl substituted with one or more $R^{3-4}$;
$R^{3-2}$ and $R^{3-3}$ are independently deuterium, halogen, OH, CN, $C_1$-$C_6$ alkoxy, "3- to 12-membered heterocycloalkyl with 1, 2, or 3 heteroatoms selected from 1, 2, or 3 of N, O, and S", $C(=O)NR^cR^d$, or $NR^bC(=O)R^a$;
$R^{3-4}$ is independently CN, $C(=O)NR^cR^d$, or $C(=O)OR^a$;
m2 is 0, 1, 2, or 3;
$R^4$ is independently oxo (=O), CN, halogen, $C_1$-$C_6$ alkyl, $C_1$-$C_6$ haloalkyl, $C_1$-$C_6$ alkoxy, or $C_1$-$C_6$ haloalkoxy;

L is a single bond, $-CR^{L1}R^{L2}-$, -O-, -S-, $-NR^{L3}-$, $-NR^{L3}CR^{L1}R^{L2}-$, $-CR^{L4}=CR^{L5}-$,

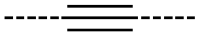,

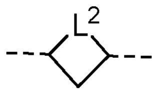, 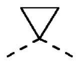, or 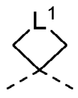;

$R^{L1}$, $R^{L2}$, and $R^{L3}$ are independently H, $C_1$-$C_6$ alkyl, or $C_3$-$C_6$ cycloalkyl;
$R^{L4}$ and $R^{L5}$ are independently H or $C_1$-$C_6$ alkyl;
$L^1$ and $L^2$ are independently $-CH_2-$, -O-, -S-, or -NH-;
ring A is a $C_3$-$C_{12}$ saturated or unsaturated carbocyclic ring or a "5- to 12-membered saturated or unsaturated heterocyclic ring with 1, 2, or 3 heteroatoms selected from 1, 2, or 3 of N, O, and S";
m3 is 0, 1, 2, or 3;
$R^5$ is independently $SF_5$, halogen, $C_1$-$C_6$ alkyl, $C_1$-$C_6$ alkyl substituted with one or more $R^{5-1}$, $SR^{5-2}$, $OR^{5-3}$, CN, $S(=O)_2R^{5-4}$, $C(=O)R^{5-5}$, $S(=O)_2NR^dR^{5-6}$, $NR^dR^{5-6}$, or $C_3$-$C_6$ cycloalkyl substituted with one or more $R^{5-7}$;
$R^{5-1}$ is independently hydroxy, CN, or halogen;
$R^{5-2}$, $R^{5-3}$, $R^{5-4}$, and $R^{5-6}$ are independently H, $C_1$-$C_6$ haloalkyl, $C_1$-$C_6$ alkyl, $C_3$-$C_6$ cycloalkyl, or $C_3$-$C_6$ cycloalkyl substituted with one or more $R^{5-8}$;
$R^{5-5}$ is independently $C_1$-$C_6$ haloalkyl, H, $C_1$-$C_6$ alkyl, $C_1$-$C_6$ alkoxy, $C_1$-$C_6$ haloalkoxy, $C_3$-$C_6$ cycloalkyl or $C_3$-$C_6$ cycloalkyl substituted with one or more $R^{5-8}$, or $NR^dR^{5-6}$;
$R^{5-7}$ and $R^{5-8}$ are independently halogen or $C_1$-$C_6$ haloalkyl;
$R^a$, $R^b$, $R^c$, and $R^d$ are independently H or $C_1$-$C_6$ alkyl.

In some embodiments, each "halogen" is independently fluorine, chlorine, bromine, or iodine, preferably fluorine or chlorine, such as F.
In some embodiments, each "$C_1$-$C_6$ alkyl" is independently methyl, ethyl, *n*-propyl, isopropyl, *n*-butyl, isobutyl, or *tert*-butyl, preferably methyl or ethyl, such as methyl.
In some embodiments, each "$C_1$-$C_6$ alkoxy" is independently methoxy, ethoxy, *n*-propoxy, isopropoxy, *n*-butoxy, isobutoxy, or *tert*-butoxy, preferably methoxy or ethoxy.
In some embodiments, each "$C_1$-$C_6$ haloalkyl" is independently $-CHF_2$, $-CH_2F$, or $-CF_3$, such as - $CF_3$.
In some embodiments, each "$C_1$-$C_6$ haloalkoxy" is independently $-OCHF_2$, $-OCH_2F$, or $-OCF_3$.
In some embodiments, each "$C_3$-$C_6$ cycloalkyl" is independently cyclopropyl, cyclobutyl, cyclopentyl, or cyclohexyl, preferably cyclopropyl or cyclobutyl, such as cyclopropyl.
In some embodiments, each "$C_6$-$C_{10}$ aryl" is independently phenyl or naphthyl, preferably phenyl.
In some embodiments, each "3- to 12-membered heterocycloalkyl with 1, 2, or 3 heteroatoms selected from 1, 2, or 3 of N, O, and S" is independently "5- to 6-membered heterocycloalkyl with 1 or 2 heteroatoms selected from 1 or 2 of N and O".
In some embodiments, the "5- to 12-membered heteroaryl with 1, 2, or 3 heteroatoms selected from 1, 2, or 3 of N, O, and S" is "5- to 6-membered heteroaryl with 1, 2, or 3 heteroatoms selected from 1, 2, or 3 of N, O, and S".
In some embodiments, in ring B, the "$C_3$-$C_{12}$ saturated or unsaturated carbocyclic ring" is a $C_6$-$C_{10}$ unsaturated carbocyclic ring, such as a benzene ring, a naphthalene ring, or a hydrindene ring, preferably a $C_6$-$C_{10}$ aromatic ring, such as a benzene ring.
In some embodiments, in ring B, the "5- to 16-membered saturated or unsaturated heterocyclic ring with 1, 2, 3, 4, or 5 heteroatoms selected from 1, 2, or 3 of N, O, and S" is a "6- to 15-membered unsaturated heterocyclic ring with 1, 2, 3, 4, or 5 heteroatoms selected from 1, 2, or 3 of N, O, and S", such as a pyridine ring (e.g.,

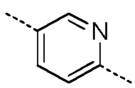

), an indole ring (e.g.,

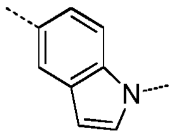

), a carbazole ring (e.g.,

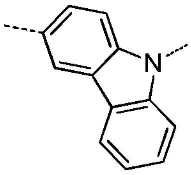), 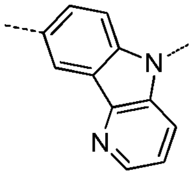, 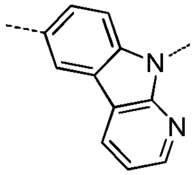, 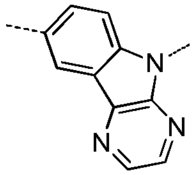, 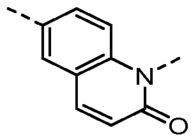,

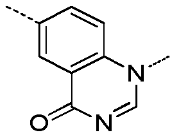, 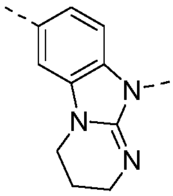, 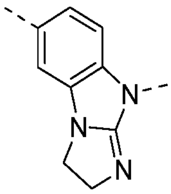, or 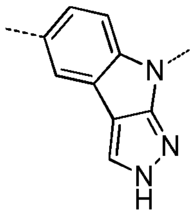.

In some embodiments, in $R^3$, the "5- to 12-membered saturated or unsaturated heterocyclyl with 1, 2, or 3 heteroatoms selected from 1, 2, or 3 of N, O, and S" is "5- to 10-membered unsaturated heterocyclyl with 1, 2, or 3 heteroatoms selected from 1, 2, or 3 of N, O, and S", for example, pyrrolyl (e.g.,

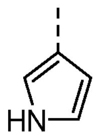

), pyrazolyl (e.g.,

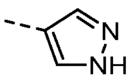, 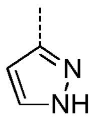, or 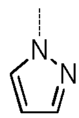

), imidazolyl (e.g.,

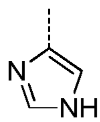

), triazolyl (e.g.,

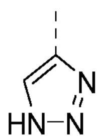

or

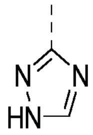

), thiazolyl (e.g.,

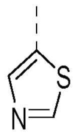

), oxazolyl (e.g.,

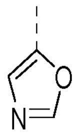

), isoxazolyl (e.g.,

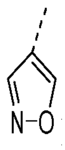

), pyridyl (e.g.,

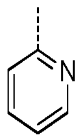

,

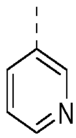

, or

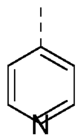

), pyrimidinyl (e.g.,

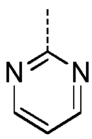

or

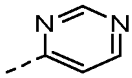

), pyrazinyl (e.g.,

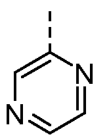

), pyridazinyl (e.g.,

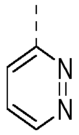), 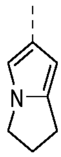, 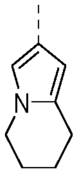, 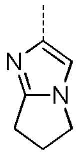, 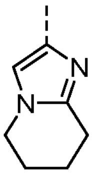, 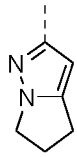, 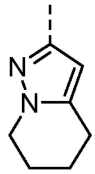, 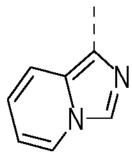, 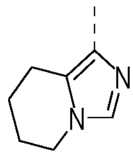,

or

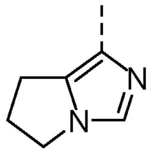,

and for another example, imidazolyl (e.g.,

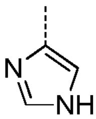

), pyrazolyl (e.g.,

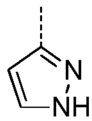 or 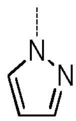

),

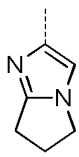,

pyridyl (e.g.,

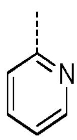

), or pyrimidinyl (e.g.,

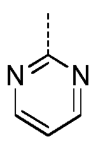

).

In some embodiments, in ring A, the "$C_3$-$C_{12}$ saturated or unsaturated carbocyclic ring" is a $C_4$-$C_{10}$ saturated or unsaturated carbocyclic ring, for example, a cyclobutane ring (e.g.,

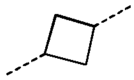

), a cyclohexane ring (e.g.,

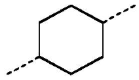

), spiro[2.5]octane (e.g.,

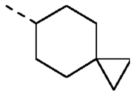

), a spiro[3.3]heptane ring (e.g.,

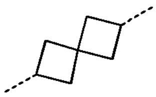

), a benzene ring (e.g.,

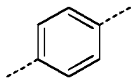

or

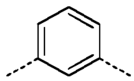

), a naphthalene ring (e.g.,

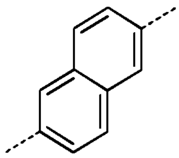

), a hydrindene ring, or a tetrahydronaphthalene ring, and for another example, a $C_4$-$C_{10}$ saturated carbocyclic ring or a $C_6$-$C_{10}$ aromatic ring, preferably a $C_6$-$C_{10}$ aromatic ring, further preferably a cyclobutane ring (e.g.,

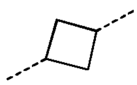

), a cyclohexane ring (e.g.,

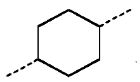

), a spiro[3.3]heptane ring (e.g.,

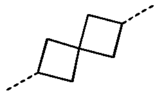

), a benzene ring (e.g.,

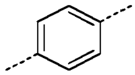

or

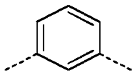

), a naphthalene ring (e.g.,

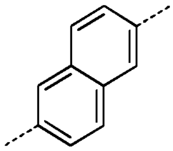

), a hydrindene ring, or a tetrahydronaphthalene ring, and even more preferably a $C_6$-$C_{10}$ aromatic ring.

In some embodiments, in ring A, the "$C_3$-$C_{12}$ saturated or unsaturated carbocyclic ring" is a cyclohexane ring (e.g.,

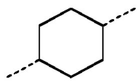

) or a benzene ring (e.g.,

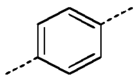

).

In some embodiments, in ring A, the "5- to 12-membered saturated or unsaturated heterocyclic ring with 1, 2, or 3 heteroatoms selected from 1, 2, or 3 of N, O, and S" (e.g., a 5- to 10-membered saturated or unsaturated heterocyclic ring) is a "5- to 6-membered monocyclic heteroaromatic ring or 9- to 10-membered bicyclic heterocyclic ring with 1, 2, or 3 heteroatoms selected from 1, 2, or 3 of N, O, and S", for example, a benzoxolane ring (e.g.,

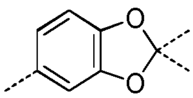

), a pyridine ring (e.g.,

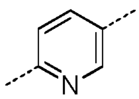

), or a thiophene ring (e.g.,

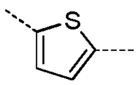

), for another example, a "5- to 6-membered heteroaromatic ring with 1, 2, or 3 heteroatoms selected from 1, 2, or 3 of N, O, and S", and for further another example, a pyridine ring (e.g.,

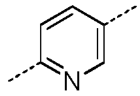

) or a thiophene ring (e.g.,

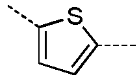

), preferably a pyridine ring (e.g.,

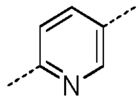

).

In some embodiments, $R^1$ and $R^2$ are independently $C_1$-$C_6$ alkyl, or $R^1$ and $R^2$, together with the atom to which they are attached, form 3- to 12-membered heterocycloalkyl, wherein the 3- to 12-membered heterocycloalkyl contains, in addition to the attached P atom, 0, 1, 2, or 3 heteroatoms selected from 1, 2, or 3 of N, O, and S, such as $C_1$-$C_6$ alkyl.

In some embodiments, $R^3$ is "5- to 12-membered saturated or unsaturated heterocyclyl with 1, 2, or 3 heteroatoms selected from 1, 2, or 3 of N, O, and S" or "5- to 12-membered saturated or unsaturated heterocyclyl with 1, 2, or 3 heteroatoms selected from 1, 2, or 3 of N, O, and S" substituted with one or more $R^{3-1}$.

In some embodiments, $R^{3-1}$ is independently $C_1$-$C_6$ alkyl or $C_1$-$C_6$ alkyl substituted with one or more $R^{3-2}$, such as $C_1$-$C_6$ alkyl.

In some embodiments, $R^{3-2}$ is independently halogen.

In some embodiments, $R^4$ is independently halogen, $C_1$-$C_6$ alkyl, or $C_1$-$C_6$ alkoxy, such as $C_1$-$C_6$ alkyl.

In some embodiments, ring B is a $C_6$-$C_{10}$ unsaturated carbocyclic ring or a "6- to 15-membered unsaturated heterocyclic ring with 1, 2, 3, 4, or 5 heteroatoms selected from 1, 2, or 3 of N, O, and S".

In some embodiments, L is a single bond, -$CR^{L1}R^{L2}$-, -O-, -S-, or -$NR^{L3}$-; preferably, L is a single bond, -O-, -S-, or -$NR^{L3}$-; more preferably, L is -$NR^{L3}$-.

In some embodiments, $R^{L3}$ is H.

In some embodiments, ring A is a $C_4$-$C_{10}$ saturated carbocyclic ring, a $C_6$-$C_{10}$ aromatic ring, or a "5- to 9-membered saturated or unsaturated heterocyclic ring with 1, 2, or 3 heteroatoms selected from 1, 2, or 3 of N, O, and S", for example, ring A is a $C_6$-$C_{10}$ aromatic ring or a "5- to 6-membered heteroaromatic ring with 1, 2, or 3 heteroatoms selected from 1, 2, or 3 of N, O, and S".

In some embodiments, m3 is 0, 1, or 2, such as 1.

In some embodiments, $R^5$ is independently $SF_5$, $C_3$-$C_6$ cycloalkyl, halogen, $C_1$-$C_6$ alkyl, $C_1$-$C_6$ alkyl substituted with one or more $R^{5-1}$, or $OR^{5-3}$, for example, $R^5$ is independently halogen, $C_1$-$C_6$ alkyl, or $C_1$-$C_6$ alkyl substituted with one or more $R^{5-1}$, such as $C_1$-$C_6$ alkyl substituted with one or more $R^{5-1}$, and for another example, $R^5$ is independently $SF_5$, halogen, or $C_1$-$C_6$ alkyl substituted with one or more $R^{5-1}$.

In some embodiments, $R^{5-1}$ is independently halogen.

In some embodiments, $R^{5-3}$ is independently $C_1$-$C_6$ haloalkyl.

In some embodiments, m2 is 0 or 1, such as 0.

In some embodiments, m1 is 1.

In some embodiments,

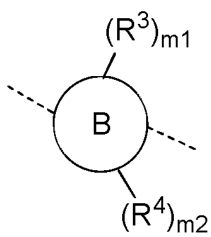

is

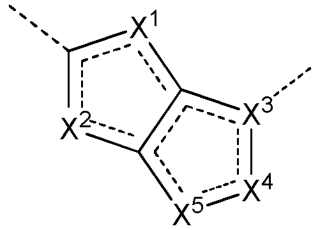

,

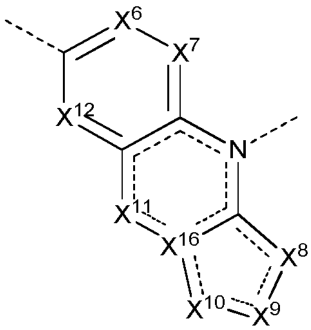

, or

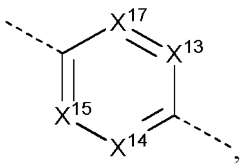

,

wherein

- - - - - represents a single bond or a double bond;

$X^1$ is S, $-CR^{X1}=CR^{X1}-$, $-N=CR^{X1}-$, or $-CR^{X1}=N-$;

$X^2$ is $CR^{X1}$, N, or S;

$X^3$ is C, $CR^{X1}$, or N;

$X^4$ is $CR^{X1}$, N, -C(O)-, or $CR^{X1}R^{X2}$;

$X^5$ is N, $NR^{X3}$, $CR^{X1}$, $CR^{X1}R^{X2}$, $-CR^{X1}=CR^{X1}-$, $-N=CR^{X1}-$, $-CR^{X1}=N-$, $-CR^{X1}R^{X2}-CR^{X1}R^{X2}-$, $-O-CR^{X1}R^{X2}-$, -C(O)-N=, $-C(O)-CR^{X1}=$, or $-NR^{X3}-CR^{X1}R^{X2}-$;

$X^6$ is $CR^{X1}$ or N;

$X^7$ is $CR^{X1}$ or N;

$X^8$ is $CR^{X1}$, $CR^{X1}R^{X2}$, N, $NR^{X3}$, -C(O)-, O, or S;

$X^9$ is $CR^{X1}$, N, $NR^{X3}$, $-CR^{X1}=CR^{X1}-$, $=CR^{X1}-CR^{X1}=$, $-CR^{X1}R^{X2}-$, $-CR^{X1}R^{X2}-CR^{X1}R^{X2}-$, $=N-CR^{X1}=$, $=CR^{X1}-N=$, $=CR^{X1}-NR^{X3}-$, $-NR^{X3}-CR^{X1}=$, $-NR^{X3}-N=$, or $=N-NR^{X3}-$;

$X^{10}$ is $CR^{X1}$, $CR^{X1}R^{X2}$, N, $NR^{X3}$, -C(O)-, O, or S;

$X^{11}$ is absent, O, or S;

$X^{12}$ is $CR^{X1}$ or N;

$X^{16}$ is C or N;

$X^{13}$ is $CR^{X1}$ or N;

$X^{14}$ is $CR^{X4}$ or N;

$X^{15}$ is $CR^{X1}$ or N;

$X^{17}$ is $CR^{X1}$ or N;

each of $R^{X1}$ and $R^{X2}$ is independently H, $C_1$-$C_6$ alkyl, halogen, or CN; or $R^{X1}$ and $R^{X2}$, together with the atom to which they are attached, form $C_3$-$C_4$ cycloalkyl;

each $R^{X3}$ is independently H, $C_1$-$C_6$ alkyl, halogen, or CN;

$R^{X4}$ is H, "5- to 12-membered saturated or unsaturated heterocyclyl with 1, 2, or 3 heteroatoms selected from 1, 2, or 3 of N, O, and S", "5- to 12-membered saturated or unsaturated heterocyclyl with 1, 2, or 3 heteroatoms selected from 1, 2, or 3 of N, O, and S" substituted with one or more $R^{X4-1}$, $C_6$-$C_{10}$ aryl, or $C_6$-$C_{10}$ aryl substituted with one or more $R^{X4-5}$;

$R^{X4-1}$ and $R^{X4-5}$ are independently H, deuterium, halogen, $C_1$-$C_6$ alkyl, $C_1$-$C_6$ alkoxy, CN, $C_1$-$C_6$ alkyl substituted with one or more $R^{X4-2}$, $C_1$-$C_6$ alkoxy substituted with one or more $R^{X4-3}$, "3- to 12-membered heterocycloalkyl with 1, 2, or 3 heteroatoms selected from 1, 2, or 3 of N, O, and S", "5- to 12-membered heteroaryl with 1, 2, or 3 heteroatoms selected from 1, 2, or 3 of N, O, and S", $C(=O)NR^cR^d$, $C_3$-$C_6$ cycloalkyl, or $C_3$-$C_6$ cycloalkyl substituted with one or more $R^{X4-4}$;

$R^{X4-2}$ and $R^{X4-3}$ are independently deuterium, halogen, OH, CN, $C_1$-$C_6$ alkoxy, "3- to 12-membered heterocycloalkyl with 1, 2, or 3 heteroatoms selected from 1, 2, or 3 of N, O, and S", $C(=O)NR^cR^d$, or $NR^bC(=O)R^a$;

$R^{X4-4}$ is independently CN, $C(=O)NR^cR^d$, or $C(=O)OR^a$;

$R^a$, $R^b$, $R^c$, and $R^d$ are independently H or $C_1$-$C_6$ alkyl.

In some embodiments,

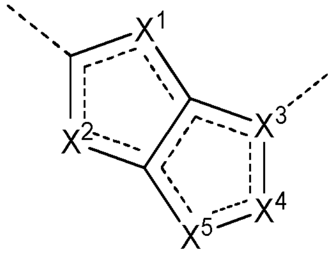

is

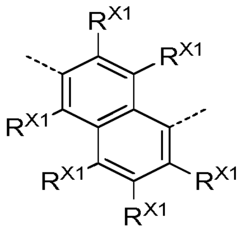

,

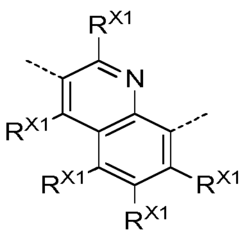

,

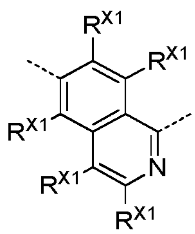

,

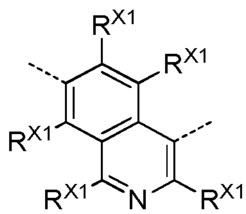

,

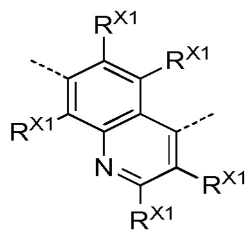

,

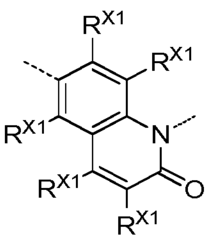

,

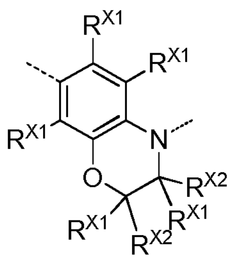

,

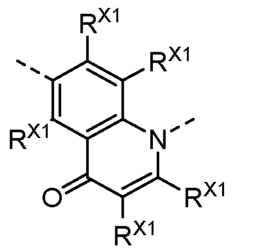

,

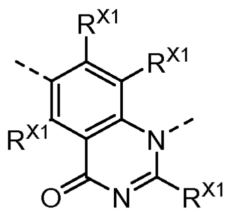

,

or

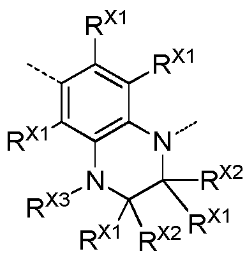

;

preferably,

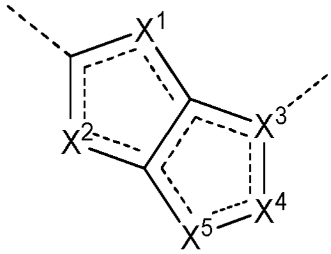

is

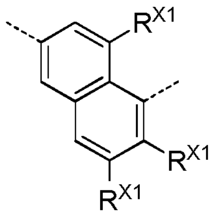

,

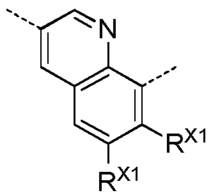 , 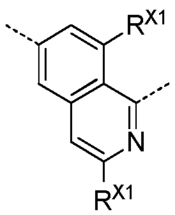 , 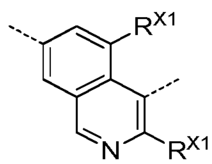 , 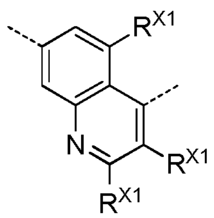 , 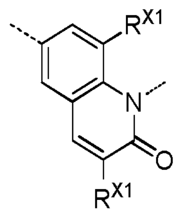 ,

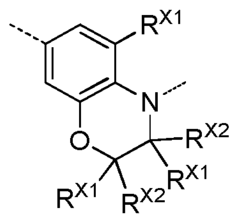 , or 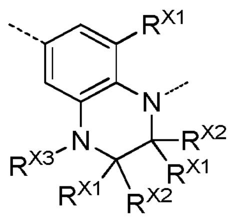 .

In some embodiments,

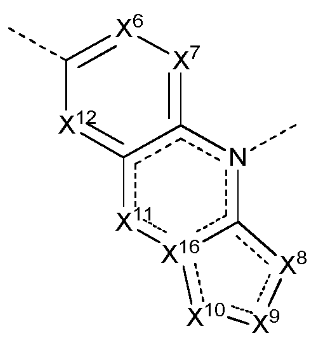

is

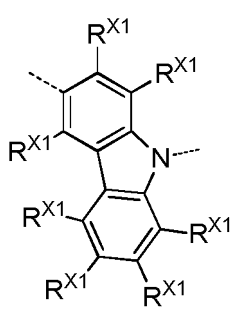
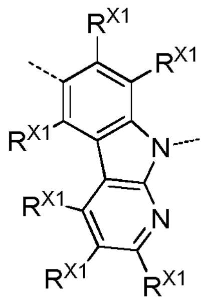

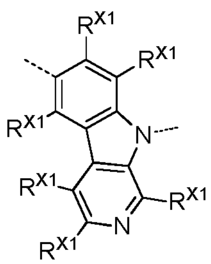
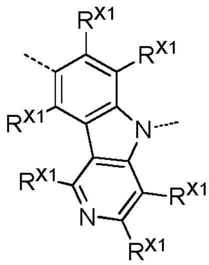
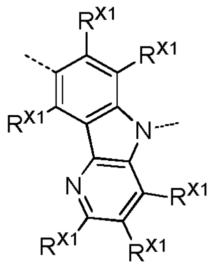
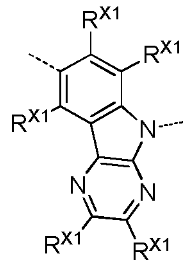
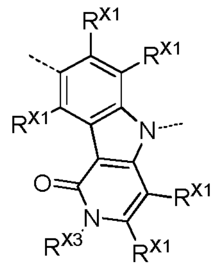

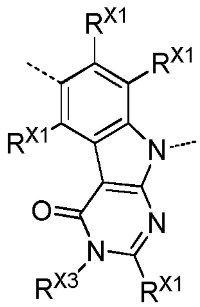
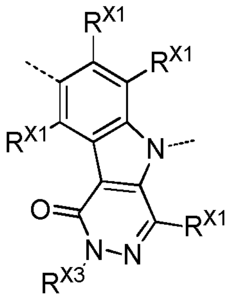
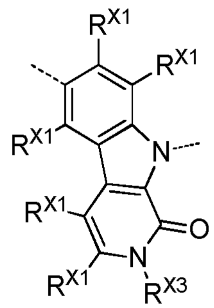
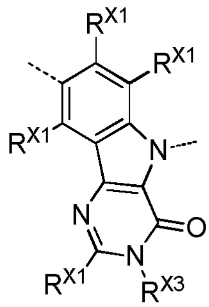
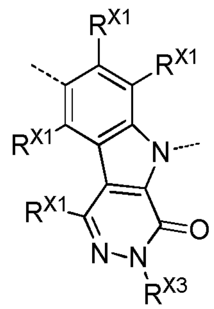

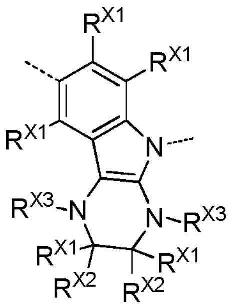
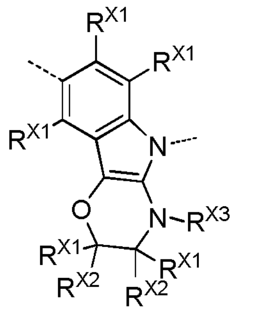
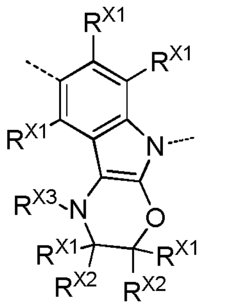

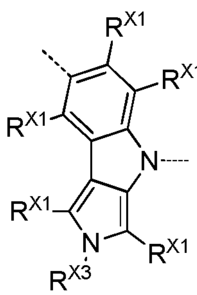
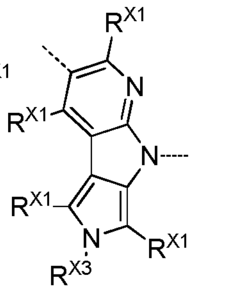
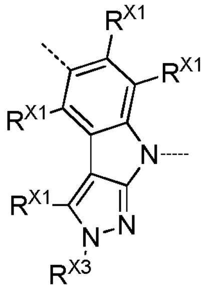
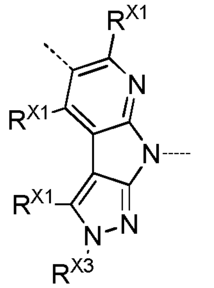
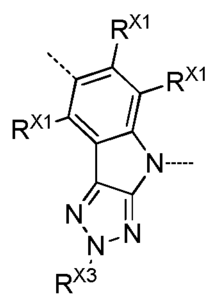

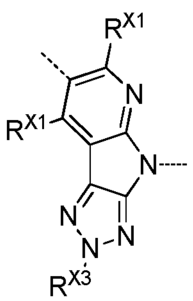 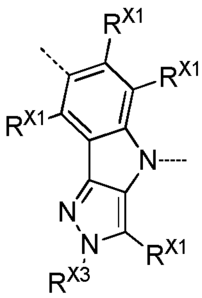 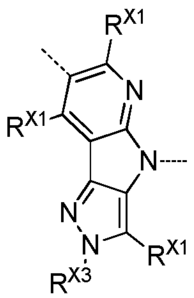 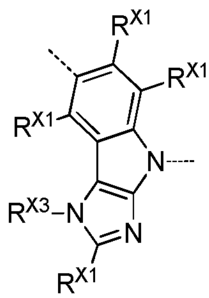 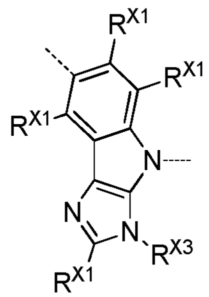

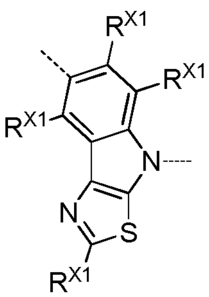 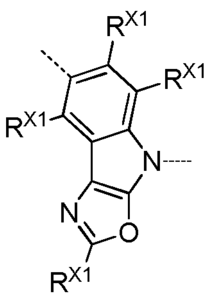 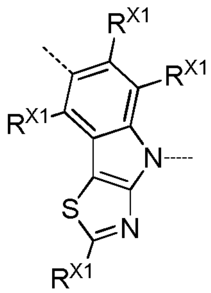 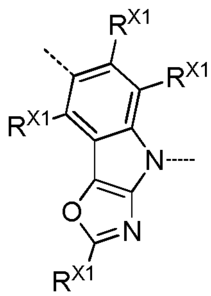 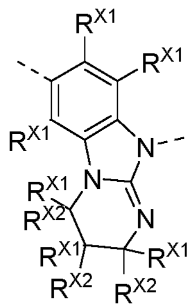

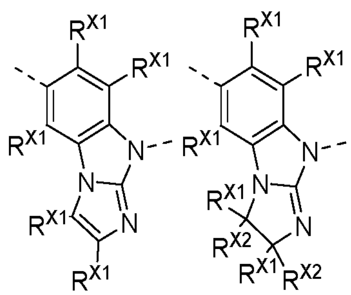

;

preferably,

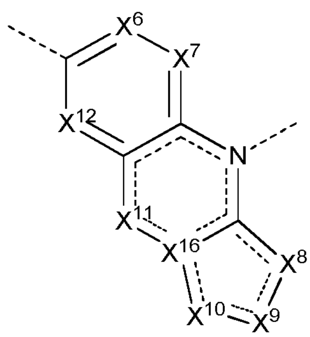

is

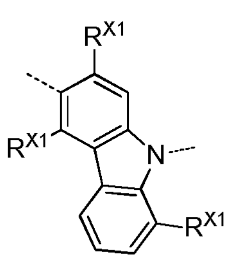 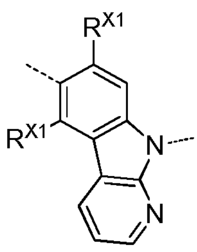 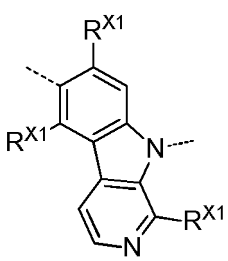

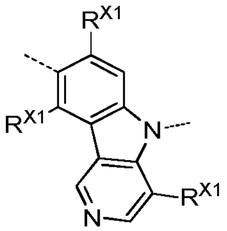 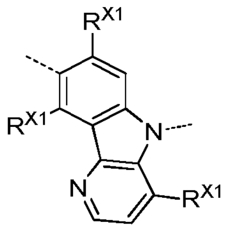 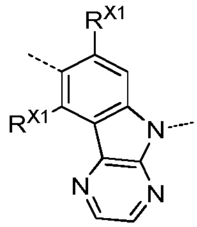

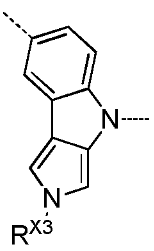 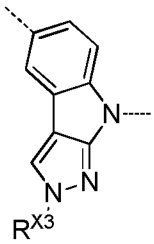 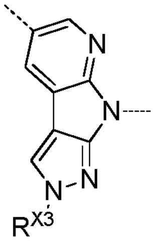 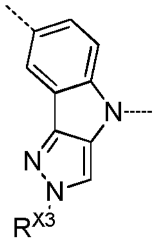 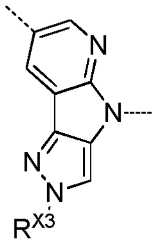

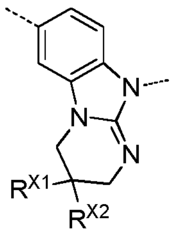 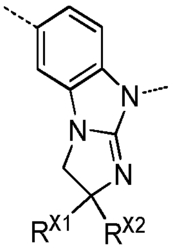 .

In some embodiments,

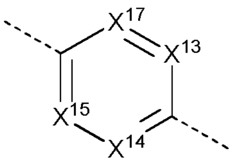 is 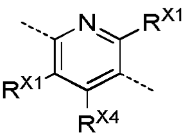 , 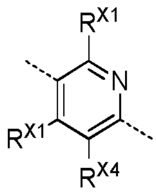 ,

or

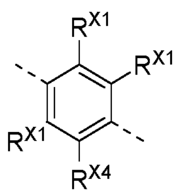 .

In some embodiments, ring B is

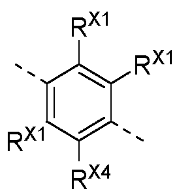 .

In some embodiments, each $R^{X1}$ is independently H.

In some embodiments, $R^{X4}$ is "5- to 12-membered saturated or unsaturated heterocyclyl with 1, 2, or 3 heteroatoms selected from 1, 2, or 3 of N, O, and S" or "5- to 12-membered saturated or unsaturated heterocyclyl with

1, 2, or 3 heteroatoms selected from 1, 2, or 3 of N, O, and S" substituted with one or more $R^{X4-1}$.

In some embodiments, $R^{X4-1}$ and $R^{X4-5}$ are independently H or $C_1$-$C_6$ alkyl, such as $C_1$-$C_6$ alkyl.

In some embodiments, in $R^{X4}$, the "5- to 12-membered saturated or unsaturated heterocyclyl with 1, 2, or 3 heteroatoms selected from 1, 2, or 3 of N, O, and S" is "5- to 10-membered unsaturated heterocyclyl with 1, 2, or 3 heteroatoms selected from 1, 2, or 3 of N, O, and S", for example, pyrrolyl (e.g.,

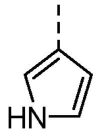

), pyrazolyl (e.g.,

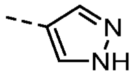, 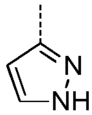, or 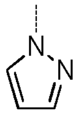

), imidazolyl (e.g.,

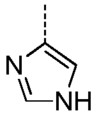

), triazolyl (e.g.,

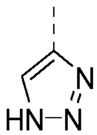

or

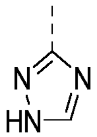

), thiazolyl (e.g.,

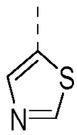

), oxazolyl (e.g.,

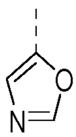

), isoxazolyl (e.g.,

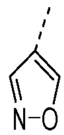

), pyridyl (e.g.,

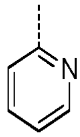

,

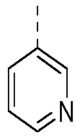

, or

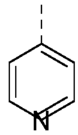

), pyrimidinyl (e.g.,

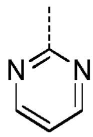

or

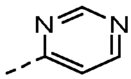

), pyrazinyl (e.g.,

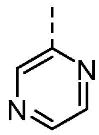

), pyridazinyl (e.g.,

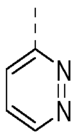

),

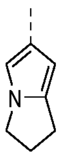

,

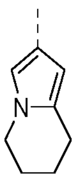

,

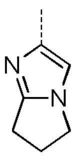

,

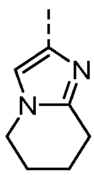

,

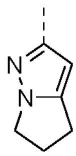

,

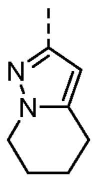

,

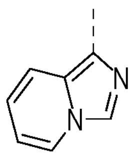

,

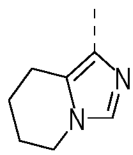

,

or

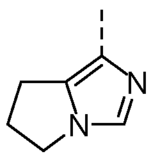

,

and for another example, imidazolyl (e.g.,

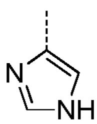

), pyrazolyl (e.g.,

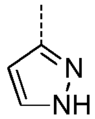 or 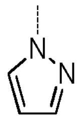

),

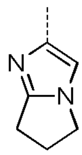,

pyridyl (e.g.,

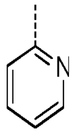

), or pyrimidinyl (e.g.,

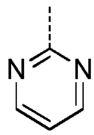

In some embodiments, $R^{X4}$ is

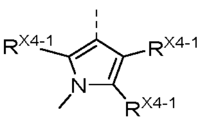 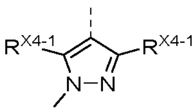 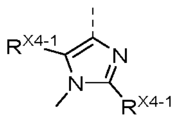 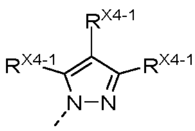

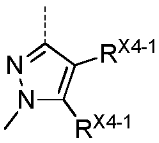 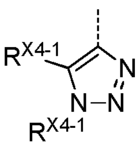 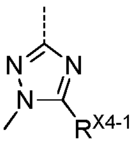 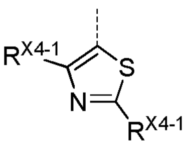 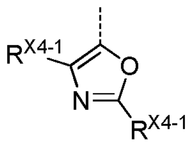 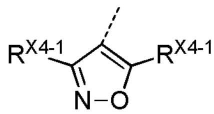

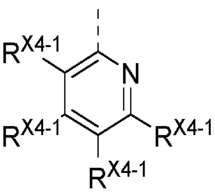 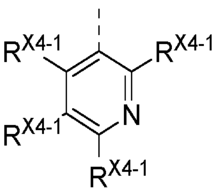 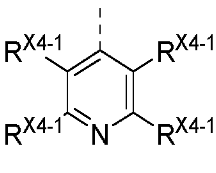 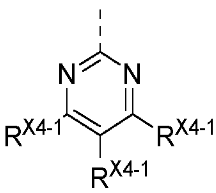 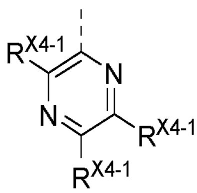

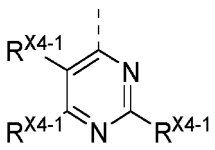 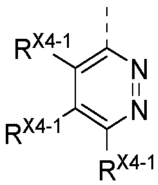 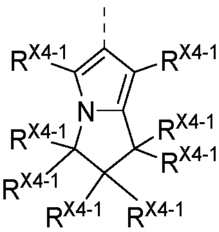 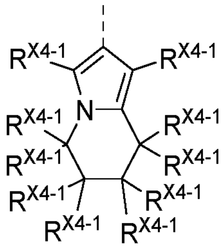 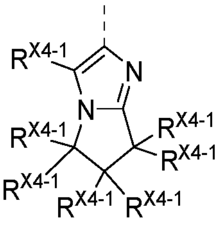

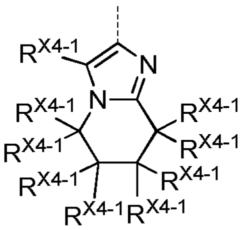 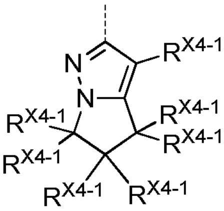 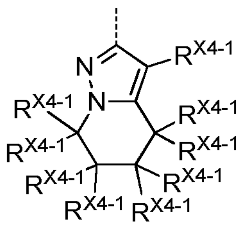 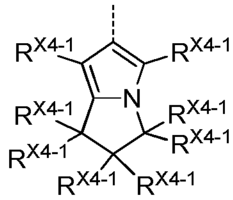

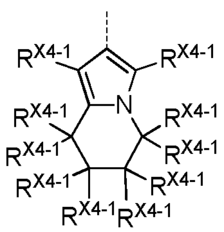 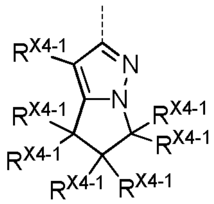 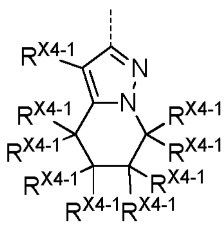 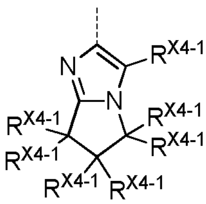 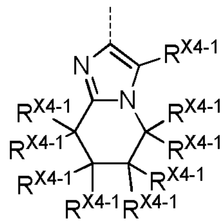

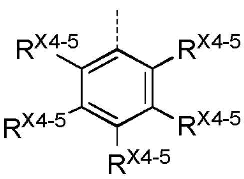 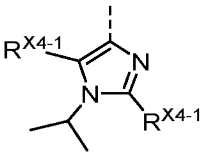 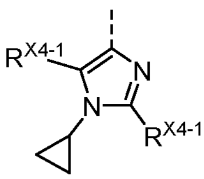 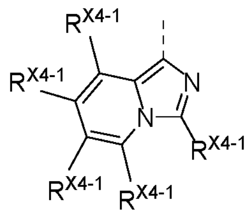 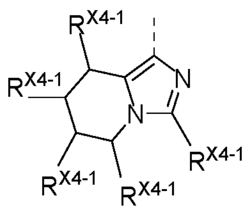

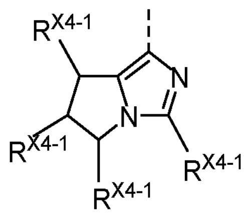

,

for example, $R^{X4}$ is

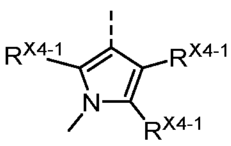 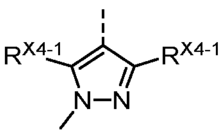 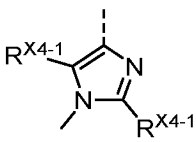

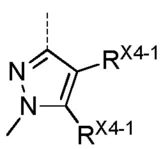 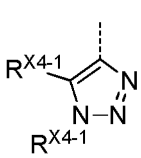 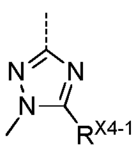 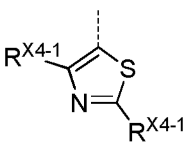 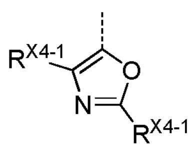 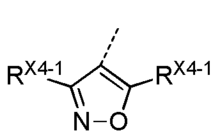

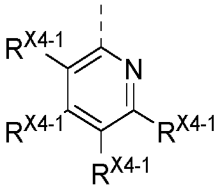 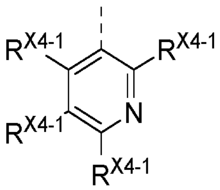 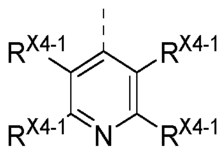 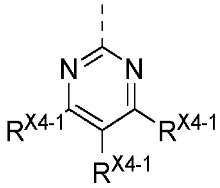 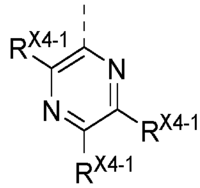

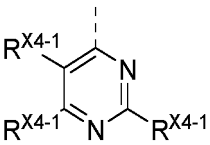 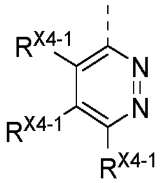 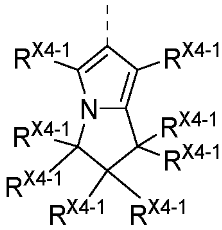 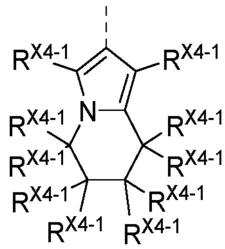 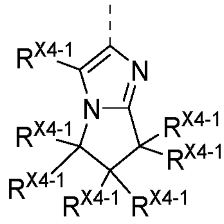

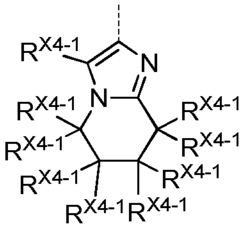 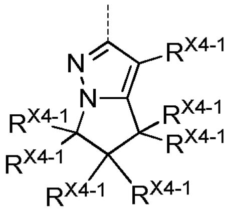 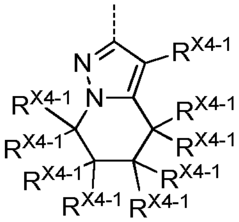 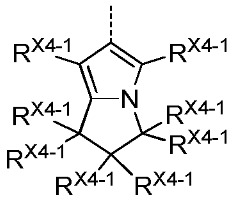

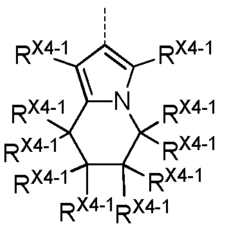 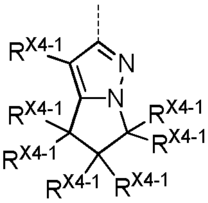 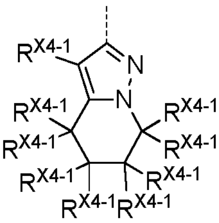 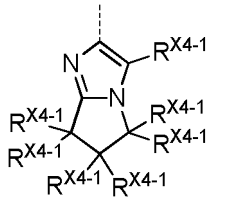 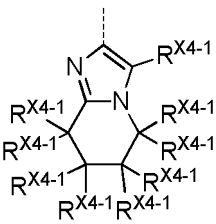

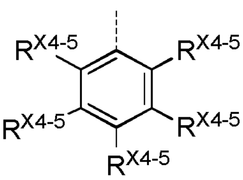 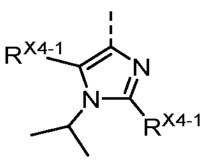 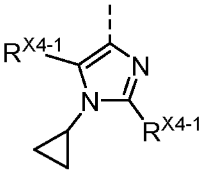 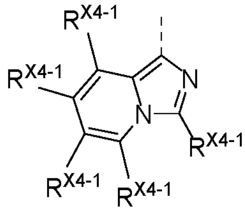 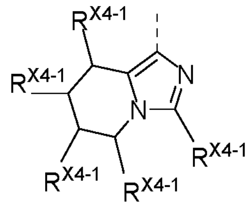

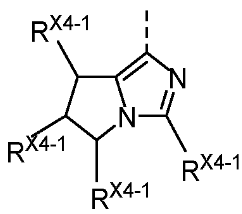

and for another example, $R^{X4}$ is

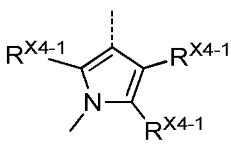 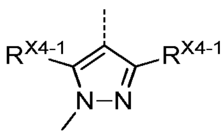 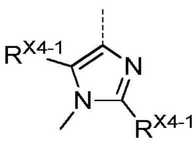

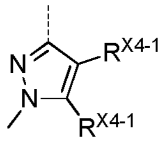 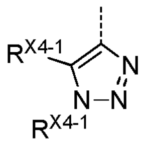 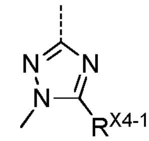 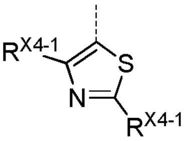 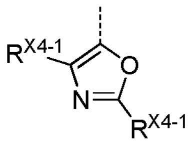 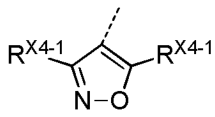

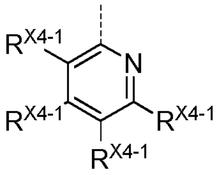 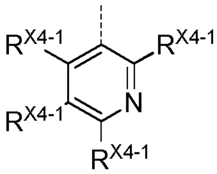 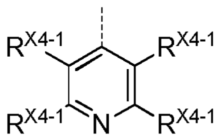 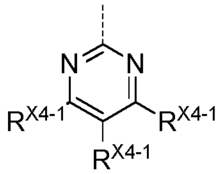 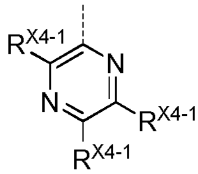

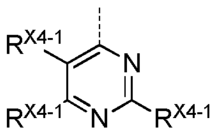 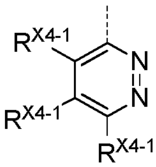 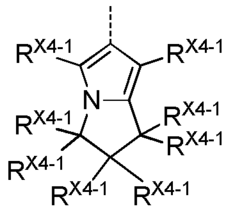 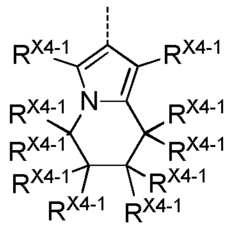 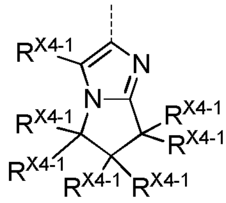

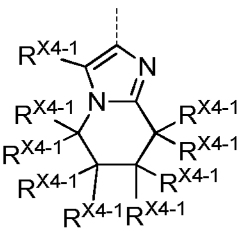 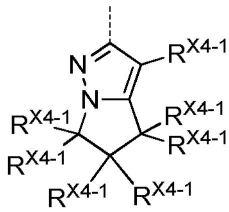 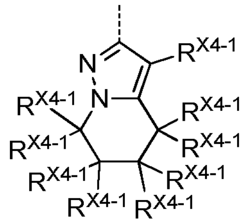 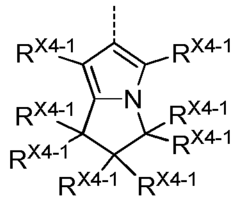

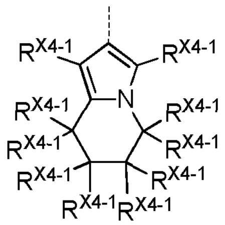 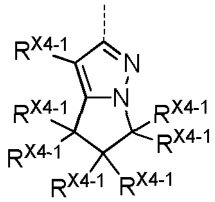 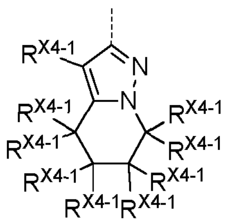 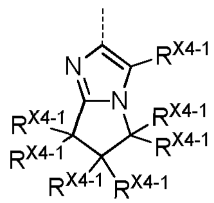 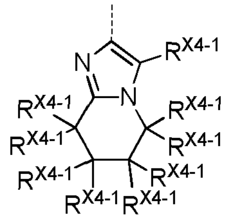

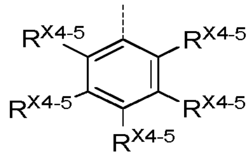.

In some embodiments,

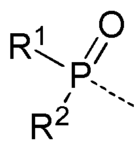

is

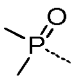, 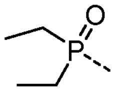, 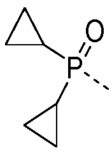, 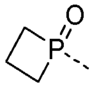, 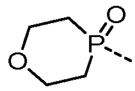,

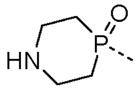, 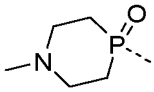, or 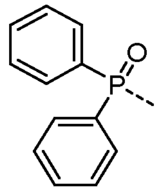.

In some embodiments,

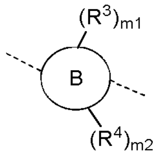

is

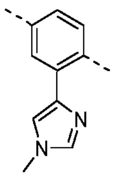 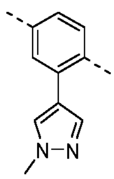 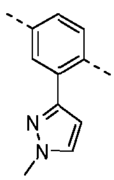 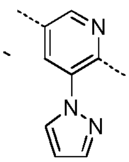 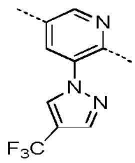

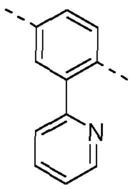 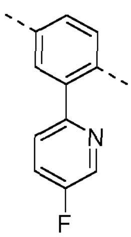 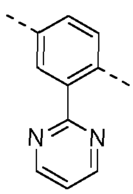 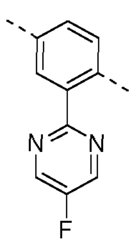 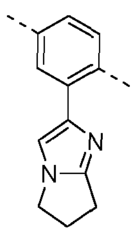 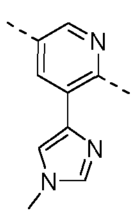 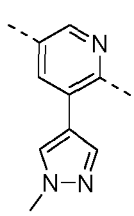

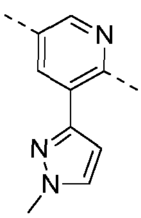 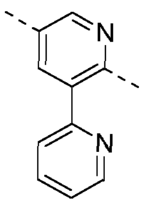 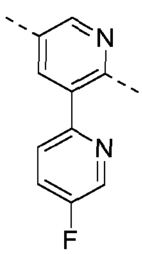 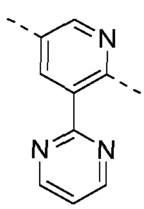 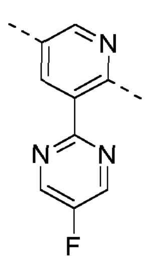 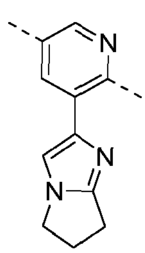

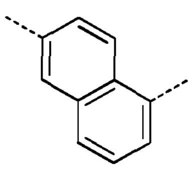 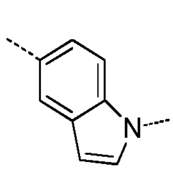 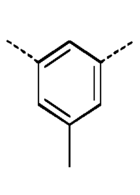 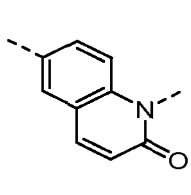 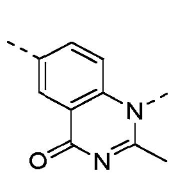

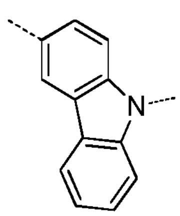
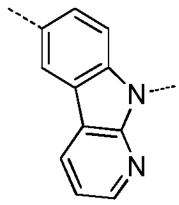
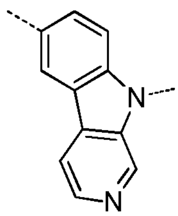
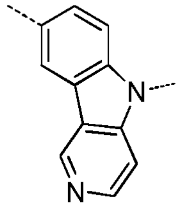
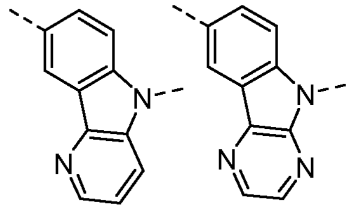
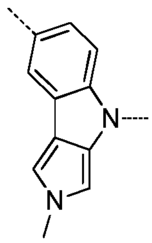
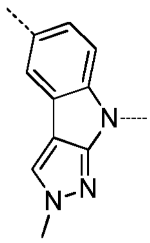
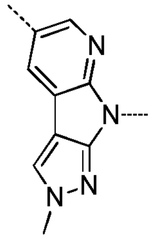
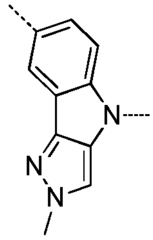
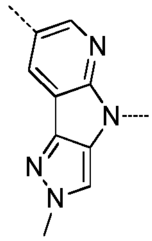
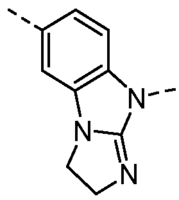
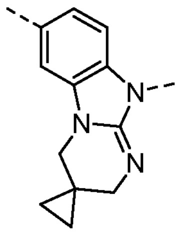
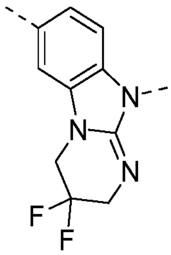
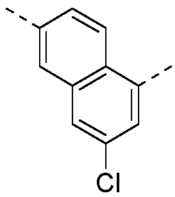
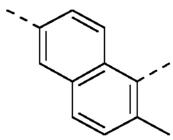
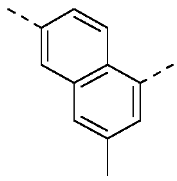
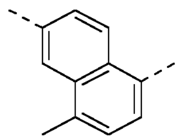
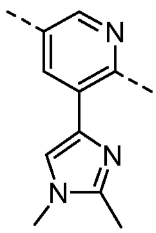
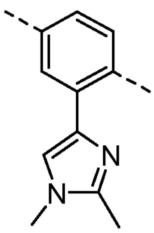
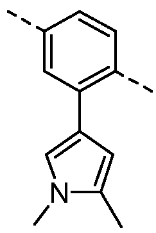
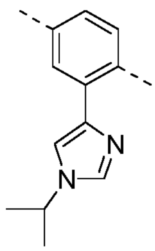
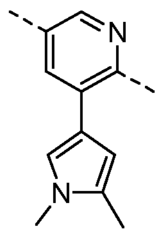
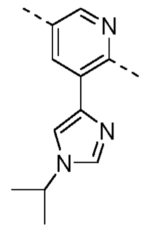
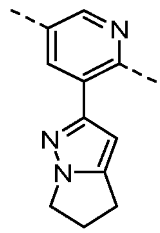
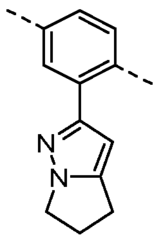
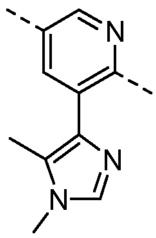
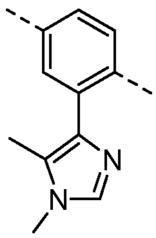
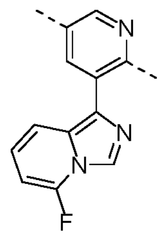
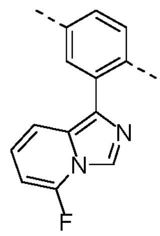
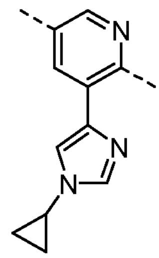
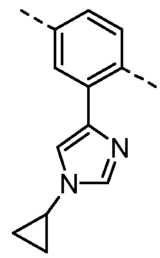

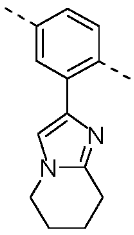
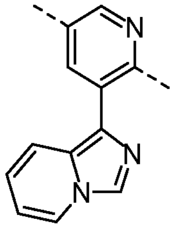
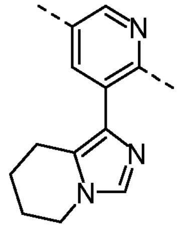
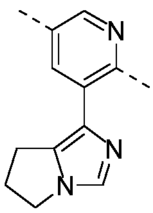
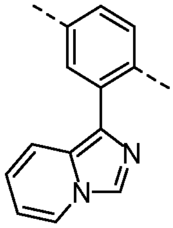
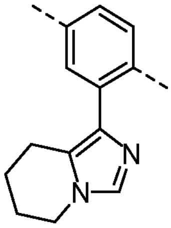
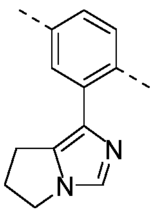

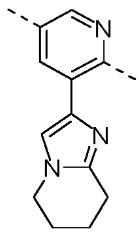
,

for example,

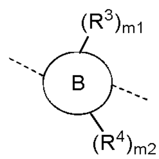

is

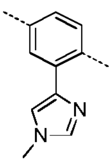
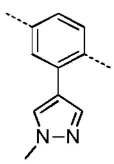
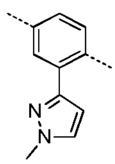
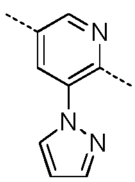
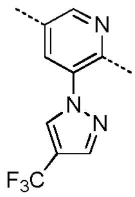

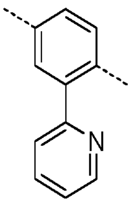
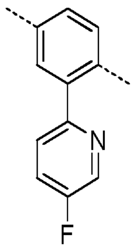
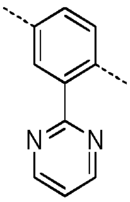
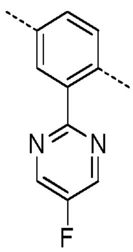
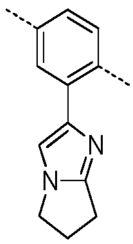
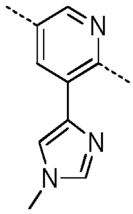
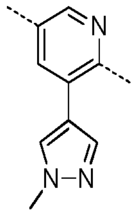

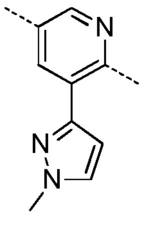
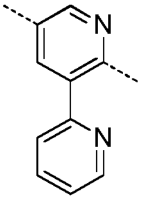
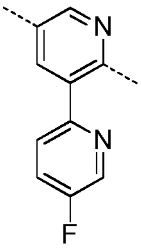
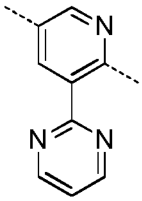
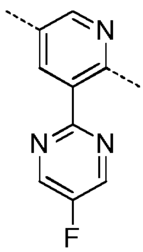
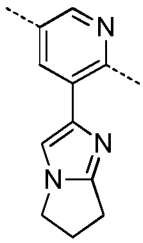

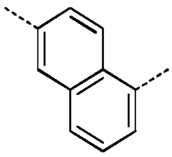 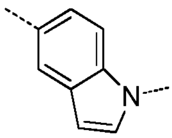 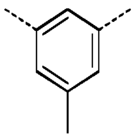 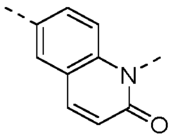 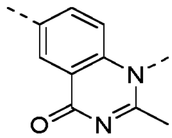

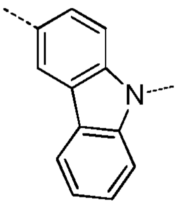 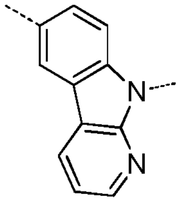 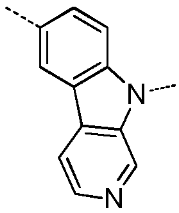 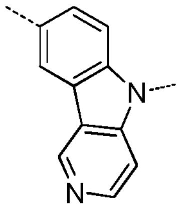

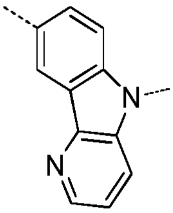 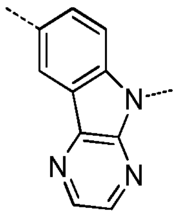

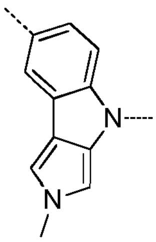 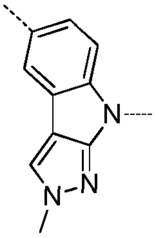 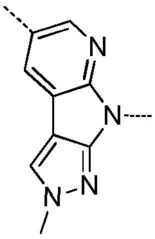 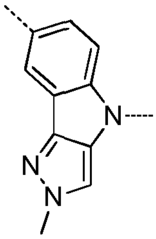 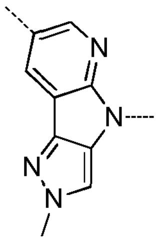 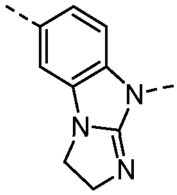

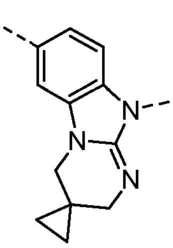 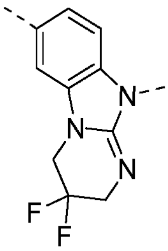 .

In some embodiments, L is a single bond, $-CR^{L1}R^{L2}-$, -O-, -S-, or -NH-, for example, L is a single bond, -O-, or -NH-.

In some embodiments,

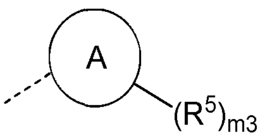

is

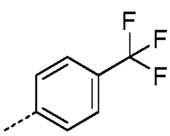, 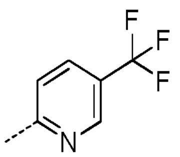,

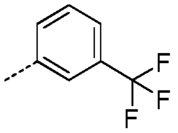, 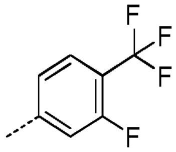, 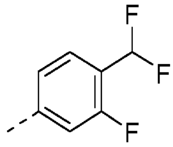, 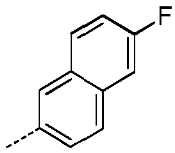, 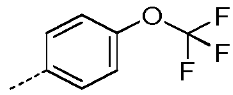, 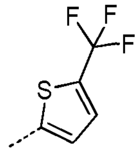,

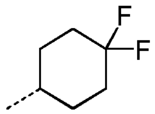, 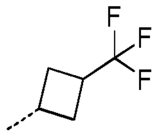, 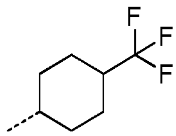, 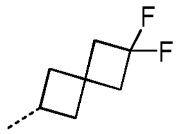, 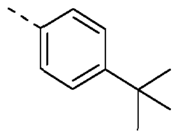, 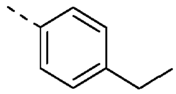,

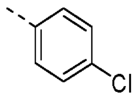, 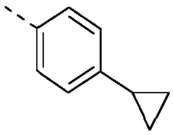, 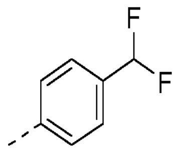, 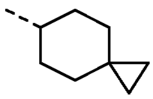, 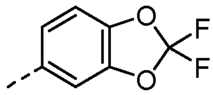, or 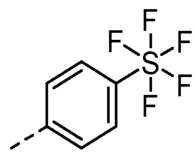,

such as

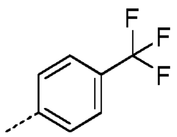, 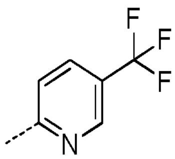, 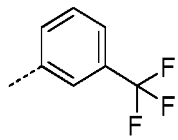, 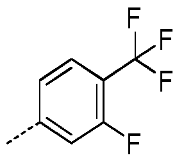, 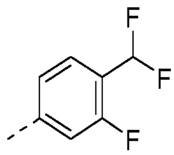,

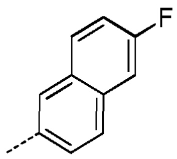, 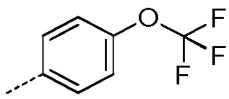, 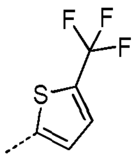, 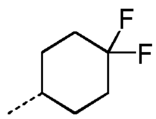, 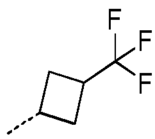, 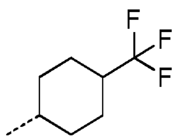,

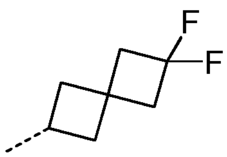, 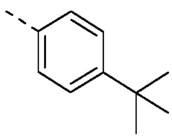, 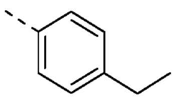, 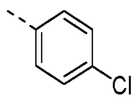, 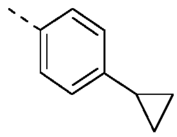, 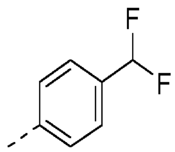,

or

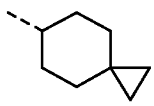;

for example,

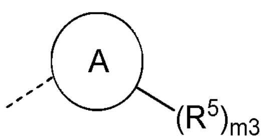

is

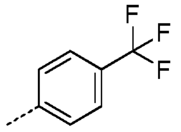, 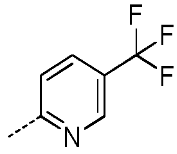,

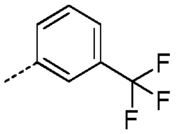, 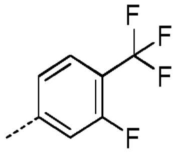, 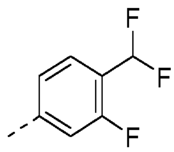, 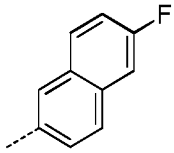, 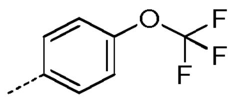, 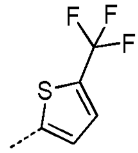,

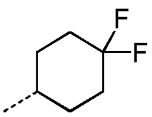, 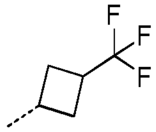, 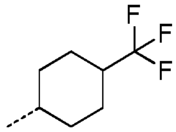, or 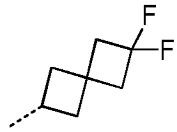.

In some embodiments, the compound represented by formula I is a compound represented by the following formula I-1, I-2, I-3, I-4, I-5, I-6, or I-7:

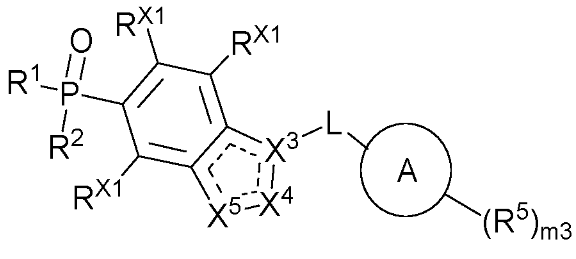

I-1

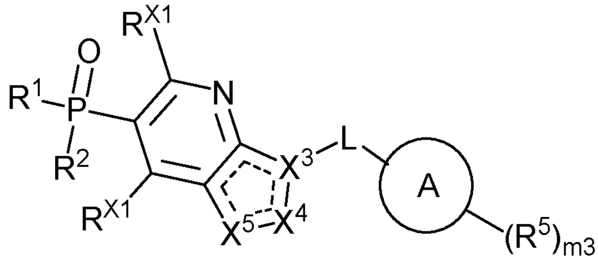

I-2

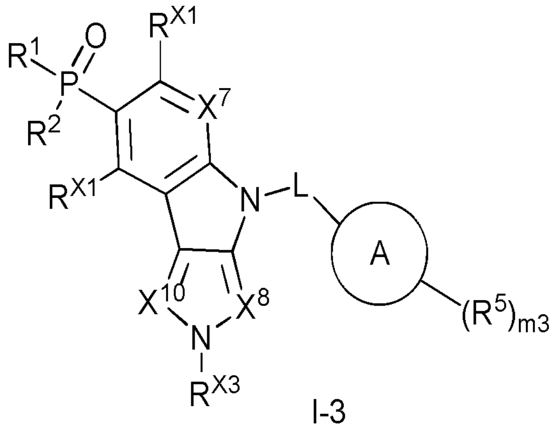

I-3

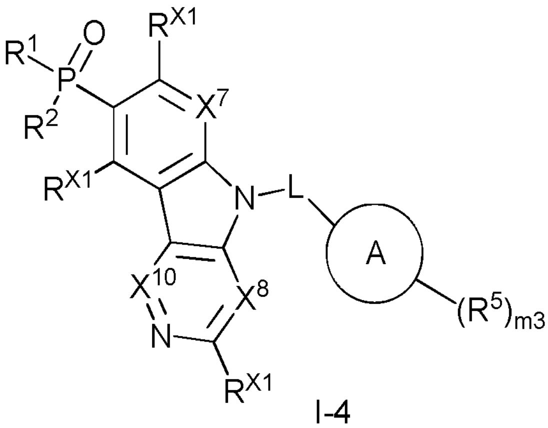

I-4

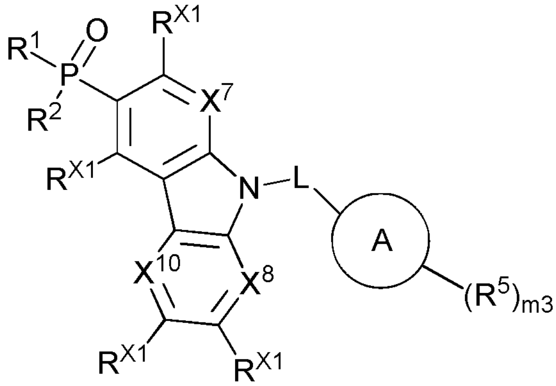

I-5

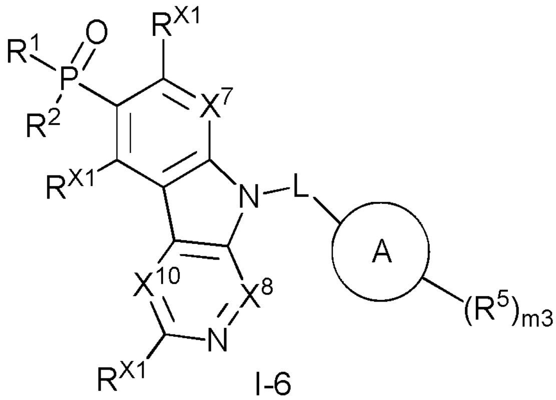

I-6

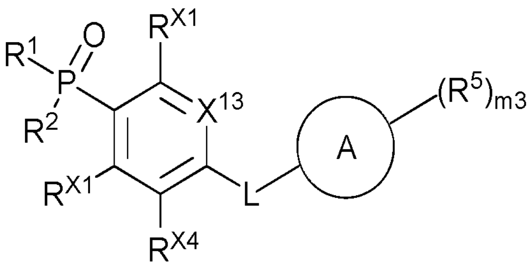

I-7

,

wherein $X^3$ is C or N;

$X^4$ is $CR^{X1}$, N, -C(O)-, or $CR^{X1}R^{X2}$;

$X^5$ is $-CR^{X1}=CR^{X1}-$, $-N=CR^{X1}-$, $-CR^{X1}=N-$, $-O-CR^{X1}R^{X2}-$, or $-NR^{X3}-CR^{X1}R^{X2}-$;

$X^8$ is $CR^{X1}$ or N;

$X^{10}$ is $CR^{X1}$ or N.

In some embodiments, the compound represented by formula I is any one of the following compounds:

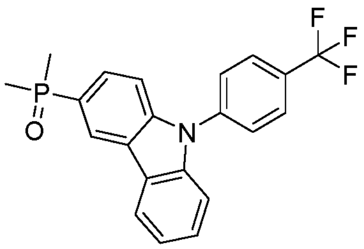

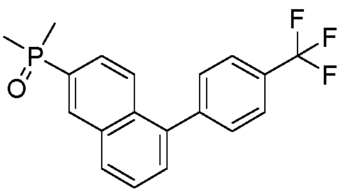

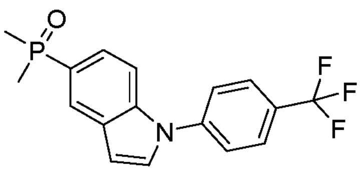

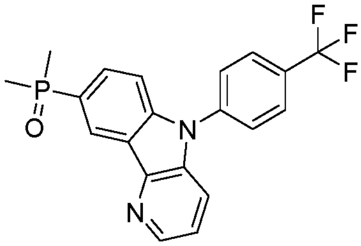

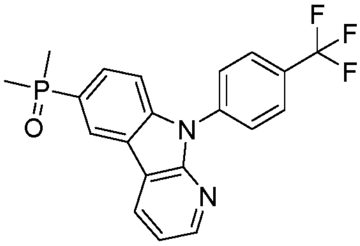

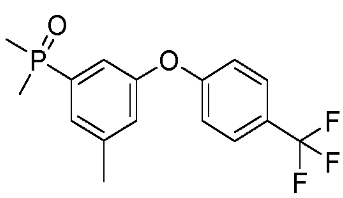

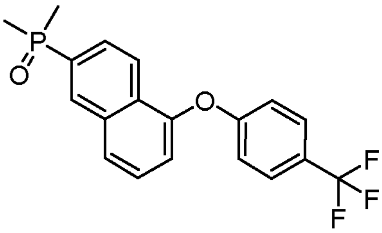

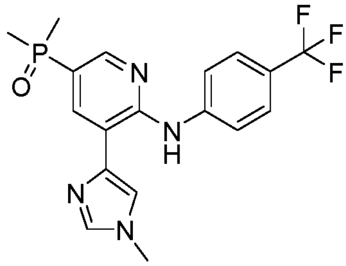

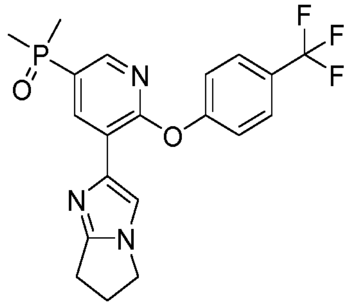

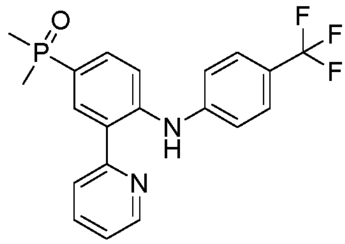

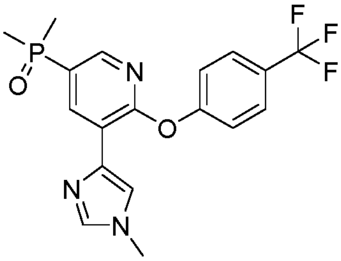

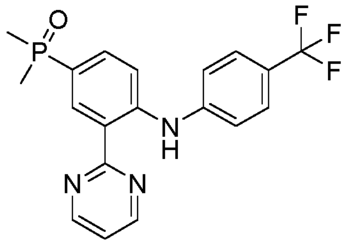

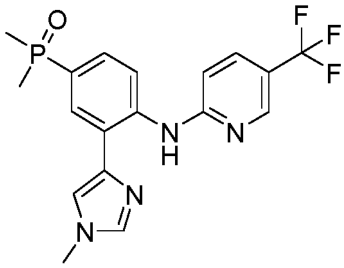

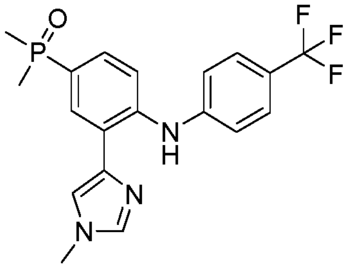

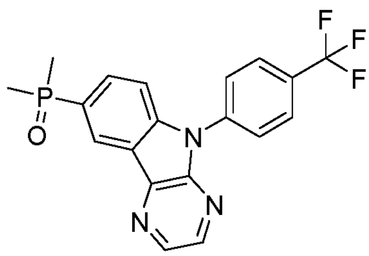

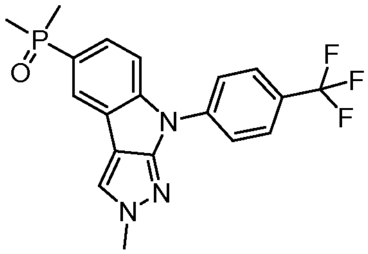

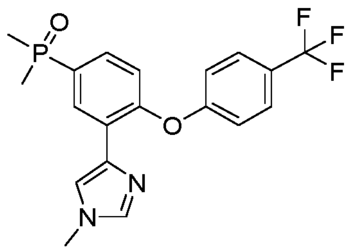

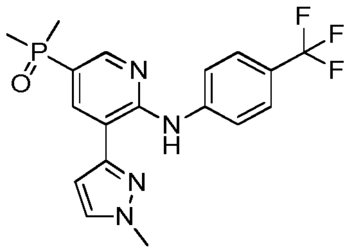

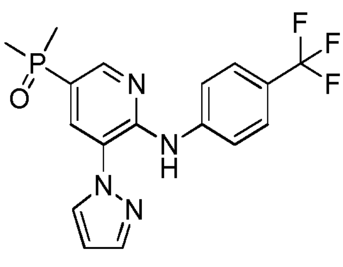

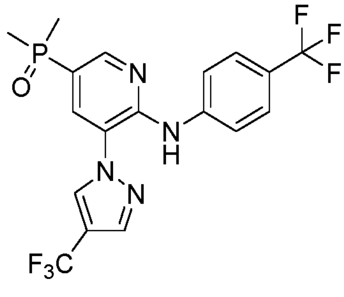

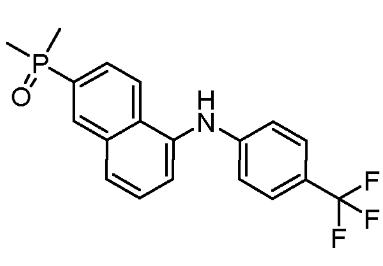

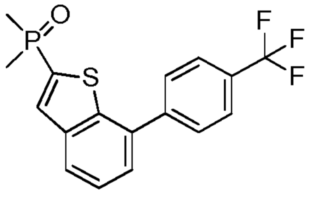

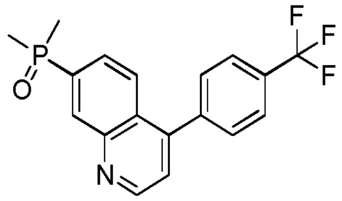
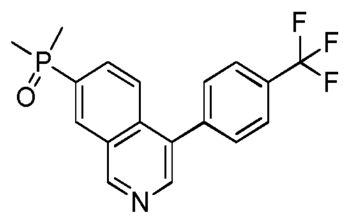
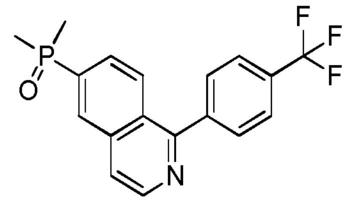

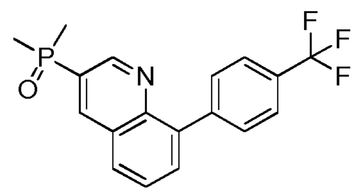

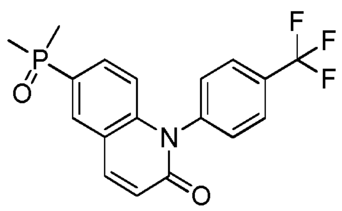
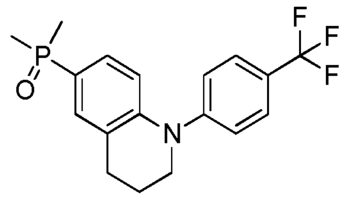
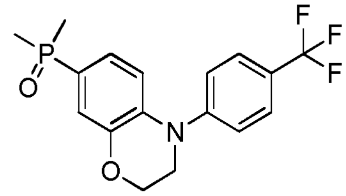

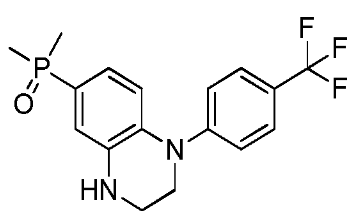
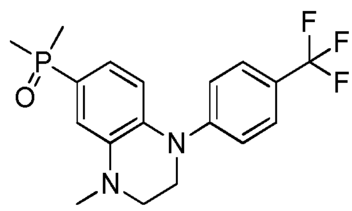
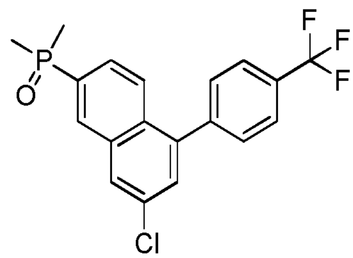

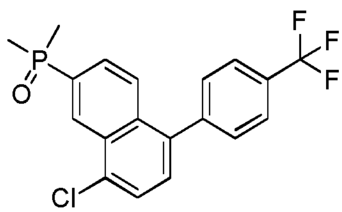
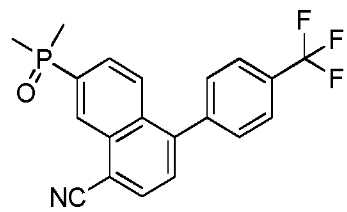
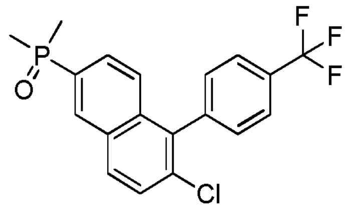

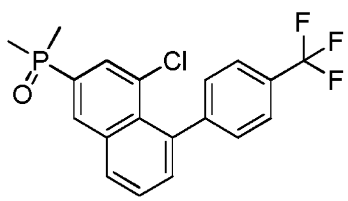
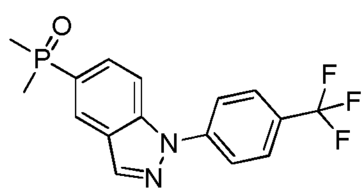
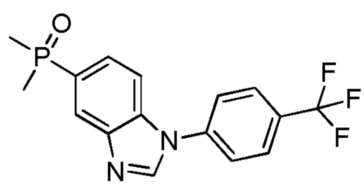

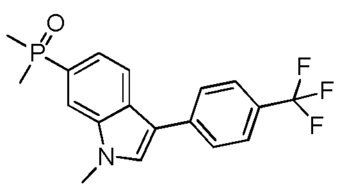
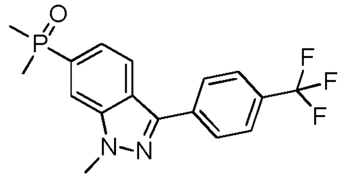

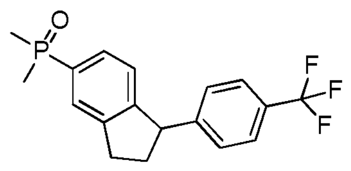
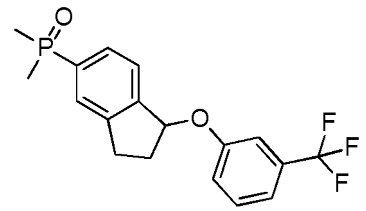

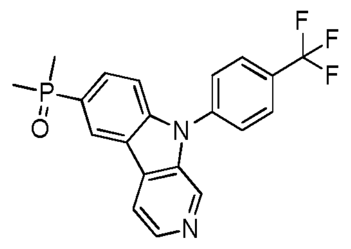
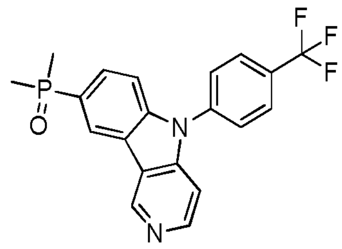

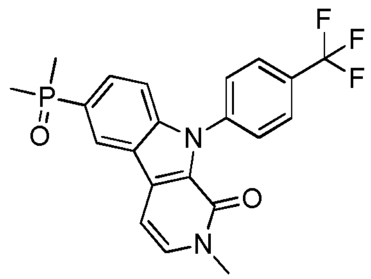
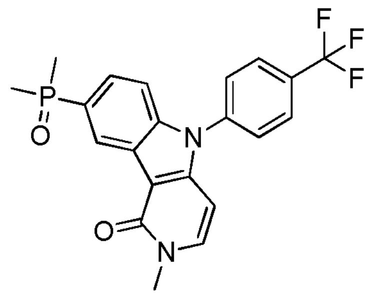

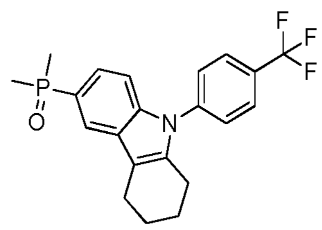
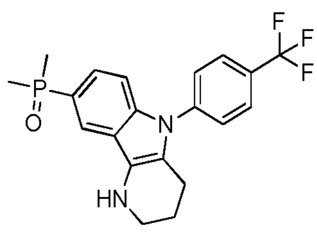
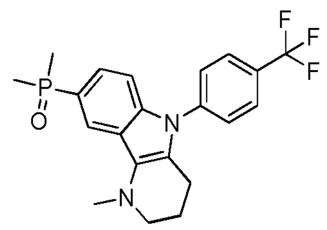

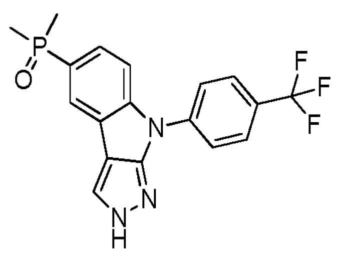
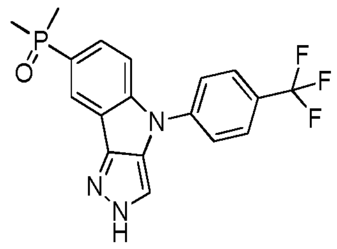
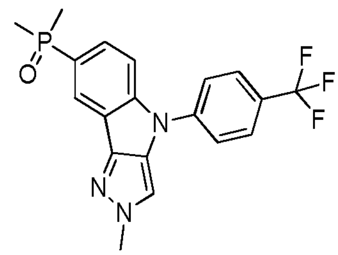

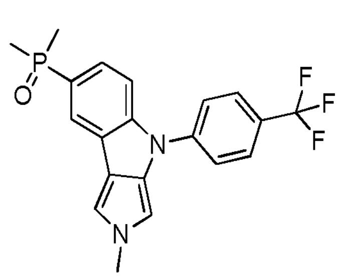
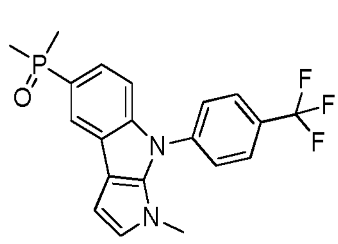
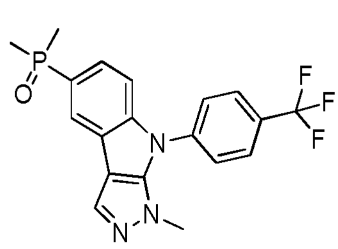

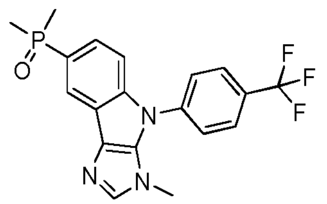
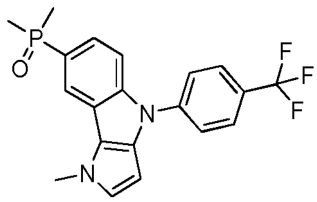
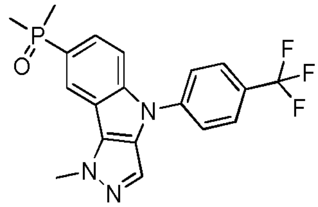

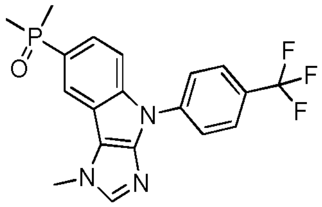
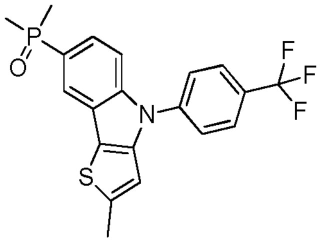
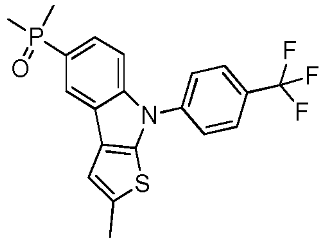

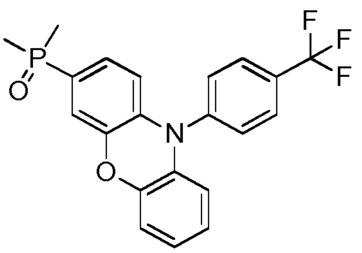
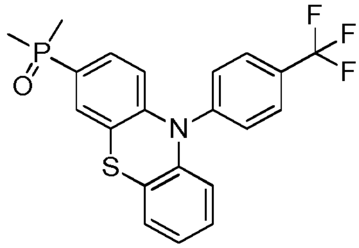

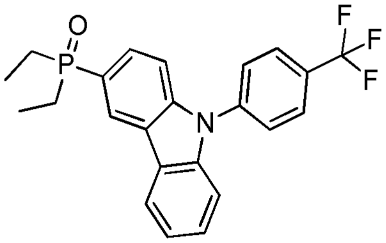
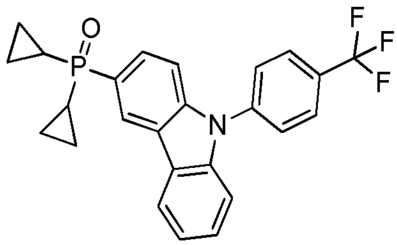
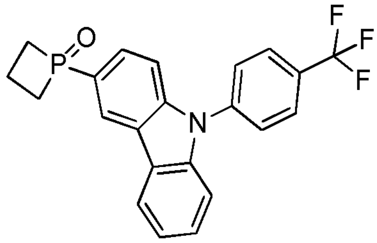

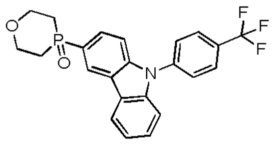
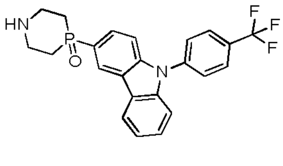
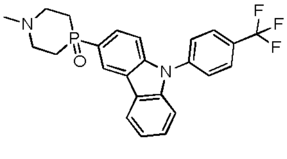
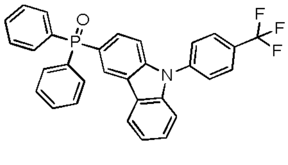

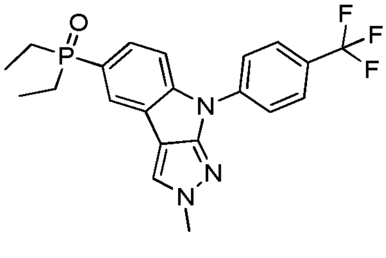
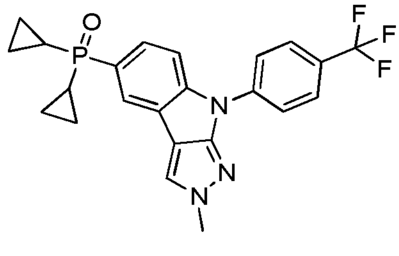
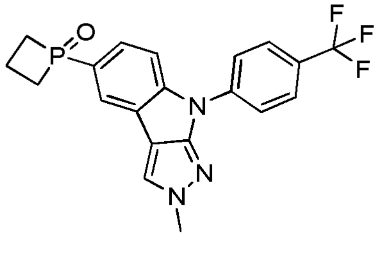

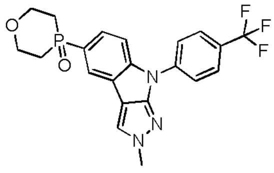
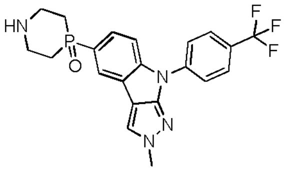
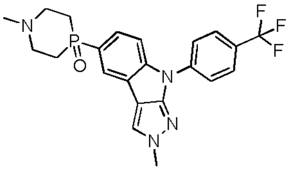
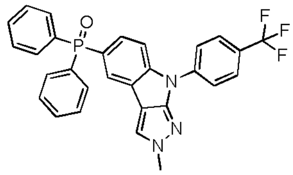

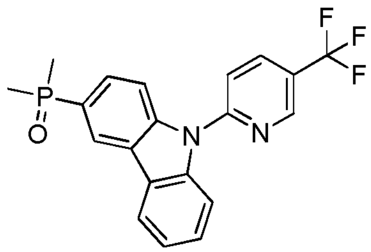
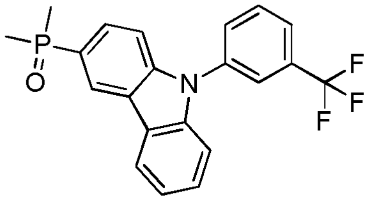
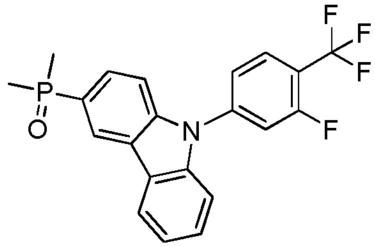

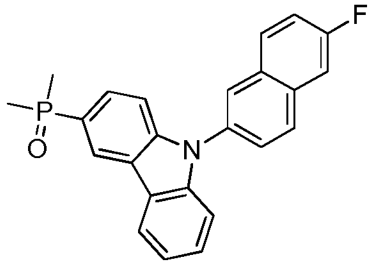
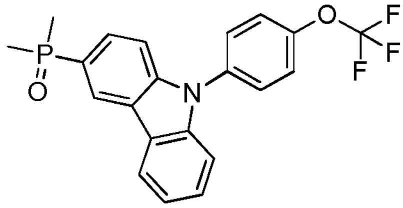
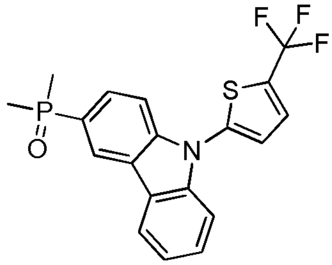

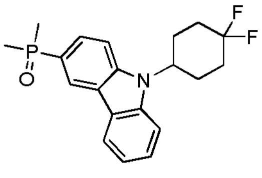
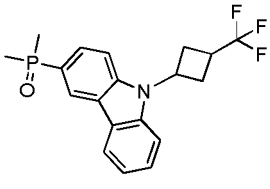
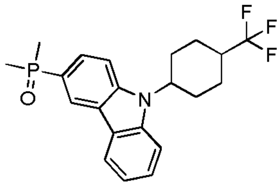
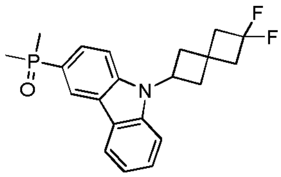

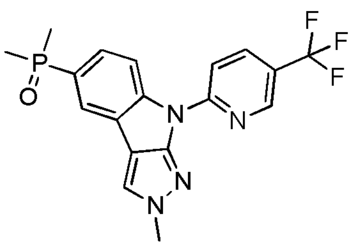
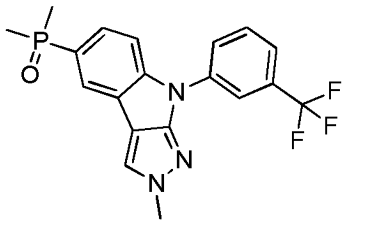
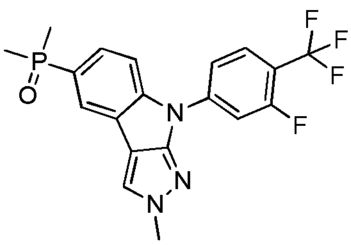

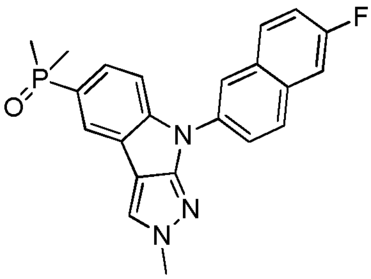
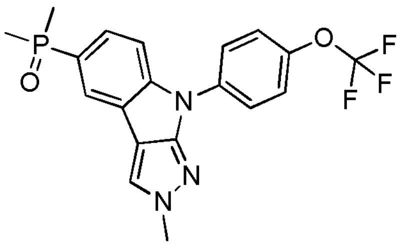
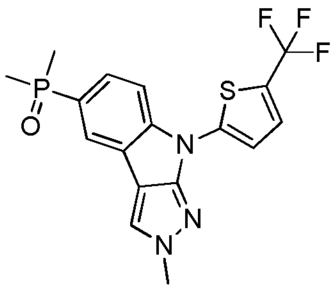

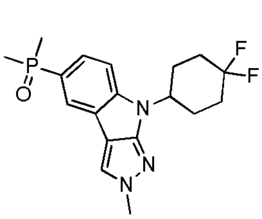
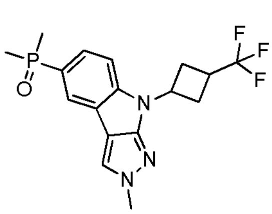
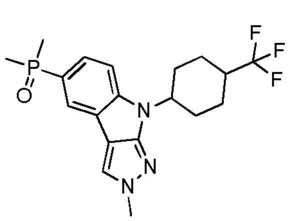
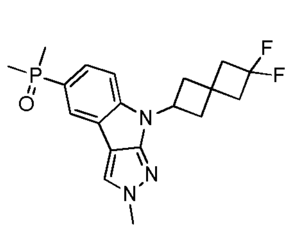

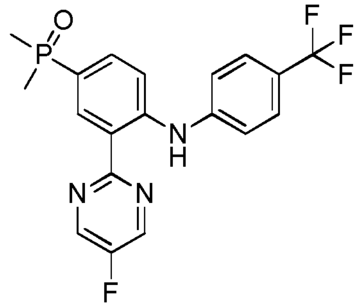

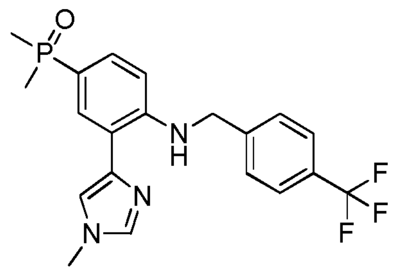

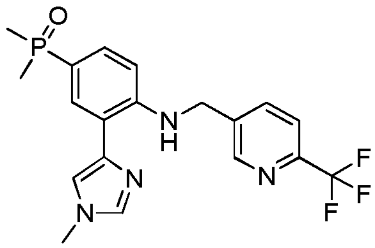

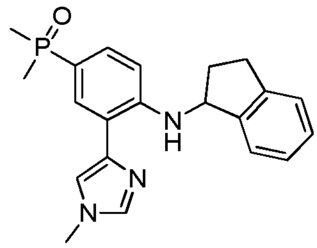

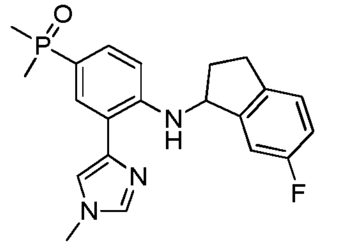

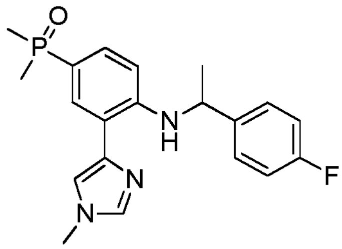

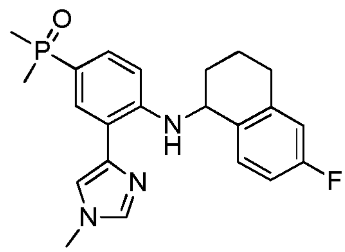

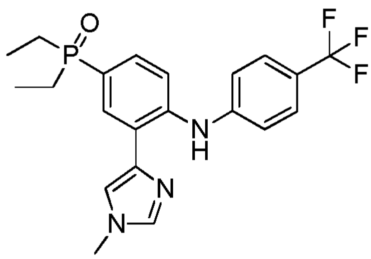

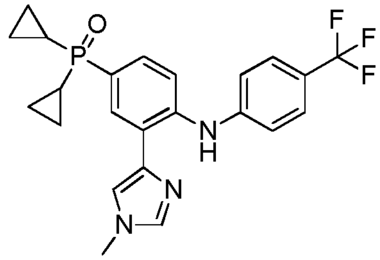

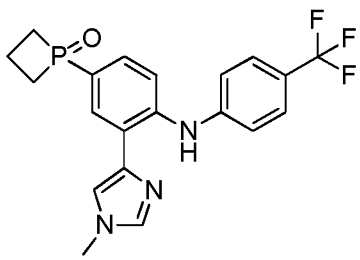

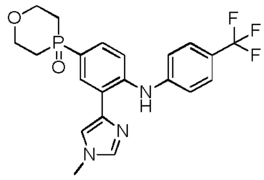

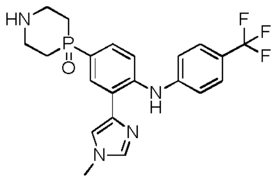

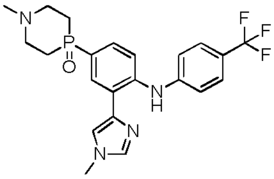

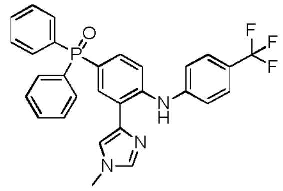

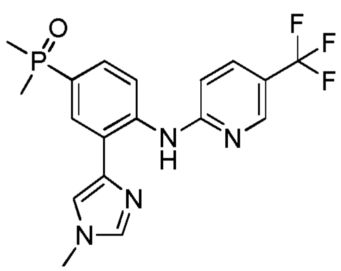

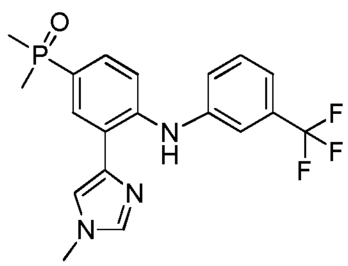

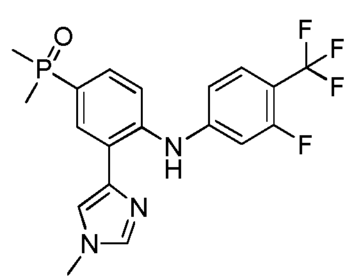

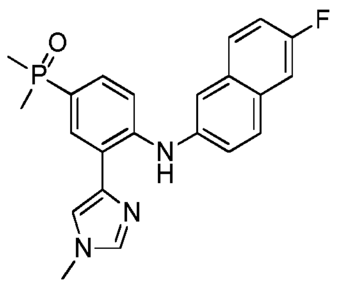
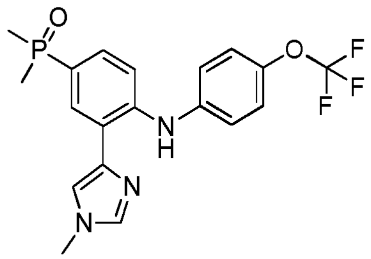
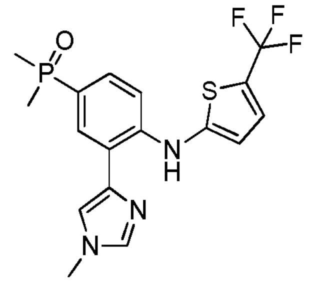

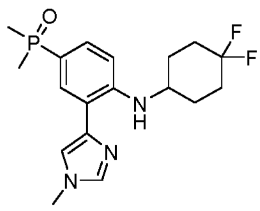
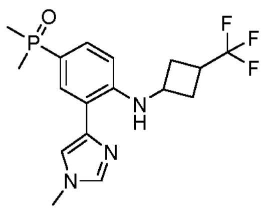
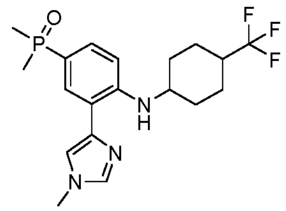
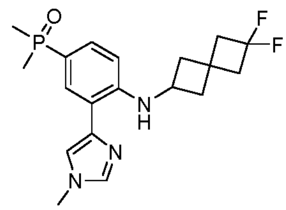

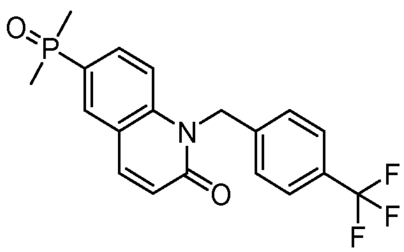
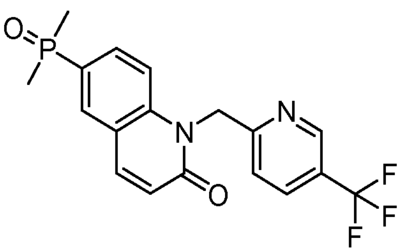

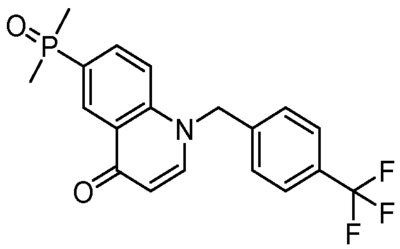
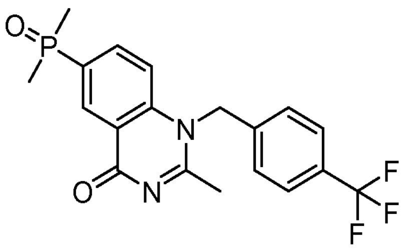
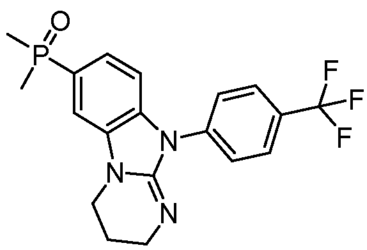

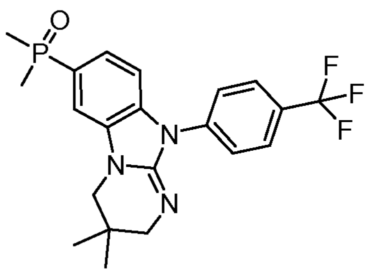
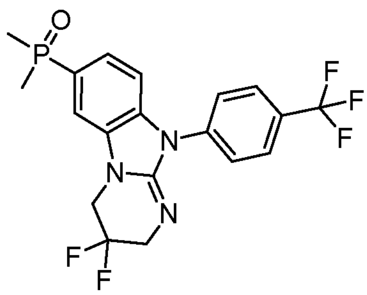
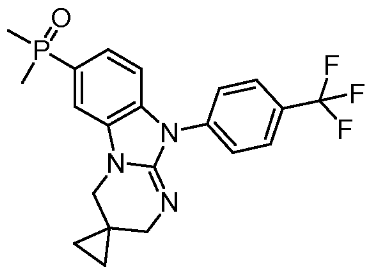

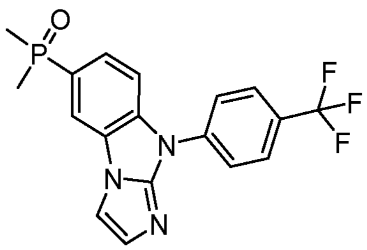
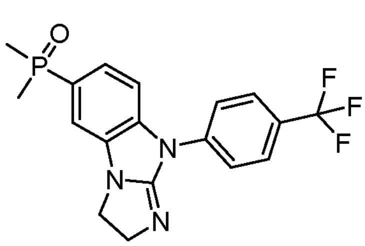
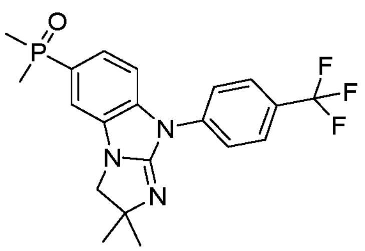

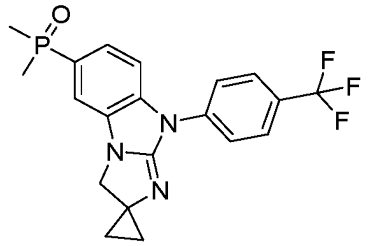

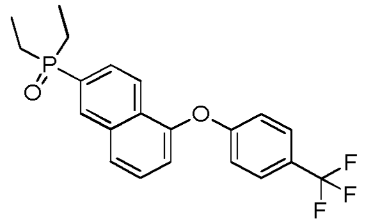

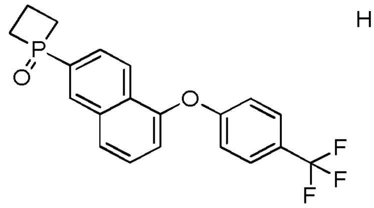

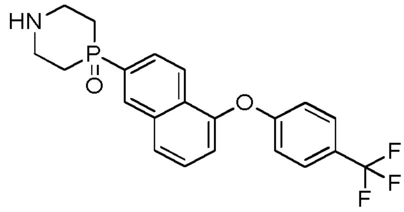

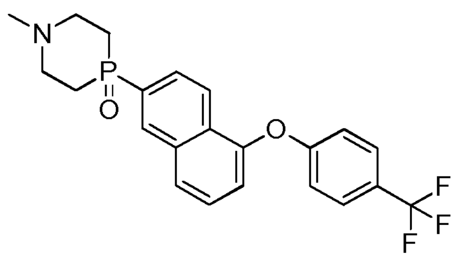

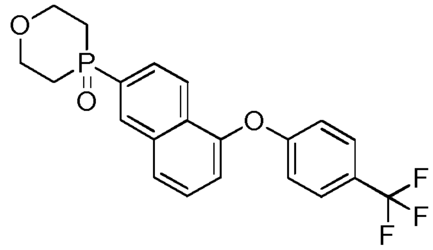

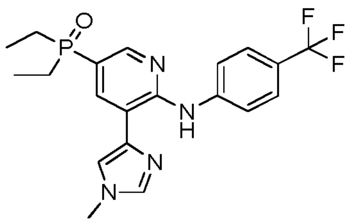

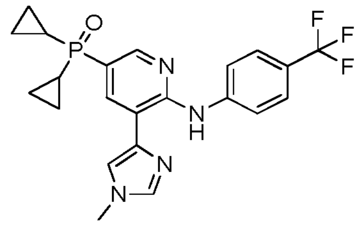

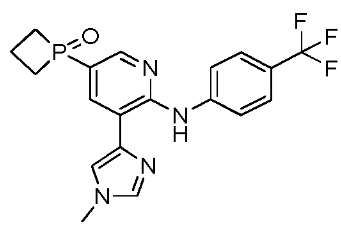

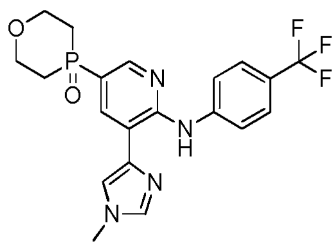

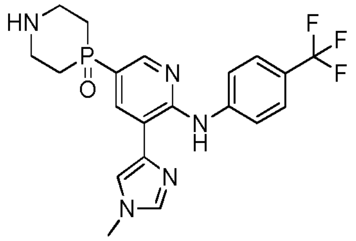

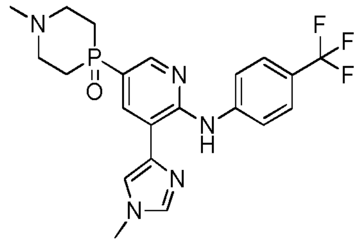

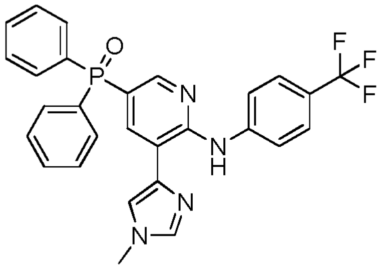

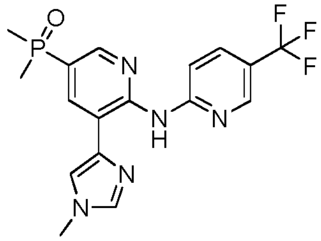

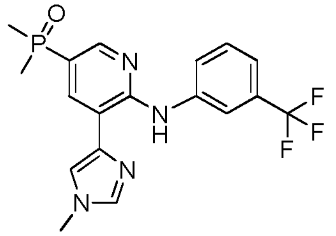

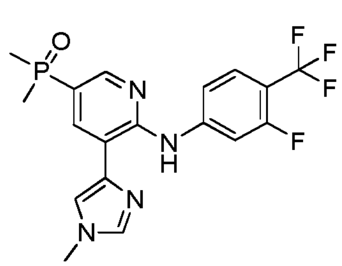
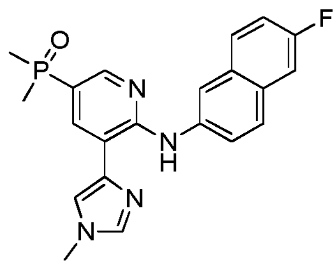
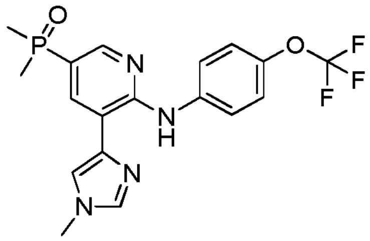

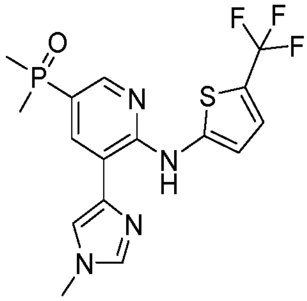
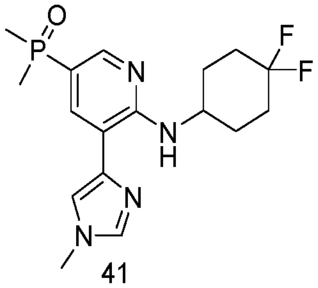

41

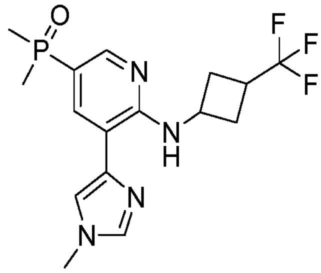

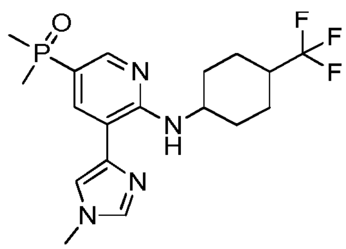
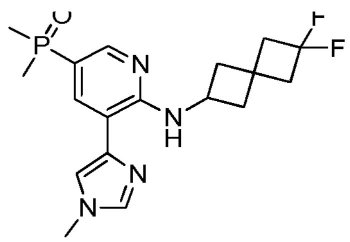

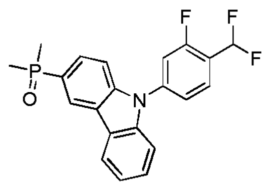
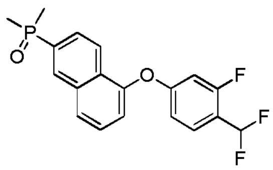
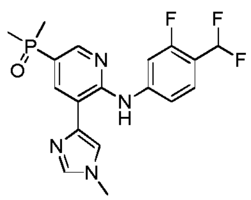
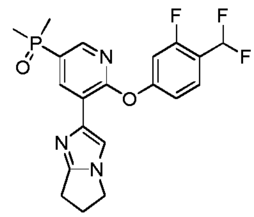

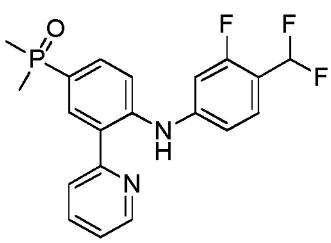
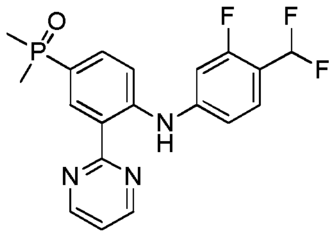
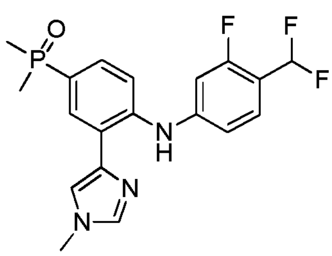

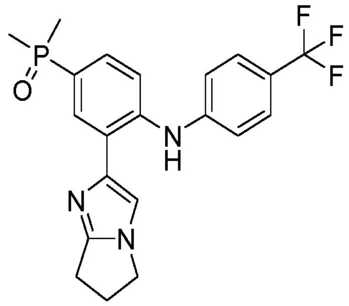
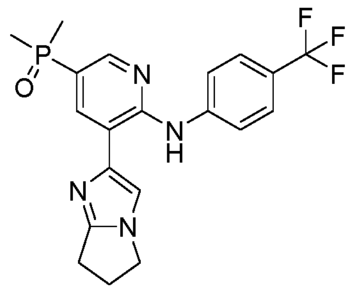
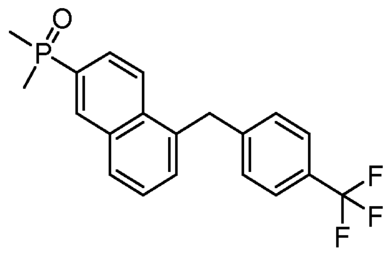

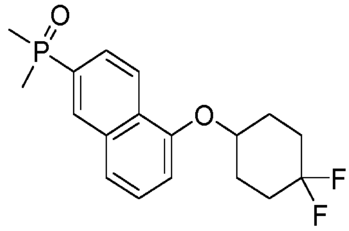
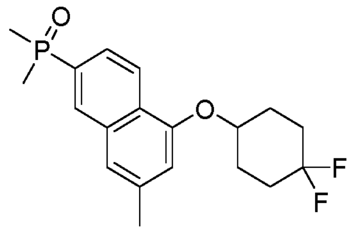
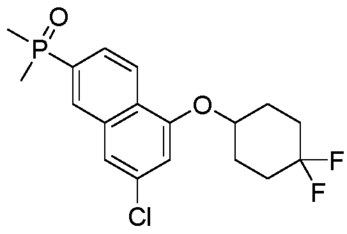

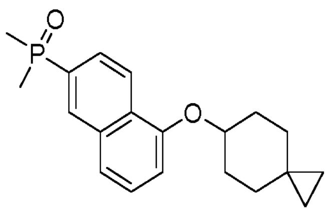
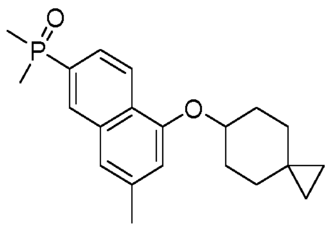
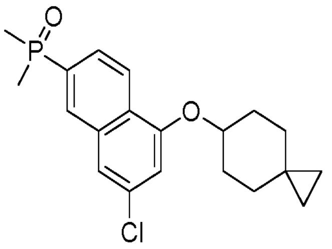

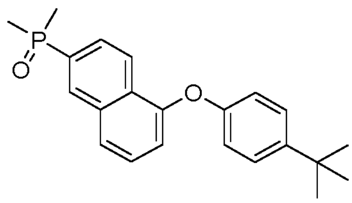
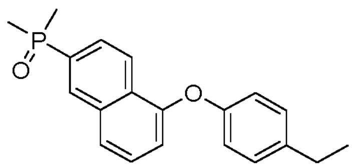
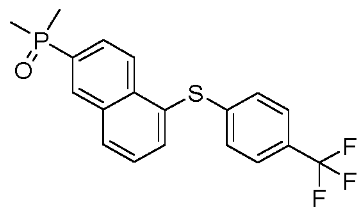

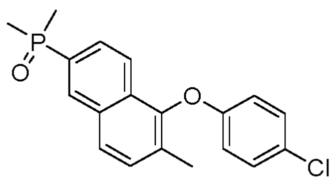
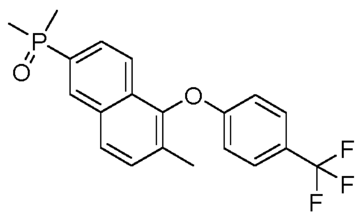
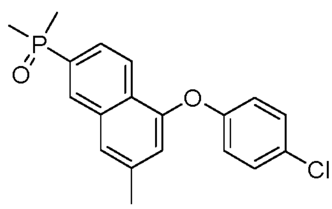

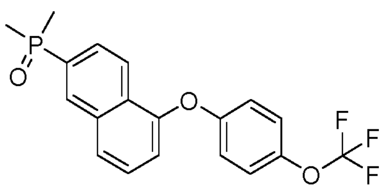
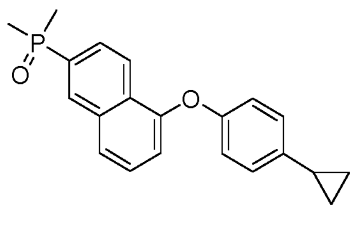
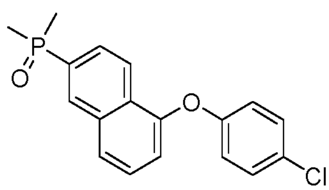

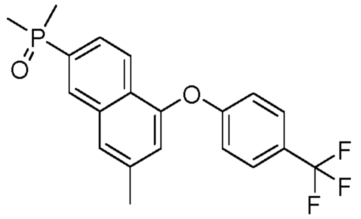

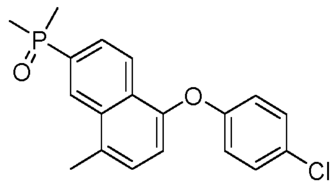

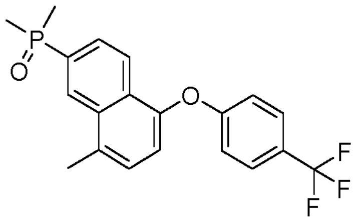

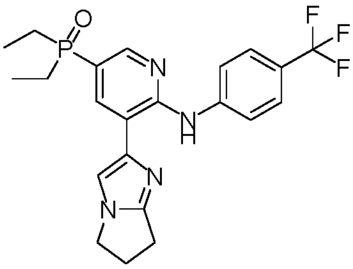

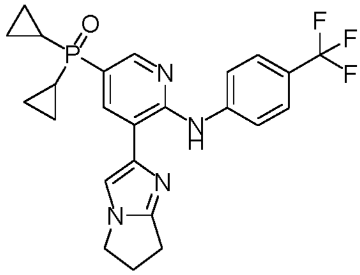

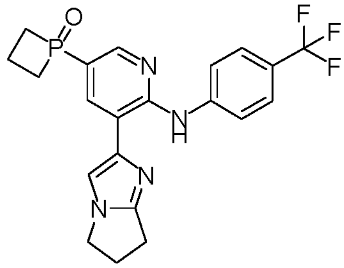

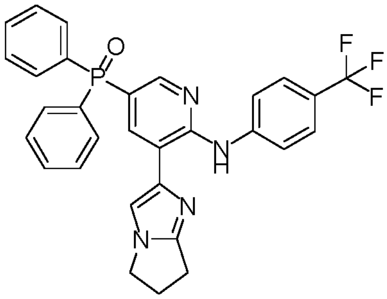

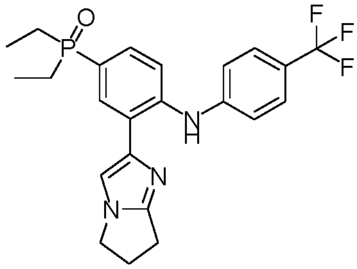

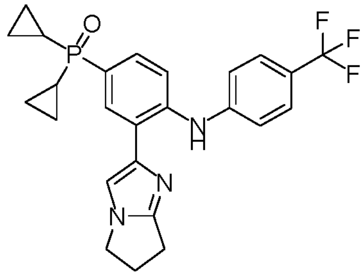

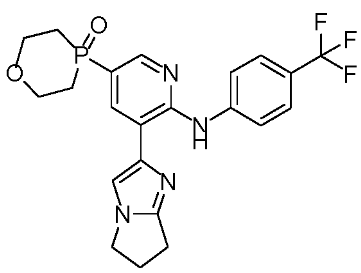

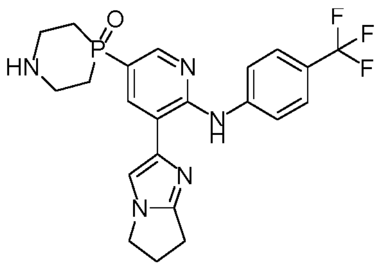

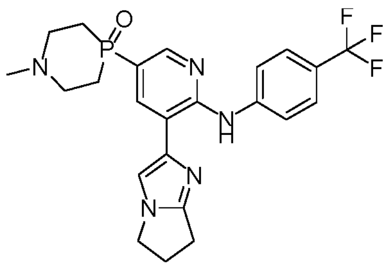

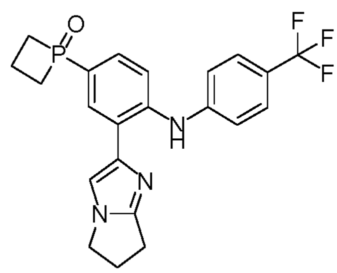

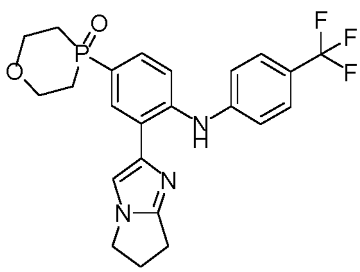

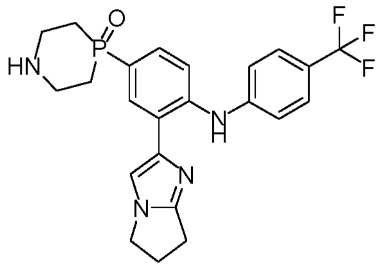

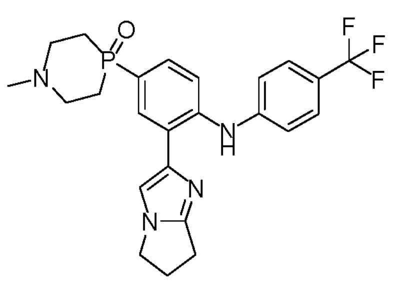
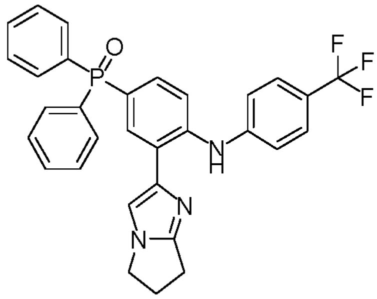
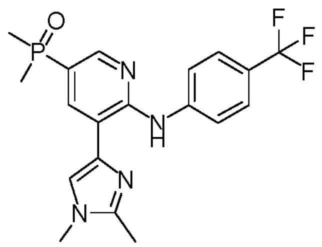
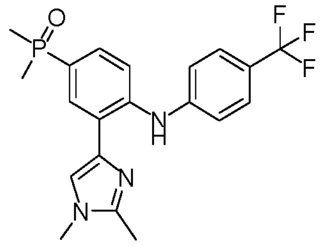
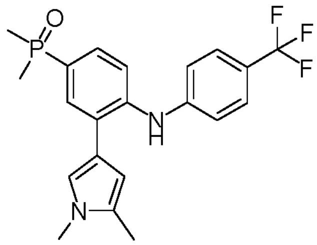
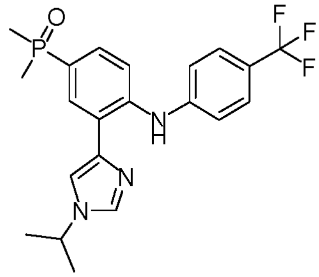
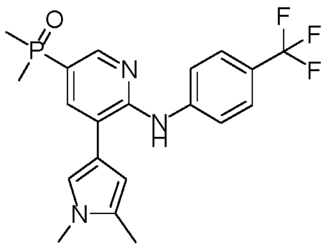
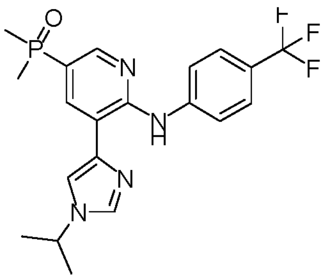
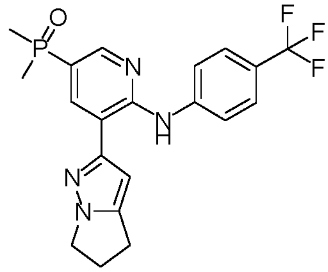
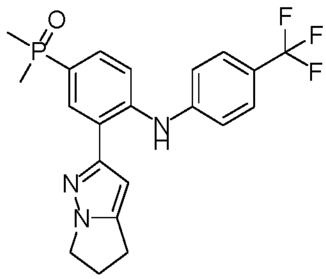
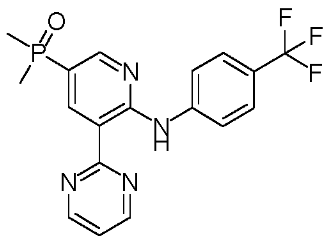
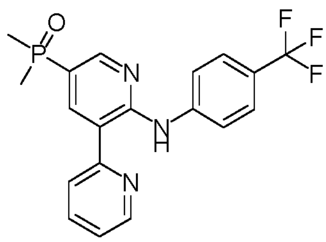
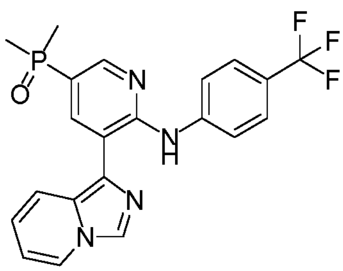
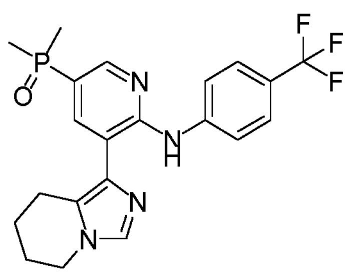
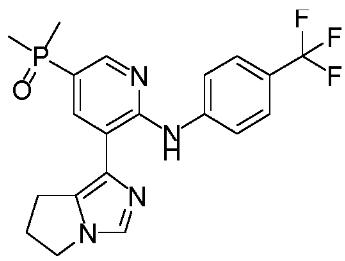

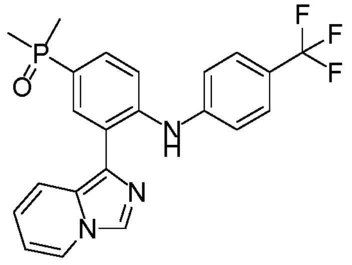
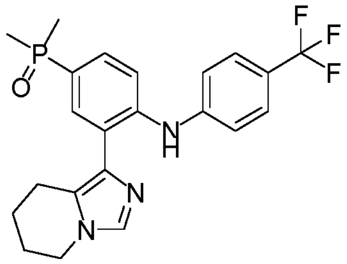
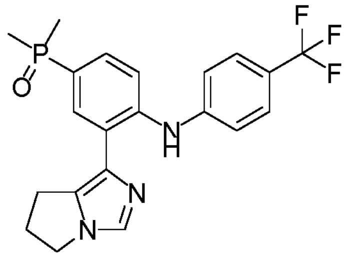
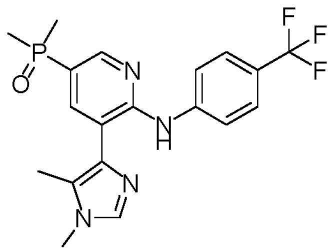
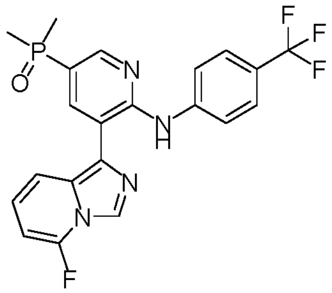
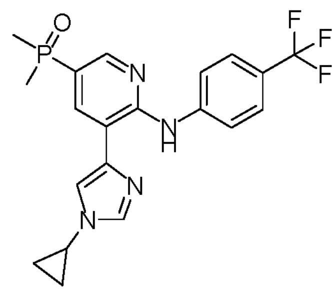
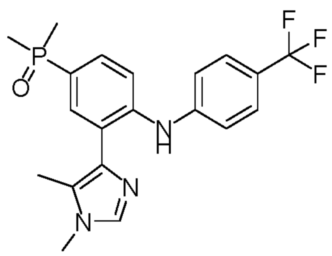
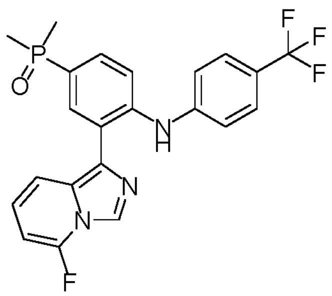
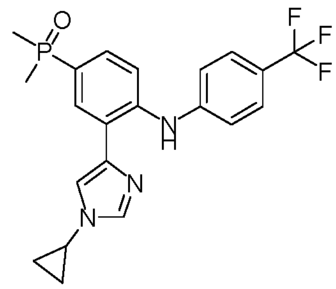
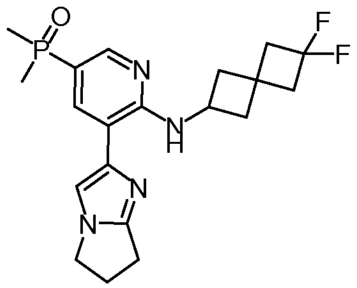
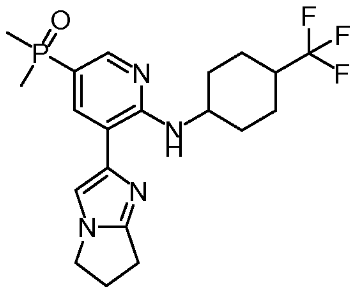
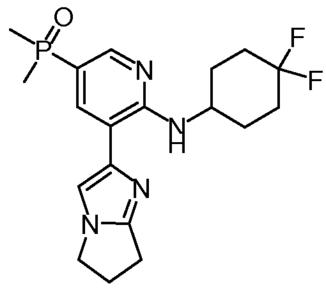
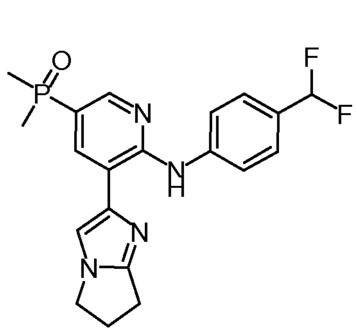
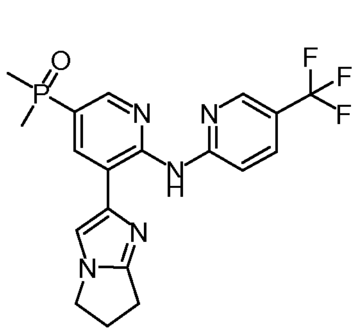
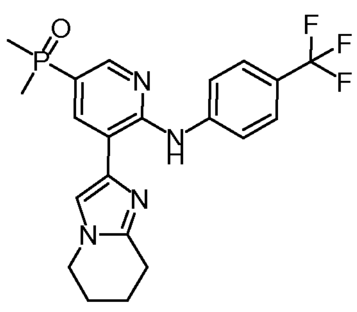

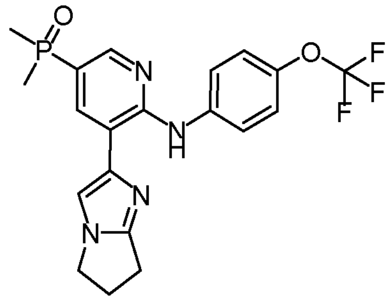
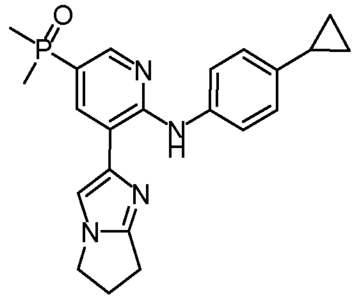
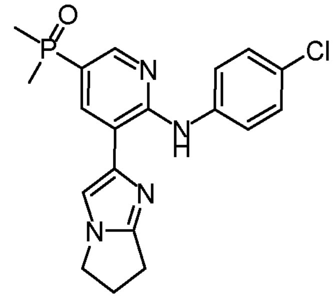

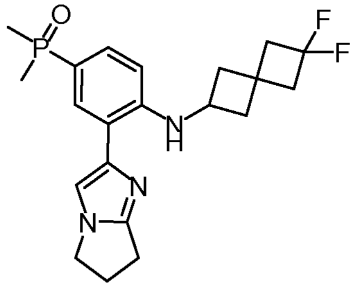
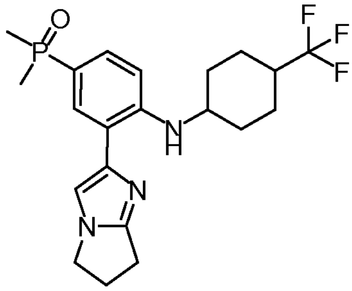
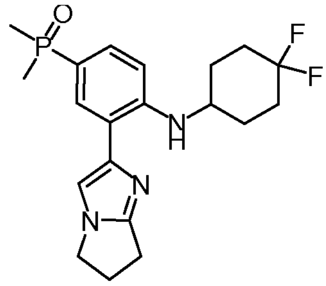

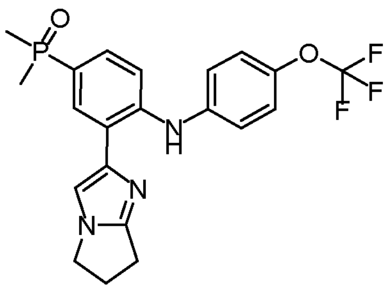
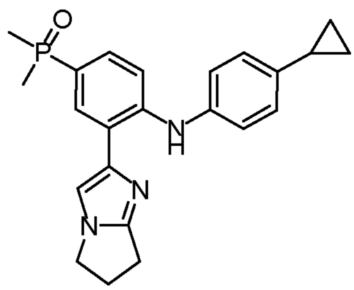
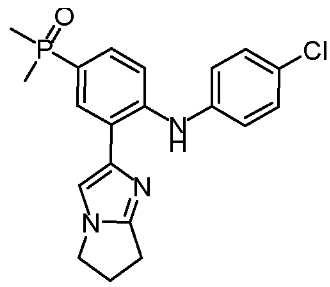

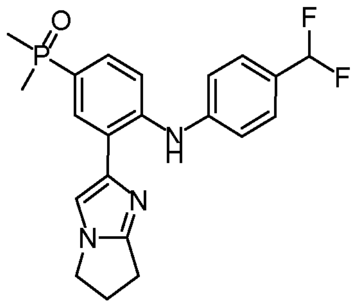
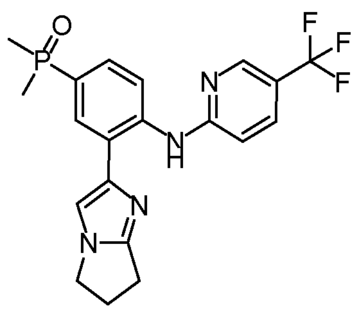
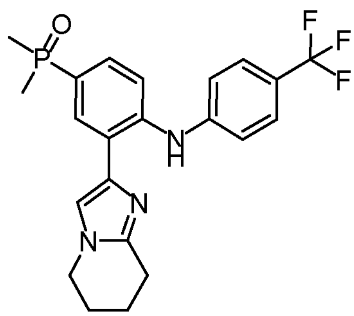

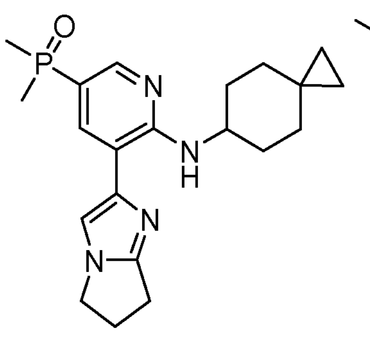
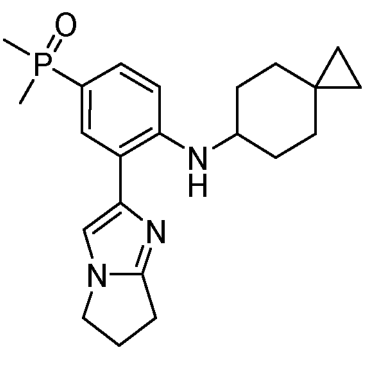
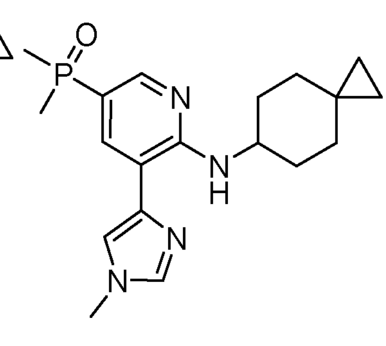

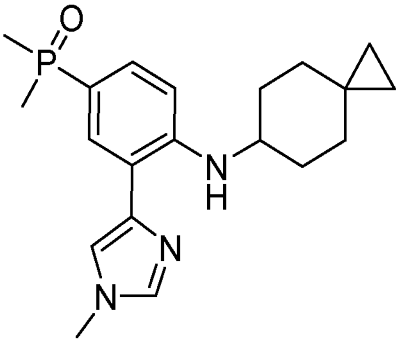
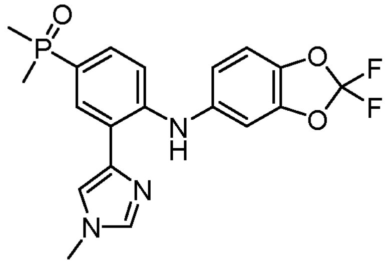
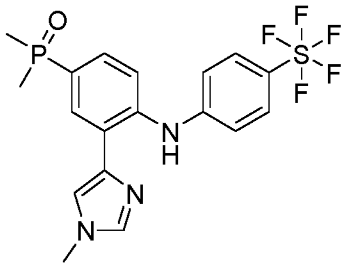
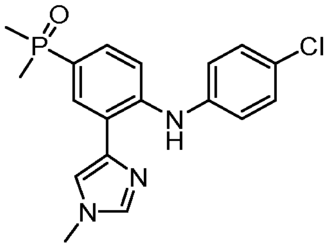
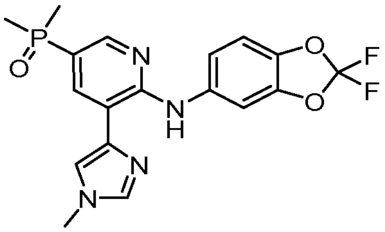
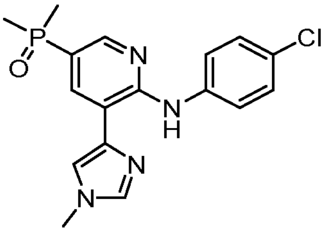
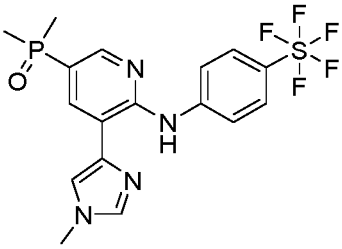
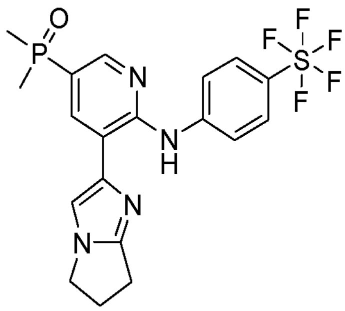
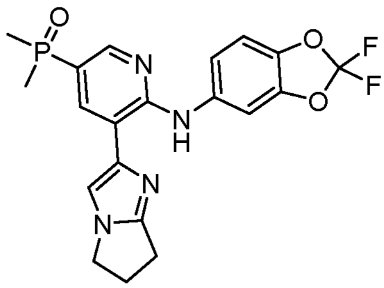

The present disclosure provides a pharmaceutical composition, which comprises:

(1) the compound represented by formula I, the pharmaceutically acceptable salt thereof, the ester thereof, the stereoisomer thereof, the tautomer thereof, the polymorph thereof, the solvate thereof, the metabolite thereof, the isotopic derivative thereof, or the prodrug thereof described above, and
(2) a pharmaceutically acceptable auxiliary material.

The present disclosure further provides use of the compound represented by formula I, the pharmaceutically acceptable salt thereof, the ester thereof, the stereoisomer thereof, the tautomer thereof, the polymorph thereof, the solvate thereof, the metabolite thereof, the isotopic derivative thereof, or the prodrug thereof described above, or the pharmaceutical composition described above in preparing a medicament, wherein the medicament is used for treating diseases and disorders mediated by TEAD, such as cancer (e.g. solid tumors and leukemia), organ regeneration, wound healing, and fibrosis.

**Terminology**

In the present disclosure, the term "pharmaceutically acceptable salt" refers to a salt obtained from the reaction of a compound with a pharmaceutically acceptable acid or base. When the compound contains a relatively acidic functional group, a base addition salt can be obtained by contacting the compound with a sufficient amount of a pharmaceutically acceptable base in a suitable inert solvent. When the compound contains a relatively basic functional group, an acid addition salt can be obtained by contacting the compound with a sufficient amount of a pharmaceutically acceptable acid in a suitable inert solvent. For more details, see Handbook of Pharmaceutical Salts: Properties, Selection, and Use (P. Heinrich Stahl, Camille G. Wermuth, 2011, 2nd Revised Edition).

The term "ester" includes physiologically hydrolyzable esters (which can be hydrolyzed under physiological conditions to release the compounds of the present disclosure in the form of free acids or alcohols). Additionally, the

compounds of the present disclosure may themselves also be esters.

The term "stereoisomer" refers to a cis-trans isomer or an optical isomer; the cis-trans isomer is an isomer caused by the inability of a double bond or a single bond of a ring-forming carbon atom to rotate freely, and the optical isomer is a stereoisomer having different optical rotation properties due to the absence of anti-axisymmetry in the molecule.

The term "tautomer" means that different functional isomers are in dynamic equilibrium at room temperature and can be rapidly converted into each other. If tautomers are possible (e.g., in a solution), the chemical equilibrium of the tautomers can be achieved. For example, a proton tautomer, also known as a prototropic tautomer, includes the interconversion by proton transfer, such as keto-enol isomerization and imine-enamine isomerization. A valence isomer includes the interconversion by recombination of some bonding electrons. A specific example of the keto-enol tautomerization is the interconversion between tautomers pentane-2,4-dione and 4-hydroxypent-3-en-2-one.

The term "polymorph" refers to a compound that presents in multiple crystal forms.

The term "solvate" refers to a substance formed by combining a compound with a solvent. Solvates are divided into stoichiometric solvates and non-stoichiometric solvates.

The term "metabolite" refers to a substance formed *in vivo* following the administration of the compound of the present disclosure.

The term "isotopic derivative" refers to a compound in which one or more atoms have an isotopic abundance that differs from their natural abundance. For example, one or more atoms in the compound are replaced by atoms with a lower natural abundance of mass number; for example, one hydrogen atom in the compound is replaced by deuterium.

The term "prodrug" refers to a derivative of a compound that contains a biologically reactive functional group such that, under biological conditions (*in vitro* or *in vivo*), the biologically reactive functional group can be cleaved from the compound or otherwise reacted to provide the compound. Typically, a prodrug is inactive, or at least less active than the compound itself, such that it does not exert its activity until the compound is cleaved from the biologically reactive functional group. The biologically reactive functional group can be hydrolyzed or oxidized under biological conditions to provide the compound. For example, the prodrug may contain a biohydrolyzable group. Examples of the biohydrolyzable group include, but are not limited to, a biohydrolyzable phosphate, a biohydrolyzable ester, a biohydrolyzable amide, a biohydrolyzable carbonate, a biohydrolyzable carbamate, and a biohydrolyzable ureide.

As used herein, a substituent may be preceded by a single dash "-" indicating that the named substituent is connected to a parent moiety by a single bond. When the direction for connection of connecting groups recited herein is not specified, the direction for connection is in the same direction as the reading sequence from left to right. For example, if the connecting group L in

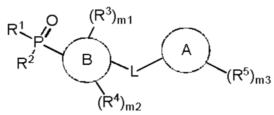

I

is -C-D-, then -C-D- connects ring B and ring A in the same direction as the reading sequence from left to right to form

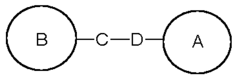,

but not to form

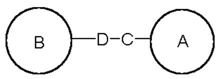.

Specifically, in the present disclosure, when L is "-$NR^{L3}CR^{L1}R^{L2}$-", it indicates that N is connected to ring B in the parent structure by a single bond, rather than the C terminus being connected to ring B in the parent structure.

In the present disclosure,

“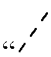”

in a structural fragment means that the structural fragment is connected to the rest of the molecule by this bond. For example,

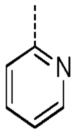

refers to pyridyl.

In the present disclosure, the term "one or more" refers to 1, 2, 3, 4, or 5, such as 1, 2, or 3.

In the present disclosure, the term "halogen" refers to fluorine, chlorine, bromine, or iodine.

The term "oxo" refers to =O, which means that an oxygen atom replaces two hydrogens on the same carbon atom, i.e., a carbonyl group replaces a methylene group.

In the present disclosure, the term "alkyl" refers to a linear or branched, saturated, monovalent hydrocarbon group with a specified number of carbon atoms (e.g., $C_1$-$C_6$). The alkyl includes, but is not limited to: methyl, ethyl, *n*-propyl, isopropyl, *n*-butyl, isobutyl, see-butyl, *tert*-butyl, *n*-pentyl, *n*-hexyl, and the like.

In the present disclosure, the term "alkoxy" refers to the group $R^Y$-O-, wherein $R^Y$ is as defined for the term "alkyl". The alkoxy includes, but is not limited to: methoxy, ethoxy, *n*-propoxy, isopropoxy, and the like.

In the present disclosure, the term "cycloalkyl" refers to a cyclic, saturated, monovalent hydrocarbon group with a specified number of carbon atoms (e.g., $C_3$-$C_6$). The cycloalkyl includes, but is not limited to:

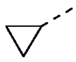 , 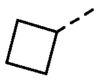 , 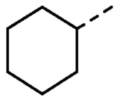 ,

and the like.

In the present disclosure, the term "heterocycloalkyl" refers to a cyclic, saturated, monovalent group with a specified number of ring atoms (e.g., 3- to 12-membered or 5- to 6-membered), a specified number of heteroatoms (e.g., 1, 2, or 3), and a specified heteroatom type (one or more of N, O, and S). The heterocycloalkyl is connected to the rest of the molecule by a carbon atom or a heteroatom. The heterocycloalkyl includes, but is not limited to:

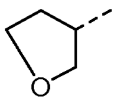 , 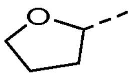 , 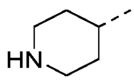 , 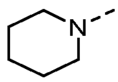 ,

and the like.

In the present disclosure, the term "heteroaryl" refers to a cyclic, unsaturated group with a specified number of ring atoms (e.g., 5- to 12-membered or 5- to 6-membered), a specified number of heteroatoms (e.g., 1, 2, or 3), and a specified heteroatom type (one or more of N, O, and S), which is either monocyclic or polycyclic; when it is polycyclic, the individual rings share two atoms and one bond, and each of the rings is aromatic. The heteroaryl is connected to the rest of the molecule by a carbon atom or a heteroatom; the heteroaryl is connected to the rest of the molecule by a ring with or without a heteroatom. The heteroaryl includes, but is not limited to,

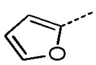 , 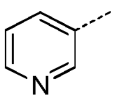 , 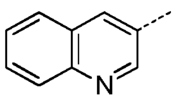 , 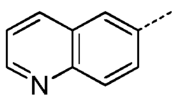 ,

and the like.

In the present disclosure, the term "aryl" refers to a cyclic, unsaturated hydrocarbon group with a specified number of carbon atoms (e.g., $C_6$-$C_{10}$), which is either monocyclic or polycyclic (e.g., bicyclic or tricyclic); when it is polycyclic, the individual rings share two atoms and one bond, and each of the rings is aromatic. The aryl includes, but is not limited to: phenyl, naphthyl, and the like.

In the present disclosure, the term "pharmaceutically acceptable auxiliary material" refers to all substances, other than the active pharmaceutical ingredient, contained in a pharmaceutical formulation, which are generally divided into two main categories: excipients and additives. For more details, see Pharmacopoeia of the People's Republic of China (2020 Edition) and Handbook of Pharmaceutical Excipients (Paul J Sheskey, Bruno C Hancock, Gary P Moss, David J Goldfarb, 2020, 9th Edition).

The preferred conditions described above may be combined arbitrarily to give preferred embodiments of the present disclosure without departing from the general knowledge in the art.

The reagents and starting materials used in the present disclosure are commercially available.

DETAILED DESCRIPTION

The present disclosure is further illustrated by the following examples; however, these examples are not intended to limit the present disclosure. Experimental procedures without specified conditions in the following examples were conducted in accordance with conventional procedures and conditions, or in accordance with product instructions.

The compound represented by formula I of the present disclosure can be obtained through the following routes:

Route 1:

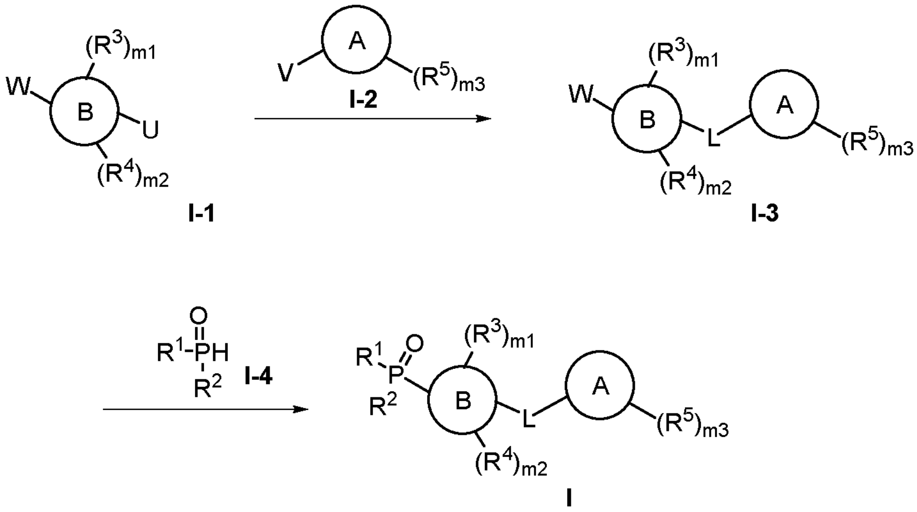

I-1
I-3
I

Step 1: Compound **I-1** reacts with compound **I-2** to give compound I-**3.**

W is selected from halogen and an alkyl/aryl sulfonate group, wherein the halogen is preferably chlorine, bromine, or iodine, and the alkyl/aryl sulfonate group includes, but is not limited to, methanesulfonate, trifluoromethanesulfonate, benzenesulfonate, p-toluenesulfonate, and naphthalenesulfonate, preferably methanesulfonate and *p*-toluenesulfonate; U is selected from H, OH, SH, $NHR^{L3}$, $CR^{L1}R^{L2}$ tin reagent, $CR^{L1}R^{L2}$ zinc reagent, $CR^{L1}R^{L2}$ magnesium reagent, etc.; V is selected from boronic acid, borate, halogen, and an alkyl/aryl sulfonate group, wherein the halogen is preferably chlorine, bromine, or iodine, and the alkyl/aryl sulfonate group includes, but is not limited to, methanesulfonate, trifluoromethanesulfonate, benzenesulfonate, *p*-toluenesulfonate, and naphthalenesulfonate, preferably methanesulfonate and*p*-toluenesulfonate;

the reaction is carried out in the presence of a suitable catalyst, such as: tetrakis(triphenylphosphine)palladium(0), bis(triphenylphosphine)palladium(II) dichloride, [1,1'-bis(diphenylphosphino)ferrocene]palladium(II) dichloride, tris(dibenzylideneacetone)dipalladium(0), palladium dichloride, palladium(II) acetate, and chloro(2-dicyclohexylphosphino-2',6'-di-isopropoxy-1,1'-biphenyl)(2-amino-1,1'-biphenyl-2-yl)palladium, preferably [1,1'-bis(diphenylphosphino)ferrocene]palladium(II) dichloride;

the reaction is carried out in the presence of a suitable base, such as: sodium carbonate, cesium carbonate, potassium *tert*-butoxide, and sodium *tert*-butoxide, preferably sodium carbonate;

the reaction is carried out in a suitable solvent, such as 1,4-dioxane, 1,4-dioxane/water, 1,2-dichloroethane, 1,2-dichloroethane/water, *N,N*-dimethylformamide, *N,N*-dimethylformamide/water, dimethyl sulfoxide, dimethyl sulfoxide/water, N-methylpyrrolidone, and *N*-methylpyrrolidone/water, preferably 1,4-dioxane/water and *N,N*-dimethylformamide;

the reaction is carried out at a suitable temperature, such as 25 °C to 140 °C, preferably 80 °C to 110 °C.

Step 2: Compound **I-3** reacts with phosphine oxide **I-4** to give compound **I.**

The reaction is carried out in the presence of a suitable catalyst, such as: tetrakis(triphenylphosphine)palladium(0), bis(triphenylphosphine)palladium(II) dichloride, [1,1'-bis(diphenylphosphino)ferrocene]palladium(II) dichloride, tris(dibenzylideneacetone)dipalladium(0), palladium dichloride, palladium(II) acetate, and chloro(2-dicyclohexylphosphino-2',6'-di-isopropoxy-1,1'-biphenyl)(2-amino-1,1'-biphenyl-2-yl)palladium, preferably chloro(2-dicyclohexylphosphino-2',6'-di-isopropoxy-1,1'-biphenyl)(2-amino-1,1'-biphenyl-2-yl)palladium and tris(dibenzylideneacetone)dipalladium(0);

the reaction is carried out in the presence of a suitable base, such as: sodium carbonate, cesium carbonate, potassium tert-butoxide, and sodium tert-butoxide, preferably cesium carbonate;
the reaction is carried out in a suitable solvent, such as 1,4-dioxane, 1,4-dioxane/water, 1,2-dichloroethane, 1,2-dichloroethane/water, *N,N*-dimethylformamide, *N,N*-dimethylformamide/water, dimethyl sulfoxide, dimethyl sulfoxide/water, N-methylpyrrolidone, and N-methylpyrrolidone/water, preferably 1,4-dioxane and *N*,*N*-dimethylformamide;
the reaction is carried out at a suitable temperature, such as 25 °C to 140 °C, preferably 40 °C to 110 °C.

Route 2:

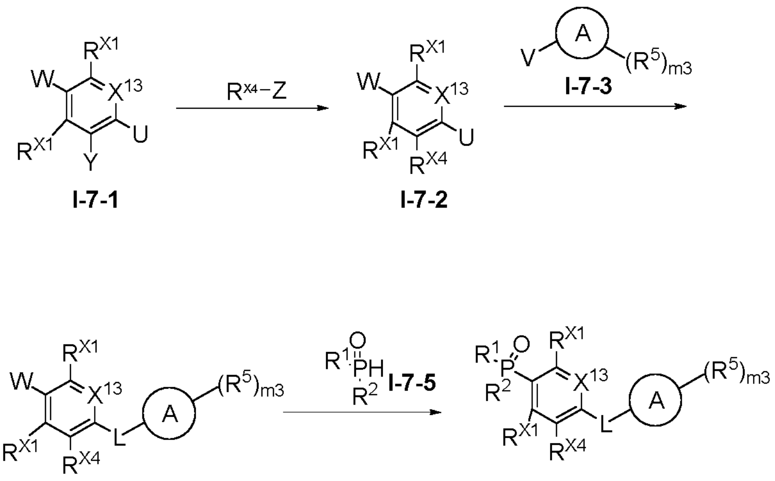


I-7-1 I-7-2 I-7-4 I-7

Step 1: Compound **I-7-1** reacts with compound $R^{X4}$-Z to give compound **1-7-2.**

Y is selected from halogen and an alkyl/aryl sulfonate group, wherein the halogen is preferably chlorine, bromine, or iodine, and the alkyl/aryl sulfonate group includes, but is not limited to, methanesulfonate, trifluoromethanesulfonate, benzenesulfonate, *p*-toluenesulfonate, and naphthalenesulfonate, preferably methanesulfonate and *p*-toluenesulfonate; Z is selected from boronic acid, borate, tin reagent, zinc reagent, and magnesium reagent, preferably boronic acid and borate;

the reaction is carried out in the presence of a suitable catalyst, such as: tetrakis(triphenylphosphine)palladium(0), bis(triphenylphosphine)palladium(II) dichloride, [1,1'-bis(diphenylphosphino)ferrocene]palladium(II) dichloride, tris(dibenzylideneacetone)dipalladium(0), palladium dichloride, palladium(II) acetate, and chloro(2-dicyclohexylphosphino-2',6'-di-isopropoxy-1,1'-biphenyl)(2-amino-1,1'-biphenyl-2-yl)palladium, preferably tetrakis(triphenylphosphine)palladium(0) and [1,1'-bis(diphenylphosphino)ferrocene]palladium(II) dichloride;
the reaction is carried out in the presence of a suitable base, such as: sodium carbonate, cesium carbonate, potassium *tert*-butoxide, and sodium *tert*-butoxide, preferably sodium carbonate;
the reaction is carried out in a suitable solvent, such as 1,4-dioxane, 1,4-dioxane/water, 1,2-dichloroethane, 1,2-dichloroethane/water, *N,N*-dimethylformamide, *N,N*-dimethylformamide/*water,* dimethyl sulfoxide, dimethyl sulfoxide/water, N-methylpyrrolidone, and N-methylpyrrolidone/water, preferably 1,4-dioxane/water and *N*,*N*-dimethylformamide;
the reaction is carried out at a suitable temperature, such as 25 °C to 140 °C, preferably 40 °C to 110 °C.

Step 2: Compound **I-7-2** reacts with compound **1-7-3** to give compound **1-7-4.**

The reaction is carried out in the presence of a suitable catalyst, such as: tetrakis(triphenylphosphine)palladium(0), bis(triphenylphosphine)palladium(II) dichloride, [1,1'-bis(diphenylphosphino)ferrocene]palladium(II) dichloride, tris(dibenzylideneacetone)dipalladium(0), palladium dichloride, palladium(II) acetate, and chloro(2-dicyclohexylphosphino-2',6'-di-isopropoxy-1,1'-biphenyl)(2-amino-1,1'-biphenyl-2-yl)palladium, preferably [1,1'-bis(diphenylphosphino)ferrocene]palladium(II) dichloride;

the reaction is carried out in the presence of a suitable base, such as: sodium carbonate, cesium carbonate, potassium *tert*-butoxide, and sodium *tert*-butoxide, preferably sodium carbonate;
the reaction is carried out in a suitable solvent, such as 1,4-dioxane, 1,4-dioxane/water, 1,2-dichloroethane, 1,2-dichloroethane/water, *N,N-dimethylformamide, N,N-dimethylformamide/water,* dimethyl sulfoxide, dimethyl sulfoxide/water, *N*-methylpyrrolidone, and N-methylpyrrolidone/water, preferably 1,4-dioxane/water and *N*,*N*-dimethylformamide;
the reaction is carried out at a suitable temperature, such as 25 °C to 140 °C, preferably 80 °C to 110 °C.

Step 3: Compound **I-7-4** reacts with phosphine oxide **I-7-5** to give compound **I-7**.

The reaction is carried out in the presence of a suitable catalyst, such as: tetrakis(triphenylphosphine)palladium(0), bis(triphenylphosphine)palladium(II) dichloride, [1,1'-bis(diphenylphosphino)ferrocene]palladium(II) dichloride, tris(dibenzylideneacetone)dipalladium(0), palladium dichloride, palladium(II) acetate, and chloro(2-dicyclohexylphosphino-2',6'-di-isopropoxy-1,1'-biphenyl)(2-amino-1,1'-biphenyl-2-yl)palladium, preferably chloro(2-dicyclohexylphosphino-2',6'-di-isopropoxy-1,1'-biphenyl)(2-amino-1,1'-biphenyl-2-yl)palladium and tris(dibenzylideneacetone)dipalladium(0);

the reaction is carried out in the presence of a suitable base, such as: sodium carbonate, cesium carbonate, potassium *tert*-butoxide, and sodium *tert*-butoxide, preferably cesium carbonate;
the reaction is carried out in a suitable solvent, such as 1,4-dioxane, 1,4-dioxane/water, 1,2-dichloroethane, 1,2-dichloroethane/water, *N,N*-dimethylformamide*, N,N*-dimethylformamide/water*,* dimethyl sulfoxide, dimethyl sulfoxide/water, N-methylpyrrolidone, and *N*-methylpyrrolidone/water, preferably 1,4-dioxane and *N*,*N*-dimethylformamide;
the reaction is carried out at a suitable temperature, such as 25 °C to 140 °C, preferably 40 °C to 110 °C.

Example 1: (Compound 1)

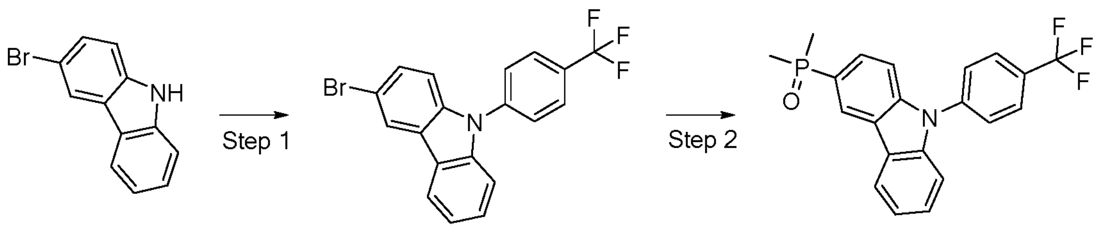


**1a** **1b** **1**

Step 1: (compound **1b**)

**1a** (1 g, 4.06 mmol), 4-iodobenzotrifluoride (1.33 g, 4.88 mmol), *N*,*N*-diisopropylethylamine (106 mg, 813 μmol), potassium hydroxide (456 mg, 8 mmol), and cuprous iodide (155 mg, 813 μmol) were dissolved in *N*,*N*-dimethylformamide (15 mL), and the mixture was purged three times with nitrogen and stirred for reaction at 110 °C for 12 h. After the reaction was completed as monitored by LCMS, the reaction solution was diluted with water (15 mL) and extracted with ethyl acetate (15 mL × 3). The organic phase was dried and then concentrated, and the resulting solid was slurried with acetonitrile (10 mL) to give **1b** as a gray-white solid (0.78 g, 1.93 mmol, yield: 48%). $^1$HNMR (400 MHz, DMSO-d6) δ 8.54 (d, *J* = 2.0 Hz, 1H), 8.33 (d, *J* = 7.6 Hz, 1H), 8.04 (d, *J* = 8.4 Hz, 2H), 7.89 (d, *J* = 8.4 Hz, 2H), 7.57 (dd, *J* = 8.8, 2.0 Hz, 1H), 7.46 - 7.51 (m, 2H), 7.43 (d, *J* = 8.8 Hz, 1H), 7.34 - 7.36 (m, 1H).

Step 2: (compound **1**)

Compound **1b** (0.1 g, 256 μmol), dimethylphosphine oxide (40 mg, 512 μmol), $Pd(dba)_2$ (15 mg, 26 μmol), 4,5-bis(diphenylphosphino)-9,9-dimethylxanthene (30 mg, 51 μmol), and cesium carbonate (84 mg, 256 μmol) were dissolved in 1,4-dioxane (3 mL), and the mixture was purged three times with nitrogen and stirred for reaction at 110 °C for 12 h. After the reaction was completed as monitored by LCMS, the reaction solution was diluted with water (5 mL) and extracted with ethyl acetate (5 mL × 3). The organic phase was dried and then concentrated. The resulting crude

product was purified by reversed-phase preparative chromatography to give **1** as a yellow gum (0.083 g, 214 μmol, yield: 83%). LCMS m/z[M+H]+: 388.3

$^1$HNMR (400 MHz, DMSO-*d6*) δ 8.71 (d, *J* = 11.6 Hz, 1H), 8.36 (d, *J* = 7.6 Hz, 1H), 8.06 (d, *J* = 8.4 Hz, 2H), 7.93 (d, *J* = 8.4 Hz, 2H), 7.81 - 7.83 (m, 1H), 7.57 (d, *J* = 8.4 Hz, 1H), 7.48 - 7.54 (m, 2H), 7.38 - 7.40 (m, 1H), 1.74 (d, *J* = 13.2 Hz, 6H).

Example 2: (Compound **2**)

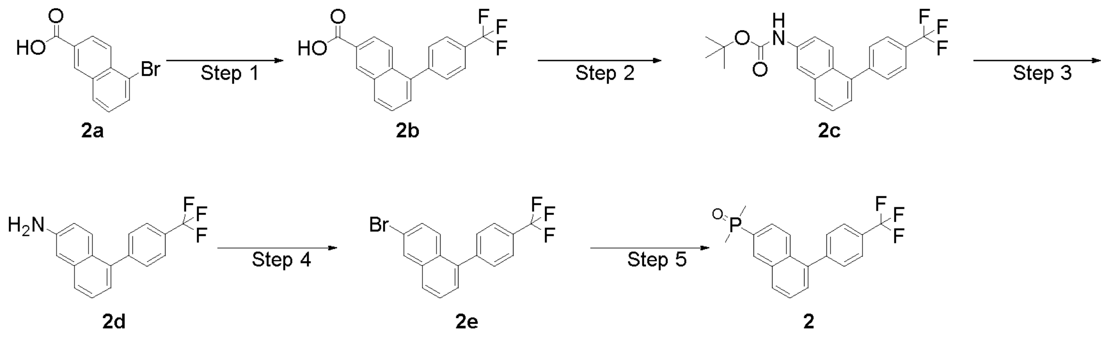


Step 1: (compound **2b**)

**2a** (5 g, 19.9 mmol), 4-(trifluoromethyl)phenylboronic acid (4.5 g, 23.9 mmol), and sodium carbonate (6.3 g, 59.7 mmol) were added to 1,4-dioxane (100 mL) and water (12 mL), and [1,1'-bis(diphenylphosphino)ferrocene]palladium(II) dichloride (0.6 g, 0.8 mmol) was added at room temperature. The mixture was purged three times with nitrogen and heated to 90 °C for reaction for 16 h. After the reaction was completed as shown by TLC, the reaction solution was cooled and filtered, and the filter cake was washed with ethyl acetate. A 1 M hydrochloric acid solution was added until the pH reached about 1, and ethyl acetate was added for washing. The organic phase was washed with brine, dried over anhydrous sodium sulfate, and concentrated. Then, the resulting crude product was separated by column chromatography to give a brown solid, and the solid was slurried with dichloromethane/methanol to give **2b** as a white solid (6 g, yield: 95%)

Step 2: (compound **2c**)

**2b** (6 g, 19 mmol) was added to *tert*-butanol (100 mL) at room temperature, and diphenylphosphoryl azide (6.3 g, 22.8 mmol) and triethylamine (5.8 g, 56.9 mmol) were added. The mixture was purged three times with nitrogen and heated to 90 °C for reaction for 16 h. After the reaction was completed as shown by LCMS, the reaction solution was cooled and washed with ethyl acetate and water. The organic phase was washed with brine, dried over anhydrous sodium sulfate, concentrated, and then separated and purified by column chromatography to give compound **2c** as a pale yellow solid (5.5 g, yield: 75%).

Step 3: (compound **2d**)

Trifluoroacetic acid (16.2 g, 142 mmol) was added dropwise to a stirred solution of compound **2c** (5.5 g, 14.2 mmol) in dichloromethane (20 mL), and the mixture was stirred at room temperature for 1 h. After the reaction was completed as shown by LCMS, the reaction solution was concentrated and then purified by column chromatography to give compound **2d** (5 g, yield: 88%). LCMS m/z[M+H]+: 288.2.

Step 4: (compound **2e**)

**2d** (300 mg, 1.04 mmol) and cupric bromide (260 mg, 1.14 mmol) were added to acetonitrile (10 mL), and the mixture was cooled to 0 °C in an ice-water bath. *tert*-Butyl nitrite (320 mg, 3.12 mmol) was added slowly, and the resulting mixture was reacted at 0 °C for 1 h. After the reaction was completed as shown by LCMS, the reaction solution was concentrated and then purified by column chromatography to give **2e** as a solid (280 mg, yield: 76.4%).

Step 2: (compound **2**)

**2e** (280 mg, 0.8 mmol), cesium carbonate (260 mg, 0.8 mmol), palladium(II) acetate (18 mg, 0.08 mmol), and 4,5-bis(diphenylphosphino)-9,9-dimethylxanthene (93 mg, 0.16 mmol) were added to 1,4-dioxane (10 mL), and dimethylphosphine oxide (120 mg, 1.6 mmol) was added. The mixture was purged three times with nitrogen and heated to 90 °C for reaction for 16 h. After the reaction was completed as shown by LCMS, the reaction solution was cooled, filtered, and washed with ethyl acetate. The filtrate and the washing solution were combined and concentrated, followed by purification by reversed-phase preparative chromatography to give **2** as a white solid (13 mg, yield: 5%). LCMS m/z$[M+H]^+$: 349.2.

$^1H$ NMR (400 MHz, DMSO-$d_6$) δ 8.49 (d, *J* = 12.8 Hz, 1H), 8.15 (d, *J* = 8.0 Hz, 1H), 7.91 (d,*J* = 8.0 Hz, 2H), 7.86 - 7.78 (m, 2H), 7.75 - 7.70 (m, 3H), 7.61 (dd, *J* = 7.2, 1.2 Hz, 1H), 1.75 (s, 3H), 1.71 (s, 3H).

Example 3: (Compound **3**)

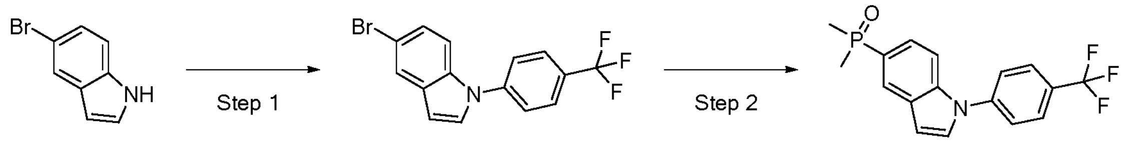


**3a** **3b** **3**

Step 1: (compound **3b**)

**3a** (1.11 g, 4.08 mmol), cuprous iodide (78 mg, 408 μmol), and potassium carbonate (564 mg, 4.08 mmol) were added to *N*,*N*-dimethylformamide (16 mL), and the mixture was heated to 140 °C and stirred for 12 h. After the reaction was completed as monitored by LCMS, the reaction solution was diluted with ethyl acetate (50 mL), and the organic phase was washed with water (20 mL × 2) and a saturated sodium chloride solution (20 mL × 2), dried over anhydrous sodium sulfate, filtered, and concentrated to give a crude product. The crude product was purified by column chromatography to give **3b** as a colorless oil (1.5 g, 3.86 mmol, yield: 94.6%). LCMS m/z$[M+H]^+$: 342.2

Step 2: (compound **3**)

Under a nitrogen atmosphere, **3b** (1.3 g, 3.82 mmol), dimethylphosphine oxide (597 mg, 7.64 mmol), $Pd(dba)_2$ (220 mg, 382 μmol), 4,5-bis(diphenylphosphino)-9,9-dimethylxanthene (442 mg, 764 μmol), and cesium carbonate (1.25 g, 3.82 mmol) were added to 1,4-dioxane (26 mL), and the mixture was heated to 80 °C and stirred for 1 h. After the reaction was completed as monitored by LCMS, the reaction solution was concentrated by rotary evaporation to give a crude product. The crude product was purified by column chromatography to give a product, and the product was subjected to reversed-phase preparative chromatography and lyophilized to give **3** as a yellow gum (28 mg, 83 μmol, yield: 6.96%). LCMS m/z$[M+H]^+$: 338.3

$^1H$NMR (400 MHz, $CDCl_3$) δ 8.16 (m, 1H), 7.84 (d, *J* = 8.4 Hz, 2H), 7.64 - 7.69 (m, 3H), 7.53 - 7.60 (m, 1H), 7.46 (d, *J* = 3.2 Hz, 1H), 6.84 (d, *J* = 3.2 Hz, 1H), 1.86 (s, 3H), 1.83 (s, 3H).

Example 4: (Compound **4**)

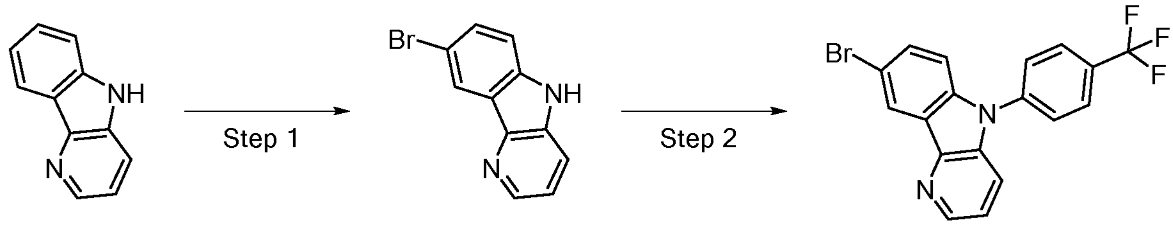


4a 4b 4c

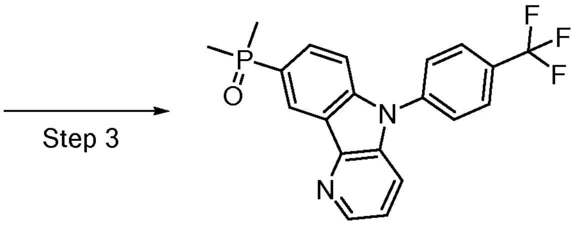


4

Step 1: (compound **4b**)

**4a** (0.9 g, 5.4 mmol), *N*-bromosuccinimide (0.95 g, 5.4 mmol), and dichloromethane (5 mL) were added to a reaction flask. The mixture was stirred at room temperature for 2 h. After the reaction was completed as shown by LCMS, the reaction solution was concentrated, and the crude product was purified by column chromatography to give **4b** as a white solid (1 g, yield: 75.6%). LCMS m/z$[M+H]^+$: 247.0

Step 2: (compound **4c**)

**4b** (500 mg, 2.0 mmol), 4-iodobenzotrifluoride (550 mg, 2.0 mmol), cuprous iodide (77 mg, 0.40 mmol), potassium hydroxide (230 mg, 4.40 mmol), *N*,*N*-diisopropylethylamine (52 mg, 0.40 mmol), and *N*,*N*-dimethylformamide (5 mL) were added to a reaction flask. The mixture was stirred at 110 °C for 5 h. After the reaction was completed as shown by LCMS, the reaction solution was concentrated, and the crude product was purified by column chromatography to give **4c** as a yellow solid (500 mg, yield: 63.2%). LCMS m/z$[M+H]^+$: 391.1

Step 3: (compound **4**)

**4c** (500 mg, 1.3 mmol), dimethylphosphine oxide (200 mg, 2.6 mmol), cesium carbonate (830 mg, 2.6 mmol), palladium(II) acetate (57 mg, 0.3 mmol), 4,5-bis(diphenylphosphino)-9,9-dimethylxanthene (300 mg, 0.5 mmol), and dioxane (5 mL) were added to a reaction flask. The mixture was stirred at 110 °C for 12 h. After the reaction was completed as shown by LCMS, the reaction solution was concentrated, and the crude product was purified by reversed-phase column chromatography to give **4** as a white solid (75 mg, yield: 15.1%). LCMS m/z$[M+H]^+$: 389.3

$^1$HNMR (400 MHz, DMSO-$d_6$) $\delta$ 8.73 (dd, *J* = 11.6, 1.2 Hz, 1H), 8.66 (dd, *J* = 4.8, 1.2 Hz, 1H), 8.08 (d, *J* = 8.4 Hz, 2H), 8.00 - 7.93 (m, 4H), 7.72 - 7.67 (m, 1H), 7.53 (dd, *J* = 8.4, 4.8 Hz, 1H), 1.77 (s, 3H), 1.74 (s, 3H).

Example 5: (Compound **5**)

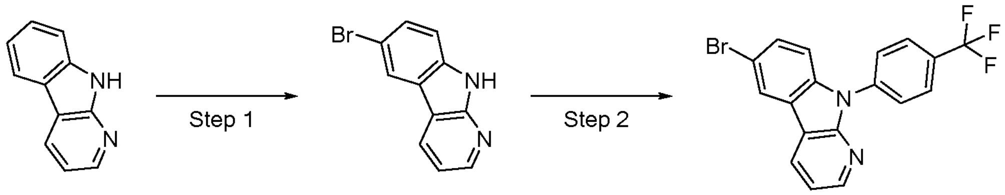


**5a** **5b** **5 c**

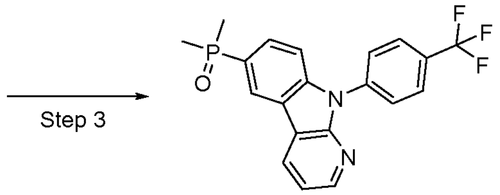


**5**

Step 1: (compound **5b**)

**5a** (3 g, 17.84 mmol) and *N*-bromosuccinimide (2.54 g, 14.27 mmol) were added to dichloromethane (30 mL). The mixture was stirred at room temperature for 2 h. After the reaction was completed as shown by LCMS, the reaction solution was concentrated, and the crude product was purified by column chromatography to give **2** as a white solid (2.4 g, yield: 54.45%). LCMS m/z$[M+H]^+$: 247.0.

Step 2: (compound **5c**)

**5b** (500 mg, 2.0 mmol), 4-iodobenzotrifluoride (660 mg, 2.4 mmol), cuprous iodide (77 mg, 0.4 mmol), potassium hydroxide (230 mg, 4.4 mmol), and *N,N*-diisopropylethylamine (52 mg, 0.4 mmol) were added to *N,N*-dimethylformamide (5 mL). The mixture was stirred at 110 °C for 5 h. After the reaction was completed as shown by LCMS, the reaction solution was concentrated, and the crude product was purified by column chromatography to give **3** as a yellow solid (500 mg, yield: 63.17%). LCMS m/z$[M+H]^+$: 391.1.

Step 3: (compound **5**)

**5c** (200 mg, 0.5 mmol), dimethylphosphine oxide (80 mg, 1.0 mmol), cesium carbonate (330 mg, 1.0 mmol), palladium(II) acetate (23 mg, 0.1 mmol), and 4,5-bis(diphenylphosphino)-9,9-dimethylxanthene (120 mg, 0.2 mmol) were added to dioxane (2 mL). The mixture was stirred at 110 °C for 12 h. After the reaction was completed as shown by LCMS, the reaction solution was concentrated, and the crude product was purified by reversed-phase column chromatography to give **5** as a white solid (110 mg, yield: 55.10%). LCMS m/z$[M+H]^+$: 389.3.

$^1H$ NMR (400 MHz, DMSO-d6) δ 8.82 (t, *J* = 1.6 Hz, 1H), 8.79 (t, *J* = 1.6 Hz, 1H), 8.53 (dd, *J* = 4.8, 1.6 Hz, 1H), 8.07 (d, *J* = 8.4 Hz, 2H), 8.02 (d, *J* = 8.4 Hz, 2H), 7.94 (ddd, *J* = 10.4, 8.4, 1.6 Hz, 1H), 7.72 (dd, *J* =8.4, 1.6 Hz, 1H), 7.49 (dd, *J* = 7.6, 4.8 Hz, 1H), 1.79 (s, 3H), 1.76 (s, 3H).

Example 6: (Compound **6**)

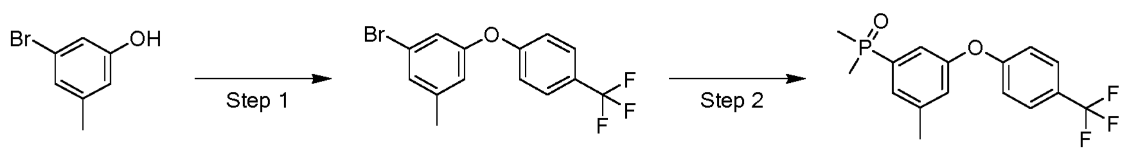


**6a** **6b** **6**

Step 1: (compound **6b**)

**6a** (202 mg, 1.1 mmol), 4-iodobenzotrifluoride (345 mg,1.3 mmol), potassium phosphate (450 mg, 2.1 mmol), 2-picolinic acid (12 mg, 101.5 μmol), and cuprous iodide (12 mg, 63.4 μmol) were added to DMSO (5 mL), and the mixture was purged with nitrogen and reacted at 120 °C for 12 h. After the reaction was completed as shown by LCMS, 50 mL of water was added to the reaction solution, and the mixture was extracted with ethyl acetate (50 mL × 2), washed with saturated brine (20 mL), dried over anhydrous sodium sulfate, concentrated, and purified by prep-TLC to give **6b** as a colorless liquid (199 mg, yield: 47%).

Step 2: (compound **6**)

**6b** (50 mg, 151 μmol), dimethylphosphine oxide (12 mg, 151 μmol), $Pd_2(dba)_3$ (14 mg, 15 μmol), 4,5-bis(diphenylphosphino)-9,9-dimethylxanthene (17 mg, 30 μmol), and cesium carbonate (148 mg, 453 μmol) were added to 1,4-dioxane (5 mL), and the mixture was purged with nitrogen and reacted at 50 °C for 12 h. After the reaction was completed as shown by LCMS, water (20 mL) was added to the reaction solution, and the mixture was extracted with ethyl acetate (20 mL × 2), washed with saturated brine (10 mL), dried over anhydrous sodium sulfate, concentrated, and purified by prep-TLC to give **6** as a colorless oily liquid (5 mg, yield: 10%). LCMS m/z$[M+H]^+$: 329.1

$^1$HNMR(400 MHz, DMSO-d6) δ 7.75 (d, *J* =8.4 Hz, 2H), 7.47 - 7.44 (d, *J* =11.2 Hz, 1H), 7.31 - 7.28 (d, *J* =12.0 Hz, 1H), 7.14 - 7.18 (m, 3H), 2.37 (s, 3H), 1.65 (s, 3H), 1.62 (s, 3H).

Example 7: (Compound 7)

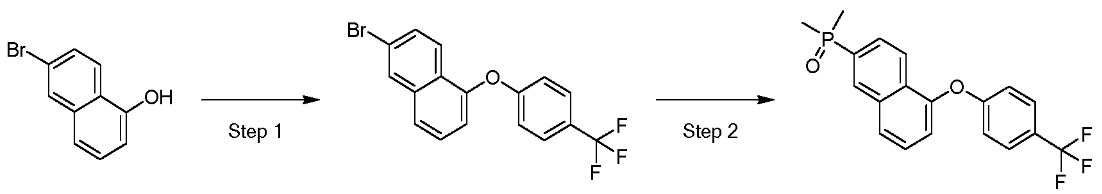


**7a** **7b** **7**

Step 1: (compound **7b**)

**7a** (0.35 g, 1.6 mmol), 4-iodobenzotrifluoride (853 mg, 3.1 mmol), potassium phosphate (556 mg, 2.6 mmol), cuprous iodide (15 mg, 78.5 μmol), and 2-picolinic acid (15 mg, 125.5 μmol) were added to dimethyl sulfoxide (18 mL). The mixture was heated to 120 °C and stirred for 12 h. After the reaction was completed as detected by LCMS, the reaction solution was poured into water (60 mL) and ethyl acetate (60 mL), and the organic phase was washed with saturated brine (60 mL × 2), dried over anhydrous sodium sulfate, filtered, concentrated, and then purified by column chromatography to give **7b** as a colorless oil (1.5 g, yield: 94.6%). $^1$HNMR (400 MHz, $CDCl_3$) δ 7.72-8.32 (m, 3H), 7.56-7.65 (m, 3H), 7.46-7.50 (m, 1H), 7.36 (d, *J* = 8.0 Hz, 1H), 7.05-7.08 (m, 2H).

Step 2: (compound 7)

**7b** (500 mg, 1.4 mmol), dimethylphosphine oxide (159 mg, 2.04 mmol), $Pd_2(dba)_3$ (125 mg, 136 μmol), 4,5-bis(diphenylphosphino)-9,9-dimethylxanthene (181 mg, 313 μmol), and cesium carbonate (1.33 g, 4.1 mmol) were added to dioxane (10 mL), and the mixture was heated to 50 °C and stirred for 1 h. After the reaction was completed as detected by LCMS, the reaction solution was concentrated and subjected to reversed-phase preparative chromatography to give **7** as a yellow solid (76 mg, yield: 15.0%). LCMS m/z$[M+H]^+$: 365.1

$^1$HNMR (400 MHz, $CDCl_3$) δ 8.50 (d, *J* = 13.2 Hz, 1H), 8.04 (dd, *J* = 2.4, 8.8 Hz, 1H), 8.00 (d, *J* = 8.0 Hz, 1H), 7.86 (td, *J* = 1.2, 9.6 Hz, 1H), 7.74 (d, *J* = 8.8 Hz, 2H), 7.66 (t, *J* = 8.8 Hz, 1H), 7.37 (t, *J* = 7.6 Hz, 1H), 7.16 (d, *J* = 7.6 Hz, 2H), 1.75 (s, 3H), 1.71 (s, 3H).

Example 8: (Compound **8**)

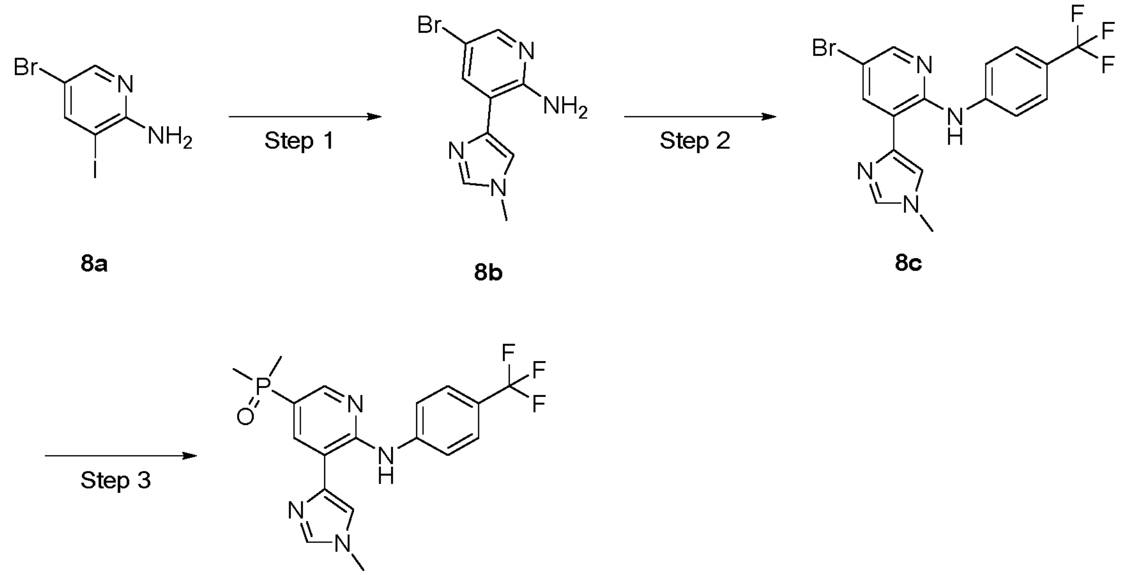


8a 8b 8c

8

Step 1: (compound **8b**)

Compound **8a** (330 mg, 1.1 mmol), *N*-methyl-4-(tributylstannyl)imidazole (408 mg, 1.1 mmol), and tetrakis(triphenylphosphine)palladium(0) (127 mg, 0.1 mmol) were added to 1,4-dioxane (10 mL), and the mixture was purged three times with nitrogen, heated to 60 °C, and stirred for 14 h. After the reaction was completed as shown by LCMS, the reaction solution was cooled to room temperature, filtered through celite, and subjected to column chromatography to give **8b** as a brilliant blue solid (263.0 mg, yield: 94.1%). LCMS m/z[M+H]$^+$: 253.1.

(1) $^1$HNMR(400 MHz, DMSO-d6) δ 7.88 (q, *J* = 2.4 Hz, 2H), 7.79 - 7.73 (m, 2H), 7.19 (s, 2H), 3.71 (s, 3H).

Step 2: (compound **8c**)

**8b** (260 mg, 1.0 mmol) was dissolved in 1,2-dichloroethane (5 mL), and 4-(trifluoromethyl)phenylboronic acid (300 mg, 1.6 mmol) and cupric acetate (920 mg, 0.1 mmol) were added. After the addition, the reaction solution was stirred for 4 days under an air atmosphere. After most of the product was generated as shown by LCMS, the reaction solution was filtered through celite, rinsed with dichloromethane, concentrated, and subjected to column chromatography to give **8c** as a brown solid (80 mg, yield: 19.6%). LCMS m/z[M+H]$^+$: 397.2.

$^1$HNMR (400 MHz, DMSO-*d6*) δ 12.06 (s, 1H), 8.24 - 8.19 (m, 2H), 8.03 (d, *J* = 1.2 Hz, 1H), 7.96 (d, *J* = 1.2 Hz, 1H), 7.92 (d, *J* = 8.4 Hz, 2H), 7.64 (d, *J* = 8.4 Hz, 2H), 3.77 (s, 3H).

Step 3: (compound **8**)

Compound **8c** (70 mg, 0.2 mmol), dimethylphosphine oxide (29 mg, 0.4 mmol), and cesium carbonate (65 mg, 0.2 mmol) were dissolved in 1,4-dioxane (7 mL), and the mixture was purged three times with nitrogen. Palladium(II) acetate (9 mg, 0.04 mmol) and 4,5-bis(diphenylphosphino)-9,9-dimethylxanthene (46 mg, 0.08 mmol) were added under a nitrogen atmosphere, and after the addition, the resulting mixture was heated to 110 °C and stirred for 12 h. After the starting materials were consumed completely as shown by LCMS, the reaction solution was poured into water and extracted with ethyl acetate. The organic phase was washed once with brine, dried over anhydrous sodium sulfate, concentrated, and subjected to column chromatography to give **8** as a brown viscous solid (20 mg, yield: 28.8%). LCMS m/z[M+H]$^+$: 395.2.

$^1$HNMR (400 MHz, DMSO-d6) δ 12.27 (s, 1H), 8.43 (dd, *J* = 6.0, 2.0 Hz, 1H), 8.25 (dd, *J* = 11.2, 2.0 Hz, 1H), 8.06 - 7.94 (m, 4H), 7.67 (d, *J* = 8.8 Hz, 2H), 3.79 (s, 3H), 1.72 (s, 3H), 1.69 (s, 3H).

Example 9: (Compound **9**)

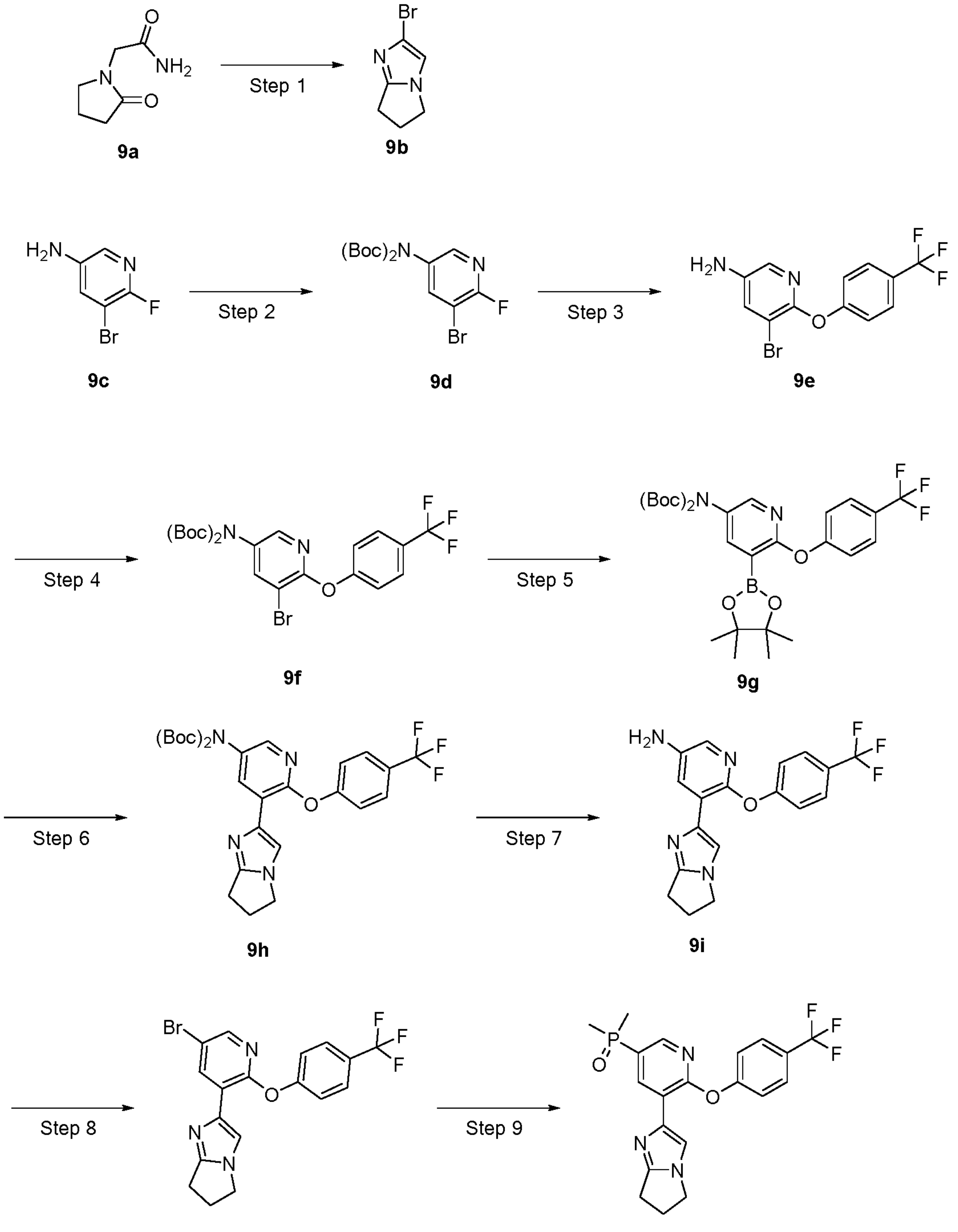


9a 9b 9c 9d 9e 9f 9g 9h 9i 9j 9

Step 1: (compound **9b**)

Compound **9a** (4 g, 28 mmol) was added to phosphoryl bromide (20 mL), and the mixture was purged three times with nitrogen, heated to 70 °C, and stirred for 14 h. After the reaction was completed as shown by LCMS, the reaction solution was poured into ice water, and an aqueous sodium bicarbonate solution and an aqueous potassium carbonate solution were added to adjust the pH to 9-10. Extraction was performed with dichloromethane, and the organic phase was washed with brine, dried over anhydrous sodium sulfate, and concentrated to give **9b** as a brown solid (4.8 g, yield: 91%). LCMS m/z[M+H]$^+$: 186.9.

$^1$HNMR (400 MHz, $CDCl_3$) δ 6.85 (d, *J* = 1.2 Hz, 1H), 4.04 - 3.92 (m, 2H), 2.86 (t, *J* = 7.6 Hz, 2H), 2.56 (q, *J* = 7.6 Hz, 2H).

Step 2: (compound **9d**)

**9c** (2.0 g, 10 mmol) was added to tetrahydrofuran (15 mL), and DMAP (3.84 g, 31 mmol) and di-*tert*-butyl dicarbonate (5.71 g, 26 mmol) were added under a nitrogen atmosphere. After the addition, the mixture was heated to 45 °C and stirred for 2 h. After no starting material remained as shown by LCMS, the reaction solution was poured into an aqueous citric acid solution and extracted with ethyl acetate. The organic phase was washed with brine, dried over anhydrous sodium sulfate, concentrated, purified by column chromatography, then slurried with petroleum ether, filtered, and dried to give **9d** as a white solid (2.3 g, yield: 75%). LCMS m/z$[M+H]^+$: 291.0.

Step 3: (compound 9e)

Compound **9d** (2.6 g, 7 mmol) was added to DMSO (20 mL), and the mixture was purged three times with nitrogen. 4-(Trifluoromethyl)phenol (1.3 g, 8 mmol) and cesium carbonate (4.34 g, 13 mmol) were added, and after the addition, the resulting mixture was heated to 110 °C and stirred for 1 h. After the reaction was completed as shown by LCMS, the reaction solution was cooled to room temperature and diluted with ethyl acetate, and a small amount of water was added, followed by phase separation and extraction with ethyl acetate. The organic phase was washed once with brine, dried over anhydrous sodium sulfate, and concentrated. The crude product was purified by column chromatography to give **9e** as a yellow solid (1.8 g, yield: 81.31%). LCMS m/z$[M+H]^+$: 333.1.

$^1$HNMR (400 MHz, DMSO-d6) δ 7.70 (d, *J* = 8.8 Hz, 2H), 7.60 (d, *J* = 2.4 Hz, 1H), 7.40 (d, *J* = 2.4 Hz, 1H), 7.10 (d, *J* = 8.8 Hz, 2H), 5.52 (s, 2H).

Step 4: (compound **9f**)

Compound **9e** (1.8 g, 5 mmol) was added to tetrahydrofuran (20 mL), and DMAP (2.0 g, 16 mmol) and di-*tert*-butyl dicarbonate (3.6 g, 16 mmol) were added under a nitrogen atmosphere. After the addition, the mixture was stirred at room temperature for 2 h. After the reaction was completed as shown by LCMS, the reaction solution was poured into an aqueous citric acid solution and extracted with ethyl acetate. The organic phase was washed with brine, dried over anhydrous sodium sulfate, and concentrated, and the crude product was slurried with petroleum ether, filtered, and dried to give **9f** as a white solid (1.2 g, yield: 41%). $^1$HNMR (400 MHz, DMSO-d6) δ 8.34 (d, *J* = 2.4 Hz, 1H), 8.08 (d, *J* = 2.4 Hz, 1H), 7.82 (d, *J* = 8.4 Hz, 2H), 7.38 (d, *J* = 8.4 Hz, 2H), 1.40 (s, 18H).

Step 5: (compound **9g**)

**9f** (1.4 g, 3 mmol), bis(pinacolato)diboron (1.38 g, 5 mmol), and potassium acetate (645 mg, 7 mmol) were added to 1,4-dioxane (28 mL), and the mixture was purged three times with nitrogen. Pd(dppf)$Cl_2$ (190 mg, 0.3 mmol) was added under a nitrogen atmosphere, and after the addition, the resulting mixture was heated to 100 °C and stirred for 2 h. After the reaction was completed as shown by LCMS, the reaction solution was cooled to room temperature, diluted with ethyl acetate, filtered through celite, and rinsed twice with ethyl acetate, and the filtrate was concentrated. The crude product was purified by column chromatography to give **9g** as a yellow solid (1.2 g, yield: 92%). LCMS m/z$[M+H]^+$: 499.3.

$^1$HNMR (400 MHz, DMSO-d6) δ 8.17 (d, *J* = 2.8 Hz, 1H), 7.89 (d, *J* = 2.8 Hz, 1H), 7.77 (d,*J* = 8.4 Hz, 2H), 7.27 (d, *J* = 8.4 Hz, 2H), 1.41 (s, 18H), 1.27 (s, 12H).

Step 6: (compound **9h**)

**9g** (500 mg, 1 mmol), **9b** (225 mg, 1 mmol), and potassium carbonate (280 mg, 2 mmol) were added to 1,4-dioxane (10 mL) and water (1 mL), and the mixture was purged three times with nitrogen. Pd(dppf)$Cl_2$ (74 mg, 0.1 mmol) was added under a nitrogen atmosphere, and after the addition, the resulting mixture was heated to 80 °C and stirred for 3 h. After the reaction was completed as shown by LCMS, the reaction solution was cooled to room temperature and filtered through celite, and the filtrate was concentrated to give a crude product. The crude product was purified by column chromatography to give **9h** as an off-white solid (400 mg, yield: 71%). LCMS m/z$[M+H]^+$: 561.3.

(1) $^1$HNMR (400 MHz, DMSO-d6) δ 8.22 (d, *J* = 2.8 Hz, 1H), 7.86 (d, *J* = 2.8 Hz, 1H), 7.81 (d, *J* = 8.4 Hz, 2H), 7.67 (s, 1H), 7.37 (d, *J* = 8.4 Hz, 2H), 4.00 (t, *J* = 7.2 Hz, 2H), 2.79 (t, *J* = 7.6 Hz, 2H), 2.53 (d, *J* = 7.2 Hz, 2H), 1.41 (s, 18H).

Step 7: (compound **9i**)

**9h** (350 mg, 0.6 mmol) was added to a solution of hydrogen chloride in dioxane (4 M, 15 mL), and after the addition, the mixture was stirred at room temperature for 2 h. After the reaction was completed as shown by LCMS, the

reaction solution was concentrated, poured into an aqueous sodium bicarbonate solution, and extracted with ethyl acetate. The organic phase was washed with brine, dried over anhydrous sodium sulfate, filtered, and concentrated to give **9i** as an off-white solid (225 mg, yield: 100%). LCMS m/z[M+H]$^+$: 361.2.

$^1$H NMR (400 MHz, DMSO-d6) δ 7.80 (d, *J* = 2.8 Hz, 1H), 7.68 (d, *J* = 8.4 Hz, 2H), 7.42 (d,*J* = 2.8 Hz, 1H), 7.36 (s, 1H), 7.11 (d, *J* = 8.4 Hz, 2H), 5.23 (s, 2H), 3.92 (t, *J* = 7.2 Hz, 2H), 2.74 (t, *J* = 7.6 Hz, 2H), 2.47 (d, *J* = 7.2 Hz, 2H).

Step 8: (compound **9j**)

**9i** (350 mg, 0.6 mmol) and cuprous bromide (320 mg, 2 mmol) were dissolved in a solution of hydrobromic acid (560 mg, 7 mmol) in water (3 mL), and at 0 °C to 5 °C, a solution of sodium nitrite (80 mg, 1 mmol) in water (1 mL) was added dropwise. After the dropwise addition, the mixture was slowly warmed to room temperature and stirred for 5 h. After the product was generated as shown by LCMS, the reaction solution was poured into an aqueous sodium bisulfite solution, and the pH was adjusted to 10 with an aqueous sodium hydroxide solution. Extraction was performed with ethyl acetate, and the organic phase was washed with saturated brine, dried over anhydrous sodium sulfate, concentrated, and purified by column chromatography to give **9j** as a white solid (100 mg, yield: 42%). LCMS m/z[M+H]$^+$: 424.1.

$^1$HNMR (400 MHz, DMSO-d6) δ 8.54 (d, *J* = 2.4 Hz, 1H), 8.08 (d, *J* = 2.4 Hz, 1H), 7.80 (d, *J* = 8.4 Hz, 2H), 7.69 (s, 1H), 7.40 (d, *J* = 8.4 Hz, 2H), 4.00 (t, *J* = 7.2 Hz, 2H), 2.80 (t, *J* = 7.6 Hz, 2H), 2.54 (t, *J* = 7.2 Hz, 2H).

Step 9: (compound **9**)

**9j** (80 mg, 0.2 mmol), dimethylphosphine oxide (30 mg, 2 mmol), and potassium phosphate (80 mg, 0.4 mmol) were dissolved in 1,4-dioxane (2 mL), and the mixture was purged three times with nitrogen. Palladium(II) acetate (10 mg, 0.05 mmol) and 4,5-bis(diphenylphosphino)-9,9-dimethylxanthene (51 mg, 0.09 mmol) were added under a nitrogen atmosphere, and after the addition, the resulting mixture was heated to 110 °C and stirred for 1 h. After the starting materials were consumed completely as shown by LCMS, the reaction solution was poured into water and extracted with ethyl acetate. The organic phase was washed once with brine, dried over anhydrous sodium sulfate, and concentrated, and the crude product was subjected to reversed-phase preparative chromatography to give **9** as an off-white solid (25 mg, yield: 31%). LCMS m/z[M+H]$^+$: 422.2.

$^1$HNMR (400 MHz, DMSO-d6) δ 8.77 (dd, *J* = 10.8, 2.4 Hz, 1H), 8.26 (dd, *J* = 6.0, 2.4 Hz, 1H), 7.82 (d, *J* = 8.4 Hz, 2H), 7.71 (s, 1H), 7.43 (d, *J* = 8.4 Hz, 2H), 4.01 (t, *J* = 7.2 Hz, 2H), 2.81 (t, *J* = 7.6 Hz, 2H), 2.54 (q, *J* = 7.2 Hz, 2H), 1.73 (s, 3H), 1.70 (s, 3H).

Example 10: (Compound **10**)

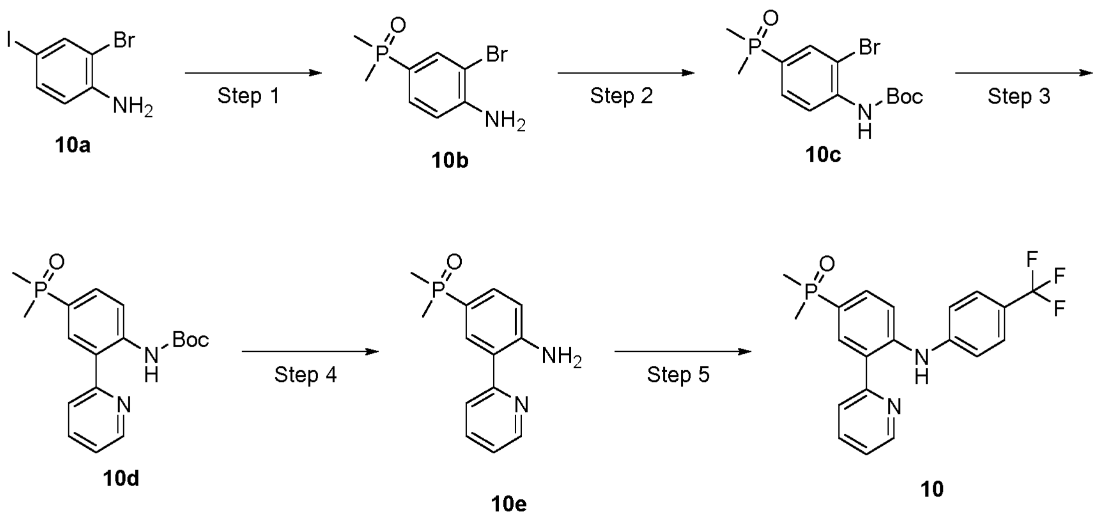


10a 10b 10c 10d 10e 10

Step 1: (compound **10b**)

**10a** (2.0 g, 7 mmol) was dissolved in 1,4-dioxane (20 mL), and dimethylphosphine oxide (0.58 g, 7 mmol), potassium phosphate (1.56 g, 7 mmol), 4,5-bis(diphenylphosphino)-9,9-dimethylxanthene (0.23 g, 0.4 mmol), and

tris(dibenzylideneacetone)dipalladium(0) (0.18 g, 0.2 mmol) were added. The mixture was purged three times with nitrogen, heated to 100 °C, and stirred for 3 h. After the reaction was finished as monitored by LCMS, the reaction solution was concentrated by rotary evaporation to give a crude product, and the crude product was separated and purified by column chromatography to give **10b** as a brown solid (1.5 g, yield: 89%). LCMS m/z[M+H]$^+$: 248.0.

Step 2: (compound **10c**)

**10b** (500 mg, 2.0 mmol) was dissolved in tetrahydrofuran (10 mL), and sodium hydride (242 mg, 6 mmol, 60 w%) was added. The mixture was heated to 65 °C and stirred for 1 h. Di-*tert*-butyl dicarbonate (660 mg, 3 mmol) was added, and the resulting mixture was stirred at 65 °C for 16 h. After the reaction was finished as monitored by LCMS, ice water (20 mL) was added to the reaction solution, and the mixture was extracted three times with ethyl acetate, washed with saturated brine, dried over anhydrous sodium sulfate, and concentrated under reduced pressure. The crude product was purified by column chromatography to give **10c** as a yellow solid (420 mg, yield: 59%). LCMS m/z[M+H]$^+$: 348.0.

Step 3: (compound **10d**)

**10c** (400 mg, 1 mmol) was dissolved in dioxane (10 mL), and 2-(tributylstannyl)pyridine (590 mg, 2 mmol), anhydrous lithium chloride (97 mg, 2 mmol), and tetrakis(triphenylphosphine)palladium(0) (132 mg, 0.1 mmol) were added. The mixture was purged three times with nitrogen and then heated to 100 °C for reaction for 16 h. After the reaction was finished as monitored by LCMS, the reaction solution was concentrated. The crude product was separated and purified by column chromatography to give **10d** as a white solid (200 mg, yield: 50%). LCMS m/z[M+H]$^+$: 347.0

Step 4: (compound **10e**)

**10d** (150 mg, 0.4 mmol) was dissolved in dichloromethane (3 mL), and then trifluoroacetic acid (1 mL) was added. The mixture was stirred at room temperature for 2 h. After the reaction was finished as monitored by LCMS, the reaction solution was concentrated and diluted with ethyl acetate, and the organic phase was washed three times with an aqueous sodium bicarbonate solution, washed with an aqueous sodium chloride solution, dried over anhydrous sodium sulfate, and concentrated under reduced pressure. The crude product was separated and purified by column chromatography to give **10e** as a yellow solid (120 mg, yield: 90%). LCMS m/z[M+H]$^+$: 247.1.

Step 5: (compound **10**)

**10e** (30 mg, 0.1 mmol) was dissolved in acetonitrile (2 mL), and 4-(trifluoromethyl)phenylboronic acid (35 mg, 0.2 mmol), 2,6-lutidine (20 mg, 0.2 mmol), cupric acetate (22 mg, 0.1 mmol), and triethylamine (19 mg, 0.2 mmol) were added. The mixture was purged three times with oxygen and stirred for reaction at room temperature for 3 h. After the reaction was finished as monitored by LCMS, the reaction solution was concentrated, and the crude product was separated and purified by column chromatography and then subjected to reversed-phase preparative chromatography to give **10** as a white solid (21 mg, yield: 44.4%). LCMS m/z[M+H]$^+$: 391.1.

$^1$HNMR (400 MHz, DMSOd6) δ 10.53 (s, 1H), 8.73 (d, *J* = 4.0 Hz, 1H), 8.07 (dd, *J* = 11.6, 1.6 Hz, 1H), 8.00 - 7.88 (m, 2H), 7.79 - 7.68 (m, 1H), 7.63 - 7.53 (m, 3H), 7.45 - 7.39 (m, 1H), 7.27 (d, *J* = 8.4 Hz, 2H), 1.69 (m, 6H).

Example 11: (Compound **11**)

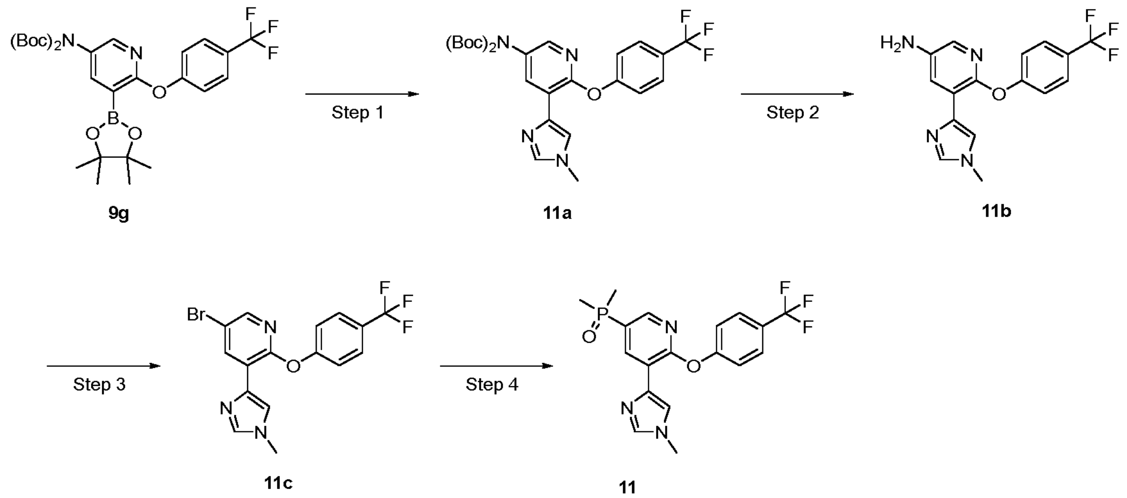


9g 11a 11b 11c 11

Step 1: (compound **11a**)

**9g** (550 mg, 1 mmol), 4-bromo-1-methyl-1H-imidazole (200 mg, 1 mmol), and potassium carbonate (265 mg, 2 mmol) were added to 1,4-dioxane (20 mL) and water (2 mL), and the mixture was purged three times with nitrogen. $Pd(dppf)Cl_2$ (76 mg, 0.1 mmol) was added under a nitrogen atmosphere, and after the addition, the resulting mixture was heated to 80 °C and stirred for 3 h. After the reaction was completed as shown by LCMS, the reaction solution was cooled to room temperature and filtered through celite, and the filtrate was concentrated and purified by column chromatography to give **11a** as a brown solid (300 mg, yield: 59%). LCMS m/z$[M+H]^+$: 535.2.

$^1$HNMR (400 MHz, DMSO-*d*6) δ 8.25 (d, *J* = 2.8 Hz, 1H), 7.87 (d, *J* = 2.8 Hz, 1H), 7.82 (d, *J* = 8.4 Hz, 2H), 7.77 (d, *J*= 1.2 Hz, 1H), 7.73 (d, *J* = 1.2 Hz, 1H), 7.40 (d, *J* = 8.4 Hz, 2H), 3.71 (s, 3H), 1.41 (s, 18H).

Step 2: (compound **11b**)

**11a** (270 mg, 0.51 mmol) was added to a solution of hydrogen chloride in dioxane (4 M, 5 mL), and after the addition, the mixture was stirred at room temperature for 2 h. After the reaction was completed as shown by LCMS, the reaction solution was concentrated, poured into an aqueous sodium bicarbonate solution (pH = 9), and extracted with ethyl acetate. The organic phase was washed with brine, dried over anhydrous sodium sulfate, filtered, and concentrated to give **11b** as a brown solid (200 mg, yield: 100%). LCMS m/z$[M+H]^+$: 335.1.

$^1$HNMR (400 MHz, DMSO-d6) δ 7.82 (d, *J* = 2.8 Hz, 1H), 7.72 - 7.65 (m, 3H), 7.43 (dd, *J* = 4.4, 2.0 Hz, 2H), 7.19 - 7.12 (m, 2H), 5.25 (s, 2H), 3.64 (s, 3H).

Step 3: (compound **11c**)

Compound **11b** (180 mg, 0.5 mmol) and cuprous bromide (306 mg, 2 mmol) were dissolved in a solution of hydrobromic acid (530 mg, 7 mmol) in water (4 mL), and at 0 °C to 5 °C, a solution of sodium nitrite (72 mg, 1 mmol) in water (1 mL) was added dropwise. After the dropwise addition, the mixture was slowly warmed to room temperature and stirred for 5 h. After the product was generated as shown by LCMS, the reaction solution was poured into an aqueous sodium bisulfite solution, and the pH was adjusted to 10 with an aqueous sodium hydroxide solution. Extraction was performed with ethyl acetate, and the organic phase was washed once with brine, dried over anhydrous sodium sulfate, concentrated, and purified by column chromatography to give **11c** as a yellow solid (40 mg, yield: 18%). LCMS m/z$[M+H]^+$: 398.0.

$^1$HNMR (400 MHz, DMSO-d6) δ 8.57 (d, *J* = 2.4 Hz, 1H), 8.10 (d, *J* = 2.4 Hz, 1H), 7.84 - 7.78 (m, 3H), 7.75 (d, *J* = 1.2 Hz, 1H), 7.43 (d, *J* = 8.4 Hz, 2H), 3.71 (s, 3H).

Step 4: (compound **11**)

**11c** (30 mg, 0.08 mmol), dimethylphosphine oxide (12 mg, 0.2 mmol), and cesium carbonate (29 mg, 0.09 mmol) were dissolved in 1,4-dioxane (3 mL), and the mixture was purged three times with nitrogen. Palladium(II) acetate (12 mg, 0.05 mmol) and 4,5-bis(diphenylphosphino)-9,9-dimethylxanthene (65 mg, 0.1 mmol) were added under a nitrogen atmosphere, and after the addition, the resulting mixture was heated to 110 °C and stirred for 4 h. After the starting materials were consumed completely as shown by LCMS, the reaction solution was poured into water and extracted with ethyl acetate. The organic phase was washed once with brine, dried over anhydrous sodium sulfate, and concentrated,

and the crude product was subjected to reversed-phase preparative chromatography to give **11** as a white solid (3 mg, yield: 10%). LCMS m/z[M+H]$^+$: 396.2.

$^1$HNMR (400 MHz, DMSO-$d_6$) δ 8.80 (dd, *J* = 10.8, 2.4 Hz, 1H), 8.28 (dd, *J* = 5.6, 2.4 Hz, 1H), 7.83 (d, *J* = 8.4 Hz, 2H), 7.79 (d, *J* = 1.2 Hz, 1H), 7.76 (d, *J* = 1.2 Hz, 1H), 7.46 (d, *J* = 8.4 Hz, 2H), 3.72 (s, 3H), 1.73 (s, 3H), 1.70 (s, 3H).

Example 12: (Compound **12**)

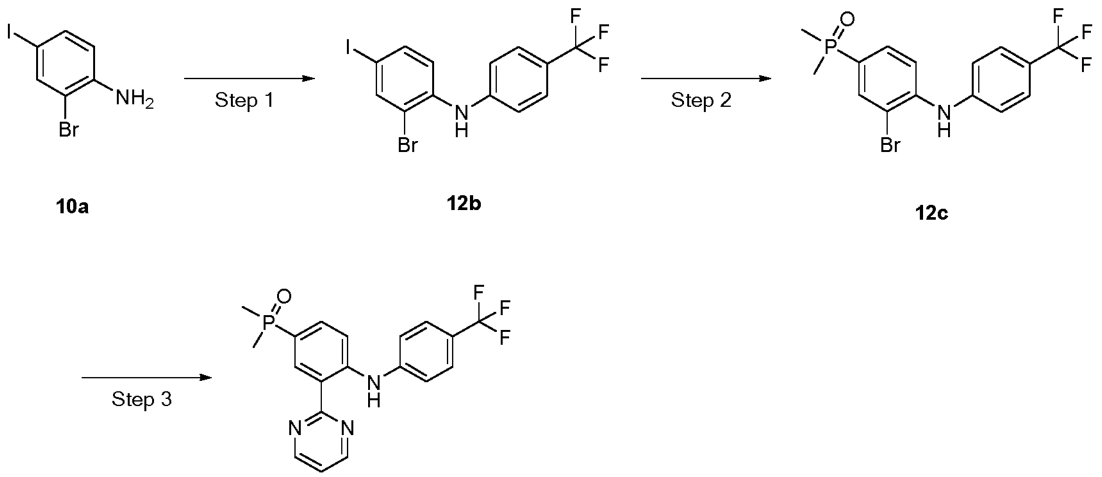


**10a** **12b** **12c**

**12**

Step 1: (compound **12b**)

**10a** (5.7 g, 19 mmol) and 4-(trifluoromethyl)phenylboronic acid (7.8 g, 38 mmol) were dissolved in dichloromethane (114 mL), and then triethylamine (3.9 g, 38 mmol) and $Cu(OAc)_2$ (5.2 g, 29 mmol) were added. The mixture was reacted at room temperature for 2 h under an oxygen atmosphere. After the product was generated as shown by LCMS, the reaction solution was concentrated and purified by column chromatography to give **12b** as an orange oily liquid (2.31 g, yield: 24.5%). LCMS m/z[M+H]$^+$: 443.9

Step 2: (compound **12c**)

**12b** (2.3 g, 5 mmol), dimethylphosphine oxide (366 mg, 5 mmol), $Pd_2(dba)_3$ (429 mg, 469 μmol), 4,5-bis(diphenylphosphino)-9,9-dimethylxanthene (543 mg, 938 μmol), and $Cs_2CO_3$ (4.6 g, 14 mmol) were added to 1,4-dioxane (231 mL), and the mixture was purged with nitrogen and reacted at 50 °C for 12 h. After the reaction was completed as shown by TLC, the reaction solution was filtered, followed by concentration and purification by column chromatography to give **12c** as a brown oily liquid (1.80 g, yield: 97.8%).

Step 3: (compound **12**)

**12c** (200 mg, 510 μmol), 2-bromopyrimidine (81 mg, 510 μmol), $Pd(dtbpf)Cl_2$ (33 mg, 51 μmol), and hexamethyldistannane (184 mg, 561 μmol) were dissolved in 1,4-dioxane (5 mL), and the mixture was reacted at 110 °C for 24 h under a nitrogen atmosphere. After the reaction was completed as shown by LCMS, water (30 mL) was added to the reaction solution, and the mixture was extracted with ethyl acetate (30 mL × 3). The organic phase was washed with a saturated potassium fluoride solution (20 mL × 2), dried over anhydrous sodium sulfate, and concentrated. After purification by prep-TLC, purification was performed by reversed-phase preparative chromatography to give **12** as a gray solid (7 mg, yield: 3.4%). LCMS m/z[M+H]$^+$: 392.1

$^1$HNMR (400 MHz, DMSO-$d_6$) δ 11.39 (s, 1H), 9.01 (d, *J* = 4.8 Hz, 2H), 8.86 (d, *J* = 12.4 Hz, 1H), 7.71 - 7.74 (m, 1H), 7.67 (d, *J* = 8.8 Hz, 2H), 7.46 - 7.53 (m, 4H), 1.67 (s, 3H), 1.64 (s, 3H).

Example 13: (Compound **13**)

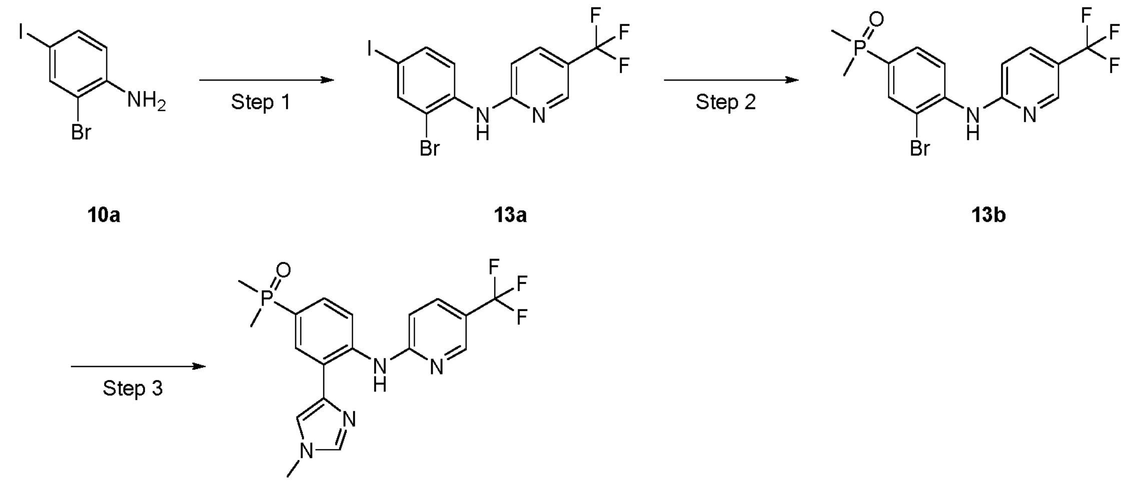


10a 13a 13b

13

Step 1: (compound **13a**)

**10a** (5.0 g, 17 mmol) and 2-chloro-5-trifluoromethylpyridine (3.1 g, 17 mmol) were dissolved in *N*,*N*-dimethylformamide (50 mL), and then sodium *tert*-butoxide (4.8 g, 50 mmol) was added. The mixture was reacted at 120 °C for 12 h. After the product was generated as shown by LCMS, the reaction solution was poured into water (200 mL), extracted with ethyl acetate (100 mL × 3), washed with saturated brine (50 mL × 2), dried over anhydrous sodium sulfate, and concentrated. Purification was performed by column chromatography to give 13a as a yellow oily liquid (490 mg, yield: 6.59%). LCMS m/z$[M+H]^+$: 445.0

Step 2: (compound **13b**)

**13a** (200 mg, 451 μmol), dimethylphosphine oxide (35 mg, 451 μmol), $Pd_2(dba)_3$ (41 mg, 45 μmol), 4,5-bis(diphenylphosphino)-9,9-dimethylxanthene (52 mg, 90 μmol), and cesium carbonate (441 mg, 1.4 mmol) were added to 1,4-dioxane (10 mL), and the mixture was purged with nitrogen and reacted at room temperature for 2 h. After the reaction was completed as shown by LCMS, water (20 mL) was added to the reaction solution, and the mixture was extracted with ethyl acetate (20 mL × 3), washed with saturated brine (5 mL × 2), dried over anhydrous sodium sulfate, and concentrated. Purification was performed by prep-TLC to give **13b** as a yellow oily liquid (70.0 mg, yield: 38.9%). LCMS m/z$[M+H]^+$: 395.1

Step 3: (compound **13**)

**13b** (65 mg, 162 μmol), 4-bromo-1-methyl-1*H*-imidazole (26 mg, 162 μmol), $Pd(dtbpf)Cl_2$ (10 mg, 16 μmol), and hexamethyldistannane (58 mg, 178 μmol) were dissolved in 1,4-dioxane (7 mL), and the mixture was reacted at 110 °C for 24 h under a nitrogen atmosphere. After the reaction was completed as shown by LCMS, the reaction solution was filtered, concentrated, and purified by reversed-phase preparative chromatography to give **13** as a gray-brown gummy solid (6.6 mg, yield: 10%). LCMS m/z$[M+H]^+$: 395.1

$^1$HNMR (400 MHz, $CDCl_3$) δ 12.03 (s, 1H), 8.77 (dd, *J* = 2.8, 8.4 Hz, 1H), 8.51 (s, 1H), 8.04 (dd, *J* = 1.6, 12.4 Hz, 1H), 7.68 (dd, *J* = 2.4, 8.8 Hz, 1H), 7.55 (s, 1H), 7.36 - 7.41 (m, 2H), 6.92 (d, *J* = 8.8 Hz, 1H), 3.78 (s, 3H), 1.76 (s, 3H), 1.73 (s, 3H).

Example 14: (Compound **14**)

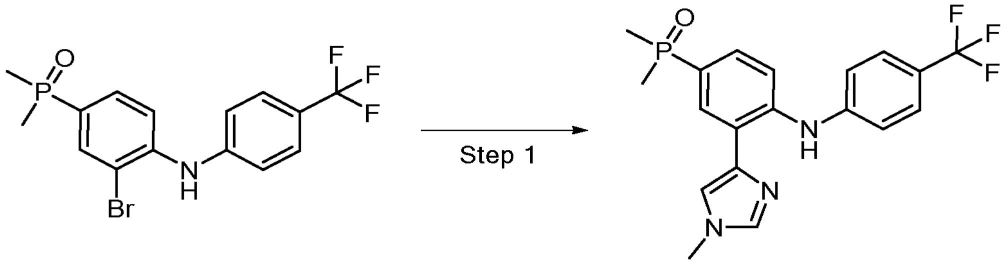


12c 14

**12c** (100 mg, 255 μmol), 4-bromo-1-methyl-1*H*-imidazole (41 mg, 255 μmol), Pd(dtbpf)$Cl_2$ (17 mg, 26 μmol), and hexamethyldistannane (92 mg, 280 μmol) were dissolved in 1,4-dioxane (3 mL), and the mixture was reacted at 110 °C for 24 h under a nitrogen atmosphere. After the reaction was completed as shown by LCMS, the reaction solution was filtered, concentrated by rotary evaporation, and purified by reversed-phase preparative chromatography to give **14** as a brown gummy solid (16 mg, yield: 16.2%). LCMS m/z$[M+H]^+$: 394.2

$^1$HNMR (400 MHz, DMSO-$d_6$) δ 10.77 (s, 1H), 7.99 - 8.02 (m, 1H), 7.85 - 7.79 (m, 2H), 7.60 - 7.51 (m, 4H), 7.29 (d, *J* = 8.4 Hz, 2H), 3.74 (s, 3H), 1.67 (s, 3H), 1.63 (s, 3H).

Example 15: (Compound **15**)

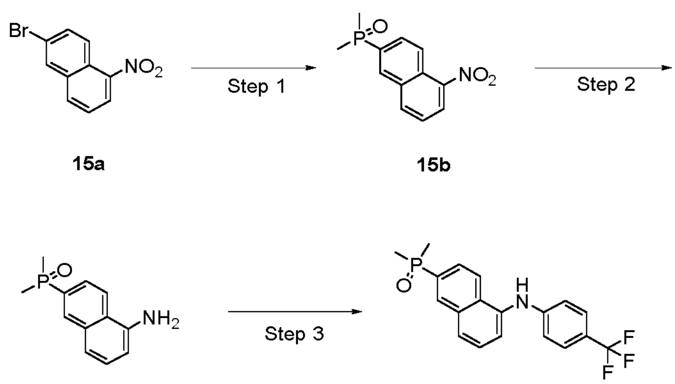


15a 15b 15c 15

Step 1: (compound **15b**)

Compound **15a** (200 mg, 0.8 mmol), dimethylphosphine oxide (120 mg, 1.6 mmol), cesium carbonate (510 mg, 1.6 mmol), palladium(II) acetate (35 mg, 0.2 mmol), and 4,5-bis(diphenylphosphino)-9,9-dimethylxanthene (180 mg, 0.3 mmol) were added to a solution of dioxane (5 mL). The mixture was purged three times with nitrogen, heated to 110 °C, and stirred for 4 h. The reaction solution was directly concentrated and separated by column chromatography to give compound **15b** (150 mg, yield: 76%). LCMS m/z$[M+H]^+$: 250.1

Step 2: (compound **15c**)

Compound **15b** (150 mg, 0.6 mmol), iron powder (0.34 g, 6 mmol), ammonium chloride (0.32 g, 6 mmol), and water (1 mL) were added to methanol (5 mL). The mixture was heated to 50 °C and stirred for 16 h. The reaction solution was filtered through celite, and the filtrate was concentrated to give a crude product of compound **15c** (0.12 g), which was used directly in the next step. LCMS m/z$[M+H]^+$: 220.1

Step 3: (compound **15**)

Compound **15c** (0.12 g, 0.54 mmol), 4-(trifluoromethyl)phenylboronic acid (0.21 g, 1.1 mmol), cupric acetate (54 mg, 0.3 mmol), and triethylamine (0.16 g, 1.6 mmol) were added to a solution of dioxane (1 mL). The mixture was heated to 60 °C and stirred open to air for 3 h. The reaction solution was directly concentrated and separated by column

chromatography to give compound **15** as a yellow solid (4 mg, yield: 2%). LCMS m/z[M+H]$^+$: 364.1

$^1$HNMR (400 MHz, DMSO-*d6*) δ 8.85 (s, 1H), 8.44 (dd, *J* = 13.1, 1.5 Hz, 1H), 8.18 (dd, *J* = 8.7, 2.5 Hz, 1H), 7.93 - 7.80 (m, 2H), 7.66 - 7.56 (m, 2H), 7.53 (d, *J* = 8.5 Hz, 2H), 7.04 (d, *J* = 8.4 Hz, 2H), 1.78 (s, 3H), 1.75 (s, 3H).

Example 16: (Compound 16)

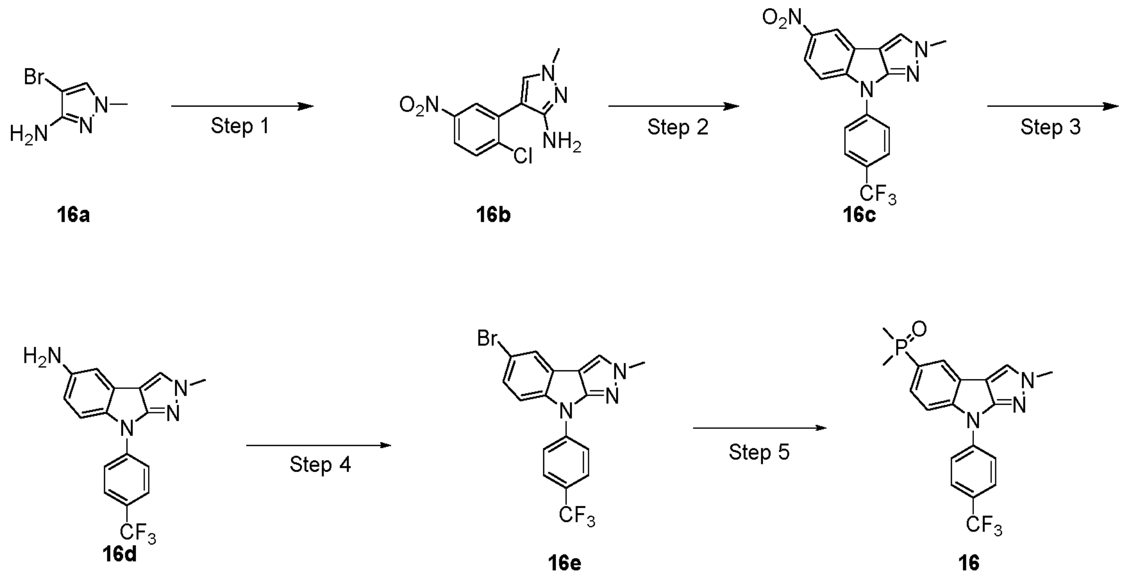


Step 1: (compound **16b**)

**16a** (2.1 g, 11.93 mmol), 2-chloro-5-nitrophenylboronic acid (3.87 g, 19.22 mmol), potassium carbonate (6.27 g, 45.33 mmol), and [1,1'-bis(diphenylphosphino)ferrocene]palladium(II)dichloride dichloromethane complex (1.75 g, 2.39 mmol) were added to 1,4-dioxane (50 mL) and water (10 mL), and the mixture was stirred at 85 °C for 6 h under a nitrogen atmosphere. The reaction solution was cooled to room temperature, and silica gel was added, followed by separation by column chromatography to give compound **16b** (700 mg, yield: 15%). LCMS m/z[M+H]$^+$: 253.1

Step 2: (compound **16c**)

**16b** (700 mg, 2.77 mmol), 4-bromobenzotrifluoride (0.81 g, 3.6 mmol), cesium carbonate (2.71 g, 8.31 mmol), and methanesulfonato(2-dicyclohexylphosphino-2',6'-di-i-propoxy-1,1'-biphenyl)(2'-methylamino-1,1'-biphenyl-2-yl) palladium(II) (0.12 g, 0.14 mmol) were added to 1,4-dioxane (20 mL), and the mixture was stirred at 120 °C for 16 h under a nitrogen atmosphere. The reaction solution was cooled to room temperature, and silica gel was added, followed by separation by column chromatography to give compound **16c** (800 mg, yield: 40%). LCMS m/z[M+H]$^+$: 361.2

Step 3: (compound **16d**)

**16c** (850 mg, 2.36 mmol), zinc powder (2.31 g, 35.4 mmol), and a saturated aqueous ammonium chloride solution (4 mL) were added to ethanol (20 mL), and the mixture was stirred at 80 °C for 2 h under a nitrogen atmosphere. The reaction solution was cooled to room temperature and filtered, and silica gel was added to the filtrate, followed by separation by column chromatography to give compound **16d** (330 mg, yield: 42%). LCMS m/z[M+H]$^+$: 331.1

Step 4: (compound **16e**)

**16d** (250 mg, 0.76 mmol) and cuprous bromide (220 mg, 1.52 mmol) were added to acetonitrile (5 mL), and at 0 °C, *tert*-butyl nitrite (240 mg, 2.28 mmol) was added. The mixture was stirred at room temperature for 2 h under a nitrogen atmosphere, and an aqueous sodium bicarbonate solution and ethyl acetate were added, followed by phase separation and extraction. The organic phase was dried over anhydrous sodium sulfate. Silica gel was added to the organic phase, followed by separation by column chromatography to give compound **16e** (100 mg, yield: 34%). LCMS m/z[M+H]$^+$: 394.0

Step 5: (compound **16**)

**16e** (42 mg, 0.11 mmol), dimethylphosphine oxide (17 mg, 0.22 mmol), cesium carbonate (220 mg, 1.52 mmol), 4,5-bis(diphenylphosphino)-9,9-dimethylxanthene (13 mg, 0.02 mmol), and tris(dibenzylideneacetone)dipalladium(0) (10 mg, 0.01 mmol) were added to 1,4-dioxane (1.5 mL), and the mixture was stirred at 110 °C for 12 h under a nitrogen atmosphere. The reaction solution was cooled to room temperature and subjected to preparative high-performance liquid chromatography to give compound 16 (12 mg, yield: 29%). LCMS m/z[M+H]$^+$: 392.1

$^1$HNMR (400 MHz, DMSO-*d6*) δ 8.21 (dd, *J* = 12.0, 1.5 Hz, 1H), 7.92 (d, *J* = 8.4 Hz, 2H), 7.82 (d, *J* = 8.4 Hz, 2H), 7.69 (q, *J* = 3.1, 2.2 Hz, 2H), 7.56 (ddd, *J* = 11.4, 8.4, 1.6 Hz, 1H), 4.08 (s, 3H), 1.80 (d, *J* = 13.0 Hz, 6H).

Example 17: (Compound **17**)

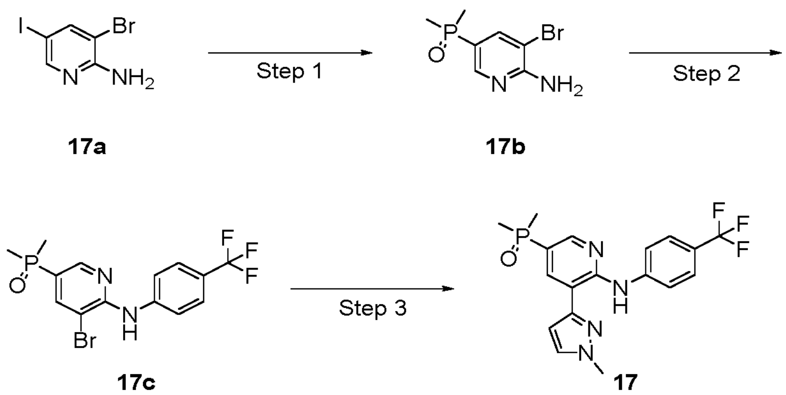


**17a** **17b** **17c** **17**

Step 1: (compound **17b**)

**17a** (3.0 g, 10 mmol) was dissolved in dioxane (50 mL), and dimethylphosphine oxide (0.86 g, 11 mmol), potassium phosphate (2.33 g, 11 mmol), 4,5-bis(diphenylphosphino)-9,9-dimethylxanthene (0.35 g, 0.6 mmol), and tris(dibenzylideneacetone)dipalladium(0) (0.27 g, 0.3 mmol) were added. The mixture was purged three times with nitrogen, heated to 100 °C, and stirred for 3 h. The reaction solution was concentrated to give a crude product, and the crude product was separated by column chromatography to give **17b** as a brown solid (2 g, yield: 80%). LCMS m/z[M+H]$^+$: 249.0

Step 2: (compound **17c**)

**17b** (500 mg, 2.01 mmol) was dissolved in acetonitrile (10 mL), and 4-(trifluoromethyl)phenylboronic acid (766.7 mg, 4.02 mmol), 2,6-lutidine (430 mg, 4.02 mmol), cupric acetate (547 mg, 3.01 mmol), and triethylamine (406.3 mg, 4.02 mmol) were added. The mixture was purged three times with oxygen, heated to 80 °C, and stirred for reaction for 24 h. The reaction solution was concentrated, and the crude product was separated by column chromatography to give **17c** as a brown solid (200 mg, yield: 25%). LCMS m/z[M+H]$^+$: 393.0

Step 3: (compound **17**)

**17c** (90 mg, 0.23 mmol) was dissolved in dioxane (3 mL) and water (1 mL), and 1-methylpyrazole-3-boronic acid (43.2 mg, 0.34 mmol), potassium carbonate (63.3 mg, 0.46 mmol), and [1,1'-bis(diphenylphosphino)ferrocene]palladium(II) dichloride (16.8 mg, 0.023 mmol) were added. The mixture was purged three times with nitrogen and then heated to 100 °C for reaction for 16 h. The reaction solution was concentrated. The crude product was separated by column chromatography and then by high-performance liquid chromatography to give **17** as a white solid (50.8 mg, yield: 56%). LCMS m/z[M+H]$^+$: 395.1

$^1$HNMR (400 MHz, MeOD) δ 8.53 (dd, *J* = 6.0, 2.1 Hz, 1H), 8.36 (dd, *J* = 11.4, 2.2 Hz, 1H), 8.06 (d, *J* = 8.5 Hz, 2H), 7.76 (d, *J* = 2.4 Hz, 1H), 7.63 (d, *J* = 8.6 Hz, 2H), 6.97 (d, *J* = 2.4 Hz, 1H), 4.09 (s, 3H), 1.89 (s, 3H), 1.86 (s, 3H).

Example 18: (Compound 18)

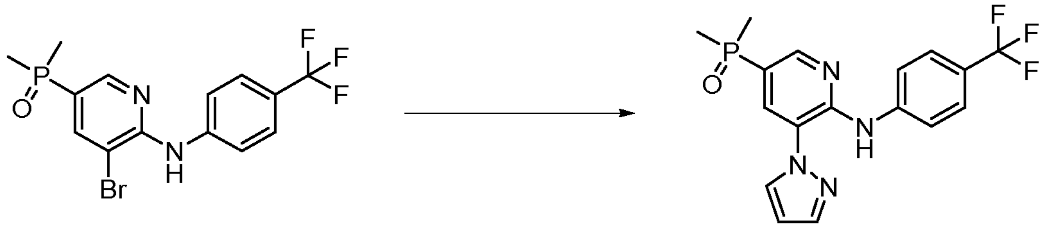

**17c** **18**

**17c** (200 mg, 0.51 mmol) was dissolved in dioxane (10 mL), and pyrazole (173.2 mg, 2.55 mmol), cuprous oxide (36.4 mg, 0.25 mmol), cesium carbonate (331.5 mg, 1.02 mmol), and salicylaldoxime (69.8 mg, 0.51 mmol) were added. The mixture was reacted at 120 °C for 60 h under a nitrogen atmosphere. The reaction solution was concentrated and separated by column chromatography, and the crude product was separated by high-performance liquid chromatography to give a white solid (53.3 mg, yield: 28%). LCMS m/z$[M+H]^+$: 381.2

$^1$HNMR (400 MHz, DMSO-d6) δ 8.53 (dd, *J* = 6.0, 2.0 Hz, 1H), 8.33 - 8.28 (m, 1H), 8.09 (dd, *J* = 11.2, 2.0 Hz, 1H), 7.91 (dd, *J* = 12.1, 5.2 Hz, 3H), 7.58 (d, *J* = 8.6 Hz, 2H), 6.66 - 6.61 (m, 1H), 1.86 (s, 3H), 1.82 (s, 3H).

Example 19: (Compound 19)

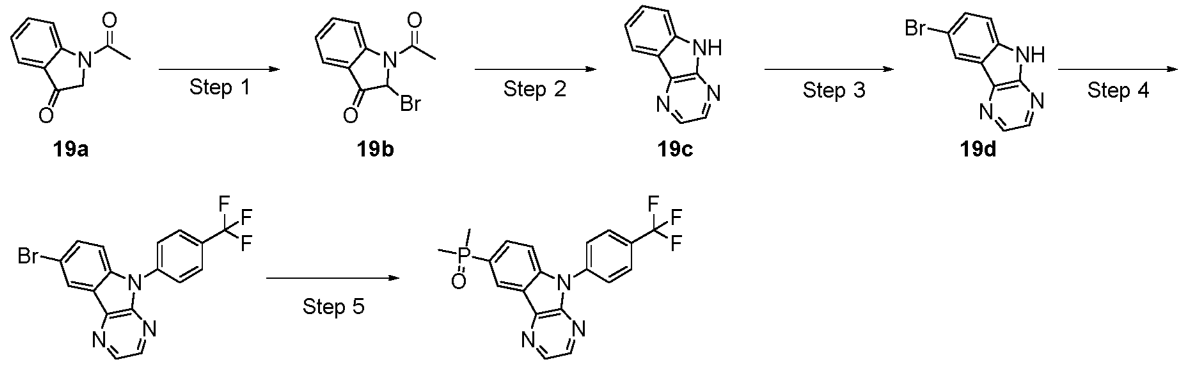


**19a** **19b** **19c** **19d**

**19e** **19**

Step 1: (compound **19b**)

19a (2.0 g, 11.45 mmol) was added to dry dichloromethane (50 mL), and the mixture was purged with argon and stirred at room temperature. Bromine (680 μL, 13.28 mmol) was added dropwise, and the resulting mixture was stirred for 20 min. The solvent was removed by distillation under reduced pressure, and the residue was dispersed in petroleum ether (20 mL). The brown solid was filtered, and the filter cake was washed with petroleum ether (2 × 10 mL) and dried under vacuum to give compound 19b (1.9 g, yield: 68%). LCMS m/z$[M+H]^+$: 253.1

Step 2: (compound 19c)

A solution (20 mL) of 3,4-diaminopyridine (1.63 g, 15.2 mmol) in 1,4-dioxane and methanol (1:1) was added dropwise to a solution of compound **19b** (1.9 g, 7.6 mmol) in 1,4-dioxane (50 mL) at room temperature under an argon atmosphere. After 2 h of reaction, the solvent was removed under reduced pressure. The resulting residue was dissolved in a mixed solvent of methanol and ethyl acetate (20 mL/20 mL), and then the mixture was heated to 80 °C under an oxygen (1 atm) atmosphere for reaction for 3 h. The solvent was removed under reduced pressure, and the residue was separated by column chromatography to give compound **19c** as a yellow solid (450 mg, yield: 37%). LCMS m/z$[M+H]^+$: 170.1

Step 3: (compound **19d**)

At 0 °C, *N*-bromosuccinimide (396 mg, 9.42 mmol) was added to a solution of compound **19c** (450 mg, 1.57 mmol) in tetrahydrofuran (12 mL), and the mixture was heated to 60 °C and stirred for 2 h. After the reaction was completed, the system was quenched with a saturated aqueous sodium bicarbonate solution and extracted with ethyl acetate, and the organic phase was concentrated and then separated by column chromatography to give **19d** as a gray-white solid (512 mg, yield: 78%). LCMS m/z$[M+H]^+$: 248.1

Step 4: (compound **19e**)

**19d** (350 mg, 1.4 mmol), 4-(trifluoromethyl)phenylboronic acid (532 mg, 2.8 mmol), cupric acetate (126 mg, 0.7 mmol), 4,5-bis(diphenylphosphino)-9,9-dimethylxanthene (404 mg, 0.7 mmol), and pyridine (332 mg, 4.2 mmol) were added to dichloromethane (40 mL), and the mixture was reacted at 60 °C for 16 h under an oxygen atmosphere. The reaction mixture was cooled and concentrated, and the resulting residue was separated by column chromatography to give **19e** as a gray-white solid (289 mg, yield: 53%). LCMS m/z$[M+H]^+$: 392.1

Step 5: (compound **19**)

**19e** (150 mg, 0.38 mmol), dimethylphosphine oxide (292 mg, 3.8 mmol), palladium(II) acetate (16 mg, 0.07 mmol), 4,5-bis(diphenylphosphino)-9,9-dimethylxanthene (45 mg, 0.076 mmol), and cesium carbonate (247 mg, 0.76 mmol) were added to 1,4-dioxane (10 mL), and the mixture was reacted at 110 °C for 16 h under a nitrogen atmosphere. The reaction mixture was cooled and concentrated, and the resulting residue was separated by column chromatography and high-performance liquid chromatography to give 19 as a white solid (67 mg, yield: 54%). LCMS m/z$[M+H]^+$: 390.1

$^1$HNMR (400 MHz, MeOD) δ 8.87 - 8.82 (m, 1H), 8.65 (d, *J* = 2.4Hz, 1H), 8.51 (d, *J* = 2.4Hz, 1H), 8.07 - 7.94 (m, 5H), 7.83 - 7.80 (m, 1H), 1.92 (s, 3H), 1.88 (s, 3H).

Example 20: (Compound 20)

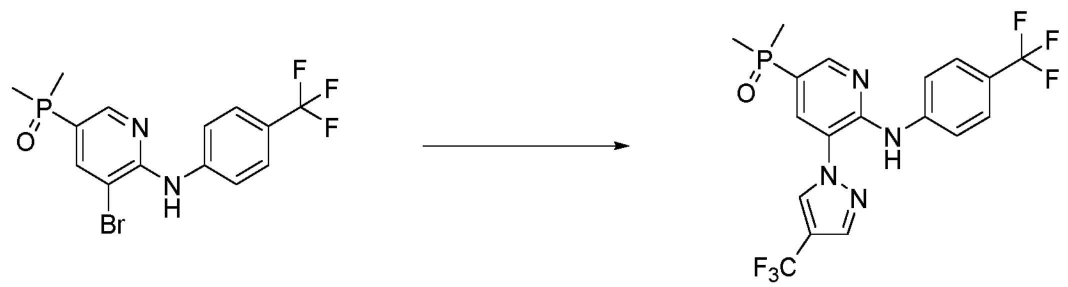

**17c** **20**

**17c** (200 mg, 0.51 mmol) was dissolved in 1,4-dioxane (10 mL), and 4-(trifluoromethyl)-1*H*-pyrazole (348.7 mg, 2.54 mmol), cuprous oxide (36.4 mg, 0.25 mmol), cesium carbonate (331.5 mg, 1.02 mmol), and salicylaldoxime (69.8 mg, 0.51 mmol) were added. The mixture was reacted at 120 °C for 72 h under a nitrogen atmosphere. The reaction solution was concentrated. The residue was separated by column chromatography, and the crude product was separated by high-performance liquid chromatography to give **20** as a white solid (28.8 mg, yield: 13%). LCMS m/z$[M+H]^+$: 449.1

$^1$HNMR (400 MHz, DMSO-d6) δ 8.82 (d, *J* = 0.7 Hz, 1H), 8.60 (dd, *J* = 5.9, 2.0 Hz, 1H), 8.20 (s, 1H), 8.14 (dd, *J* = 11.1, 2.0 Hz, 1H), 7.86 (d, *J* = 8.5 Hz, 2H), 7.58 (d, *J* = 8.6 Hz, 2H), 1.86 (s, 3H), 1.82 (s, 3H).

Example 21: (Compound **21**)

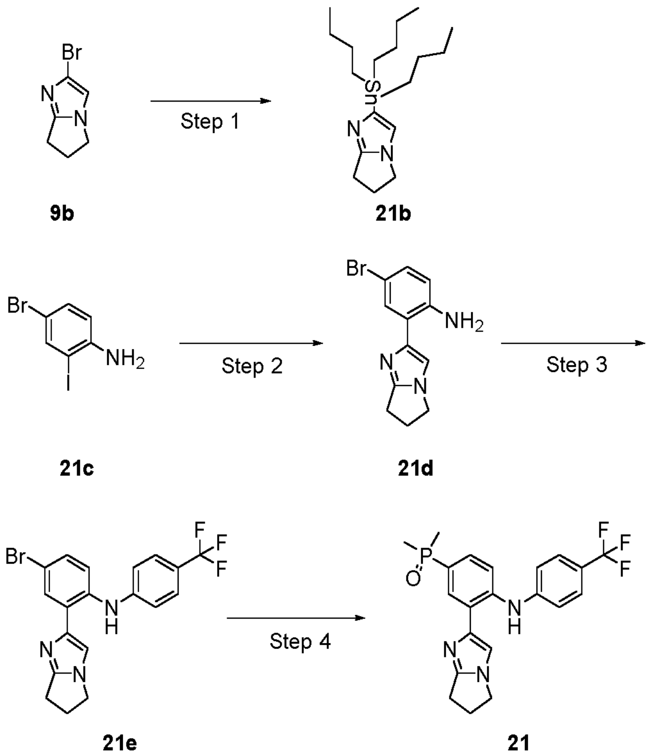


9b 21b 21c 21d 21e 21

Step 1: (compound **21b**)

Compound **9b** (500 mg, 2.67 mmol) was dissolved in anhydrous tetrahydrofuran (10 mL), and the mixture was purged three times with argon and cooled to -5 °C to 0 °C. A solution (2 M) of isopropylmagnesium chloride (412 mg, 4.01 mmol) in tetrahydrofuran was added dropwise, and after the dropwise addition, the mixture was stirred for 1 h. Butyltin trichloride (956 mg, 2.94 mmol) was added dropwise, and after the dropwise addition, the resulting mixture was stirred for 4 h. An aqueous potassium fluoride solution was added dropwise to the reaction solution, and the reaction mixture was stirred for 1 h and filtered through celite. The filtrate was extracted with ethyl acetate, and the organic phase was washed once with brine, dried over anhydrous sodium sulfate, and concentrated to give a crude product of **21b** as an oil (1060 mg, yield: 100%). LCMS m/z$[M+H]^+$: 399.2

Step 2: (compound **21d**)

Compound **21c** (950 mg, 3.2 mmol), **21b** (1270 mg, 3.2 mmol), tetrakis(triphenylphosphine)palladium(0) (370 mg, 0.32 mmol), and cuprous iodide (61 mg, 0.32 mmol) were added to 1,4-dioxane (10 mL), and the mixture was purged three times with argon, heated to 80 °C, and stirred for 16 h. The reaction solution was concentrated and separated by column chromatography to give **21d** as a brown solid (740 mg, yield: 83%). LCMS m/z$[M+H]^+$: 278.0

Step 3: (compound **21e**)

Compound **21d** (300 mg, 1.08 mmol) was dissolved in acetonitrile (6 mL), and 4-(trifluoromethyl)phenylboronic acid (308 mg, 1.62 mmol), triethylamine (110 mg, 1.09 mmol), 2,6-lutidine (12 mg, 0.11 mmol), and cupric acetate (20 mg, 0.11 mmol) were added. After the addition, the reaction solution was stirred for 3 h under an air atmosphere, diluted with ethyl acetate, concentrated, and separated by column chromatography to give **21e** as a brown oil (180 mg, yield: 39%). LCMS m/z$[M+H]^+$: 422.1

Step 4: (compound **21**)

Compound **21e** (180 mg, 0.43 mmol), dimethylphosphine oxide (50 mg, 0.64 mmol), and potassium phosphate (183 mg, 0.86 mmol) were dissolved in 1,4-dioxane (10 mL), and the mixture was purged three times with argon. Tris(dibenzylideneacetone)dipalladium(0) (40 mg, 0.044 mmol) and 4,5-bis(diphenylphosphino)-9,9-dimethylxanthene (50 mg, 0.086 mmol) were added under an argon atmosphere, and after the addition, the resulting mixture was heated to 110 °C and stirred for 12 h. The reaction solution was combined, filtered through celite, and rinsed with ethyl acetate, and

the organic phase was washed once with brine, dried over anhydrous sodium sulfate, concentrated, and separated by column chromatography to give a white solid. The white solid was slurried with petroleum ether/ethyl acetate = 5/1 (5 mL) and filtered, and the filter cake was dried under vacuum to give 21 as a yellow solid (100 mg, yield: 56%). LCMS m/z[M+H]+: 420.2

$^1$HNMR (400 MHz, DMSO-d6) δ 10.79 (s, 1H), 8.04 - 7.94 (m, 1H), 7.72 (s, 1H), 7.59 (d, *J* = 8.4 Hz, 2H), 7.54 - 7.46 (m, 2H), 7.27 (d, *J* = 8.4 Hz, 2H), 4.03 (t, *J* = 7.1 Hz, 2H), 2.86 (t, *J* = 7.5 Hz, 2H), 2.56 (q, *J* = 7.3 Hz, 2H), 1.66 (s, 3H), 1.63 (s, 3H).

Example 22: (Compound **22**)

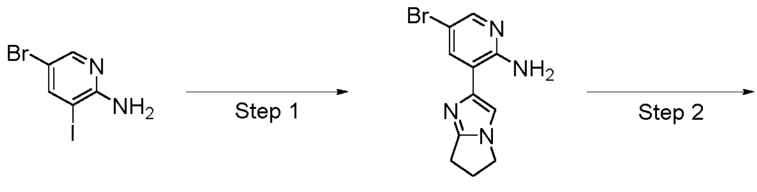


**8a** **22a**

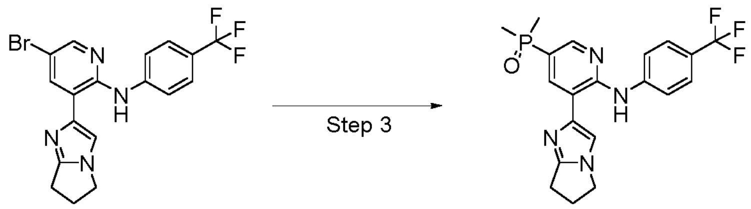


**22b** **22**

Step 1: (compound **22a**)

Compound **8a** (980 mg, 3.28 mmol), **21b** (1200 mg, 2.72 mmol), tetrakis(triphenylphosphine)palladium(0) (314 mg, 0.27 mmol), and cuprous iodide (52 mg, 0.27 mmol) were added to 1,4-dioxane (10 mL), and the mixture was purged three times with argon, heated to 80 °C, and stirred for 26 h. The reaction solution was concentrated and separated by column chromatography to give **22a** as a brown solid (450 mg, yield: 59%). LCMS m/z[M+H]+: 279.1

Step 2: (compound **22b**)

Compound **22a** (330 mg, 1.18 mmol) was dissolved in 1,2-dichloroethane (10 mL), and 4-(trifluoromethyl) phenylboronic acid (240 mg, 1.26 mmol) and cupric acetate (200 mg, 1.1 mmol) were added. After the addition, the reaction solution was stirred for 3 h under an air atmosphere, diluted with ethyl acetate, concentrated, and separated by column chromatography to give **22b** as a yellow solid (250 mg, yield: 50%). LCMS m/z[M+H]+: 423.1

Step 3: (compound **22**)

Compound **22b** (170 mg, 0.40 mmol), dimethylphosphine oxide (51 mg, 0.65 mmol), and potassium phosphate (170 mg, 0.80 mmol) were dissolved in 1,4-dioxane (10 mL), and the mixture was purged three times with argon. Tris(dibenzylideneacetone)dipalladium(0) (44 mg, 0.048 mmol) and 4,5-bis(diphenylphosphino)-9,9-dimethylxanthene (54 mg, 0.093 mmol) were added under an argon atmosphere, and after the addition, the resulting mixture was heated to 110 °C and stirred for 12 h. The reaction solution was combined, filtered through celite, and rinsed with ethyl acetate, and the organic phase was washed once with brine, dried over anhydrous sodium sulfate, concentrated, and separated by column chromatography to give a white solid. The white solid was slurried with petroleum ether/ethyl acetate = 5/1 (5 mL) and filtered, and the filter cake was dried under vacuum to give 22 as a yellow solid (120 mg, yield: 71%). LCMS m/z[M+H]+: 421.2

$^1$HNMR (400 MHz, DMSO-d6) δ 12.35 (d, *J* = 3.5 Hz, 1H), 8.41 (dq, *J* = 4.4, 2.1 Hz, 1H), 8.22 (dq, *J* = 11.1, 2.1 Hz, 1H), 7.99 (dt, *J* = 8.7, 3.5 Hz, 3H), 7.70 - 7.61 (m, 2H), 4.09 (q, *J* = 6.0, 3.7 Hz, 2H), 2.98 - 2.88 (m, 2H), 2.60 (d, *J* = 10.0 Hz, 2H), 1.72 (d, J= 3.8 Hz, 3H), 1.69 (d, *J* = 3.6 Hz, 3H).

Example 23: (Compound **23**)

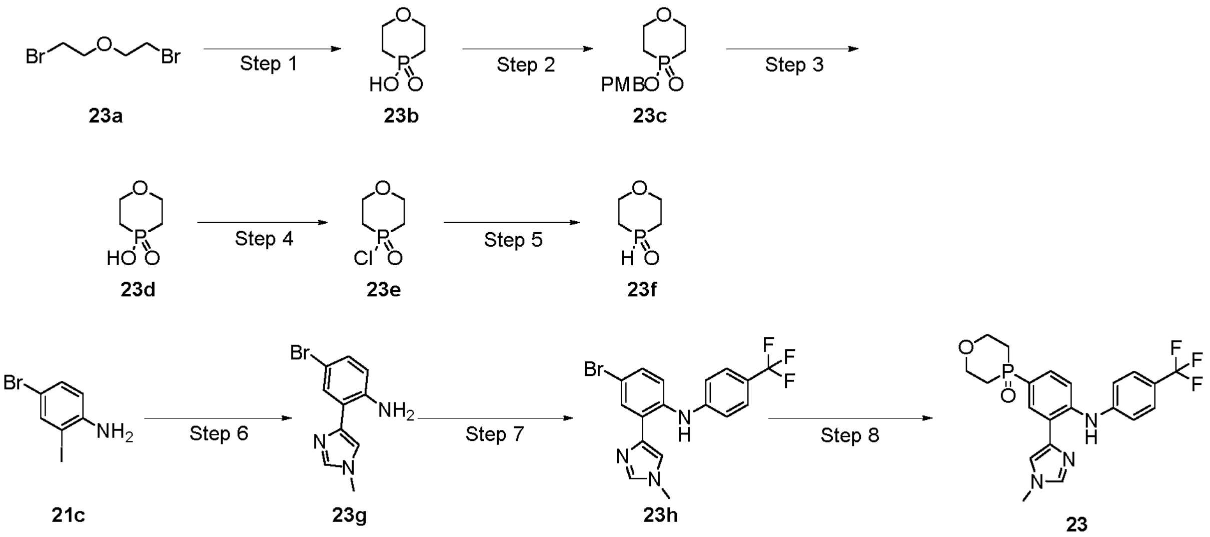


23a 23b 23c 23d 23e 23f 21c 23g 23h 23

Step 1: (compound **23b**)

**23a** (6 g, 25.87 mmol), ammonium hypophosphite (3.22 g, 38.8 mmol), and hexamethyldisilazane (16.7 g, 103.48 mmol) were added to 1,2,4-trimethylbenzene (42 mL), and the mixture was stirred at 150 °C for 12 h under a nitrogen atmosphere. The reaction solution was cooled to room temperature and filtered, and the filtrate was concentrated under reduced pressure, resulting in the precipitation of a solid. The solid was filtered, and the resulting filtrate was concentrated under reduced pressure, leading to the precipitation of another solid. After filtration, another concentration was performed to give **23b** as an oil (4 g, yield: 114%). LCMS m/z$[M+H]^+$: 137.0

Step 2: (compound **23c**)

**23b** (3.3 g, 24.25 mmol), cesium carbonate (15.8 g, 48.5 mmol), and 4-methoxybenzyl chloride (5.7 g, 36.38 mmol) were added to N,N-dimethylformamide (24 mL), and the mixture was stirred at 110 °C for 16 h under a nitrogen atmosphere. The reaction solution was cooled to room temperature, and silica gel was added, followed by separation by column chromatography to give **23c** (1.5 g, yield: 24%). LCMS m/z$[M+H]^+$: 257.2

Step 3: (compound **23d**)

**23c** (1.4 g, 2.36 mmol) and palladium on carbon (200 mg) were added to tetrahydrofuran (40 mL), and the mixture was purged five times with hydrogen and stirred at room temperature for 16 h under a hydrogen atmosphere. The reaction solution was filtered, and the filtrate was concentrated to give **23d** (700 mg, yield: 94%).

Step 4: (compound **23e**)

**23d** (125 mg, 0.92 mmol) and N,N-dimethylformamide (15 mg, 0.21 mmol) were added to dichloromethane (4 mL), and oxalyl chloride (0.35 g, 2.76 mmol) was added slowly. The mixture was stirred at room temperature for 16 h under a nitrogen atmosphere, and the reaction solution was concentrated to give **23e** (130 mg, yield: 92%), which was used directly in the next step.

Step 5: (compound **23f**)

**23e** (125 mg, 0.81 mmol) was added to dichloromethane (3 mL), and the mixture was cooled to - 78 °C. Then, a 1 M solution of diisobutylaluminium hydride in hexane (1.62 mL) was added, followed by stirring at -78 °C for 2 h under a nitrogen atmosphere. The reaction solution was diluted with 10 mL of dichloromethane, then silica gel was added, and the reaction mixture was vigorously stirred. 2 mL of methanol and 0.5 mL of acetic acid were added, and the resulting mixture was warmed to room temperature, stirred for 1 h, concentrated, and then separated by column chromatography to give **23f**

(35 mg, yield: 36%). LCMS m/z[M+H]$^+$: 121.0

Step 6: (compound **23g**)

21c (23.0 g, 77.20 mmol), *N*-methyl-4-(tributylstannyl)imidazole (25 g, 70.0 mmol), tetrakis(triphenylphosphine) palladium(0) (2.0 g, 1.75 mmol), and cuprous iodide (0.7 g, 3.5 mmol) were added to 1,4-dioxane (220 mL), and the mixture was purged three times with argon, heated to 80 °C, and stirred for 26 h. The reaction solution was concentrated and separated by column chromatography to give 23g as a brown solid (14 g, yield: 79%). LCMS m/z[M+H]$^+$: 252.1

Step 7: (compound **23h**)

**23g** (2.0 g, 7.93 mmol) was dissolved in acetonitrile (20 mL), and 4-(trifluoromethyl)phenylboronic acid (2.26 g, 11.89 mmol), triethylamine (0.8 g, 7.93 mmol), 2,6-lutidine (85 mg, 0.79 mmol), and cupric acetate (140 mg, 0.79 mmol) were added. After the addition, the reaction solution was stirred for 4 h under an air atmosphere, combined, concentrated, and separated by column chromatography to give **23h** as a brown oil (10 g, yield: 46%). LCMS m/z[M+H]$^+$: 396.2

Step 8: (compound **23**)

**23h** (30 mg, 0.076 mmol) and **23f** (11 mg, 0.091 mmol) were added to 1,4-dioxane (1 mL), and potassium phosphate (32 mg, 0.15 mmol), 4,5-bis(diphenylphosphino)-9,9-dimethylxanthene (8.8 mg, 0.015 mmol), and tris(dibenzylideneacetone)dipalladium(0) (7 mg, 0.0076 mmol) were added. The mixture was stirred at 110 °C for 8 h under a nitrogen atmosphere, and silica gel was added to the reaction solution, followed by separation by column chromatography to give **23** (30.5 mg, yield: 92%). LCMS m/z[M+H]$^+$: 436.2

$^1$HNMR (400 MHz, DMSO-d6) δ 10.95 (s, 1H), 8.04 (dd, *J* = 11.9, 1.7 Hz, 1H), 7.88 (dd, *J* = 15.1, 1.3 Hz, 2H), 7.66 - 7.50 (m, 4H), 7.33 (d, *J* = 8.4 Hz, 2H), 4.07 - 3.83 (m, 4H), 3.75 (s, 3H), 2.38 (tt, *J*= 9.9, 5.0 Hz, 2H), 1.93 (q, *J* = 16.0 Hz, 2H).

Example 24: (Compound 24)

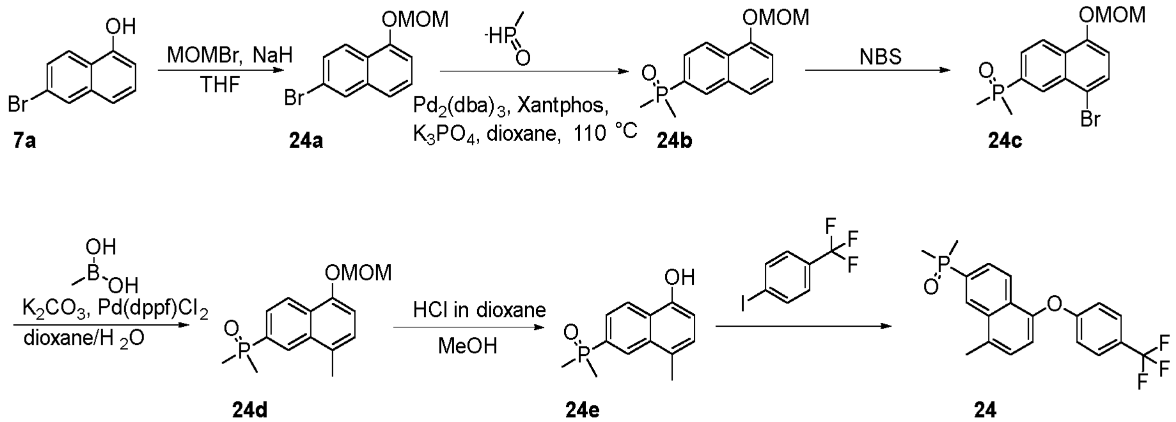


7a 24a 24b 24c

24d 24e 24

Step 1: (compound 24a)

Compound 7a (1000 mg, 4.48 mmol) was dissolved in THF (15 mL), and the mixture was purged three times with argon and cooled to 0 °C. Sodium hydride (270 mg, 6.75 mmol) was added slowly, and after the addition, the resulting mixture was stirred for 30 min. Bromomethyl methyl ether (672 mg, 5.38 mmol) was added, and the reaction mixture was stirred for 1 h. After the reaction was completed as shown by LC-MS, the reaction solution was poured into ice water and extracted with ethyl acetate, and the organic phase was washed with brine, dried over anhydrous sodium sulfate, and concentrated to give 24a as a brown oily liquid (1100 mg, yield: 91%).

Step 2: (compound 24b)

Compound 24a (1100 mg, 4.12 mmol), dimethylphosphine oxide (386 mg, 4.95 mmol), and potassium phosphate (1750 mg, 8.24 mmol) were dissolved in 1,4-dioxane (22 mL), and the mixture was purged three times with argon. Pd$_2$

(dba)$_3$ (377 mg, 0.41 mmol) and Xantphos (238 mg, 0.41 mmol) were added, and after the addition, the reaction solution was heated to 110 °C and stirred for 18 h. After the reaction was completed as shown by LC-MS, the reaction solution was cooled, poured into water, and extracted with ethyl acetate. The organic phase was washed with brine, dried over anhydrous sodium sulfate, and concentrated, and the crude product was purified by column chromatography to give 24b as a brown oily liquid (800 mg, yield: 73%). LCMS(ESI)m/z =529.3 $[2M+1]^+$. 1H NMR (400 MHz, DMSO-d6) δ 8.35 (dt, J = 13.1, 1.0 Hz, 1H), 8.28 (dd, J = 8.6, 2.6 Hz, 1H), 7.82 (ddd, J = 9.9, 8.6, 1.5 Hz, 1H), 7.67 (d, J = 8.2 Hz, 1H), 7.52 (t, J = 8.0 Hz, 1H), 7.23 (dd, J = 7.7, 0.9 Hz, 1H), 5.45 (s, 2H), 3.46 (s, 3H), 1.74 (s, 3H), 1.71 (s, 3H).

Step 3: (compound 24c)

Compound 24b (800 mg, 3.03 mmol) was dissolved in acetonitrile (20 mL), and NBS (600 mg, 3.37 mmol) was added under stirring. The reaction mixture was stirred at room temperature for 1 h. After the starting materials were consumed completely as shown by LCMS, the reaction solution was poured into water, extracted with ethyl acetate, dried over anhydrous sodium sulfate, filtered, and concentrated under reduced pressure to give a crude product. The crude product was purified by column chromatography to give 24c as a yellow oily liquid (935 mg, yield: 90%). LCMS(ESI)m/z =685.1 $[2M+1]^+$. 1H NMR (400 MHz, DMSO-d6) δ 8.54 (dt, J = 13.3, 0.9 Hz, 1H), 8.35 (dd, J = 8.6, 2.7 Hz, 1H), 7.95 - 7.92 (m, 1H), 7.89 (d, J = 8.4 Hz, 1H), 7.19 (d, J = 8.4 Hz, 1H), 5.46 (s, 2H), 3.45 (s, 3H), 1.76 (s, 3H), 1.73 (s, 3H).

Step 4: (compound 24d)

Compound 24c (1200 mg, 3.5 mmol), methylboronic acid (420 mg, 7.02 mmol), potassium carbonate (1210 mg, 8.75 mmol), and Pd(dppf)$Cl_2$ (256 mg, 0.35 mmol) were dissolved in 1,4-dioxane (25 mL) and water (2.5 mL), and the reaction mixture was purged three times with argon, heated to 90 °C, and stirred for 5 h. After the starting materials were consumed completely as shown by LCMS, the reaction solution was concentrated under reduced pressure to give a crude product. The crude product was purified by column chromatography to give 24d as a brown solid (768 mg, yield: 79%). LCMS(ESI)m/z =557.3 $[2M+1]^+$.

Step 5: (compound 24e)

Compound 24d (1000 mg, 2.7 mmol) was dissolved in methanol (20 mL), and a solution of hydrochloric acid in dioxane (4 mL, 4 mol/L in dioxane) was added at room temperature. The reaction mixture was stirred at room temperature for 1 h. After the starting materials were consumed completely as shown by LCMS, the reaction solution was concentrated under reduced pressure to give a crude product, and the crude product was purified by column chromatography to give 24e as a yellow oily liquid (500 mg, yield: 79%). LCMS(ESI)m/z =235.2 $[M+H]^+$.

Step 6: (compound 24)

Compound 24e (600 mg, 2.56 mmol), 4-iodobenzotrifluoride (836 mg, 3.07 mmol), potassium carbonate (425 mg, 3.08 mmol), 2-picolinic acid (32 mg, 0.26 mmol), and cuprous iodide (50 mg, 0.26 mmol) were dissolved in DMSO (10 mL), and the reaction mixture was purged three times with nitrogen, heated to 120 °C and stirred for 14 h. After the starting materials were consumed completely as shown by LCMS, the reaction solution was poured into water and extracted with ethyl acetate. The organic phase was washed with saturated brine, dried over anhydrous sodium sulfate, filtered, and concentrated under reduced pressure to give a crude product. Purification was performed by column chromatography and Prep-HPLC to give compound 24 as a yellow colloid (302 mg, yield: 31%). LCMS(ESI)m/z=379.2 $[M+H]^+$. 1H NMR (400 MHz, DMSO-d6) δ 8.52 - 8.47 (m, 1H), 8.02 (dd, J = 8.6, 2.7 Hz, 1H), 7.86 (td, J = 9.0, 8.5, 1.4 Hz, 1H), 7.71 (d, J = 8.6 Hz, 2H), 7.51 (dd, J = 7.7, 1.2 Hz, 1H), 7.30 (d, J = 7.7 Hz, 1H), 7.12 (d, J = 8.5 Hz, 2H), 2.74 (s, 3H), 1.76 (s, 3H), 1.72 (s, 3H).

Example 25: (Compound 25)

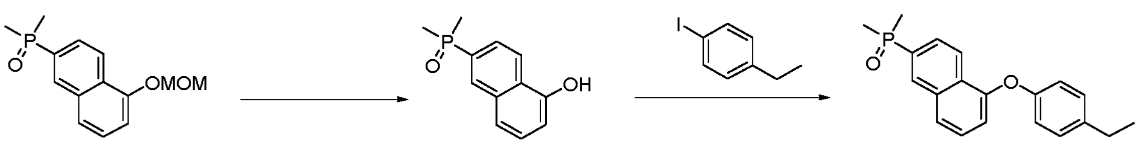

24b 25a 25

Step 1: (compound 25a)

24b (11.1 g, 0.04 mmol) and 40 mL of a 4 M solution of HCl in 1,4-dioxane were added to a 100 mL single-necked flask in sequence, and the mixture was stirred at room temperature for 4 h, resulting in the precipitation of a solid. The solid was filtered to give 25a as a white solid (8.3 g, 79%).

Step 2: (compound 25)

25a (70 mg, 0.32 mmol), 1-ethyl-4-iodobenzene (148 mg, 0.64 mmol), potassium carbonate (110 mg, 0.77 mmol), dimethyl sulfoxide (1.5 mL), picolinic acid hydrochloride (25 mg, 0.15 mmol), and cuprous iodide (18 mg, 0.096 mmol) were added to a 25 mL single-necked flask in sequence, and the mixture was purged 5 times with a nitrogen balloon and stirred at 120 °C for 16 h under a nitrogen atmosphere. 20 mL of water was added, and extraction was performed twice with ethyl acetate (25 mL). The organic phases were combined, washed once with saturated brine (20 mL), dried over anhydrous sodium sulfate, concentrated, and subjected to column chromatography to give a product of 25 (50 mg, yield: 56%). LCMS(ESI)m/z =325.1 $[M+H]^+$ $^1H$ NMR (400 MHz, DMSO-d6) δ 8.45 (d, J = 13.0 Hz, 1H), 8.20 (dd, J = 8.6, 2.4 Hz, 1H), 7.85 (td, J = 8.7, 2.2 Hz, 2H), 7.55 (t, J = 7.9 Hz, 1H), 7.24 (d, J = 8.5 Hz, 2H), 7.04 (d, J = 7.6 Hz, 1H), 7.01 - 6.94 (m, 2H), 2.60 (q, J = 7.6 Hz, 2H), 1.73 (d, J = 13.3 Hz, 6H), 1.18 (t, J = 7.6 Hz, 3H).

Example 26: (Compound 26)

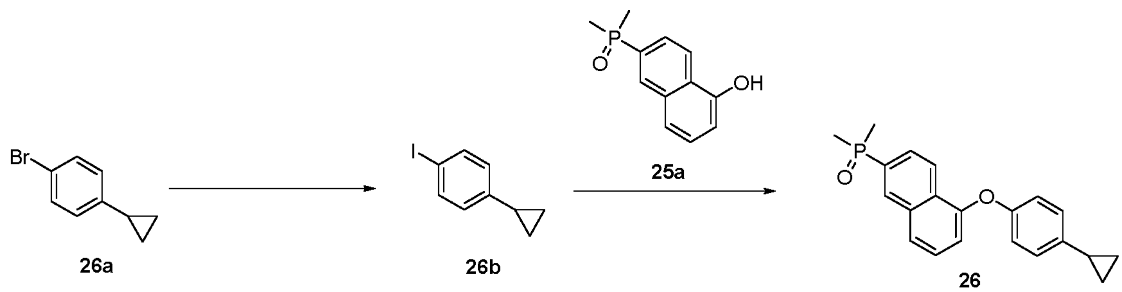


26a 26b 26

Step 1: (compound 26b) 26a (160 mg, 0.81 mmol), sodium iodide (360 mg, 2.43 mmol), n-butanol (1.5 mL), *trans*-*N*,*N'*-dimethylcyclohexane-1,2-diamine (23 mg, 0.16 mmol), and cuprous iodide (15.4 mg, 0.081 mmol) were added to a 25 mL single-necked flask in sequence, and the mixture was purged 5 times with a nitrogen balloon and stirred at 120 °C for 16 h under a nitrogen atmosphere. 20 mL of water was added, and extraction was performed twice with ethyl acetate (25 mL). The organic phases were combined, washed once with saturated brine (20 mL), dried over anhydrous sodium sulfate, concentrated, and subjected to column chromatography to give a product of 26b (180 mg, yield: 91%), which was used directly in the next step.

Step 2: (compound 26c)

25a (70 mg, 0.32 mmol), 26b (160 mg, 0.64 mmol), potassium carbonate (110 mg, 0.77 mmol), dimethyl sulfoxide (1.5 mL), picolinic acid hydrochloride (25 mg, 0.15 mmol), and cuprous iodide (18 mg, 0.096 mmol) were added to a 25 mL single-necked flask in sequence, and the mixture was purged 5 times with a nitrogen balloon and stirred at 120 °C for 16 h under a nitrogen atmosphere. 20 mL of water was added, and extraction was performed twice with ethyl acetate (25 mL). The organic phases were combined, washed once with saturated brine (20 mL), dried over anhydrous sodium sulfate, concentrated, and subjected to column chromatography to give a product of 26 (72 mg, yield: 67%). LCMS(ESI)m/z =337.1 $[M+H]^+$. $^1H$ NMR (400 MHz, DMSO-d6) δ 8.47 - 8.39 (m, 1H), 8.19 (dd, J = 8.6, 2.6 Hz, 1H), 7.88 - 7.79 (m, 2H), 7.54 (t, J = 7.9 Hz, 1H), 7.15 - 7.08 (m, 2H), 7.02 (dd, J = 7.6, 0.9 Hz, 1H), 6.98 - 6.91 (m, 2H), 1.93 (tt, J = 8.3, 5.3 Hz, 1H), 1.73 (d, J = 13.3 Hz, 6H), 0.97 - 0.89 (m, 2H), 0.68 - 0.60 (m, 2H).

Example 27: (Compound 27)

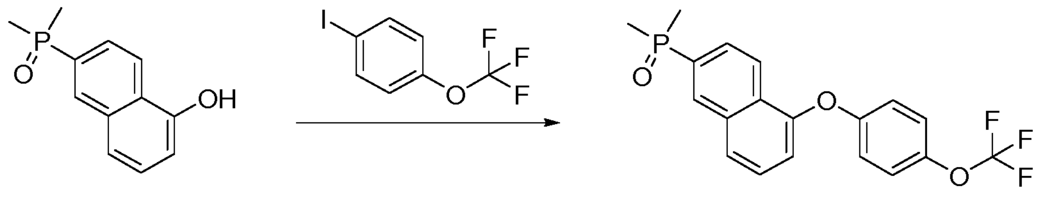

25a

27

25a (70 mg, 0.32 mmol), 4-(trifluoromethoxy)iodobenzene (184 mg, 0.64 mmol), potassium carbonate (110 mg, 0.77 mmol), dimethyl sulfoxide (1.5 mL), picolinic acid hydrochloride (25 mg, 0.15 mmol), and cuprous iodide (18 mg, 0.096 mmol) were added to a 25 mL single-necked flask in sequence, and the mixture was purged 5 times with a nitrogen balloon and stirred at 120 °C for 16 h under a nitrogen atmosphere. 20 mL of water was added, and extraction was performed twice with ethyl acetate (25 mL). The organic phases were combined, washed once with saturated brine (20 mL), dried over anhydrous sodium sulfate, concentrated, and subjected to column chromatography to give a product of 27 (35 mg, yield: 34%). LCMS(ESI)m/z =381.1 $[M+H]^+$. $^1H$ NMR (400 MHz, DMSO-d6) δ 8.48 (d, J = 13.0 Hz, 1H), 8.13 (dd, J = 8.6, 2.4 Hz, 1H), 7.94 (d, J = 8.3 Hz, 1H), 7.91 - 7.82 (m, 1H), 7.61 (t, J = 7.9 Hz, 1H), 7.40 (d, J = 8.7 Hz, 2H), 7.22 (d, J = 7.6 Hz, 1H), 7.18 - 7.10 (m, 2H), 1.73 (d, J = 13.3 Hz, 6H).

Example 28: (Compound 28)

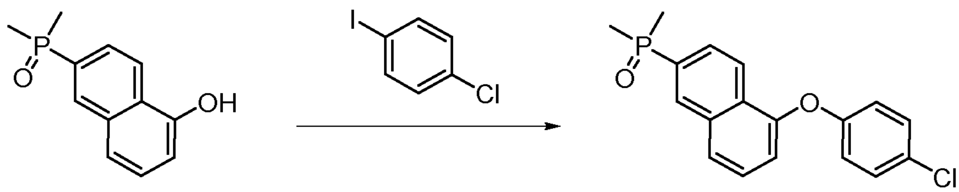

25a

28

25a (70 mg, 0.32 mmol), 1-chloro-4-iodobenzene (150 mg, 0.64 mmol), potassium carbonate (110 mg, 0.77 mmol), dimethyl sulfoxide (1.5 mL), picolinic acid hydrochloride (25 mg, 0.15 mmol), and cuprous iodide (18 mg, 0.096 mmol) were added to a 25 mL single-necked flask in sequence, and the mixture was purged 5 times with a nitrogen balloon and stirred at 120 °C for 16 h under a nitrogen atmosphere. 20 mL of water was added, and extraction was performed twice with ethyl acetate (25 mL). The organic phases were combined, washed once with saturated brine (20 mL), dried over anhydrous sodium sulfate, concentrated, and subjected to column chromatography to give a product of 28 (17 mg, yield: 16.2%). LCMS(ESI)m/z =331.0 [M+H]+. $^1H$ NMR (400 MHz, DMSO-d6) δ 8.47 (dd, J = 13.0, 1.3 Hz, 1H), 8.12 (dd, J = 8.6, 2.5 Hz, 1H), 7.92 (d, J = 8.3 Hz, 1H), 7.86 (ddd, J = 9.9, 8.6, 1.5 Hz, 1H), 7.60 (t, J = 7.9 Hz, 1H), 7.49-7.41 (m, 2H), 7.18 (dd, J = 7.6, 0.9 Hz, 1H), 7.10-7.03 (m, 2H), 1.73 (d, J = 13.3 Hz, 6H).

Example 29: (Compound 29)

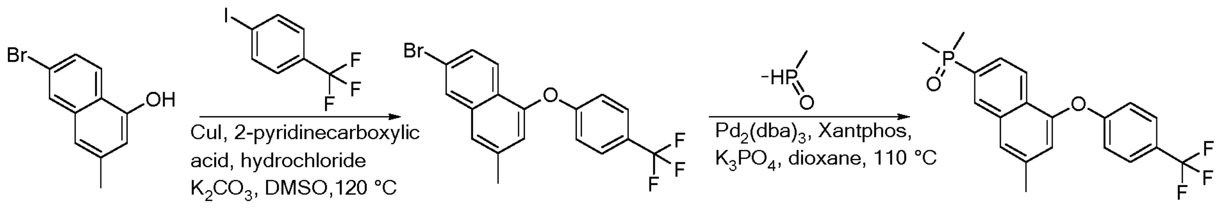


29a

29b

29

Step 1: (compound 29b)

Compound 29a (500 mg, 2.11 mmol, synthesized with reference to Example 20 in Patent WO2013103738A1), 4-iodobenzotrifluoride (688 mg, 2.53 mmol), and potassium carbonate (584 mg, 4.23 mmol) were dissolved in DMSO (10 mL), and the mixture was purged three times with argon. CuI (81 mg, 0.43 mmol) and 2-pyridinecarboxylic acid (104 mg, 0.84 mmol) were added, and after the addition, the reaction solution was heated to 120 °C and stirred for 12 h. After the starting materials were consumed completely as monitored by LC-MS, the reaction solution was poured into water and extracted with ethyl acetate. The organic phase was washed once with brine, dried over anhydrous sodium sulfate, concentrated, and subjected to column chromatography to give 29b as a colorless oily liquid (360 mg, yield: 45%).

Step 2: (compound 29)

Compound 29b (200 mg, 0.52 mmol), dimethylphosphine oxide (61 mg, 0.78 mmol), and potassium phosphate (220 mg, 1.04 mmol) were dissolved in 1,4-dioxane (3 mL), and the mixture was purged three times with argon. $Pd_2(dba)_3$ (48 mg, 0.05 mmol) and Xantphos (60 mg, 0.1 mmol) were added, and after the addition, the reaction solution was heated to 110 °C and stirred for 14 h. After the reaction was completed as shown by LC-MS, the reaction solution was cooled, filtered through celite, and washed twice with ethyl acetate, and the filtrate was concentrated. The crude product was purified by column chromatography to give another crude product, which was dissolved in a small amount of ethyl acetate, and then petroleum ether was added for crystallization. After the solid was completely precipitated, the solid was filtered and dried to give 29 as a brown solid (160 mg, yield: 80%). LCMS(ESI)m/z =379.2 $[M+H]^+$. $^1H$ NMR (400 MHz, Methanol-d4) δ 8.41 - 8.33 (m, 1H), 8.09 (dd, J = 8.6, 2.8 Hz, 1H), 7.75 - 7.68 (m, 2H), 7.66 (s, 1H), 7.64 (s, 1H), 7.13 (dd, J = 4.7, 1.2 Hz, 2H), 7.10 (s, 1H), 2.51 (s, 3H), 1.87 (s, 3H), 1.84 (s, 3H).

Example 30: (Compound 30)

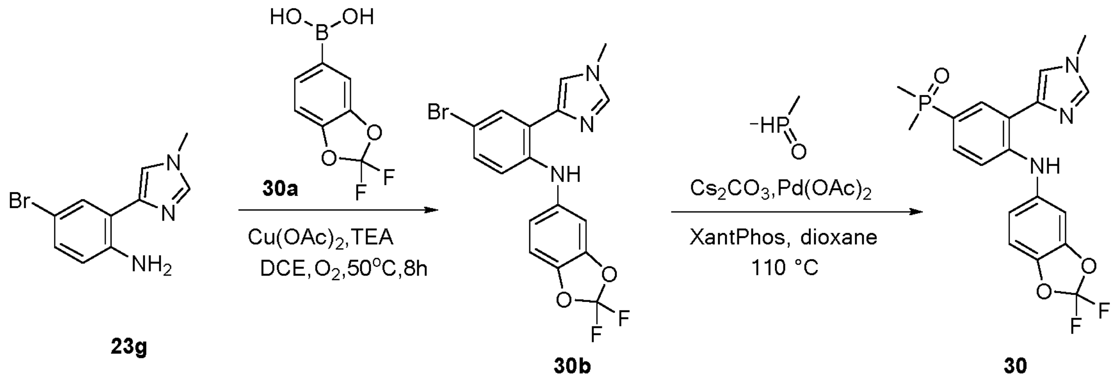


23g 30b 30

Step 1: (compound 30b)

Compound 23g (310 mg, 1.23 mmol), compound 30a (397.43 mg, 1.97 mmol), triethylamine (124.23 mg, 1.23 mmol), and cupric acetate (45.5 mg, 0.25 mmol) were added to 1,2-dichloroethane (10 mL). The mixture was purged three times with oxygen, and then heated to 50 °C and stirred for 8 h under an oxygen atmosphere. The reaction solution was concentrated under reduced pressure, and the residue was purified by column chromatography to give 30b as a yellow solid (162 mg, yield: 32.29%). LCMS(ESI)m/z=407.9$[M+H]^+$

Step 2: (compound 30) Compound 30b (110 mg, 0.27 mmol), dimethylphosphine oxide (52.61 mg, 0.67 mmol), cesium carbonate (131.63 mg, 0.41 mmol), Xantphos (62.49 mg, 0.11 mmol), and palladium(II) acetate (12.07 mg, 0.05 mmol) were added to 1,4-dioxane (10 mL). The mixture was purged three times with nitrogen, and then heated to 110 °C and stirred overnight under a nitrogen atmosphere. The reaction solution was cooled to room temperature and concentrated under reduced pressure, and the residue was purified by column chromatography to give 30 as a gray-white solid (50 mg, yield: 45.78%). LCMS(ESI)m/z=406.1$[M+H]^+$ .$^1H$ NMR (400 MHz, DMSO-d6): δ 10.61 (s, 1H), 7.95-7.91 (m, 1H), 7.83 (s, 1H), 7.80 (d, J=0.8 Hz, 1H), 7.46-7.41 (m, 1H), 7.34-7.31 (m, 2H), 7.27-7.24 (m, 1H), 7.00-6.97 (m, 1 H), 3.76 (s, 3 H), 1.66 (s, 3 H), 1.62 (s, 3 H).

Example 31: (Compound 31)

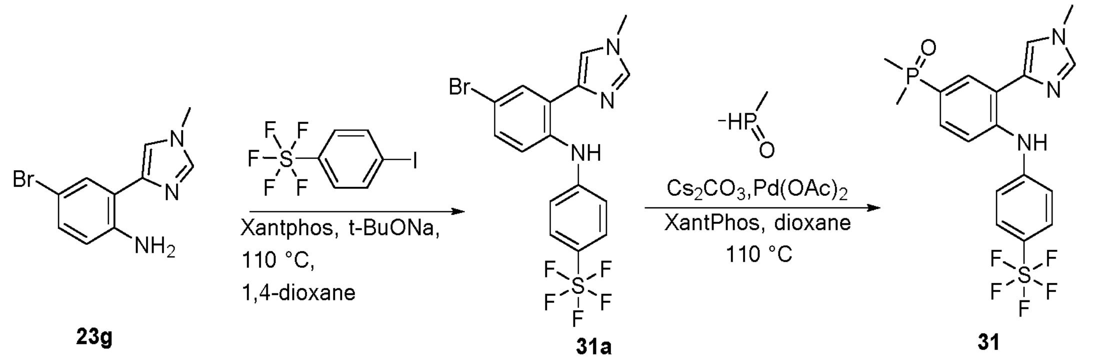


23g 31a 31

Step 1: (compound 31a)

Compound 23g (100 mg, 0.40 mmol), 4-iodophenylsulphur pentafluoride (261.83 mg, 0.79 mmol), chloro[(4,5-bis(diphenylphosphino)-9,9-dimethylxanthene)-2-(2'-amino-1,1'-biphenyl)]palladium(II) (35.22 mg, 0.04 mmol), and sodium *tert*-butoxide (76.8 mg, 0.80 mmol) were added to 1,4-dioxane (15 mL). The mixture was purged three times with nitrogen, and then heated to 110 °C and stirred overnight under a nitrogen atmosphere. After the starting materials were consumed completely as shown by LC-MS, the reaction solution was cooled to room temperature and concentrated under reduced pressure, and the residue was purified by column chromatography to give 31a as a brown solid (160 mg, yield: 89%). LCMS(ESI)m/z=455.9$[M+H]^+$

Step 2: (compound 31)

Compound 31a (100 mg, 0.22 mmol), dimethylphosphine oxide (43.04 mg, 0.55 mmol), cesium carbonate (107.25 mg, 0.33 mmol), Xantphos (50.92 mg, 0.08 mmol), and palladium(II) acetate (9.85 mg, 0.04 mmol) were added to 1,4-dioxane (10 mL). The mixture was purged three times with nitrogen, and then heated to 110 °C and stirred overnight under a nitrogen atmosphere. The reaction solution was cooled to room temperature and concentrated under reduced pressure, and the residue was purified by column chromatography to give 31 as a white solid (65 mg, yield: 65.59%). LCMS(ESI)m/z=452.1$[M+H]^+$. $^1$H NMR (400 MHz, DMSO-d6): $\delta$ 10.76 (s, 1H), 8.03 (d, J=11.6 Hz, 1H), 7.85 (s, 1H), 7.78-7.34 (m, 3H), 7.54 (d, J=6.0 Hz, 2H), 7.25 (d, J=8.8 Hz, 2H), 3.74 (s, 3 H), 1.69 (s, 3 H), 1.65 (s, 3 H).

Example 32: (Compound 32)

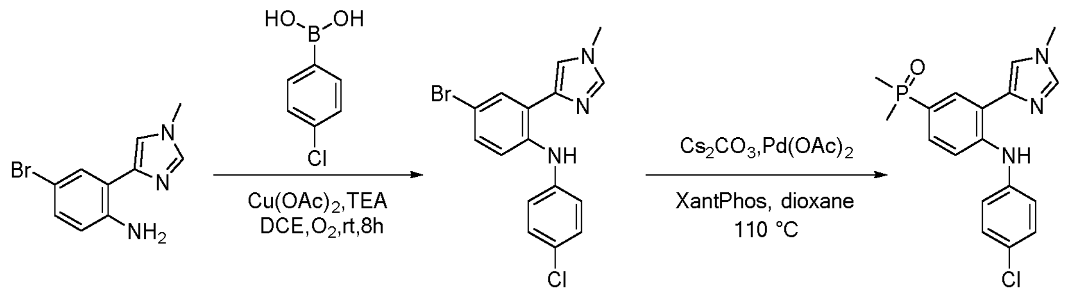


23g 32a 32

Step 1: (compound 32a)

Compound 23g (300 mg, 1.18 mmol), 4-chlorophenylboronic acid (185 mg, 1.18 mmol), triethylamine (120 mg, 1.18 mmol), and cupric acetate (22 mg, 0.12 mmol) were added to 1,2-dichloroethane (20 mL). The mixture was purged three times with oxygen and stirred at room temperature for 8 h under an oxygen atmosphere. After the starting materials were consumed completely as shown by LC-MS, the reaction solution was concentrated under reduced pressure, and the residue was purified by column chromatography to give 32a as a yellow solid (129 mg, yield: 30.3%). LCMS(ESI) m/z=362.1$[M+H]^+$

Step 2: (compound 32)

Compound 32a (129 mg, 0.35 mmol), dimethylphosphine oxide (56 mg, 0.71 mmol), cesium carbonate (117 mg, 0.35 mmol), Xantphos (83 mg, 0.14 mmol), and palladium(II) acetate (16 mg, 0.07 mmol) were added to 1,4-dioxane (10 mL). The mixture was purged three times with nitrogen, and then heated to 110 °C and stirred overnight under a nitrogen atmosphere. After the starting materials were consumed completely as shown by LC-MS, the reaction solution was cooled to room temperature and concentrated under reduced pressure, and the residue was purified by column chromatography to give 32 as a white solid (52 mg, yield: 40.9%). LCMS(ESI)m/z=360.1[M+H]$^+$. $^1$H NMR (400 MHz, DMSO-d6): δ 10.62 (s, 1H), 7.94-7.91 (m, 1H), 7.83 (d, J=0.8 Hz, 1H), 7.79 (d, J=1.2 Hz, 1H), 7.47-7.42 (m, 1H), 7.35-7.30 (m, 3H), 7.22-7.19 (m, 2H), 3.74 (s, 3 H), 1.64 (s, 3 H), 1.61 (s, 3 H).

Example 33: (Compound 34)

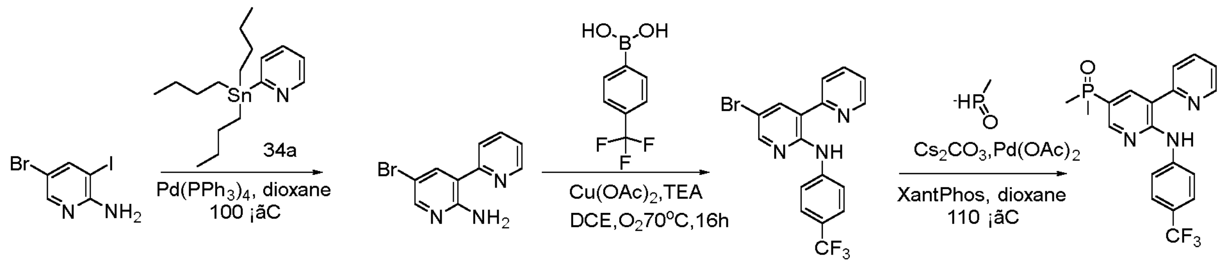


8a 34b 34c 34

Step 1: (compound 34b)

Compound 8a (1.0 g, 3.34 mmol), compound 34a (1.23 g, 3.34 mmol), and tetrakis(triphenylphosphine) palladium(0) (0.37 g, 0.03 mmol) were added to 1,4-dioxane (40 mL). The mixture was purged three times with nitrogen, and then heated to 100 °C and stirred overnight under a nitrogen atmosphere. Some of the starting materials were not consumed completely as shown by LC-MS. The reaction solution was cooled to room temperature and concentrated under reduced pressure, and the residue was purified by column chromatography (PE/EA = 5:1 to 3:1) to give 34b as a yellow solid (250 mg, yield: 30%). LCMS(ESI)m/z=252.0[M+H]$^+$.

Step 2: (compound 34c)

Compound 34b (250 mg, 1.0 mmol), 4-(trifluoromethyl)phenylboronic acid (303.84 mg, 1.6 mmol), triethylamine (101.19 mg, 1.0 mmol), and cupric acetate (18.2 mg, 0.1 mmol) were added to 1,2-dichloroethane (10 mL). The mixture was purged three times with oxygen, and then heated to 70 °C and stirred for 16 h under an oxygen atmosphere. The reaction solution was concentrated under reduced pressure, and the residue was purified by column chromatography to give 34c as a yellow solid (180 mg, yield: 4.7%). LCMS(ESI)m/z=394.0[M+H]$^+$.

Step 3: (compound 34)

Compound 34c (100 mg, 0.25 mmol), dimethylphosphine oxide (49.5 mg, 0.63 mmol), cesium carbonate (121.87 mg, 0.38 mmol), Xantphos (57.86 mg, 0.1 mmol), and palladium(II) acetate (11.2 mg, 0.05 mmol) were added to 1,4-dioxane (10 mL). The mixture was purged three times with nitrogen, and then heated to 110 °C and stirred overnight under a nitrogen atmosphere. The reaction solution was cooled to room temperature and concentrated under reduced pressure, and the residue was purified by column chromatography to give 34 as a white solid (45 mg, yield: 45.35%). LCMS(ESI) m/z=392.1[M+H]$^+$. $^1$HNMR (400 MHz, DMSO-d6): δ 12.68 (s, 1H), 8.86-8.85 (m, 1H), 8.63-8.62 (s, 1H), 8.56-8.52 (s, 1H), 8.26 (d, J=8.0 Hz, 1H), 8.10-8.04 (m, 3H), 7.68 (d, J=8.4 Hz, 2H), 7.55-7.52 (m, 1H), 1.78 (s, 3 H), 1.74 (s, 3 H).

Example 34: (Compound 35)

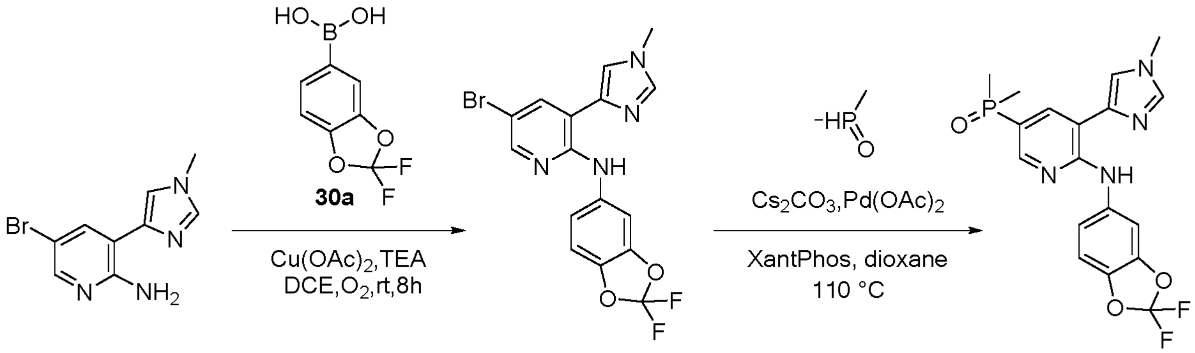


8b 35a 35

Step 1: (compound 35a)

Compound 8b (300 mg, 1.18 mmol), compound 30a (238 mg, 1.18 mmol), triethylamine (120 mg, 1.18 mmol), and cupric acetate (22 mg, 0.12 mmol) were added to 1,2-dichloroethane (20 mL). The mixture was purged three times with oxygen and stirred at room temperature for 8 h under an oxygen atmosphere. The reaction solution was concentrated under reduced pressure, and the residue was purified by column chromatography to give 35a as a yellow solid (185 mg, yield: 38.5%). LCMS(ESI)m/z=409.1$[M+H]^+$.

Step 2: (compound 35)

Compound 35a (143 mg, 0.35 mmol), dimethylphosphine oxide (56 mg, 0.71 mmol), cesium carbonate (117 mg, 0.35 mmol), Xantphos (83 mg, 0.14 mmol), and palladium(II) acetate (16 mg, 0.07 mmol) were added to 1,4-dioxane (10 mL). The mixture was purged three times with nitrogen, and then heated to 110 °C and stirred overnight under a nitrogen atmosphere. The reaction solution was cooled to room temperature and concentrated under reduced pressure, and the residue was purified by column chromatography to give 35 as a white solid (92.5 mg, yield: 65.1%). LCMS(ESI)m/z=407.1 $[M+H]^+$. $^1$H NMR (400 MHz, DMSO-d6): δ 12.00 (s, 1H), 8.39-8.36 (m, 1H), 8.22-8.19 (m, 1H), 8.15(d, J=2.4 Hz, 1H), 8.01 (d, J=1.2 Hz, 1H), 7.93 (d, J=0.8 Hz, 1H), 7.35-7.25 (m, 2H), 3.78 (s, 3 H), 1.71 (s, 3 H), 1.67 (s, 3 H).

Example 35: (Compound 36)

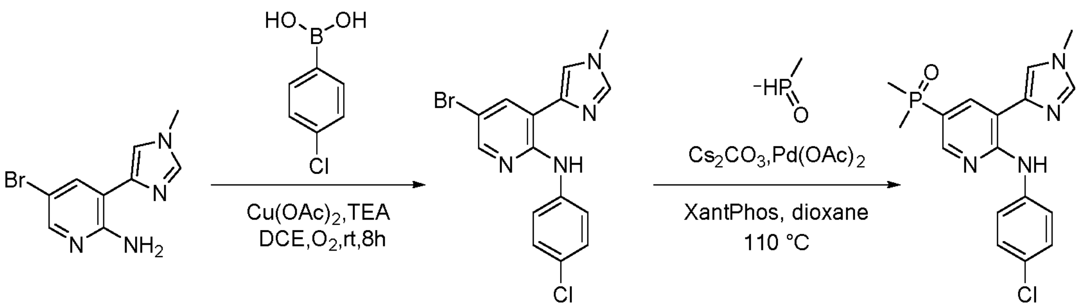


8b 36a 36

Step 1: (compound 36a)

Compound 8b (225 mg, 0.89 mmol), 4-chlorophenylboronic acid (223 mg, 1.42 mmol), triethylamine (90 mg, 0.89 mmol), and cupric acetate (16 mg, 0.09 mmol) were added to 1,2-dichloroethane (40 mL). The mixture was purged three times with oxygen and stirred at room temperature for 8 h under an oxygen atmosphere. After the starting materials were consumed completely as shown by LC-MS, the reaction solution was concentrated under reduced pressure, and the residue was purified by column chromatography to give 36a as a yellow solid (180 mg, yield: 55.5%). LCMS(ESI) m/z=363.0$[M+H]^+$.

Step 2: (compound 36)

Compound 36a (100 mg, 0.27 mmol), dimethylphosphine oxide (43 mg, 0.55 mmol), cesium carbonate (90 mg, 0.27 mmol), Xantphos (64 mg, 0.11 mmol), and palladium(II) acetate (11 mg, 0.05 mmol) were added to 1,4-dioxane (5 mL). The mixture was purged three times with nitrogen, and then heated to 110 °C and stirred overnight under a nitrogen atmosphere. After the starting materials were consumed completely as shown by LC-MS, the reaction solution was cooled to room temperature and concentrated under reduced pressure, and the residue was purified by column chromatography to give 36 as a white solid (48 mg, yield: 48.4%). LCMS(ESI)m/z=361.0[M+H]$^+$. $^1$H NMR (400 MHz, DMSO-d6): δ 11.97 (s, 1H), 8.38-8.36 (m, 1H), 8.21-8.18 (m, 1H), 8.00 (d, J=1.2 Hz, 1H), 7.94 (d, J=0.8 Hz, 1H), 7.82-7.80 (m, 2H), 7.37-7.35 (m, 2H), 3.78 (s, 3 H), 1.70 (s, 3 H), 1.67 (s, 3 H).

Example 36: (Compound 37)

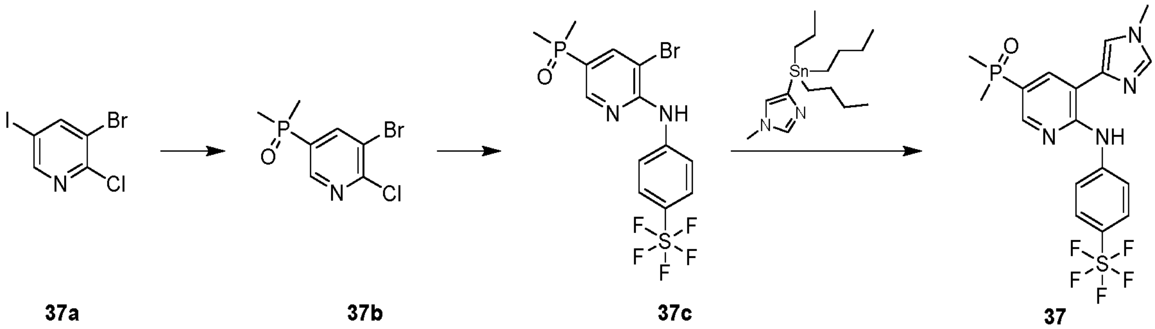

37a 37b 37c 37

Step 1: (compound 37b)

Compound 37a (4.0 g, 12.6 mmol) was dissolved in 1,4-dioxane (40 mL), and dimethylphosphine oxide (1.5 g, 18.9 mmol), 4,5-bis(diphenylphosphino)-9,9-dimethylxanthene (1.5 g, 2.52 mmol), triethylamine (3.8 g, 37.8 mmol), and tris(dibenzylideneacetone)dipalladium(0) (2.3 g, 2.52 mmol) were added. The mixture was then stirred at 50 °C for 16 h under a nitrogen atmosphere. After the reaction was completed as shown by TLC, the reaction solution was cooled to room temperature and filtered, and the filtrate was diluted with water (100 mL) and extracted with a mixed solution of 10% methanol in ethyl acetate (100 mL × 6). The organic phases were combined, dried over anhydrous sodium sulfate, filtered, and desolventized under reduced pressure, and the residue was purified by silica gel column chromatography to give a crude product (6 g), which was subjected to reversed-phase preparative chromatography to give compound 37b (1 g, yellow solid, yield: 29.64%). MS (ESI): m/z =267.8 [M+1]$^+$

Step 2: (compound 37c)

Compound 37b (500 mg, 1.86 mmol) was dissolved in dimethyl sulfoxide (10 mL), and 4-aminophenylsulfur pentafluoride (490 mg, 2.23 mmol) and cesium carbonate (1.2 g, 3.72 mmol) were added. The mixture was stirred at 110 °C for 16 h under a nitrogen atmosphere. After the reaction was completed as shown by TLC, the reaction solution was diluted with water (100 mL) and extracted with ethyl acetate (50 mL × 3). The organic phases were combined, washed with saturated brine (50 mL × 2), dried over anhydrous sodium sulfate, filtered, and desolventized under reduced pressure, and the residue was purified by silica gel column chromatography to give compound 37c (450 mg, yellow oil, yield: 53.55%). MS (ESI): m/z = 450.9 [M+1]$^+$

Step 3: (compound 37)

Compound 37c (50 mg, 0.11 mmol) was dissolved in 1,4-dioxane (2 mL), and *N*-methyl-4-(tributylstannyl) imidazole (41 mg, 0.11 mmol), cuprous iodide (3 mg, 0.02 mmol), and tetrakis(triphenylphosphine)palladium(0) (12.8 mg, 0.01 mmol) were added. The mixture was stirred at 80 °C for 16 h under a nitrogen atmosphere. After the reaction was completed as shown by TLC, the reaction solution was cooled to room temperature, diluted with water (10 mL), and extracted with ethyl acetate (8 mL × 3). The organic phases were combined, washed with saturated brine (5 mL × 2), and desolventized under reduced pressure, and the residue was separated by prep-TLC to give 37 (5 mg, green solid). MS (ESI): m/z = 452.9 [M+1]$^+$. $^1$H NMR (400 MHz, Chloroform-d) δ(ppm) 11.96 (s, 1H), 8.29 (d, J = 5.5 Hz, 1H), 8.24 - 8.16 (m,

1H), 7.89 (d, J = 8.7 Hz, 2H), 7.68 (d, J = 8.8 Hz, 2H), 7.57 (s, 1H), 7.46 (s, 1H), 3.80 (s, 3H), 1.79 (s, 3H), 1.76 (s, 3H).

Example 37: (Compound 38)

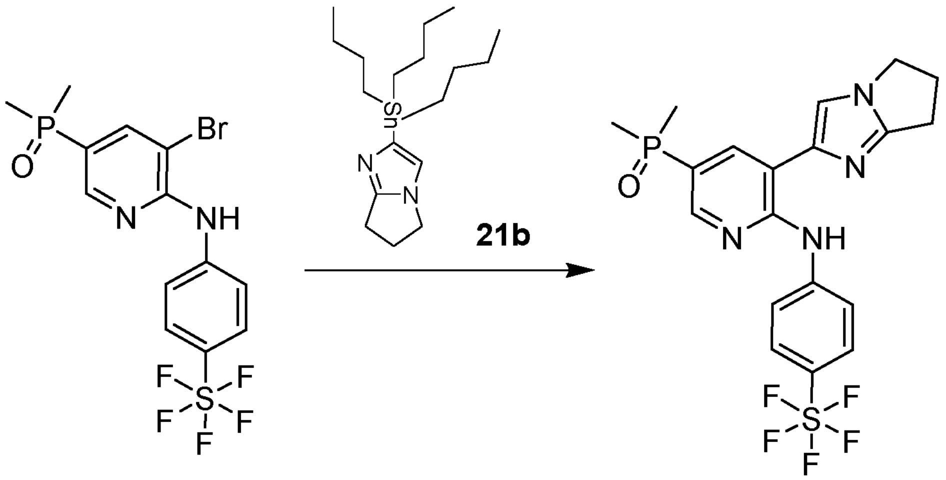


37c

38

Compound 37c (100 mg, 0.22 mmol) was dissolved in 1,4-dioxane (5 mL), and compound 21b (146 mg, 0.22 mmol, 60%), cuprous iodide (6 mg, 0.03 mmol), and tetrakis(triphenylphosphine)palladium(0) (25.6 mg, 0.02 mmol) were added. The mixture was stirred at 80 °C for 16 h under a nitrogen atmosphere. After the reaction was completed as shown by TLC, the reaction solution was cooled to room temperature, diluted with water (15 mL), and extracted with ethyl acetate (10 mL × 3). The organic phases were combined, washed with saturated brine (8 mL × 2), and desolventized under reduced pressure, and the residue was separated by prep-TLC to give a crude product (100 mg). The crude compound was further purified by HPLC to give compound 38 (20 mg, off-white solid). MS (ESI): m/z = 479.1 $[M+1]^+$. $^1H$ NMR (300 MHz, DMSO-$d_6$) δ(ppm) 12.42 (s, 1H), 8.50 - 8.35 (m, 1H), 8.24 (d, J = 11.1 Hz, 1H), 8.07 - 7.91 (m, 3H), 7.83 (d, J = 8.9 Hz, 2H), 4.09 (t, J = 7.2 Hz, 2H), 2.94 (t, J = 7.4 Hz, 2H), 2.59 (t, J = 7.5 Hz, 2H), 1.73 (s, 3H) 1.68 (s, 3H).

Example 38: (Compound 39)

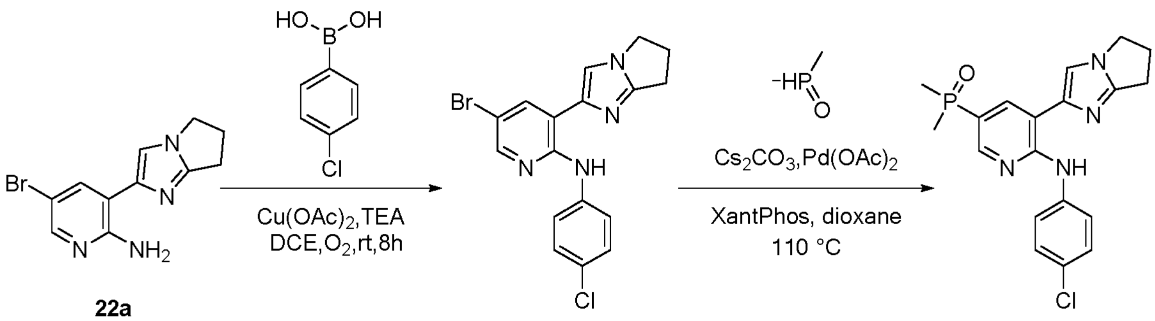


22a

39a

39

Step 1: (compound 39a)

Compound 22a (182 mg, 0.64 mmol), 4-chlorophenylboronic acid (164 mg, 1.04 mmol), triethylamine (68 mg, 0.64 mmol), and cupric acetate (24 mg, 0.02 mmol) were added to 1,2-dichloroethane (10 mL). The mixture was purged three times with oxygen and stirred at room temperature for 8 h under an oxygen atmosphere. After the starting materials were consumed completely as shown by LC-MS, the reaction solution was concentrated under reduced pressure, and the residue was purified by column chromatography to give 39a as a yellow solid (130 mg, yield: 51.1%). LCMS(ESI)m/z=389.1[M+H]+

Step 2: (compound 39)

Compound 39a (130 mg, 0.33 mmol), dimethylphosphine oxide (53 mg, 0.67 mmol), cesium carbonate (109 mg, 0.33 mmol), Xantphos (78 mg, 0.13 mmol), and palladium(II) acetate (15 mg, 0.06 mmol) were added to 1,4-dioxane (15 mL). The mixture was purged three times with nitrogen, and then heated to 110 °C and stirred overnight under a nitrogen atmosphere. After the starting materials were consumed completely as shown by LC-MS, the reaction solution was cooled to room temperature and concentrated under reduced pressure, and the residue was purified by column chromatography to give 39 as a brown solid (80 mg, yield: 62.0%). LCMS(ESI)m/z=387.0[M+H]+. $^1$H NMR (400 MHz, DMSO-d6): δ 12.04 (s, 1H), 7.35 (d, J=4.4 Hz, 1H), 8.18-8.15 (m, 1H), 7.94 (s, 1H), 7.81 (d, J=8.8 Hz, 2H), 7.36 (d, J=8.4 Hz, 2H), 4.10-4.06 (m, 2H), 2.94-2.90 (m, 2H), 2.60-2.56 (m, 2 H), 1.70 (s, 3 H), 1.66 (s, 3 H).

Example 39: (Compound 40)

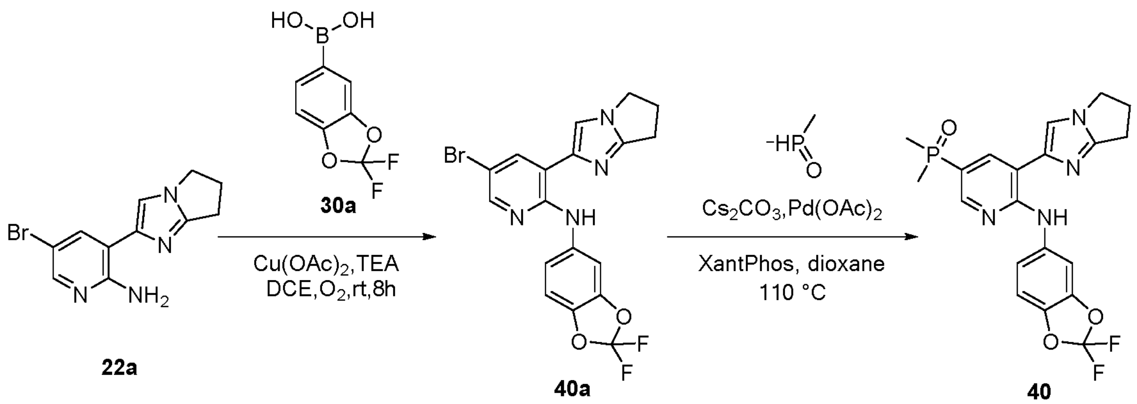


30a

22a

40a

40

Step 1: (compound 40a)

Compound 22a (182 mg, 0.64 mmol), compound 30a (210.1 mg, 1.04 mmol), triethylamine (68 mg, 0.64 mmol), and cupric acetate (24 mg, 0.02 mmol) were added to 1,2-dichloroethane (10 mL). The mixture was purged three times with oxygen and stirred at room temperature for 8 h under an oxygen atmosphere. The reaction solution was concentrated under reduced pressure, and the residue was purified by column chromatography to give 40a as a yellow solid (130 mg, yield: 46.9%). LCMS(ESI)m/z=435.1[M+H]+

Step 2: (compound 40)

Compound 40a (130 mg, 0.33 mmol), dimethylphosphine oxide (53 mg, 0.67 mmol), cesium carbonate (109 mg, 0.33 mmol), Xantphos (78 mg, 0.13 mmol), and palladium(II) acetate (15 mg, 0.06 mmol) were added to 1,4-dioxane (15 mL). The mixture was purged three times with nitrogen, and then heated to 110 °C and stirred overnight under a nitrogen atmosphere. The reaction solution was cooled to room temperature and concentrated under reduced pressure, and the residue was purified by column chromatography to give 40 as a brown solid (65 mg, yield: 45.8%). LCMS(ESI)m/z=433.1 [M+H]+. $^1$H NMR (400 MHz, DMSO-d6): δ 12.09 (s, 1H), 8.37-8.35 (m, 1H), 8.19-8.16 (m, 2H), 7.94(s, 1H), 7.34 (d, J=8.8 Hz, 1H), 7.26-7.23 (m, 1H), 4.10-4.06 (m, 2H), 2.94-2.90 (m, 2H), 2.60-2.56 (m, 2 H), 1.70 (s, 3 H), 1.67 (s, 3 H).

Example 40: (Compound 41)

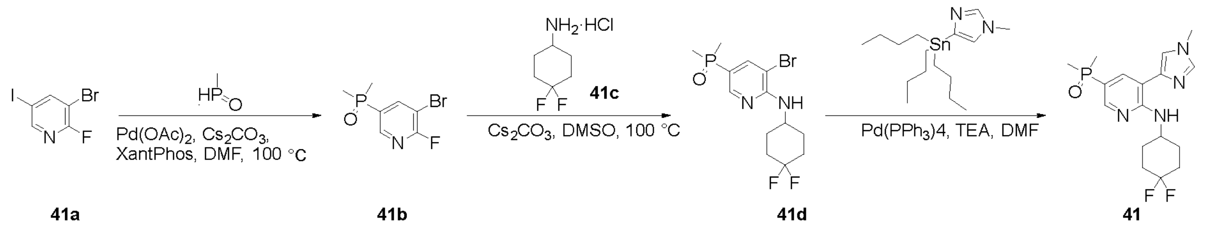


41a 41b 41d 41

Step 1: (compound 41b)

Compound 41a (1 g, 3.14 mmol), dimethylphosphine oxide (245 mg, 3.14 mmol), cesium carbonate (2 g, 6.28 mmol), palladium(II) acetate (78 g, 0.35 mmol), and XantPhos (382 g, 0.66 mmol) were added to a solution of DMF (5 mL). The mixture was purged three times with argon, heated to 100 °C, and stirred for 16 h. After the reaction was completed as shown by LCMS, water (50 mL) was added to the reaction solution, and the mixture was extracted with ethyl acetate (20 mL × 3). The organic phases were combined, washed with saturated brine, dried over anhydrous sodium sulfate, filtered, and concentrated to give a product of 41b as a brown oil (0.3 g, yield: 51%).

Step 2: (compound 41d)

Compound 41b (300 mg, 2.64 mmol), compound 41c (0.36 g, 2.64 mmol), and cesium carbonate (1.7 g, 5.28 mmol) were added to a solution of DMSO (3 mL), and the mixture was purged with argon for 1 min, heated to 100 °C, and stirred for 16 h. After the reaction was completed as shown by LCMS, the reaction solution was concentrated and purified by column chromatography to give a product of 41d as an off-white solid (0.5 g, purity: 78%). LCMS(ESI)m/z = 367.1 $[M+H]^+$

Step 3: (compound 41)

Compound 41d (150 mg, 0.45 mmol), N-methyl-4-(tributylstannyl)imidazole (330 mg, 0.90 mmol), tetrakis(triphenylphosphine)palladium(0) (52 mg, 0.045 mmol), and triethylamine (140 mg, 1.35 mmol) were added to a DMF (2 mL) solution. After the reaction was completed as shown by LCMS, the reaction solution was removed under vacuum, and the residue was subjected to column chromatography to give a product of 41 as a white solid (130 mg, yield: 86%, purity: 100%). LCMS(ESI)m/z = 369.2 $[M+H]^+$. $^1H$ NMR (400 MHz, DMSO-d6) δ 9.34 (d, J = 7.2 Hz, 1H), 8.24 (dd, J = 6.1, 2.1 Hz, 1H), 7.99 (dd, J = 10.9, 2.1 Hz, 1H), 7.85 (dd, J = 10.8, 1.3 Hz, 2H), 4.24 (d, J = 9.0 Hz, 1H), 3.76 (s, 3H), 2.13 -1.95 (m, 8H), 1.67 (s, 3H), 1.64 (s, 3H).

**Efficacy Experimental Examples**

The structure of comparative compound B1 is

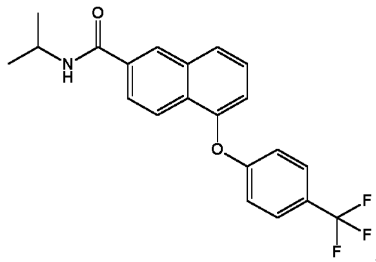

,

and the structure of comparative compound B2 is

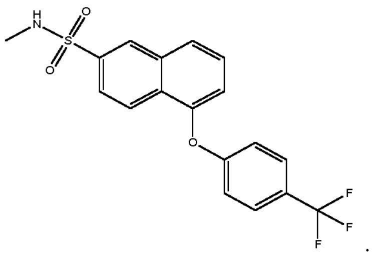

Comparative compound B1 and comparative compound B2 were synthesized with reference to the synthetic routes described in Patent WO2020214734A1. The structure of comparative compound B3 (VT103) is

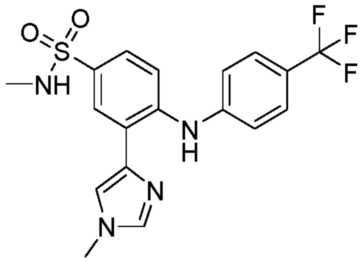

(CAS No.: 2290608-13-6, synthesized with reference to the synthetic route described in Patent WO2019040380A1). The structure of comparative compound B4 (MRK-A) was

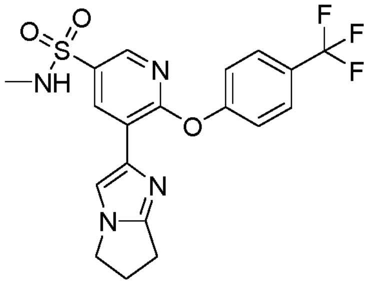

(CAS No.: 2821763-12-4, purchased from: Jiangsu Aikon Biopharmaceutical R&D Co., Ltd., batch No.: AK23-1338803-1-1).

Efficacy Experimental Example 1:

YAP-TEAD reporter gene

The YAP-TEAD reporter gene assay was performed as follows. Firstly, the 8xGTIIC promoter sequence (GAGCTCT TACGCGTGCTAGCCCGGCCAGTGCCAAGTTGAGACACATTCCACACATTCCACTGC AAGCTTGAGACACATTCC ACACATTCC ACTGCAAGCTTGGCCAGTGCCAAGTTGAGACACATTCCACACATTCCACTGCAAGCTTGAGAC AC ATTCCACACATTCCACTGCAAGCTT CTAGAGATCTGCAGGTCGAGGTCGACGGTATCGATAAGCTTGGGGGTGG GCGCCGGGGGGAC CTTAAAGCCTCTGCCCCCCAAGGAGCCC TTCCCAGACAGCCGCCGGCACCCACCGCTC CGTGGGACGATCCCCCAAGAGCTTGGCATTCCG GTACTGTTGGTAAA) was constructed into a pGL4.20(luc2-Puro) plasmid, and the TK promoter (AAATGAGTCTTCGGACCTCGCGGGGGCCGCTTAAGCGGTGGTTAGGGTTTGTCT GACGCGGG GGGAGGGGGAAGGAACGAAACACTCTCA TTCGGAGGCGGCTCGGGGTTTGGTCTTGGTGGCCA CGGGCACGCAGAAGAGCGCCGCGATCC TCTTAAGCACCCCCCCGCCCTCCGTGGA GGCGGGGGTTTGGTCG GCGGGTGGTAACTGGCGGGCCGCTGACTCGGGCGGGTCGCGCGCCC CAGAGTGTGACCTTTTCGGTCTGCTC GC AGACCCCCGGGCGGCGCCGCCGCGGCGGCGACGGGCTCGCTGGGTCCTAGGCTCCATGGGGA CCGTATA CGTGGACAGGCTCTGGAGCAT CCGCACGACTGCGGTGATATTACCGGAGACCTTCTGCGGGACGAGCCGGGTC ACGCGGCTGAC GCGGAGCGTCCGTTGGGCGACAAACAC CAGGACGGGGCACAGGTACACTATCTTGTCACCC GGAGGCGCGAGGGACTGCAGGAGCTTCA GGGAGTGGCGCAGCTGCTTCATCCCCGT GGCCCGTTGCTCGCG TTTGCTGGCGGTGTCCCCGGAAGAAATATATTTGCATGTCTTTAGTTCTA TGATGACACAAACCCCGCCCAGCGT CTTGTCATTGGCGAATTCGAACACGCAGATGCAGTCGGGGCGGCGCGGTCCCAGGTCCACTTC GCATATTAAGG

TGACGCGTGTGGCCTC GAACACCGAGCGACCCTGCAGCGACCCGCTTAA) unrelated to the hippo pathway was constructed into a pGL4.78(hRlucCP/Hygro) plasmid, thereby constructing stably transfected 293T cells that can stably and continuously express the transcriptional activity of the YAP-TEAD transcription complex. After selection and identification of individual clones, the clones were grown and maintained in a DMEM medium containing 10% fetal bovine serum, penicillin-streptomycin solution, 1 μg/mL puromycin, and 150 μg/mL hygromycin. To perform reporter gene assays with the compounds to be tested, the well-growing cells described above were plated in a 384-well plate at a cell density of 3000 cells per well, with 90 μL per well. The periphery was sealed with an appropriate amount of PBS to prevent evaporation of water from the peripheral wells. Administration was performed on the next day after the cell plating. The test compounds were added at different concentration gradients, and three replicate wells were set for each concentration point. The compound concentration started at 5 μM and was diluted seven times with a 5-fold gradient. A corresponding negative control group treated with DMSO was also set up. After 24 h of drug treatment, the detection reagents were prepared first. The Dual-Lumi™ Firefly luciferase detection reagent and Dual-Lumi™ Renilla luciferase detection buffer were dissolved and brought to room temperature. The Dual-Lumi™ Renilla luciferase detection substrate (100X) was placed in an ice bath or an ice box for later use. According to the amount of 100 μL of Dual-Lumi™ Renilla luciferase detection working solution required for the detection of each sample, an appropriate amount of Dual-Lumi™ Renilla luciferase detection working solution was prepared. The Dual-Lumi™ Renilla luciferase detection substrate (100X) and the Dual-Lumi™ Renilla luciferase detection buffer were mixed in a ratio of 1:100 to prepare the Dual-Lumi™ Renilla luciferase detection working solution. The cell culture plate was taken out and allowed to equilibrate at room temperature for 10 min (usually no more than 30 min). Then, 100 μL of Dual-Lumi™ Firefly luciferase detection reagent was added to each well of a 96-well plate (25 μL per well for the 384-well plate), and the mixture was mixed well. The mixture was then incubated at room temperature (about 25 °C) for 10 min to stabilize the luminescent signal, and then chemiluminescence was detected using a multimode microplate reader with a chemiluminescence detection function. Subsequently, the detection of Renilla luciferase began. Firstly, 100 μL of Dual-Lumi™ Renilla luciferase detection working solution was added to each well of a 6-well plate (25 μL per well for the 384-well plate), and the mixture was mixed well. The mixture was then incubated at room temperature (about 25 °C) for 10 min to stabilize the luminescent signal. Finally, chemiluminescence was detected using a multimode microplate reader with a chemiluminescence detection function. The obtained data were analyzed using GraphPad Prism 7.0 software. A sigmoidal dose-survival rate curve was plotted using a non-linear regression model, and the $IC_{50}$ values were calculated. The test results are shown in Table 1:

Table 1

| Example No. | $IC_{50}$ (nM) |
|---|---|
| Example 1 | 39.71 |
| Example 4 | 123.4 |
| Example 5 | 57.07 |
| Example 7 | 4.09 |
| Example 8 | 42.38 |
| Example 9 | 160 |
| Example 10 | 100.4 |
| Example 12 | 92.13 |
| Example 13 | 343.6 |
| Example 14 | 20.24 |
| Example 16 | 31.75 |
| Example 17 | 11.81 |
| Example 20 | 438.8 |
| Example 21 | 7.46 |
| Example 22 | 13.64 |
| Example 23 | 27.42 |
| Example 24 | 134.9 |
| Example 28 | 283.2 |
| Example 29 | 4.65 |

(continued)

| Example No. | $IC_{50}$ (nM) |
|---|---|
| Example 30 | 23.71 |
| Example 31 | 61.85 |
| Example 32 | 78.5 |
| Example 33 | 19.76 |
| Example 34 | 190.9 |
| Example 36 | 39.16 |
| Example 37 | 5.93 |
| Example 38 | 28.7 |
| Example 39 | 32.44 |

Efficacy Experimental Example 2: Inhibition of Cell Proliferation

NCI-H2052 (NF2 mutant), NCI-H226 (NF2 mutant), NCI-H2452 (NF2 wide-type), NCI-H28 (NF2 wide-type), and MSTO-211H (NF2 wide-type) cells were cultured in RPMI 1640 complete media.

The normally growing cells described above were digested with a trypsin cell-digesting solution, centrifuged, counted, and then plated in a 96-well plate at a density of 1000 cells per well. Administration was performed 24 h after the cell plating. Inhibitors at different concentration gradients were added at 10 μL per well, and three replicate wells were set for each concentration point. The starting concentration was 20 μM and diluted 8 times with a 4-fold gradient. A corresponding negative control group treated with 0.1% DMSO was also set up. After 72 h of drug treatment, the cell culture plate to be tested was taken out of the incubator. The culture medium in the 96-well plate was pipetted, and 190 μL of fresh RPMI 1640 complete medium was added. Another administration was performed, and the cells were cultured for another 72 h. The 96-well plate was taken out and allowed to equilibrate at room temperature for 10 min. The medium in the 96-well plate was pipetted, and 200 μL of CellTiter-Lumi™ luminescent cell viability detection solution (mixed with a medium in a ratio of 1:1) was added. After shaking for two minutes, the mixture was reacted at room temperature for 10 min. Then, 150 μL of liquid was pipetted from the transparent 96-well plate and transferred to a white 96-well plate for chemiluminescence reading. The background value was subtracted from the value for each well to calculate the cell survival rate. Survival rate (%) = (Sample/Vehicle - 1) × 100. Sample is the chemiluminescence of the drug-treated group, and Vehicle is the absorbance of the DMSO control group. A sigmoidal dose-survival rate curve was plotted using a non-linear regression model in the GraphPad Prism 7.0 software, and the $IC_{50}$ values or $GI_{50}$ values were calculated. The test results are shown in Table 2:

Table 2

| Example No. | NCI-H2052 (NF2 mutant) $IC_{50}$ (nM) | NCI-H226 (NF2 mutant) $IC_{50}$ (nM) | NCI-H2452 (NF2 wide-type) $IC_{50}$ (μM) | NCI-H28 (NF2 wide-type) $IC_{50}$ (μM) | MSTO-211H (NF2 wide-type) $IC_{50}$ (μM) |
|---|---|---|---|---|---|
| Example 1 | 14 | 25 | >5 | >5 | >5 |
| Example 2 | 277 | 740 | >5 | >5 | >20 |
| Example 4 | 214 | 394 | >5 | >1.25 | >1.25 |
| Example 5 | 27 | 88 | >20 | >1.25 | >5 |
| Example 7 | 39 | 50 | >5 | >5 | >1.25 |
| Example 8 | 20 | 56 | >5 | 1.6 | >5 |
| Example 9 | 75 | 119 | >5 | >5 | >1.25 |
| Example 10 | 51 | 86 | >5 | >1.25 | >1.25 |
| Example 12 | 91 | 121 | >20 | >5 | >20 |
| Example 13 | 398 | 442 | >20 | >5 | >20 |
| Example 14 | 118 | 132 | >5 | >1.25 | >5 |

(continued)

| Example No. | NCI-H2052 (NF2 mutant) $IC_{50}$ (nM) | NCI-H226 (NF2 mutant) $IC_{50}$ (nM) | NCI-H2452 (NF2 wide-type) $IC_{50}$ ($\mu$M) | NCI-H28 (NF2 wide-type) $IC_{50}$ ($\mu$M) | MSTO-211H (NF2 wide-type) $IC_{50}$ ($\mu$M) |
|---|---|---|---|---|---|
| Example 16 | 22 | 48 | >5 | 0.876 | 0.112 |
| Example 17 | 10 | 28 | >1.25 | 0.958 | - |
| Example 18 | 791 | - | >1.25 | >20 | >1.25 |
| Example 19 | 176 | 250 | >5 | >5 | 0.628 |
| Example 20 | 794 | 161 | >20 | >20 | >1.25 |
| Example 21 | 5.1 | 11 | >5 | 0.24 | - |
| Example 22 | 4 | 11 | >5 | >1.25 | - |
| Example 23 | 9.9 | 25 | >5 | >5 | 0.192 |
| Example 24 | 45 | 43 | >5 | >5 | |
| Example 25 | | 214 | >20 | >20 | |
| Example 26 | 478 | 431 | >5 | >5 | |
| Example 27 | | 425 | >20 | >5 | |
| Example 28 | 164 | 342 | >50 | >20 | |
| Example 29 | 6.7 | 4 | >1.25 | >1.25 | |
| Example 30 | 27 | 19 | >5 | >5 | |
| Example 32 | 61 | 32 | >5 | >5 | |
| Example 33 | 91 | 29 | >5 | >5 | |
| Example 34 | 156 | 97 | >5 | >5 | |
| Example 35 | 87 | 53 | >5 | >5 | |
| Example 36 | 28 | 17 | >20 | >20 | |
| Example 37 | 11 | 6 | >1.25 | >1.25 | |
| Example 38 | 14 | 10 | >1.25 | >1.25 | |
| Example 39 | 30 | 19 | >1.25 | >1.25 | |

Efficacy Experimental Example 3: Stability Test in Liver Microsomes

Phase I metabolic stability of the test compounds in liver microsomes of CD-1 mice, Sprague-Dawley rats, and humans was assessed.

**Experimental system:**

The animal and human liver microsomes used in the test system were purchased from Xenotech, Corning, or other qualified suppliers and stored in a freezer at a temperature below -60 °C prior to use. The information of the animal and human liver microsomes used was as follows:

| Type | Product information | Manufacturer | Abbreviation |
|---|---|---|---|
| Human | Cat No. 452117<br>Lot No. 3 8297 | Corning | HLM |
| SD rat | Cat No. R1000<br>Lot No. 2110178 | Xenotech | RLM |
| CD-1 mouse | Cat No. M1000<br>Lot No. 2110092 | Xenotech | MLM |

**Brief introduction of the experiment:**

The test samples and the reference compounds were separately incubated with animal and human liver microsomes at 37±1 °C for a period of incubation time of up to 60 min. The samples were taken out at the indicated time points, and the reaction was stopped with acetonitrile or another organic solvent containing an internal standard. After centrifugation, the resulting supernatants were assayed by the liquid chromatography-tandem mass spectrometry (LC-MS/MS) method.

**Experimental method:**

1. Preparation of buffer

73.21 g of dipotassium hydrogen phosphate trihydrate and 10.78 g of potassium dihydrogen phosphate were dissolved in 4000 mL of ultrapure water. The solution was adjusted to pH 7.40±0.10 with 10% phosphoric acid or 1 M potassium hydroxide to make a final concentration of 100 mM.

2. Preparation of working solutions

The test sample powder was prepared into a stock solution at a certain concentration with DMSO or another organic solvent, which was then further diluted with a suitable organic solvent.
The reference compounds testosterone, diclofenac, and propafenone were each prepared into a 10 mM stock solution with DMSO, which was then further diluted with a suitable organic solvent.

3. Preparation of liver microsome solution

Microsomes of each species were diluted into a 2× working solution with a 100 mM potassium phosphate buffer. The final concentration of the microsomes in the reaction system was 0.5 mg/mL.

4. Preparation of reduced nicotinamide adenine dinucleotide phosphate (NADPH) regeneration system

An appropriate amount of nicotinamide adenine dinucleotide phosphate (NADP) and isocitric acid (ISO) powder was weighed out, dissolved in a magnesium chloride solution, and mixed well by shaking. An appropriate amount of isocitrate dehydrogenase (IDH) was added, and the mixture was mixed well by gently turning upside down. The final concentrations in the reaction system were: 1 mM NADP, 1 mM magnesium chloride, 6 mM ISO, and 1 unit/mL IDH.

5. Preparation of stop solution

The stop solution was prepared with acetonitrile or another organic solvent containing an internal standard (tolbutamide or another suitable compound). The prepared stop solution was stored in a freezer at 2-8 °C.

6. Incubation process

Incubation was performed in a 96-well plate. Eight incubation plates were prepared and designated T0, T5, T15, T30, T45, T60, Blank60, and NCF60, respectively. The first 6 plates corresponded to reaction time points of 0 min, 5 min, 15 min, 30 min, 45 min, and 60 min, respectively. No test sample or reference compound was added to the Blank60 plate, and samples were taken after 60 min of incubation. In the NCF60 plate, incubation was performed for 60 min using the potassium phosphate buffer instead of the NADPH regeneration system solution. Three replicates were made for samples in all conditions.
The microsomes and the test sample or reference compound were mixed, and then the incubation plates Blank60, T5, T15, T30, T45, and T60 except for T0 and NCF60 were placed in a 37 °C water bath for pre-incubation for about 10 min. In the incubation plate T0, a stop solution was first added, followed by the addition of the NADPH regeneration system working solution, and 98 μL of the potassium phosphate buffer was added to each sample well of the incubation plate NCF60 to initiate the reaction. After the pre-incubation of the incubation plates Blank60, T5, T15, T30, T45, and T60 was completed, 98 μL of the NADPH regeneration system working solution was added to each sample well to initiate the reaction. The reaction temperature was 37±1 °C, the final volume of the reaction was 200 μL, and the reaction system included 0.5 mg/mL microsomes, 1.0 μM substrate, 1 mM NADP, 6 mM ISO, and 1 unit/mL IDH.
At 5 min, 15 min, 30 min, 45 min, and 60 min, a cold stop solution containing an internal standard was added to the reaction plate to stop the reaction.

All the reaction plates after the reaction was stopped were shaken well and centrifuged at 3220× g at 4 °C for 20 min. After the supernatant was diluted at a certain ratio, LC-MS/MS analysis was performed.

**Sample analysis**

Sample analysis was performed by the liquid chromatography-tandem mass spectrometry (LC-MS/MS) method without standard curves and quality control samples. Semi-quantitative determination was performed according to the ratio of the analyte peak area to the internal standard peak area. The retention times of the analyte and internal standard, the chromatogram acquisition, and chromatogram integration were processed using the Analyst software (Sciex, Framingham, Massachusetts, USA).
The CV for the internal standard peak area in each matrix in each analysis batch should be within 20%.

**Data analysis**

The *in vitro* elimination rate constant ke of the compound was obtained by converting the ratio of the peak area of the compound to the internal standard peak area in the following formula into the remaining rate:

$$\text{Remaining rate (\%)}=\frac{\text{Peak area ratio of compound to internal standard at any time point}}{\text{0Peak area ratio of compound to internal standard at 0 min}}\times 100$$

$$C_t = C_0 \bullet e^{-k_e t}$$

*when*

$$C_t = \frac{1}{2}C_0 \ ,$$

$$T_{1/2} = \frac{Ln2}{k_e} = \frac{0.693}{k_e}$$

$$CL_{int(mic)} = 0.693/T_{1/2}/\text{microsome protein content (microsome concentration during incubation in mg/mL)}$$

$$CL_{int(liver)} = CL_{int(mic)} \times \text{microsome protein amount in liver (mg/g)} \times \text{liver-to-body weight ratio}$$

According to the well stir model, the hepatic intrinsic clearance and hepatic clearance can be converted through the following formula.

$$CL_{(liver)} = (CL_{int(liver)} \times Q_h)/(CL_{int(liver)} + Q_h)$$

Parameters in the formula are shown in Table 3 below.

Table 3

| Species | Liver-to-body weight ratio (g/kg body weight) | Liver blood flow volume ($Q_h$) (mL/min/kg) | Microsome protein amount (mg/g liver weight) |
|---|---|---|---|
| Mouse | 88 | 90.0 | |
| Rat | 40 | 55.2 | 45 |
| Human | 20 | 20.7 | |

The test results are shown in Table 4:

Table 4

| Example No. | T1/2(min) (Human/rat/mouse) | CLint(liver) ($\mu$L/min/mg) (Human/rat/mouse) |
|---|---|---|
| Example 1 | >145/>145/>145 | <8.6/<17.3/<38.0 |
| Example 7 | >145/>145/>145 | <8.6/<17.3/<38.0 |
| Example 8 | >145/>145/>145 | <8.6/<17.3/<38.0 |
| Example 10 | >145/>145/>145 | <8.6/<17.3/<38.0 |
| Example 12 | >145/>145/>145 | <8.6/<17.3/<38.0 |
| Example 14 | >145/>145/>145 | <8.6/<17.3/<38.0 |
| Example 16 | >145/>145/>145 | <8.6/<17.3/<38.0 |
| Example 22 | >145/>145/>145 | <8.6/<17.3/<38.0 |
| Example 24 | 166/82.0/119 | 10.5/30.3/45.7 |

Efficacy Experimental Example 4: hERG

HEK293 cells were cultured in a DMEM medium containing 10% fetal bovine serum and 0.8 mg/mL G418 at 37 °C with 5% $CO_2$. The cells were digested by TrypLE™ Express and then centrifuged. The cell density was adjusted to $2 \times 10^6$ cells/mL. The cells were then gently mixed for 15-20 min using a shaker equilibrated at room temperature, and subjected to patch clamping on a machine. The medium of the prepared cells was replaced with extracellular fluid. The intracellular fluid and the extracellular fluid were taken from the fluid pool and added to the intracellular fluid pool and the cell and test substance pool of the QPlate chip, respectively. The voltage stimulation of the whole-cell hERG potassium current was recorded by the whole-cell patch clamp, and the experimental data were collected and stored by Qpatch. The compound was subjected to a 3-fold dilution starting from 30 $\mu$M to obtain 6 concentration points, each for two administrations over a period of at least 5 min. The current detected in compound-free extracellular fluid for each cell served as its own control group, and the detection was repeated at least twice independently using two cells per concentration. All electrophysiological experiments were performed at room temperature.

For data analysis, the current after the action of each drug concentration and the current of the blank control were standardized ($\frac{\text{Peak tail current}_{\text{compound}}}{\text{Peak tail current}_{\text{vehicle}}}$), and then the inhibition rate corresponding to each drug concentration was calculated ($1-\frac{\text{Peak tail current}_{\text{compound}}}{\text{Peak tail current}_{\text{vehicle}}}$). The mean and standard error were calculated for each concentration, and the half maximal inhibitory concentration of each compound was calculated:

$$Y = \text{Bottom} + \frac{\text{Top} - \text{Bottom}}{1 + 10\text{\^{}}((\text{LogIC}_{50} - \text{C}) \times \text{HillSlope})}$$

. The dose-dependent effect was subjected to non-linear fitting using the above equation, wherein Y represents the inhibition rate, C represents the concentration of the test substance, $IC_{50}$ is the half maximal inhibitory concentration, and HillSlope represents the Hill coefficient. Curve fitting and calculation of $IC_{50}$ were performed using Graphpad software.

Efficacy Experimental Example 5: Inhibition of Cytochrome Oxidase P450

1) Preparation of buffer:

100 mM K-Buffer: 9.5 mL of stock solution A was mixed with 40.5 mL of stock solution B, the total volume was adjusted to 500 mL with ultrapure water, and the buffer was titrated to pH 7.4 with KOH or $H_3PO_4$.

Starting material A (1 M potassium dihydrogen phosphate): 136.5 g of potassium dihydrogen phosphate in 1 L of water;

stock B (1 M potassium dihydrogen phosphate): 174.2 g of potassium dihydrogen phosphate in 1 L of water.

2) Preparation of test substance

The test substance powder was prepared into a stock solution at a certain concentration with DMSO or another organic solvent, which was then further diluted with a suitable organic solvent.

*3) In vitro* incubation

The liver microsomal *in vitro* incubation system for the CYP450 enzyme metabolism phenotype study was a biochemical reaction of prepared liver microsomes supplemented with a redox coenzyme and an enzyme-specific selective inhibitor under simulated physiological temperature and environment conditions.

4) Detection of parent drug or metabolite

The concentration of the parent drug or a metabolite thereof in the incubation solution was determined by LC-MS/MS. The test results are shown in Table 5:

Table 5

| Example No. | 1A2/2D6/2C9/2C19/3A4-M $IC_{50}$ (μM) |
|---|---|
| Example 1 | >10/>10/>10/>10/>10 |
| Example 7 | >10/>10/>10/>10/>10 |
| Example 8 | >10/>10/>10/>10/>10 |
| Example 14 | >10/>10/>10/>10/>10 |
| Example 22 | >10/>10/>10/>10/>10 |

Efficacy Experimental Example 6: Pharmacokinetic Test in Mice

The compounds of the present disclosure were administered to Balb/c mice by single intravenous injection and oral gavage, and the plasma of the mice at each time point was collected. The concentration of the compounds to be tested in BALB/C plasma was detected using the LC-MS/MS analysis method, and pharmacokinetic analysis was performed.

Preparation of formulations for administration (low dose)

Vehicle of formulations for oral and intravenous administration: preparation procedures for a 25% sulfobutylether-β-cyclodextrin solution containing 3% dimethyl sulfoxide and 5% polyoxyl 15 hydroxystearate: An appropriate amount of a solution of the compound to be tested (1 mg/mL) in 25% sulfobutylether-β-cyclodextrin was taken, and 3% sulfobutylether-β-cyclodextrin solution in total volume was added. The mixture was vortexed, shaken, and ultrasonicated. The remaining volume of 25% sulfobutylether-β-cyclodextrin (sterilized) was added to prepare the final concentration solution or suspension.

Intravenous injection formulations will be directly administered in animal experiments without filtration. The IV drug preparation vials were disinfected in an infrared sterilization cabinet one day in advance.

Administration

Intravenous injection: Administration was performed via the tail vein. After the needle was inserted, the syringe was slowly retracted. If blood was drawn back, it indicated that the needle had entered the vein, and the drug liquid could be injected.

Gavage: Gavage needle and syringe of an appropriate type were selected. The gavage needle was inserted from the root of the tongue into the esophagus until it reached near the cardia of the stomach, then the drug formulation was injected, and the gavage needle was withdrawn. When disposable plastic gavage needles were used, one needle was used for each group.

Blood sample collection and processing

At each time point, 60 μL of whole blood was collected from the submandibular vein or tail vein (except for the IV group). The blood sample was added to a pre-cooled anticoagulant blood collection tube containing anticoagulant EDTA-$K_2$ (1 μL, 15% EDTA-$K_2$ solution), placed on wet ice, and centrifuged (3000 g, 4 °C, 10 min) within 1 h. The plasma was immediately collected after centrifugation, transferred to an EP tube, and stored at -90 °C to -60 °C.

4. Sample detection

Formulation concentration detection: The HPLC or LCMS/MS method was used to determine the concentrations of the formulations.

Plasma concentration detection: A developed LC-MS/MS method was used to detect the compounds in plasma.

5. Data analysis

The plasma concentration-time data were analyzed using a non-compartmental model in the Phoenix® WinNonlin® (Version 8.2, Pharsight, Mountain View, CA). The compounds in Table 6 were administered at 2 mg/kg by IV and 10 mg/kg by PO, and the test results are shown in Table 6:

Table 6

| Example No. | Administration mode | $AUC_{last}$ (nM*h) | $T_{1/2}$ (h) | Cl (L/h/kg) | $C_{max}$ (nM*h) | MRT (h) | F% |
|---|---|---|---|---|---|---|---|
| Example 1 | IV | 30719 | 4.2 | 0.17 | \ | 6.1 | \ |
| | PO | 181895 | 3.9 | \ | 12870 | 6.5 | 118 |
| Example 4 | IV | 9234 | 6.3 | 0.5 | \ | 6.4 | \ |
| | PO | 79024 | 3.3 | \ | 9363 | 5.3 | 163 |
| Example 5 | IV | 9374 | 1.8 | 0.5 | \ | 2.5 | \ |
| | PO | 155083 | 2.6 | \ | 15544 | 5.3 | 318 |
| Example 7 | IV | 50658 | 119.7 | 0.015 | \ | 170.9 | \ |
| | PO | 267210 | 146.5 | \ | 12680 | 211.2 | 106 |
| Example 8 | IV | 7936 | 5.6 | 0.6 | \ | 6.4 | \ |
| | PO | 119205 | 34.2 | \ | 14559 | 47.2 | 300 |
| Example 9 | IV | 5737 | 1.4 | 0.8 | \ | 1.7 | \ |
| | PO | 30475 | 1.9 | \ | 9370 | 3.2 | 111 |
| Example 10 | IV | 59324 | 52 | 0.024 | \ | 75 | \ |
| | PO | 290094 | 28.2 | \ | 14559 | 75.6 | 98 |
| Example 12 | IV | 58827 | 17.2 | 0.06 | \ | 23.4 | \ |
| | PO | 295169 | 25.6 | \ | 16069 | 34.5 | 100 |
| Example 14 | IV | 21159 | 19.0 | 0.14 | \ | 26.2 | \ |
| | PO | 112116 | 12.3 | \ | 8081 | 17.8 | 106 |
| Example 16 | IV | 5435 | 2.7 | 0.8 | \ | 3.6 | \ |
| | PO | 63501 | 3.1 | \ | 7174 | 5.3 | 206 |
| Example 22 | IV | 16403 | 8.9 | 0.25 | \ | 10.8 | \ |
| | PO | 96371 | 7.6 | \ | 7065 | 9.9 | 114 |
| Example 24 | IV | 22115 | 11 | 0.198 | \ | | \ |
| | PO | 132517 | 16.2 | \ | 7822 | | 120 |
| Example 29 | IV | 50811 | 63.5 | 0.024 | \ | | \ |
| | PO | 250810 | 17 | \ | 13349 | | 99 |
| Comparative compound B1 | IV | 1243 | 1.4 | 4.25 | \ | | \ |
| | PO | 4867 | 5.6 | \ | 653 | | 81 |
| Comparative compound B2 | IV | 8749 | 19.6 | 0.362 | \ | | \ |
| | PO | 52774 | 9.3 | \ | 4210 | | 87.5 |

As can be seen from the above table, the compounds of the present disclosure have high oral exposure and good bioavailability.

High-dose (100 mpk or 200 mpk) pharmacokinetic test of compounds in mice: Vehicle of formulations for oral administration: preparation procedures for a 10% hydroxypropyl-β-cyclodextrin solution containing 5% dimethyl sulfoxide and 15% polyoxyl 15 hydroxystearate: An appropriate amount of the compound was weighed out, and the total volume of the solution was calculated based on the administration dose (for example, if the administration dose was 200 mg/kg and the fixed administration volume for mice was 10 mL/kg, the concentration was calculated to be 20 mg/mL). After adding 5% DMSO, the mixture was vortexed. 15% HS15 was then added, and the mixture was vortexed again. Finally, a 10% aqueous cyclodextrin solution was added, and the resulting mixture was vortexed to prepare the final concentration solution or suspension. The remaining procedures referred to the pharmacokinetic procedures for mice with an administration dose of 10 mpk. The test results are shown in Table 7.

Table 7

| Example No. | Administration mode | $AUC_{last}$ (nM*h) | $C_{max}$ (nM*h) |
|---|---|---|---|
| Example 7 | PO<br>100 mpk | 2832599 | 137376 |
| Example 8 | PO<br>200 mpk | 2433185 | 113607 |
| Example 10 | PO | 3379803 | 147509 |
| | 200 mpk | | |
| Example 14 | PO<br>200 mpk | 3143278 | 141079 |
| Example 22 | PO<br>200 mpk | 2521694 | 128253 |
| Example 29 | PO<br>100 mpk | 2163106 | 115931 |
| Comparative compound B2 | PO<br>200 mpk | 901871 | 47808 |
| Comparative compound B3 | PO<br>200 mpk | 1042083 | 54706 |

As can be seen from the above table, the compounds of the present disclosure still have high oral exposure at high doses (100 mpk or 200 mpk), with a linear dependence of exposure on dose compared to the 10 mpk PO results in Table 6.

Efficacy Experimental Example 7: Pharmacokinetic Test in Rats

In this experimental example, the pharmacokinetic behavior of the test compounds after administration by intravenous injection (IV) and oral gavage (PO) in SD rats was tested. Preparation of formulations for administration referred to the low-dose pharmacokinetic test in mice.

On the day of administration, rats were weighed for actual body weight, and the administration volume was calculated. Each compound was tested in two groups with 3 rats in each group; one group was subjected to administration by single intravenous injection, and the other group was subjected to administration by single gavage. The whole blood samples were collected via the jugular vein at prescribed time points (0.25 h, 0.5 h, 1 h, 2 h, 4 h, 8 h, and 24 h after administration). After the blood sample collection, the samples were immediately transferred to labeled commercial sample tubes containing K2-EDTA (0.85-1.15 mg), followed by centrifugation (3200× g, 4 °C, 10 min) and plasma collection. The plasma was transferred to a pre-cooled centrifuge tube, frozen in dry ice, and then stored in an ultra-low temperature freezer at -60 °C or lower until the LC-MS/MS analysis was performed.

Plasma concentrations were determined by the LC-MS/MS method. Plasma drug concentration data for the compounds were processed using a non-compartmental model in the WinNonlin Version 6.3 (Pharsight, Mountain View, CA) pharmacokinetic software. The relevant pharmacokinetic parameters were calculated by a linear-log trapezoidal method. The compounds in Table 8 were administered at 2 mg/kg by IV and 10 mg/kg by PO, and the test results are shown in Table 8:

Table 8

| Example No. | Administration mode | $AUC_{last}$ (nM*h) | $T_{1/2}$ (h) | Cl (L/h/kg) | MRT (h) | F% |
|---|---|---|---|---|---|---|
| Example 1 | IV | 112664 | 32.4 | 0.02 | 46.3 | \ |
| | PO | 604952 | 66.9 | \ | 69.9 | 107 |
| Example 7 | IV | 44470 | 15.7 | 0.12 | 20.5 | \ |
| | PO | 307971 | 19.5 | \ | 28.5 | 142 |
| Example 8 | IV | 8887 | 6.2 | 0.54 | 7.8 | \ |
| | PO | 59272 | 14.7 | \ | 20.6 | 133 |
| Example 10 | IV | 21744 | 19.0 | 0.18 | 26.5 | \ |
| | PO | 152322 | 17.8 | \ | 25.7 | 140 |
| Example 12 | IV | 34929 | 54.2 | 0.07 | 76.3 | \ |
| | PO | 113637 | 21.8 | \ | 24.9 | 65 |
| Example 14 | IV | 16908 | 8.1 | 0.28 | 10.2 | \ |
| | PO | 83224 | 10.9 | \ | 15.0 | 98 |
| Example 22 | IV | 6417 | 5.8 | 0.66 | 7.6 | \ |
| | PO | 32225 | 7.3 | \ | 9.6 | 95 |

The compounds in the above table were administered at 2 mg/kg by IV and 10 mg/kg by PO. As can be seen from the results, the compounds of the present disclosure have high oral exposure and good bioavailability.

Efficacy Experimental Example 8: Pharmacodynamic Test

Healthy female nude mice (BALB/c nu/nu) aged 6-8 weeks were housed in an SPF environment in accordance with animal ethical guidelines. The well-growing $10^7$ H226 cells were harvested through trypsin digestion, washed in phosphate-buffered saline (PBS), and then resuspended in PBS containing 50% matrigel (BD Biosciences).

Subcutaneous injection was then performed at the dorsolateral sites of the mice, and the tumor volume was measured once every three days thereafter. When the tumor volume reached around 150-200 $mm^3$, the mice were randomly assigned to different treatment groups. Any one of the compounds described herein was prepared accordingly and administered at an appropriate dose. Finally, the tumor growth in the nude mice was analyzed after 4 to 6 weeks based on the growth of the tumors. The test results are shown in Table 9:

Table 9

| Example No. | TGI |
|---|---|
| Example 1 | 50mpk QD TGI = 108.39%<br>100mpk QD TGI = 113.05% |
| Example 7 | 5mpk QD TGI = 111.84% |
| Example 8 | 10mpk QD TGI = 115.60% |
| Example 10 | 25mpk QD TGI = 100.89% |
| Example 12 | 50mpk QD TGI = 111.58% |
| Example 14 | 10mpk QD TGI = 100.38% |
| Example 22 | 10mpk QD TGI = 106.26% |
| | 30mpk QD TGI = 113.45% |

Healthy female nude mice (BALB/c nu/nu) aged 6-8 weeks were housed in an SPF environment in accordance with animal ethical guidelines. The well-growing 5M MSTO-211H cells were harvested through trypsin digestion, washed in phosphate-buffered saline (PBS), and then resuspended in PBS containing 50% matrigel (BD Biosciences).

Subcutaneous injection was then performed at the dorsolateral sites of the mice, and the tumor volume was

measured once every three days thereafter. When the tumor volume reached around 150-200 $mm^3$, the mice were randomly assigned to different treatment groups. Any one of the compounds described herein was prepared accordingly and administered at an appropriate dose. Finally, the tumor growth in the nude mice was analyzed after 4 to 6 weeks based on the growth of the tumors. The test results are shown in Table 10:

Table 10

| Example No. | TGI |
|---|---|
| Example 7 | 25mpk QD TGI = 107.93% |
| Example 8 | 50mpk QD TGI = 104.28% |
| Example 14 | 50mpk QD TGI = 100.43%<br>150mpk QD TGI = 118.27% |
| Example 22 | 150mpk QD TGI = 102.62% |

Efficacy Experimental Example 9: Test of Saturated Solubility of Samples in FaSSIF Solution

1. Experimental process

1.1 Preparation of FaSSIF solution

Preparation of buffer (pH 6.5):

About 0.21 g of sodium hydroxide, about 2.24 g of sodium dihydrogen phosphate dihydrate, and 3.09 g of sodium chloride were weighed out and dissolved in 500 mL of water, and after dissolution, the pH was adjusted to 6.5 with 1 N sodium hydroxide or 1 N hydrochloric acid.
112 mg of FaSSIF solid was weighed out and placed in a 50 mL volumetric flask, and the buffer described above was added for dissolution. The resulting solution was diluted to volume, mixed well, and left to stand at room temperature for 2 h or more.

1.2 Preparation of samples

Test sample: About 1 mg of the test sample was taken, and 1 mL of FaSSIF solution was added. The mixture was stirred at room temperature overnight and centrifuged, and the supernatant was collected and injected for analysis.
Reference sample solution: About 1.5 mg of the test sample was precisely weighed out and placed in a 50 mL volumetric flask, and DMF was added for dissolution. The resulting solution was diluted to volume and mixed well.

1.3 Preparation of mobile phases

Mobile phase A: 1000 mL of purified water was accurately weighed out, 1 mL of formic acid was added, and the mixture was mixed well and degassed by ultrasonication.
Mobile phase B: Acetonitrile.

1.4 Chromatographic conditions (Table 11)

Table 11

| Instrument | HPLC |
|---|---|
| Chromatographic column | Waters XBridge C18, 4.6*50mm 3.5um |
| Sample tray (°C) | off |
| Column temperature (°C) | 30 |
| UV wavelength (nm) | 254 |
| Needle washing solution | MeOH |

(continued)

| Instrument | HPLC | |
|---|---|---|
| Flow rate (mL/min) | 1.0 | |
| Run time (min) | 6 | |
| Gradient elution table | | |
| Time (min) | Mobile phase A (%) | Mobile phase B (%) |
| 0 | 90 | 10 |
| 0.5 | 90 | 10 |
| 6 | 10 | 90 |
| 7 | 10 | 90 |
| 7.1 | 90 | 10 |
| 10 | 90 | 10 |

**2. Results**

The peak of the main component of the reference sample and the peak of the test sample with the same retention time were integrated, and the concentration thereof in the FaSSIF solution was calculated according to the external standard method. The test results are shown in Table 12.

Table 12

| Example No. | FaSSIF(pH = 6.5) (ug/mL) |
|---|---|
| Example 1 | >950.5 |
| Example 5 | 781.7 |
| Example 7 | 1499.5 |
| Example 8 | 433.97 |
| Example 10 | 89.75 |
| Example 14 | 674.6 |
| Example 22 | 337.6 |
| Example 24 | 193.3 |
| Example 29 | 223.4 |
| Comparative compound B2 | 16.4 |
| Comparative compound B3 | 14.0 |
| Comparative compound B4 | 36.2 |

**Claims**

1. A compound represented by formula I, a pharmaceutically acceptable salt thereof, an ester thereof, a stereoisomer thereof, a tautomer thereof, a polymorph thereof, a solvate thereof, a metabolite thereof, an isotopic derivative thereof, or a prodrug thereof,

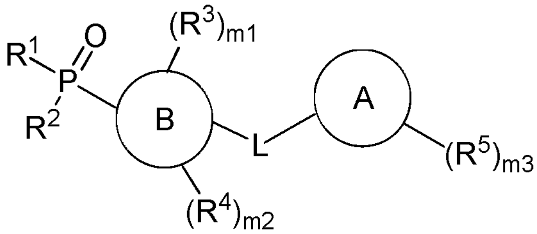

I

wherein

$R^1$ and $R^2$ are independently $C_1$-$C_6$ alkyl, $C_3$-$C_6$ cycloalkyl, or $C_6$-$C_{10}$ aryl;
or $R^1$ and $R^2$, together with the atom to which they are attached, form 3- to 12-membered heterocycloalkyl or 3- to 12-membered heterocycloalkyl substituted with one or more $R^{1-1}$, wherein the 3- to 12-membered heterocycloalkyl contains, in addition to the attached P atom, 0, 1, 2, or 3 heteroatoms selected from 1, 2, or 3 of N, O, and S;
$R^{1-1}$ is independently $C_1$-$C_6$ alkyl;
ring B is a $C_3$-$C_{12}$ saturated or unsaturated carbocyclic ring or a "5- to 16-membered saturated or unsaturated heterocyclic ring with 1, 2, 3, 4, or 5 heteroatoms selected from 1, 2, or 3 of N, O, and S";
ring B is monocyclic or polycyclic; when ring B is monocyclic, ring B is an aromatic ring; when ring B is polycyclic, the ring connected to

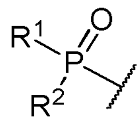

is an aromatic ring, with the remaining ring(s) being saturated or unsaturated ring(s);
m1 is 0 or 1;
$R^3$ is "5- to 12-membered saturated or unsaturated heterocyclyl with 1, 2, or 3 heteroatoms selected from 1, 2, or 3 of N, O, and S", "5- to 12-membered saturated or unsaturated heterocyclyl with 1, 2, or 3 heteroatoms selected from 1, 2, or 3 of N, O, and S" substituted with one or more $R^{3-1}$, $C_6$-$C_{10}$ aryl, or $C_6$-$C_{10}$ aryl substituted with one or more $R^{3-5}$;
$R^{3-1}$ and $R^{3-5}$ are independently H, deuterium, halogen, $C_1$-$C_6$ alkyl, $C_1$-$C_6$ alkoxy, CN, $C_1$-$C_6$ alkyl substituted with one or more $R^{3-2}$, $C_1$-$C_6$ alkoxy substituted with one or more $R^{3-3}$, "3- to 12-membered heterocycloalkyl with 1, 2, or 3 heteroatoms selected from 1, 2, or 3 of N, O, and S", "5- to 12-membered heteroaryl with 1, 2, or 3 heteroatoms selected from 1, 2, or 3 of N, O, and S", C(=O)NR$^c$R$^d$, $C_3$-$C_6$ cycloalkyl, or $C_3$-$C_6$ cycloalkyl substituted with one or more $R^{3-4}$;
$R^{3-2}$ and $R^{3-3}$ are independently deuterium, halogen, OH, CN, $C_1$-$C_6$ alkoxy, "3- to 12-membered heterocycloalkyl with 1, 2, or 3 heteroatoms selected from 1, 2, or 3 of N, O, and S", C(=O)NR$^c$R$^d$, or NR$^b$C(=O)R$^a$;
$R^{3-4}$ is independently CN, C(=O)NR$^c$R$^d$, or C(=O)OR$^a$;
m2 is 0, 1, 2, or 3;
$R^4$ is independently oxo (=O), CN, halogen, $C_1$-$C_6$ alkyl, $C_1$-$C_6$ haloalkyl, $C_1$-$C_6$ alkoxy, or $C_1$-$C_6$ haloalkoxy; L is a single bond, -CR$^{L1}$R$^{L2}$-, -O-, -S-, -NR$^{L3}$-, -NR$^{L3}$CR$^{L1}$R$^{L2}$-, -CR$^{L4}$=CR$^{L5}$-,

 ,

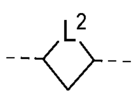 , 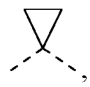 , or 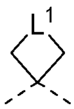 ;

$R^{L1}$, $R^{L2}$, and $R^{L3}$ are independently H, $C_1$-$C_6$ alkyl, or $C_3$-$C_6$ cycloalkyl;
$R^{L4}$ and $R^{L5}$ are independently H or $C_1$-$C_6$ alkyl;
$L^1$ and $L^2$ are independently -$CH_2$-, -O-, -S-, or -NH-;
ring A is a $C_3$-$C_{12}$ saturated or unsaturated carbocyclic ring or a "5- to 12-membered saturated or unsaturated heterocyclic ring with 1, 2, or 3 heteroatoms selected from 1, 2, or 3 of N, O, and S";

m3 is 0, 1, 2, or 3;
$R^5$ is independently $SF_5$, halogen, $C_1$-$C_6$ alkyl, $C_1$-$C_6$ alkyl substituted with one or more $R^{5-1}$, $SR^{5-2}$, $OR^{5-3}$, CN, $S(=O)_2R^{5-4}$, $C(=O)R^{5-5}$, $S(=O)_2NR^dR^{5-6}$, $NR^dR^{5-6}$, $C_3$-$C_6$ cycloalkyl, or $C_3$-$C_6$ cycloalkyl substituted with one or more $R^{5-7}$;
$R^{5-1}$ is independently hydroxy, CN, or halogen;
$R^{5-2}$, $R^{5-3}$, $R^{5-4}$, and $R^{5-6}$ are independently H, $C_1$-$C_6$ haloalkyl, $C_1$-$C_6$ alkyl, $C_3$-$C_6$ cycloalkyl, or $C_3$-$C_6$ cycloalkyl substituted with one or more $R^{5-8}$;
$R^{5-5}$ is independently $C_1$-$C_6$ haloalkyl, H, $C_1$-$C_6$ alkyl, $C_1$-$C_6$ alkoxy, $C_1$-$C_6$ haloalkoxy, $C_3$-$C_6$ cycloalkyl or $C_3$-$C_6$ cycloalkyl substituted with one or more $R^{5-8}$, or $NR^dR^{5-6}$;
$R^{5-7}$; and $R^{5-8}$ are independently halogen or $C_1$-$C_6$ haloalkyl;
$R^a$, $R^b$, $R^c$, and $R^d$ are independently H or $C_1$-$C_6$ alkyl.

**2.** The compound represented by formula I, the pharmaceutically acceptable salt thereof, the ester thereof, the stereoisomer thereof, the tautomer thereof, the polymorph thereof, the solvate thereof, the metabolite thereof, the isotopic derivative thereof, or the prodrug thereof according to claim 1, wherein

$R^1$ and $R^2$ are independently $C_1$-$C_6$ alkyl, $C_3$-$C_6$ cycloalkyl, or $C_6$-$C_{10}$ aryl;
or $R^1$ and $R^2$, together with the atom to which they are attached, form 3- to 12-membered heterocycloalkyl or 3- to 12-membered heterocycloalkyl substituted with one or more $R^{1-1}$, wherein the 3- to 12-membered heterocycloalkyl contains, in addition to the attached P atom, 0, 1, 2, or 3 heteroatoms selected from 1, 2, or 3 of N, O, and S;
$R^{1-1}$ is independently $C_1$-$C_6$ alkyl;
ring B is a $C_3$-$C_{12}$ saturated or unsaturated carbocyclic ring or a "5- to 16-membered saturated or unsaturated heterocyclic ring with 1, 2, 3, 4, or 5 heteroatoms selected from 1, 2, or 3 of N, O, and S";
ring B is monocyclic or polycyclic; when ring B is monocyclic, ring B is an aromatic ring; when ring B is polycyclic, the ring connected to

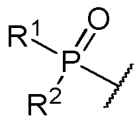

is an aromatic ring, with the remaining ring(s) being saturated or unsaturated ring(s);
m1 is 0 or 1;
$R^3$ is "5- to 12-membered saturated or unsaturated heterocyclyl with 1, 2, or 3 heteroatoms selected from 1, 2, or 3 of N, O, and S", "5- to 12-membered saturated or unsaturated heterocyclyl with 1, 2, or 3 heteroatoms selected from 1, 2, or 3 of N, O, and S" substituted with one or more $R^{3-1}$, $C_6$-$C_{10}$ aryl, or $C_6$-$C_{10}$ aryl substituted with one or more $R^{3-5}$;
$R^{3-1}$ and $R^{3-5}$ are independently H, deuterium, halogen, $C_1$-$C_6$ alkyl, $C_1$-$C_6$ alkoxy, CN, $C_1$-$C_6$ alkyl substituted with one or more $R^{3-2}$, $C_1$-$C_6$ alkoxy substituted with one or more $R^{3-3}$, "3- to 12-membered heterocycloalkyl with 1, 2, or 3 heteroatoms selected from 1, 2, or 3 of N, O, and S", "5- to 12-membered heteroaryl with 1, 2, or 3 heteroatoms selected from 1, 2, or 3 of N, O, and S", $C(=O)NR^cR^d$, $C_3$-$C_6$ cycloalkyl, or $C_3$-$C_6$ cycloalkyl substituted with one or more $R^{3-4}$;
$R^{3-2}$ and $R^{3-3}$ are independently deuterium, halogen, OH, CN, $C_1$-$C_6$ alkoxy, "3- to 12-membered heterocycloalkyl with 1, 2, or 3 heteroatoms selected from 1, 2, or 3 of N, O, and S", $C(=O)NR^cR^d$, or $NR^bC(=O)R^a$;
$R^{3-4}$ is independently CN, $C(=O)NR^cR^d$, or $C(=O)OR^a$;
m2 is 0, 1, 2, or 3;
$R^4$ is independently oxo (=O), CN, halogen, $C_1$-$C_6$ alkyl, $C_1$-$C_6$ haloalkyl, $C_1$-$C_6$ alkoxy, or $C_1$-$C_6$ haloalkoxy;
L is a single bond, $-CR^{L1}R^{L2}-$, -O-, -S-, $-NR^{L3}-$, $-NR^{L3}CR^{L1}R^{L2}-$, $-CR^{L4}=CR^{L5}-$,

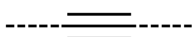
,

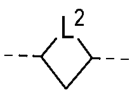
, 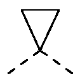, or 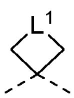;

$R^{L1}$, $R^{L2}$, and $R^{L3}$ are independently H, $C_1$-$C_6$ alkyl, or $C_3$-$C_6$ cycloalkyl;
$R^{L4}$ and $R^{L5}$ are independently H or $C_1$-$C_6$ alkyl;

$L^1$ and $L^2$ are independently $-CH_2-$, -O-, -S-, or -NH-;
ring A is a $C_3$-$C_{12}$ saturated or unsaturated carbocyclic ring or a "5- to 12-membered saturated or unsaturated heterocyclic ring with 1, 2, or 3 heteroatoms selected from 1, 2, or 3 of N, O, and S";
m3 is 0, 1, 2, or 3;
$R^5$ is independently $SF_5$, halogen, $C_1$-$C_6$ alkyl, $C_1$-$C_6$ alkyl substituted with one or more $R^{5-1}$, $SR^{5-2}$, $OR^{5-3}$, CN, $S(=O)_2R^{5-4}$, $C(=O)R^{5-5}$, $S(=O)_2NR^dR^{5-6}$, $NR^dR^{5-6}$, or $C_3$-$C_6$ cycloalkyl substituted with one or more $R^{5-7}$;
$R^{5-1}$ is independently hydroxy, CN, or halogen;
$R^{5-2}$, $R^{5-3}$, $R^{5-4}$, and $R^{5-6}$ are independently H, $C_1$-$C_6$ haloalkyl, $C_1$-$C_6$ alkyl, $C_3$-$C_6$ cycloalkyl, or $C_3$-$C_6$ cycloalkyl substituted with one or more $R^{5-8}$;
$R^{5-5}$ is independently $C_1$-$C_6$ haloalkyl, H, $C_1$-$C_6$ alkyl, $C_1$-$C_6$ alkoxy, $C_1$-$C_6$ haloalkoxy, $C_3$-$C_6$ cycloalkyl or $C_3$-$C_6$ cycloalkyl substituted with one or more $R^{5-8}$, or $NR^dR^{5-6}$;
$R^{5-7}$; and $R^{5-8}$ are independently halogen or $C_1$-$C_6$ haloalkyl;
$R^a$, $R^b$, $R^c$, and $R^d$ are independently H or $C_1$-$C_6$ alkyl.

**3.** The compound represented by formula I, the pharmaceutically acceptable salt thereof, the ester thereof, the stereoisomer thereof, the tautomer thereof, the polymorph thereof, the solvate thereof, the metabolite thereof, the isotopic derivative thereof, or the prodrug thereof according to claim 1 or 2, wherein one or two or more of the following conditions are satisfied:

(1) each "halogen" is independently fluorine, chlorine, bromine, or iodine;
(2) each "$C_1$-$C_6$ alkyl" is independently methyl, ethyl, n-propyl, isopropyl, n-butyl, isobutyl, or *tert*-butyl;
(3) each "$C_1$-$C_6$ alkoxy" is independently methoxy, ethoxy, n-propoxy, isopropoxy, n-butoxy, isobutoxy, or tert-butoxy;
(4) each "$C_1$-$C_6$ haloalkyl" is independently $-CHF_2$, $-CH_2F$, or $-CF_3$;
(5) each "$C_1$-$C_6$ haloalkoxy" is independently $-OCHF_2$, $-OCH_2F$, or $-OCF_3$;
(6) each "$C_3$-$C_6$ cycloalkyl" is independently cyclopropyl, cyclobutyl, cyclopentyl, or cyclohexyl;
(7) each "$C_6$-$C_{10}$ aryl" is independently phenyl or naphthyl;
(8) each "3- to 12-membered heterocycloalkyl with 1, 2, or 3 heteroatoms selected from 1, 2, or 3 of N, O, and S" is independently "5- to 6-membered heterocycloalkyl with 1 or 2 heteroatoms selected from 1 or 2 of N and O";
(9) the "5- to 12-membered heteroaryl with 1, 2, or 3 heteroatoms selected from 1, 2, or 3 of N, O, and S" is "5- to 6-membered heteroaryl with 1, 2, or 3 heteroatoms selected from 1, 2, or 3 of N, O, and S";
(10) in ring B, the "$C_3$-$C_{12}$ saturated or unsaturated carbocyclic ring" is a $C_6$-$C_{10}$ unsaturated carbocyclic ring, preferably a $C_6$-$C_{10}$ aromatic ring;
(11) in ring B, the "5- to 16-membered saturated or unsaturated heterocyclic ring with 1, 2, 3, 4, or 5 heteroatoms selected from 1, 2, or 3 of N, O, and S" is a "6- to 15-membered unsaturated heterocyclic ring with 1, 2, 3, 4, or 5 heteroatoms selected from 1, 2, or 3 of N, O, and S";
(12) in $R^3$, the "5- to 12-membered saturated or unsaturated heterocyclyl with 1, 2, or 3 heteroatoms selected from 1, 2, or 3 of N, O, and S" is "5- to 10-membered unsaturated heterocyclyl with 1, 2, or 3 heteroatoms selected from 1, 2, or 3 of N, O, and S";
(13) in ring A, the "$C_3$-$C_{12}$ saturated or unsaturated carbocyclic ring" is a $C_4$-$C_{10}$ saturated or unsaturated carbocyclic ring, preferably a $C_6$-$C_{10}$ aromatic ring; and
(14) in ring A, the "5- to 12-membered saturated or unsaturated heterocyclic ring with 1, 2, or 3 heteroatoms selected from 1, 2, or 3 of N, O, and S" is a "5- to 6-membered heteroaromatic ring with 1, 2, or 3 heteroatoms selected from 1, 2, or 3 of N, O, and S".

**4.** The compound represented by formula I, the pharmaceutically acceptable salt thereof, the ester thereof, the stereoisomer thereof, the tautomer thereof, the polymorph thereof, the solvate thereof, the metabolite thereof, the isotopic derivative thereof, or the prodrug thereof according to claim 1 or 2, wherein one or two or more of the following conditions are satisfied:

(1) each "halogen" is independently fluorine or chlorine;
(2) each "$C_1$-$C_6$ alkyl" is independently methyl or ethyl;
(3) each "$C_1$-$C_6$ alkoxy" is independently methoxy or ethoxy;
(4) each "$C_3$-$C_6$ cycloalkyl" is independently cyclopropyl or cyclobutyl;
(5) each "$C_6$-$C_{10}$ aryl" is independently phenyl;
(6) in ring B, the "$C_3$-$C_{12}$ saturated or unsaturated carbocyclic ring" is a benzene ring, a naphthalene ring, or a hydrindene ring;
(7) in ring B, the "5- to 16-membered saturated or unsaturated heterocyclic ring with 1, 2, 3, 4, or 5 heteroatoms

selected from 1, 2, or 3 of N, O, and S" is a pyridine ring (e.g.,

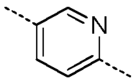

), an indole ring (e.g.,

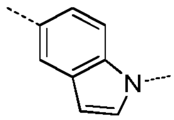

), a carbazole ring (e.g.,

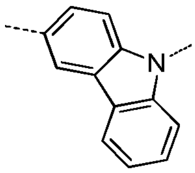

),

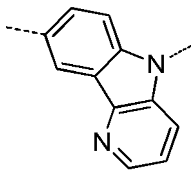
,
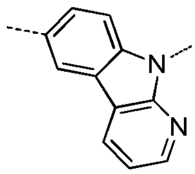
,

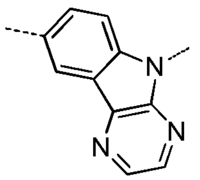
,
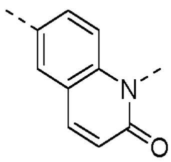
,
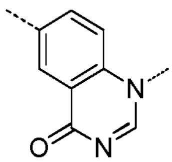
,
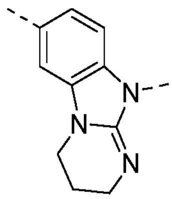
,
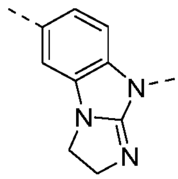
,

or

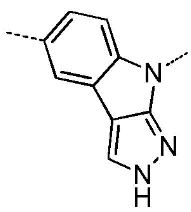
;

(8) in $R^3$, the "5- to 12-membered saturated or unsaturated heterocyclyl with 1, 2, or 3 heteroatoms selected from 1, 2, or 3 of N, O, and S" is imidazolyl (e.g.,

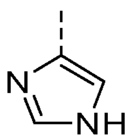

), pyrazolyl (e.g.,

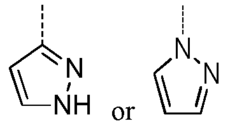

),

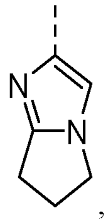

,

pyridyl (e.g.,

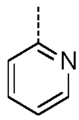

), or pyrimidinyl (e.g.,

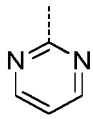

);

(9) in ring A, the "$C_3$-$C_{12}$ saturated or unsaturated carbocyclic ring" is a cyclobutane ring (e.g.,

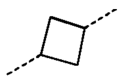

), a cyclohexane ring (e.g.,

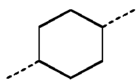

), a spiro[3.3]heptane ring (e.g.,

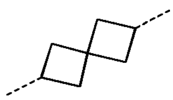

), a benzene ring (e.g.,

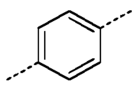

or

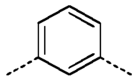

), a naphthalene ring (e.g.,

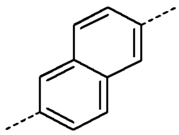

), a hydrindene ring, or a tetrahydronaphthalene ring; and

(10) in ring A, the "5- to 12-membered saturated or unsaturated heterocyclic ring with 1, 2, or 3 heteroatoms selected from 1, 2, or 3 of N, O, and S" is a pyridine ring (e.g.,

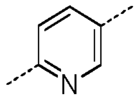

), or a thiophene ring (e.g.,

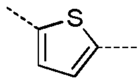

).

5. The compound represented by formula I, the pharmaceutically acceptable salt thereof, the ester thereof, the stereoisomer thereof, the tautomer thereof, the polymorph thereof, the solvate thereof, the metabolite thereof, the isotopic derivative thereof, or the prodrug thereof according to claim 1, wherein one or two or more of the following conditions are satisfied:

(1) in $R^3$, the "5- to 12-membered saturated or unsaturated heterocyclyl with 1, 2, or 3 heteroatoms selected from 1, 2, or 3 of N, O, and S" is pyrrolyl (e.g.,

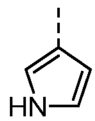

), pyrazolyl (e.g.,

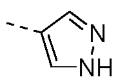
,
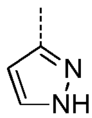
, or
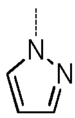

), imidazolyl (e.g.,

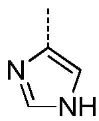

), triazolyl (e.g.,

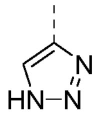 or 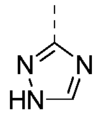

), thiazolyl (e.g.,

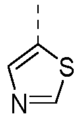

), oxazolyl (e.g.,

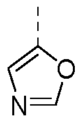

), isoxazolyl (e.g.,

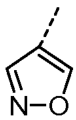

), pyridyl (e.g.,

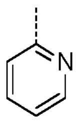, 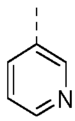, or 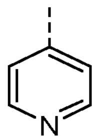

), pyrimidinyl (e.g.,

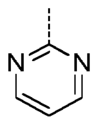

or

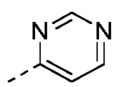

), pyrazinyl (e.g.,

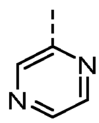

), pyridazinyl (e.g.,

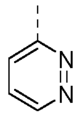), 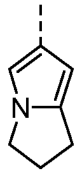, 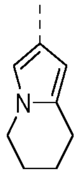, 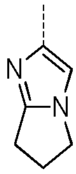, 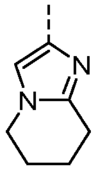,

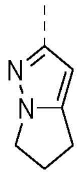, 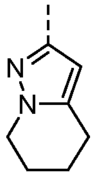, 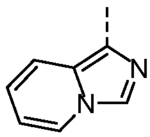, 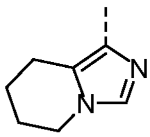, or 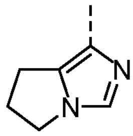;

and

(2) in ring A, the "$C_3$-$C_{12}$ saturated or unsaturated carbocyclic ring" is a cyclobutane ring (e.g.,

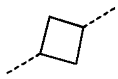

), a cyclohexane ring (e.g.,

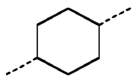

), spiro[2.5]octane (e.g.,

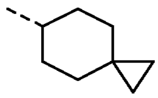

), a spiro[3.3]heptane ring (e.g.,

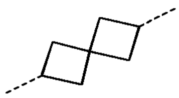

), a benzene ring (e.g.,

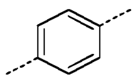 or 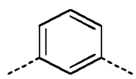

), a naphthalene ring (e.g.,

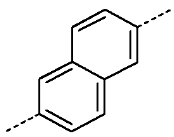

), a hydrindene ring, or a tetrahydronaphthalene ring.

**6.** The compound represented by formula I, the pharmaceutically acceptable salt thereof, the ester thereof, the stereoisomer thereof, the tautomer thereof, the polymorph thereof, the solvate thereof, the metabolite thereof, the isotopic derivative thereof, or the prodrug thereof according to claim 1 or 2, wherein one or two or more of the following

conditions are satisfied:

(1) $R^1$ and $R^2$ are independently $C_1$-$C_6$ alkyl;
(2) $R^3$ is "5- to 12-membered saturated or unsaturated heterocyclyl with 1, 2, or 3 heteroatoms selected from 1, 2, or 3 of N, O, and S" or "5- to 12-membered saturated or unsaturated heterocyclyl with 1, 2, or 3 heteroatoms selected from 1, 2, or 3 of N, O, and S" substituted with one or more $R^{3-1}$;
(3) $R^{3-1}$ is independently $C_1$-$C_6$ alkyl or $C_1$-$C_6$ alkyl substituted with one or more $R^{3-2}$;
(4) $R^{3-2}$ is independently halogen;
(5) $R^4$ is independently halogen, $C_1$-$C_6$ alkyl, or $C_1$-$C_6$ alkoxy;
(6) ring B is a $C_6$-$C_{10}$ unsaturated carbocyclic ring or a "6- to 15-membered unsaturated heterocyclic ring with 1, 2, 3, 4, or 5 heteroatoms selected from 1, 2, or 3 of N, O, and S";
(7) L is a single bond, $-CR^{L1}R^{L2}-$, -O-, -S-, or $-NR^{L3}-$; preferably, L is a single bond, -O-, -S-, or $-NR^{L3}-$;
(8) $R^{L3}$ is H;
(9) ring A is a $C_6$-$C_{10}$ aromatic ring or a "5- to 6-membered heteroaromatic ring with 1, 2, or 3 heteroatoms selected from 1, 2, or 3 of N, O, and S";
(10) m3 is 1;
(11) $R^5$ is independently halogen, $C_1$-$C_6$ alkyl, or $C_1$-$C_6$ alkyl substituted with one or more $R^{5-1}$
(12) $R^{5-1}$ is independently halogen.

**7.** The compound represented by formula I, the pharmaceutically acceptable salt thereof, the ester thereof, the stereoisomer thereof, the tautomer thereof, the polymorph thereof, the solvate thereof, the metabolite thereof, the isotopic derivative thereof, or the prodrug thereof according to claim 1, wherein $R^5$ is independently $C_3$-$C_6$ cycloalkyl.

**8.** The compound represented by formula I, the pharmaceutically acceptable salt thereof, the ester thereof, the stereoisomer thereof, the tautomer thereof, the polymorph thereof, the solvate thereof, the metabolite thereof, the isotopic derivative thereof, or the prodrug thereof according to claim 1, wherein one or two or more of the following conditions are satisfied:

(1) $R^1$ and $R^2$ are independently $C_1$-$C_6$ alkyl, or $R^1$ and $R^2$, together with the atom to which they are attached, form 3- to 12-membered heterocycloalkyl, wherein the 3- to 12-membered heterocycloalkyl contains, in addition to the attached P atom, 0, 1, 2, or 3 heteroatoms selected from 1, 2, or 3 of N, O, and S;
(2) $R^4$ is independently $C_1$-$C_6$ alkyl;
(3) $R^5$ is independently $C_1$-$C_6$ alkyl substituted with one or more $R^{5-1}$; and
(4) m2 is 0 or 1.

**9.** The compound represented by formula I, the pharmaceutically acceptable salt thereof, the ester thereof, the stereoisomer thereof, the tautomer thereof, the polymorph thereof, the solvate thereof, the metabolite thereof, the isotopic derivative thereof, or the prodrug thereof according to claim 1 or 2, wherein

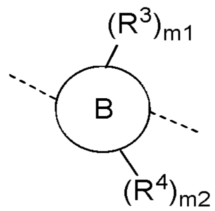

is

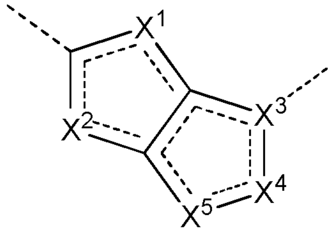

,

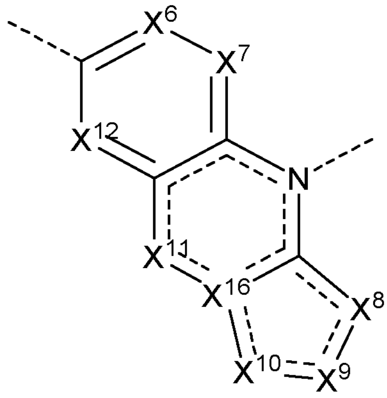

,

or

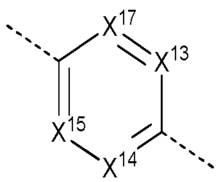

,

wherein

- - - - - represents a single bond or a double bond;

$X^1$ is S, $-CR^{X1}=CR^{X1}-$, $-N=CR^{X1}-$, or $-CR^{X1}=N-$;

$X^2$ is $CR^{X1}$, N, or S;

$X^3$ is C, $CR^{X1}$, or N;

$X^4$ is $CR^{X1}$, N, -C(O)-, or $CR^{X1}R^{X2}$;

$X^5$ is N, $NR^{X3}$, $CR^{X1}$, $CR^{X1}R^{X2}$, $-CR^{X1}=CR^{X1}-$, $-N=CR^{X1}-$, $-CR^{X1}=N-$, $-CR^{X1}R^{X2}-CR^{X1}R^{X2}-$, $-O-CR^{X1}R^{X2}-$, -C(O)-N=, $-C(O)-CR^{X1}=$, or $-NR^{X3}-CR^{X1}R^{X2}-$;

$X^6$ is $CR^{X1}$ or N;

$X^7$ is $CR^{X1}$ or N;

$X^8$ is $CR^{X1}$, $CR^{X1}R^{X2}$, N, $NR^{X3}$, -C(O)-, O, or S;

$X^9$ is $CR^{X1}$, N, $NR^{X3}$, $-CR^{X1}=CR^{X1}-$, $=CR^{X1}-CR^{X1}=$, $-CR^{X1}R^{X2}-$, $-CR^{X1}R^{X2}-CR^{X1}R^{X2}-$, $=N-CR^{X1}=$, $=CR^{X1}-N=$, $=CR^{X1}-R^{X3}-$, $-NR^{X3}-CR^{X1}=$, $-NR^{X3}-N=$, or $=N-NR^{X3}-$;

$X^{10}$ is $CR^{X1}$, $CR^{X1}R^{X2}$, N, $NR^{X3}$, -C(O)-, O, or S;

$X^{11}$ is absent, O, or S;

$X^{12}$ is $CR^{X1}$ or N;

$X^{16}$ is C or N;

$X^{13}$ is $CR^{X1}$ or N;

$X^{14}$ is $CR^{X4}$ or N;

$X^{15}$ is $CR^{X1}$ or N;

$X^{17}$ is $CR^{X1}$ or N;

each of $R^{X1}$ and $R^{X2}$ is independently H, $C_1$-$C_6$ alkyl, halogen, or CN; or $R^{X1}$ and $R^{X2}$, together with the atom to which they are attached, form $C_3$-$C_4$ cycloalkyl;

each $R^{X3}$ is independently H, $C_1$-$C_6$ alkyl, halogen, or CN;

$R^{X4}$ is H, "5- to 12-membered saturated or unsaturated heterocyclyl with 1, 2, or 3 heteroatoms selected from 1, 2, or 3 of N, O, and S", "5- to 12-membered saturated or unsaturated heterocyclyl with 1, 2, or 3 heteroatoms selected from 1, 2, or 3 of N, O, and S" substituted with one or more $R^{X4-1}$, $C_6$-$C_{10}$ aryl, or $C_6$-$C_{10}$ aryl substituted with one or more $R^{X4-5}$;

$R^{X4-1}$ and $R^{X4-5}$ are independently H, deuterium, halogen, $C_1$-$C_6$ alkyl, $C_1$-$C_6$ alkoxy, CN, $C_1$-$C_6$ alkyl substituted with one or more $R^{X4-2}$, $C_1$-$C_6$ alkoxy substituted with one or more $R^{X4-3}$, "3- to 12-membered heterocycloalkyl with 1, 2, or 3 heteroatoms selected from 1, 2, or 3 of N, O, and S", "5- to 12-membered heteroaryl with 1, 2, or 3 heteroatoms selected from 1, 2, or 3 of N, O, and S", $C(=O)NR^cR^d$, $C_3$-$C_6$ cycloalkyl, or $C_3$-$C_6$ cycloalkyl substituted with one or more $R^{X4-4}$;

$R^{X4-2}$ and $R^{X4-3}$ are independently deuterium, halogen, OH, CN, $C_1$-$C_6$ alkoxy, "3- to 12-membered heterocycloalkyl with 1, 2, or 3 heteroatoms selected from 1, 2, or 3 of N, O, and S", $C(=O)NR^cR^d$, or $NR^bC(=O)R^a$;
$R^{X4-4}$ is independently CN, $C(=O)NR^cR^d$, or $C(=O)OR^a$;
$R^a$, $R^b$, $R^c$, and $R^d$ are independently H or $C_1$-$C_6$ alkyl.

**10.** The compound represented by formula I, the pharmaceutically acceptable salt thereof, the ester thereof, the stereoisomer thereof, the tautomer thereof, the polymorph thereof, the solvate thereof, the metabolite thereof, the isotopic derivative thereof, or the prodrug thereof according to claim 9, wherein one or two or more of the following conditions are satisfied:

(1)

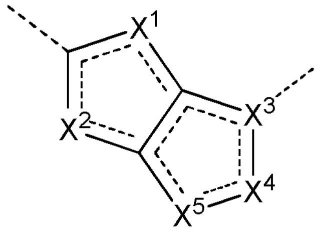

is

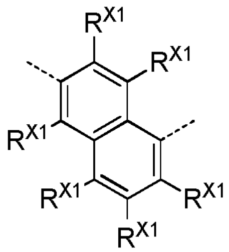
,
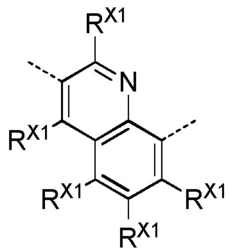
,
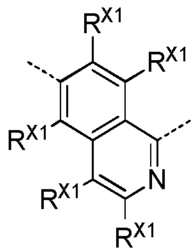
,

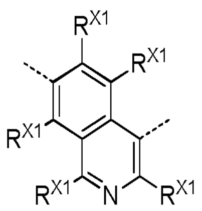
,
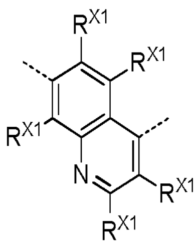
,
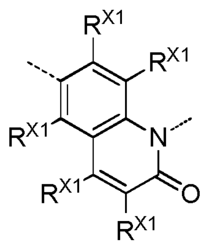
,
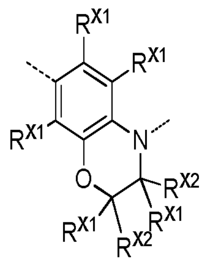
,
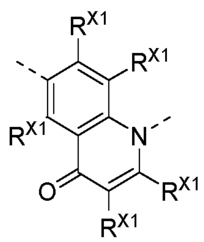
,

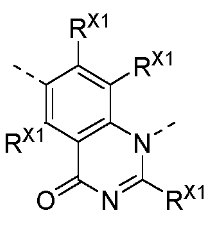
, or
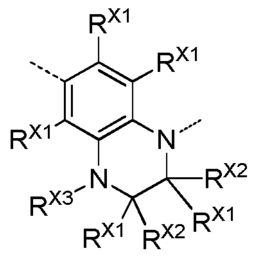
;

preferably,

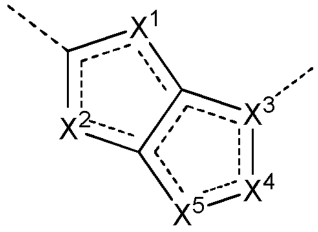

is

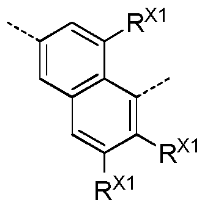 , 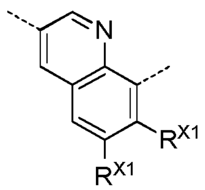 , 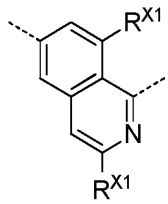 , 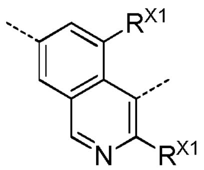 , 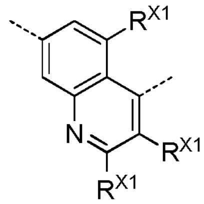 ,

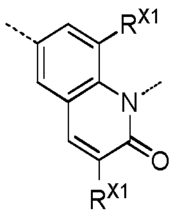 , 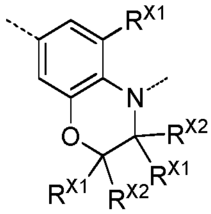 , or 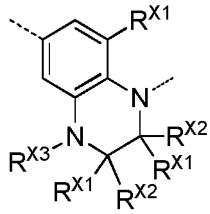 ;

(2)

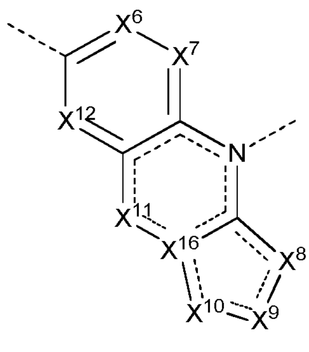

is

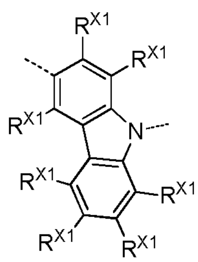 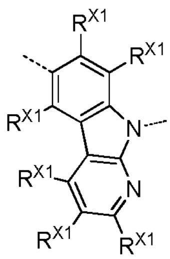

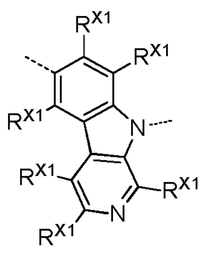
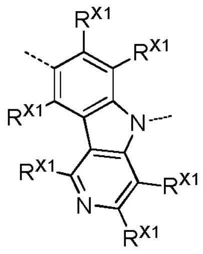
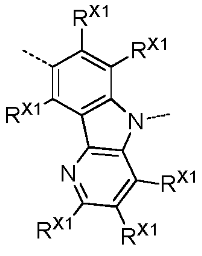
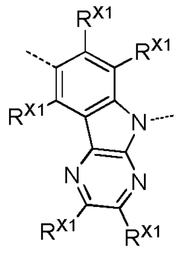
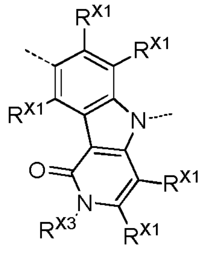

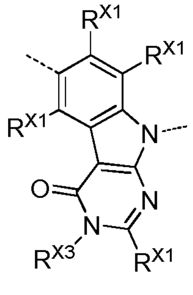
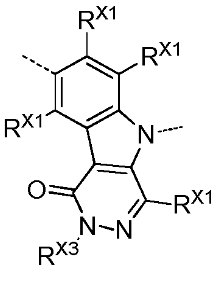
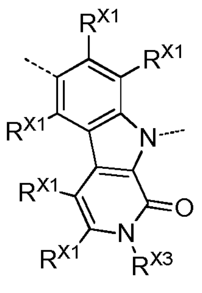
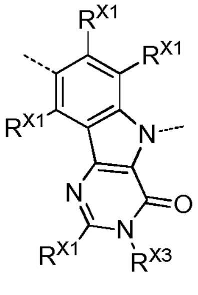
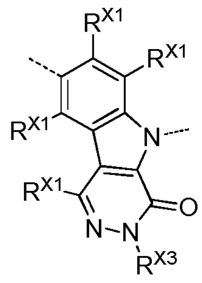

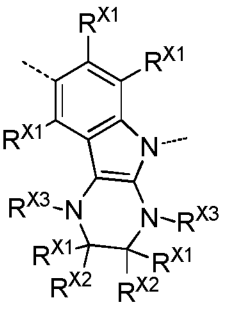
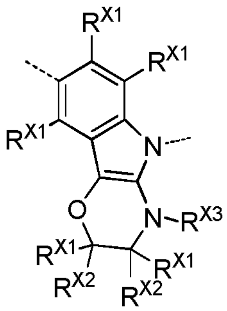
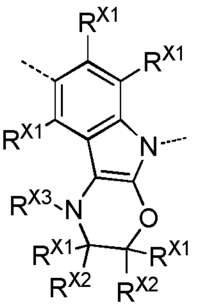

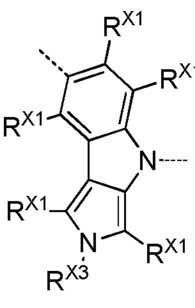
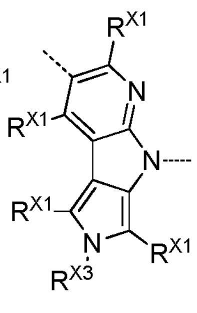
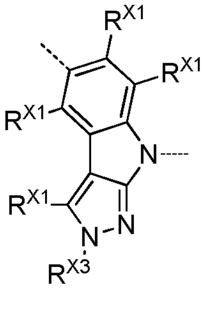
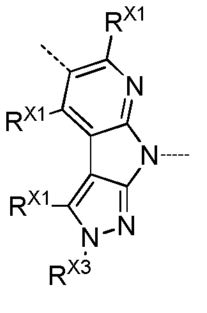
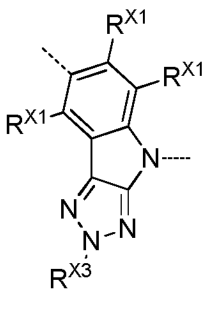

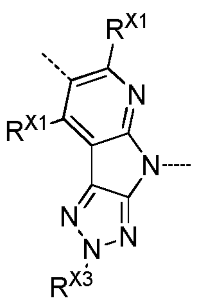
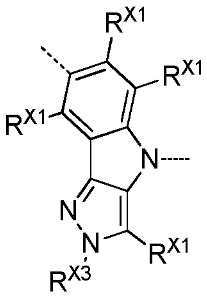
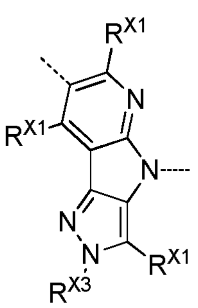
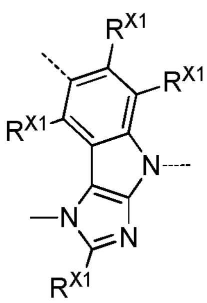
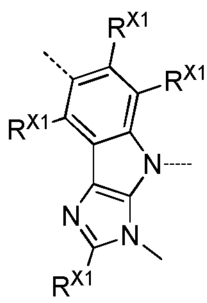

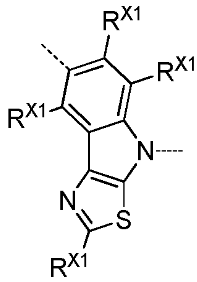 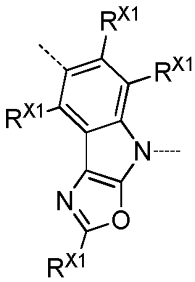 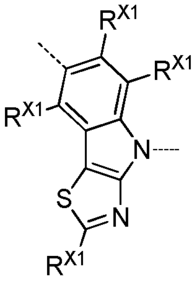 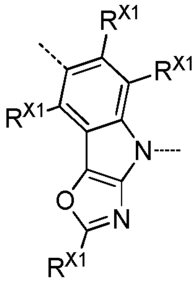 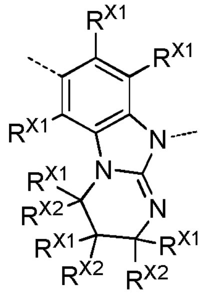

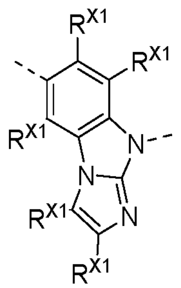 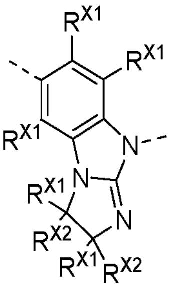 ;

preferably,

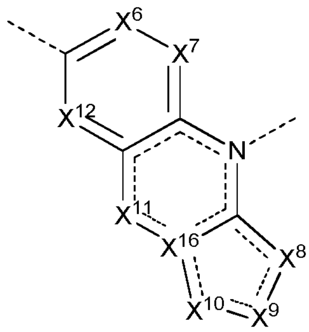

is

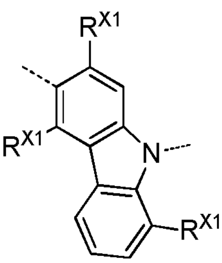 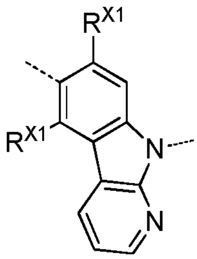 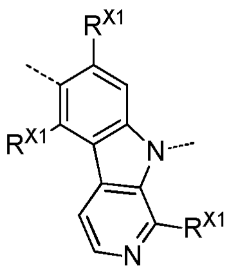

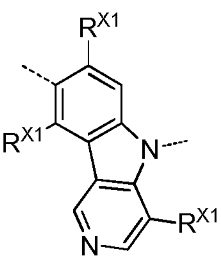 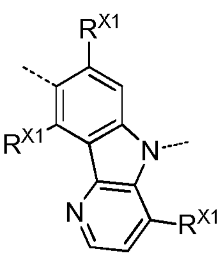 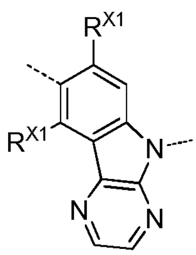

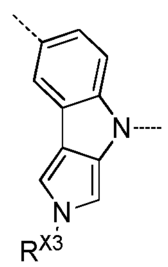 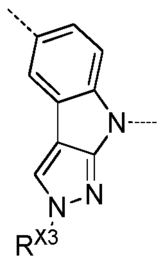 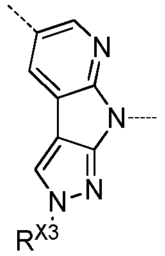 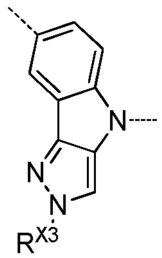 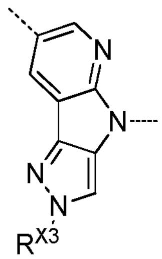

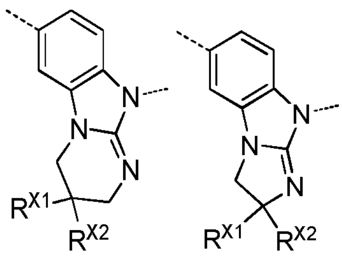 ;

(3)

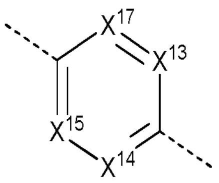 is 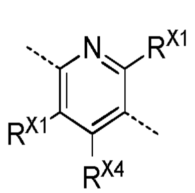 , 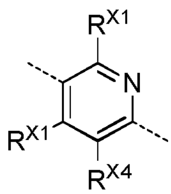 , or 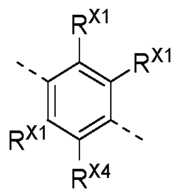 ;

and
(4) $R^{X4}$ is

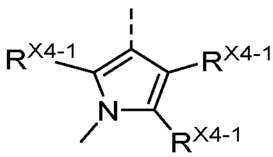 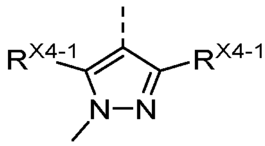 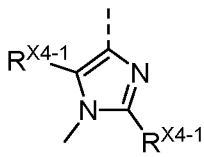

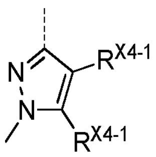 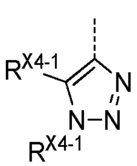 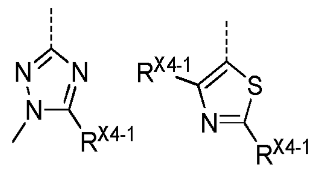 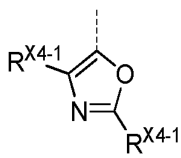 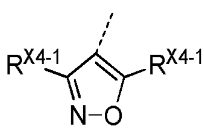

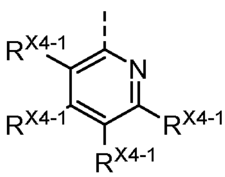

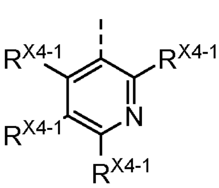 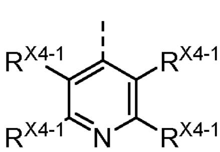 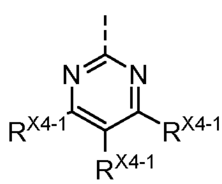 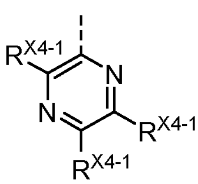

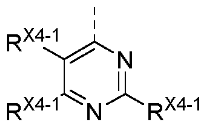 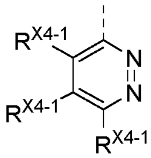 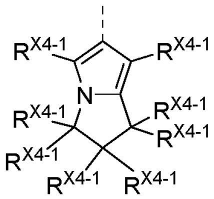 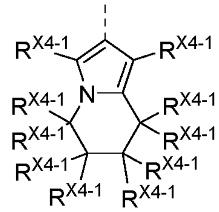 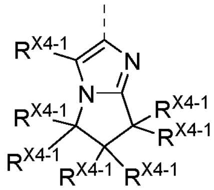

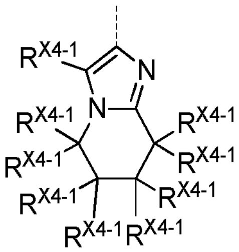 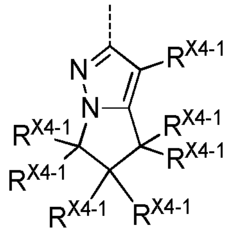 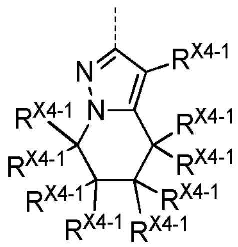 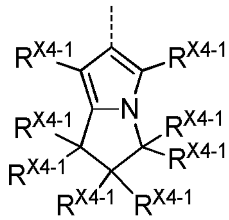

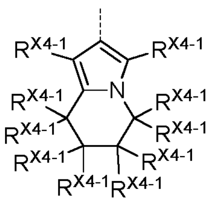 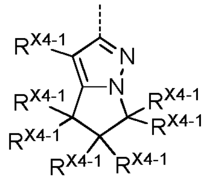 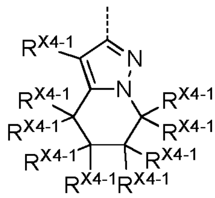 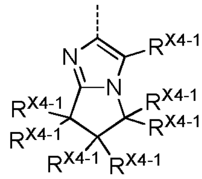 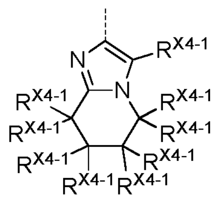

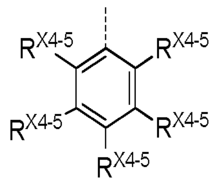 .

**11.** The compound represented by formula I, the pharmaceutically acceptable salt thereof, the ester thereof, the stereoisomer thereof, the tautomer thereof, the polymorph thereof, the solvate thereof, the metabolite thereof, the isotopic derivative thereof, or the prodrug thereof according to claim 9, wherein one or two or more of the following conditions are satisfied:

(1)

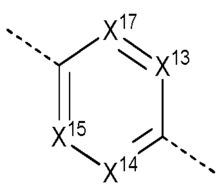 is 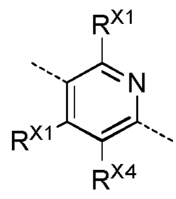 or 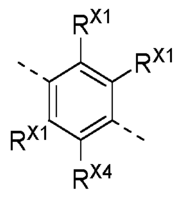 ;

and

(2) $R^{X4}$ is

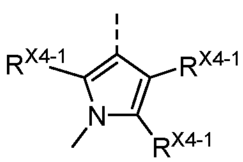 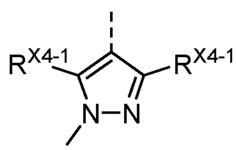 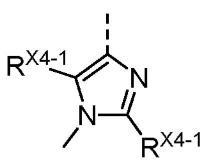

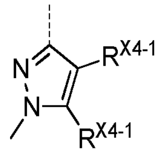 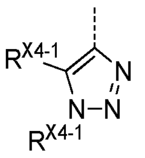 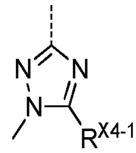 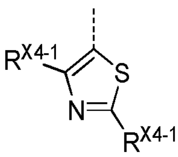 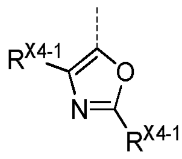 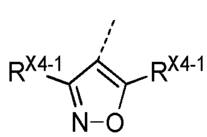

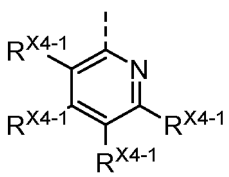

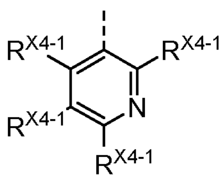 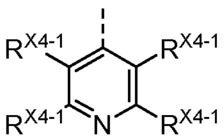 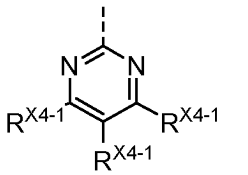 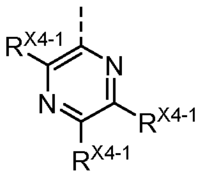

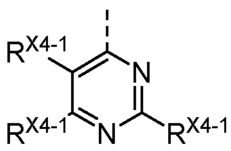 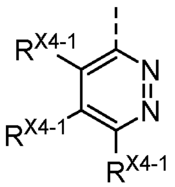 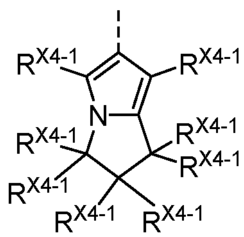 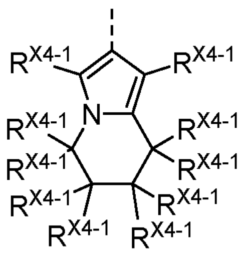

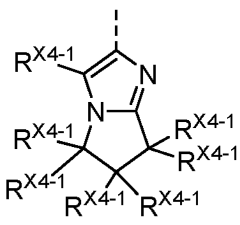

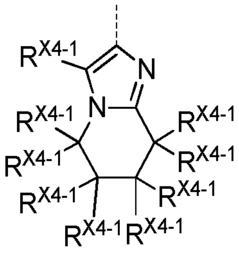 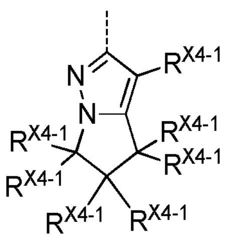 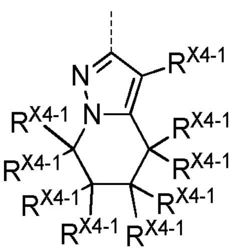 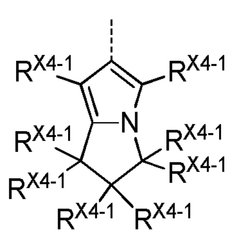

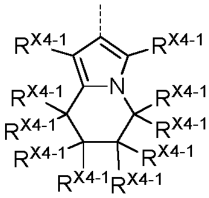 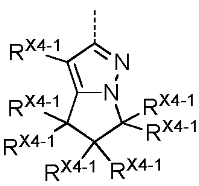 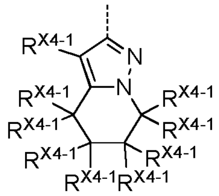 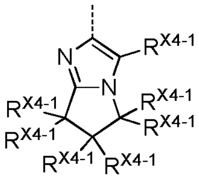 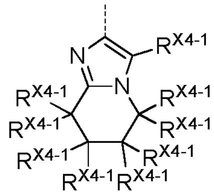

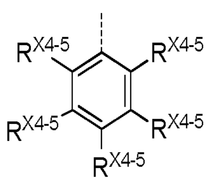 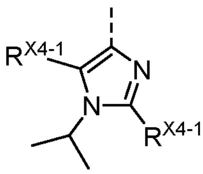 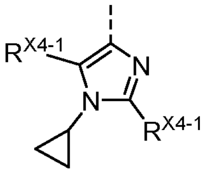 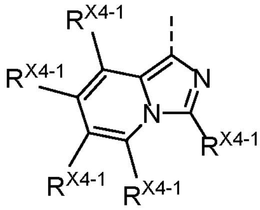 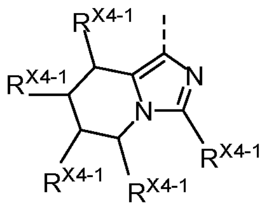

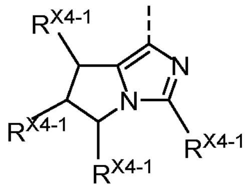 ;

preferably, $R^{X4}$ is

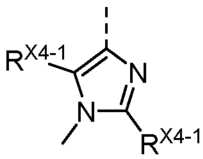 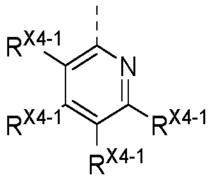 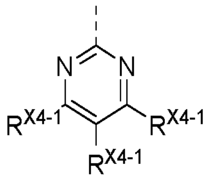

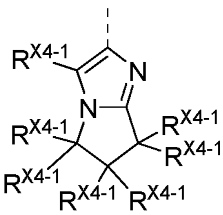 .

**12.** The compound represented by formula I, the pharmaceutically acceptable salt thereof, the ester thereof, the stereoisomer thereof, the tautomer thereof, the polymorph thereof, the solvate thereof, the metabolite thereof, the isotopic derivative thereof, or the prodrug thereof according to claim 9, wherein one or two or more of the following conditions are satisfied:

(1) ring B is

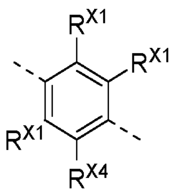 ;

(2) each $R^{X1}$ is independently H;
(3) $R^{X4}$ is "5- to 12-membered saturated or unsaturated heterocyclyl with 1, 2, or 3 heteroatoms selected from 1, 2, or 3 of N, O, and S" or "5- to 12-membered saturated or unsaturated heterocyclyl with 1, 2, or 3 heteroatoms selected from 1, 2, or 3 of N, O, and S" substituted with one or more $R^{X4-1}$; and
(4) $R^{X4-1}$ and $R^{X4-5}$ are independently H or $C_1$-$C_6$ alkyl, such as $C_1$-$C_6$ alkyl.

**13.** The compound represented by formula I, the pharmaceutically acceptable salt thereof, the ester thereof, the stereoisomer thereof, the tautomer thereof, the polymorph thereof, the solvate thereof, the metabolite thereof, the isotopic derivative thereof, or the prodrug thereof according to claim 9, wherein one or two or more of the following conditions are satisfied: in $R^{X4}$, the "5- to 12-membered saturated or unsaturated heterocyclyl with 1, 2, or 3

heteroatoms selected from 1, 2, or 3 of N, O, and S" is "5- to 10-membered unsaturated heterocyclyl with 1, 2, or 3 heteroatoms selected from 1, 2, or 3 of N, O, and S", for example, pyrrolyl (e.g.,

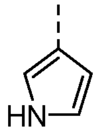

), pyrazolyl (e.g.,

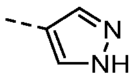
,
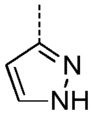
, or
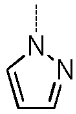

), imidazolyl (e.g.,

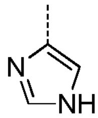

), triazolyl (e.g.,

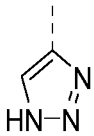
or
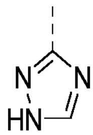

), thiazolyl (e.g.,

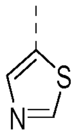

), oxazolyl (e.g.,

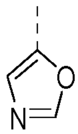

), isoxazolyl (e.g.,

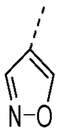

), pyridyl (e.g.,

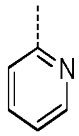, 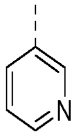, or 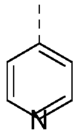,

), pyrimidinyl (e.g.,

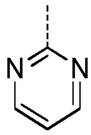 or 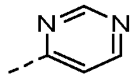

), pyrazinyl (e.g.,

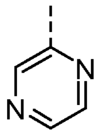

), pyridazinyl (e.g.,

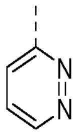), 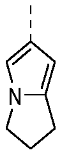, 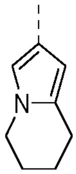, 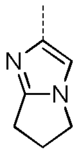, 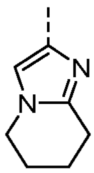, 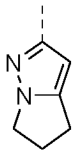, 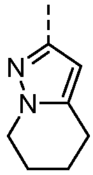,

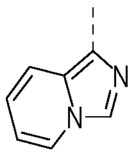, 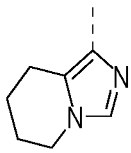, or 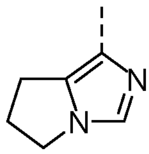,

and for another example, imidazolyl (e.g.,

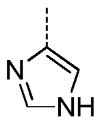

), pyrazolyl (e.g.,

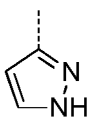 or 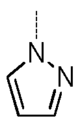), 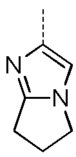,

pyridyl (e.g.,

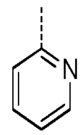

), or pyrimidinyl (e.g.,

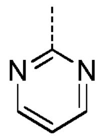

).

**14.** The compound represented by formula I, the pharmaceutically acceptable salt thereof, the ester thereof, the stereoisomer thereof, the tautomer thereof, the polymorph thereof, the solvate thereof, the metabolite thereof, the isotopic derivative thereof, or the prodrug thereof according to claim 1 or 2, wherein one or two or more of the following conditions are satisfied:

(1)

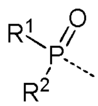 is 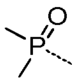, 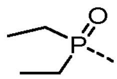, 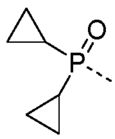, 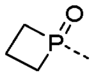, 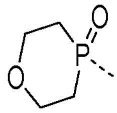, 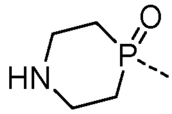, 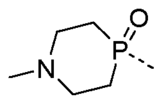,

or

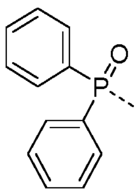;

(2)

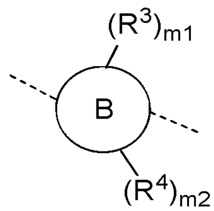

is

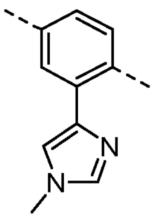 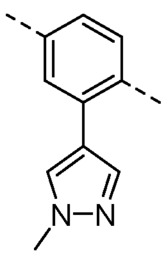 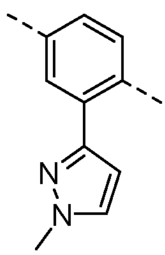 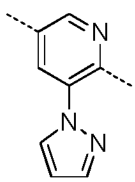 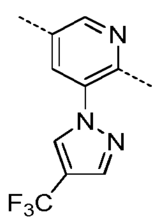

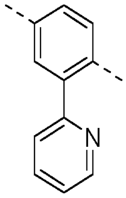 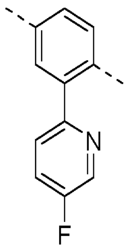 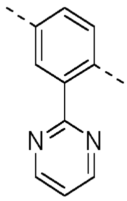 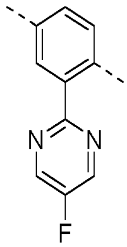 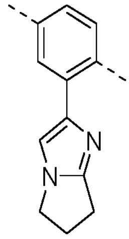 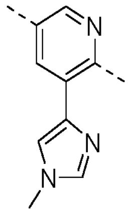 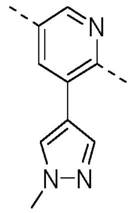

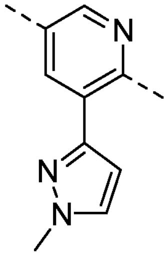 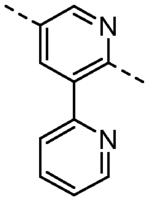 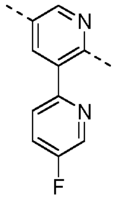 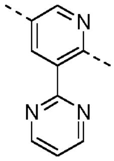 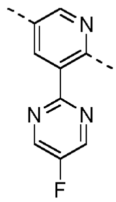 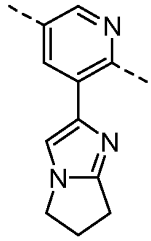 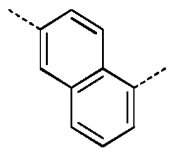

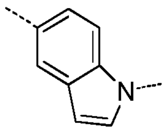 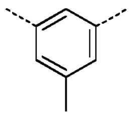 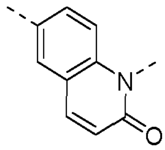 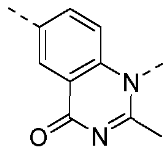

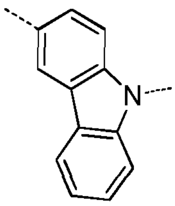 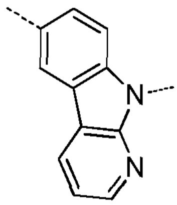 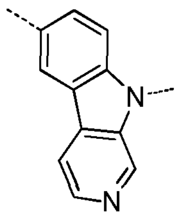 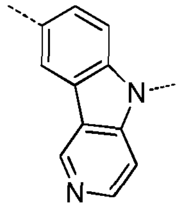

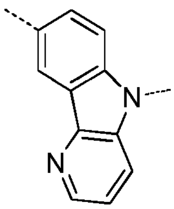 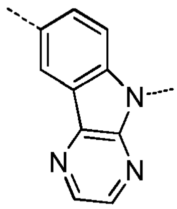

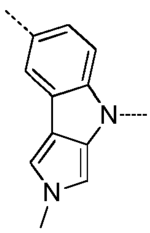 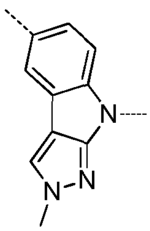 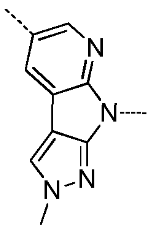 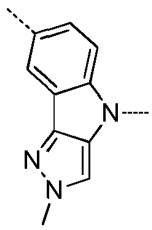 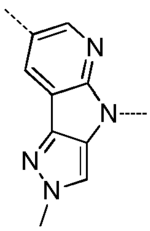 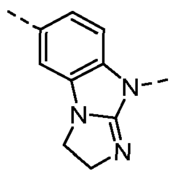

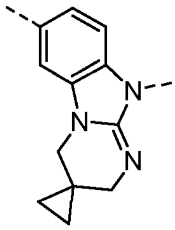 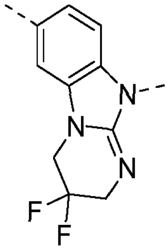 ;

(3) L is a single bond, -O-, or -NH-; and

(4) 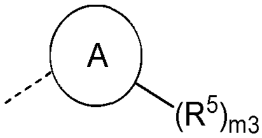

is

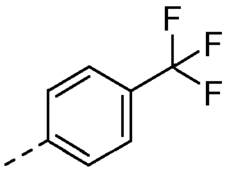 , 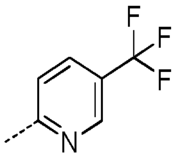 , 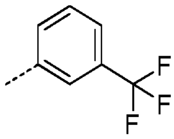 , 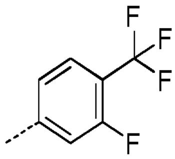 ,

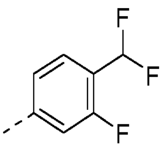 , 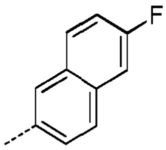 , 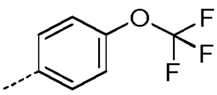 , 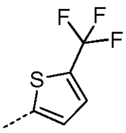 , 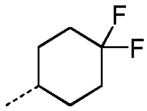 , 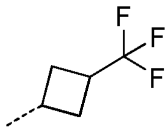 ,

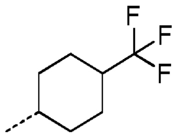 , or 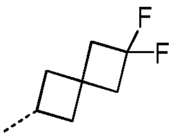 .

**15.** The compound represented by formula I, the pharmaceutically acceptable salt thereof, the ester thereof, the stereoisomer thereof, the tautomer thereof, the polymorph thereof, the solvate thereof, the metabolite thereof, the isotopic derivative thereof, or the prodrug thereof according to claim 1 or 2, wherein one or two or more of the following conditions are satisfied:

(1)

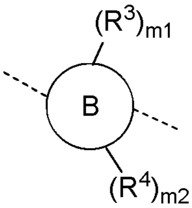

is

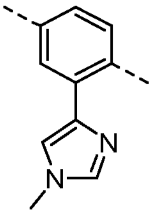
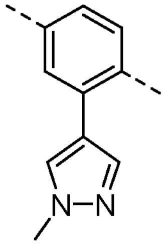
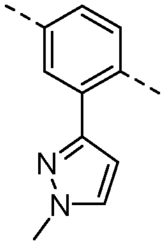
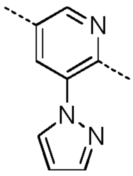
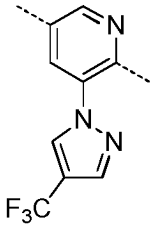

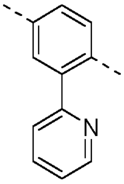
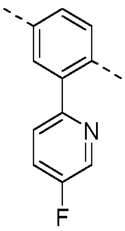
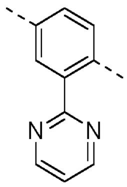
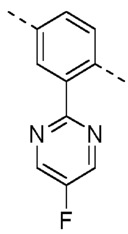
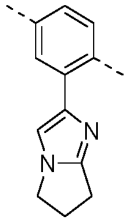
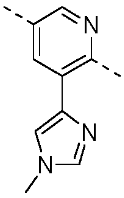
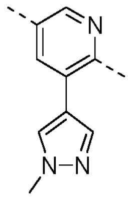

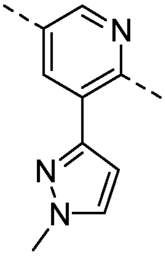
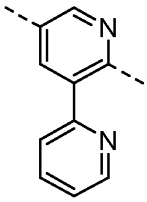
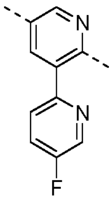
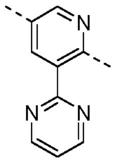
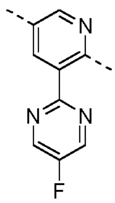
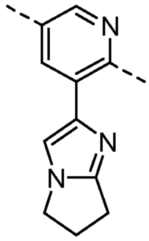
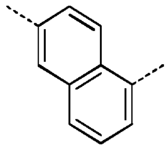

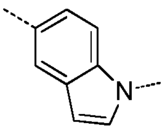
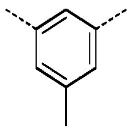
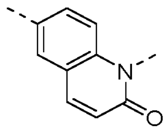
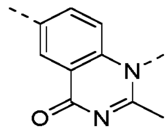

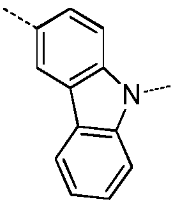
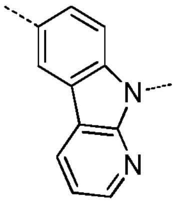
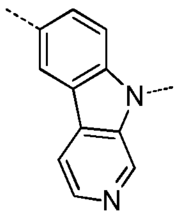
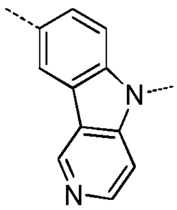

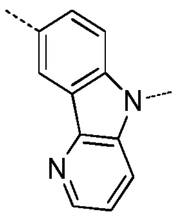
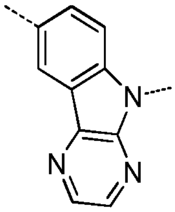

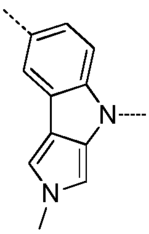
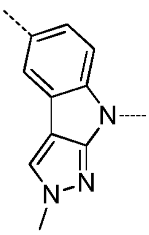
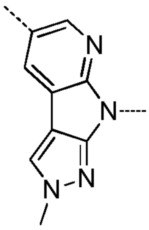
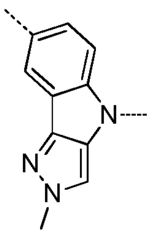
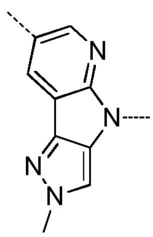
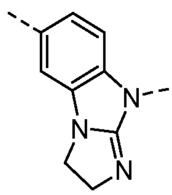

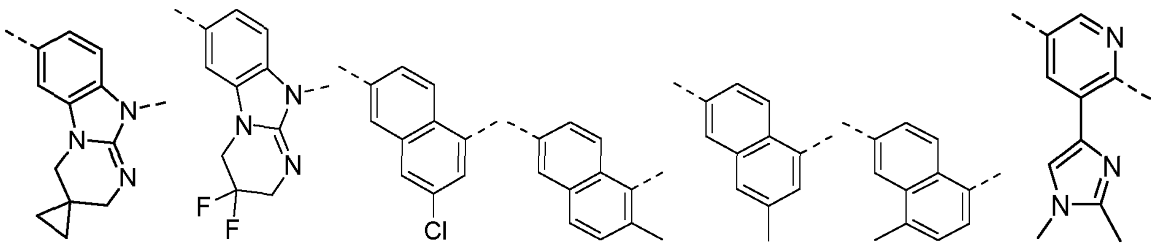

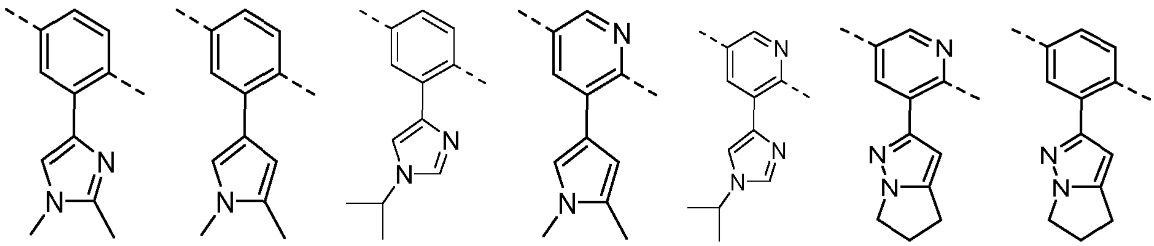

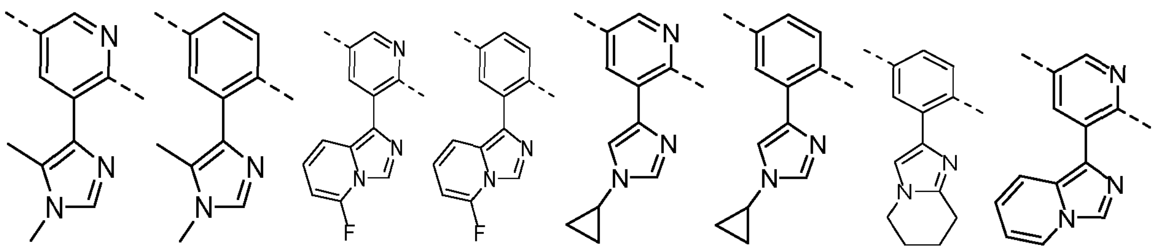

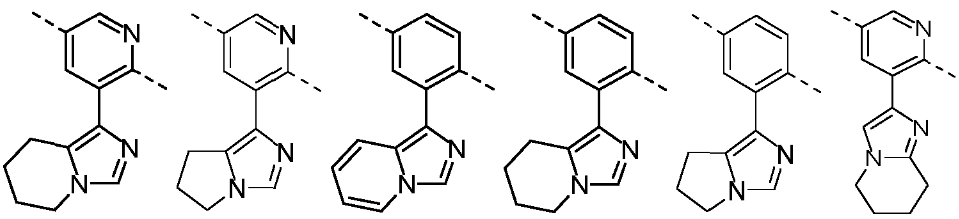 ;

(2) L is a single bond, -$CR^{L1}R^{L2}$-, -O-, -S-, or -NH-; and
(3)

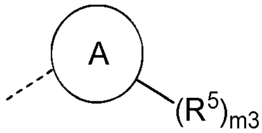

is

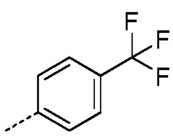 , 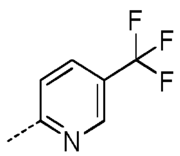 , 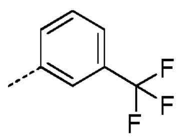 , 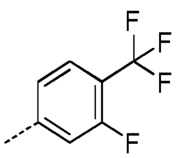 ,

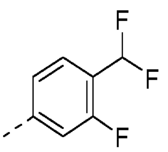 , 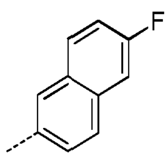 , 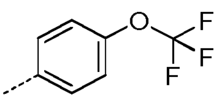 , 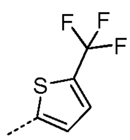 , 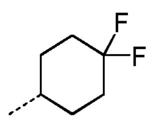 , 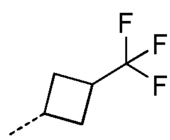 ,

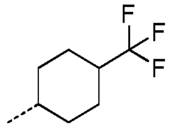, 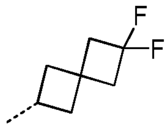, 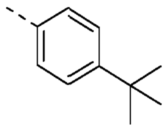, 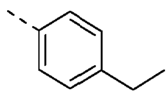, 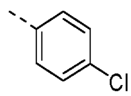, 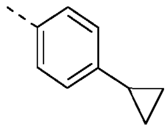,

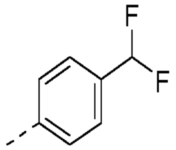, or 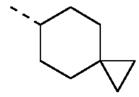.

**16.** The compound represented by formula I, the pharmaceutically acceptable salt thereof, the ester thereof, the stereoisomer thereof, the tautomer thereof, the polymorph thereof, the solvate thereof, the metabolite thereof, the isotopic derivative thereof, or the prodrug thereof according to any one of claims 1-15, wherein the compound represented by formula I is a compound represented by the following formula I-1, I-2, 1-3, I-4, I-5, I-6, or I-7:

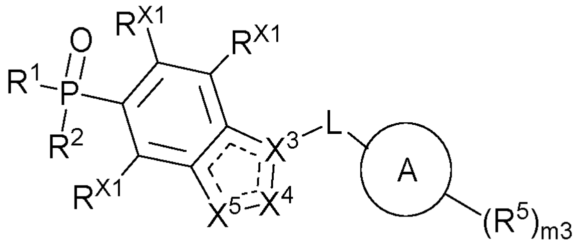

I-1

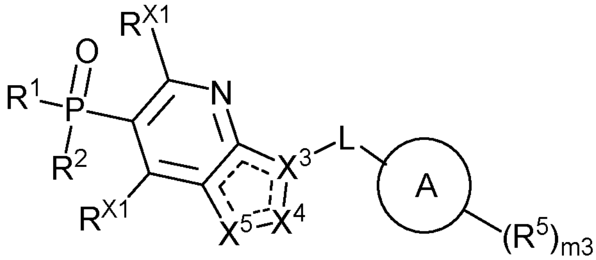

I-2

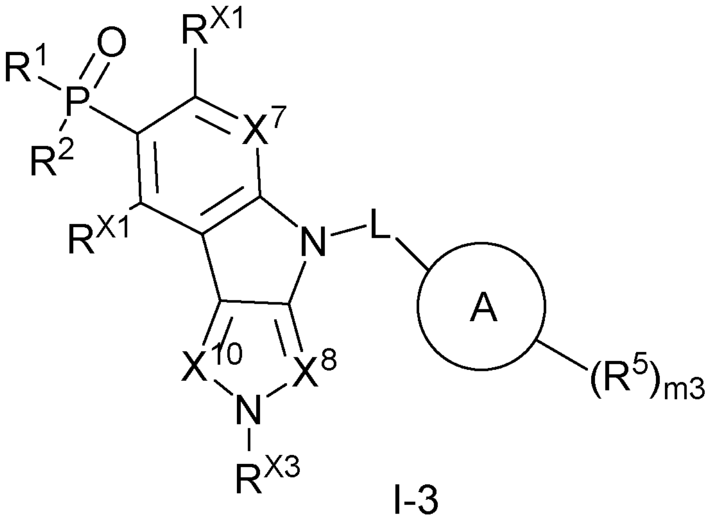

I-3

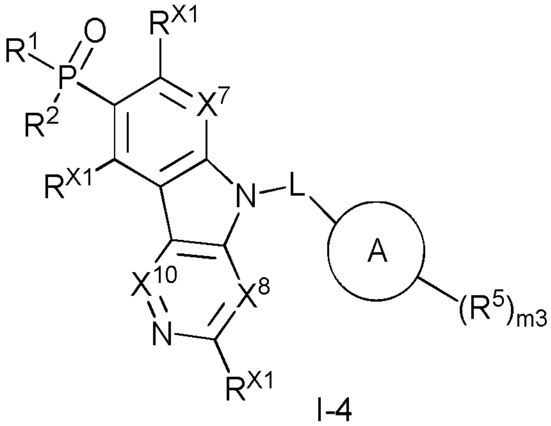

I-4

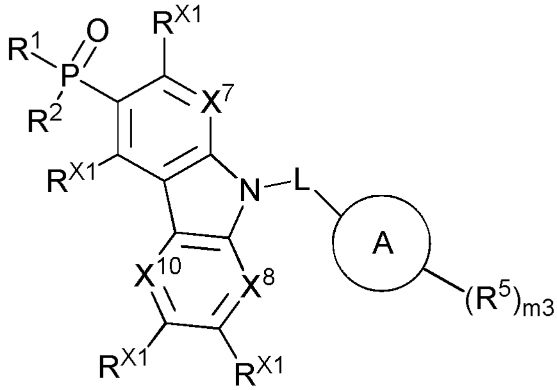

I-5

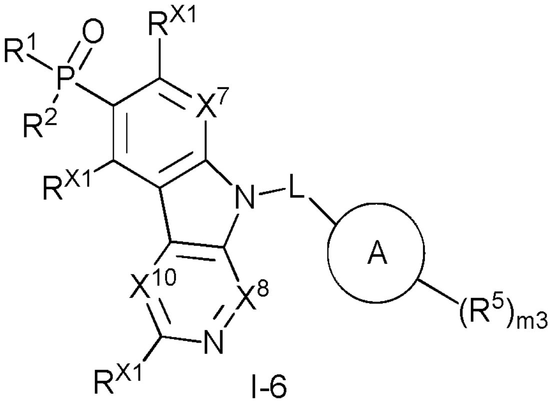

I-6

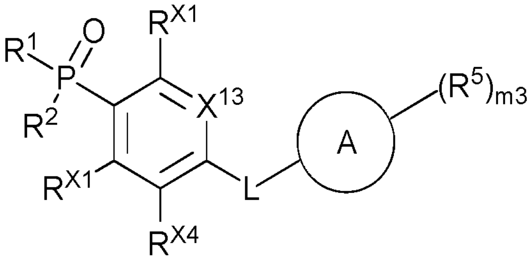

I-7

wherein $X^3$ is C or N;
$X^4$ is $CR^{X1}$, N, -C(O)-, or $CR^{X1}R^{X2}$;
$X^5$ is $-CR^{X1}=CR^{X1}-$, $-N=CR^{X1}-$, $-CR^{X1}=N-$, $-O-CR^{X1}R^{X2}-$, or $-NR^{X3}-CR^{X1}R^{X2}-$;
$X^8$ is $CR^{X1}$ or N;
$X^{10}$ is $CR^{X1}$ or N;
$R^1$, $R^2$, L, ring A, $R^5$, and m3 are as defined in any one of claims 1-15;
$X^7$, $X^{13}$, $R^{X1}$, $R^{X2}$, $R^{X3}$, and $R^{X4}$ are as defined in any one of claims 9-15.

**17.** The compound represented by formula I, the pharmaceutically acceptable salt thereof, the ester thereof, the stereoisomer thereof, the tautomer thereof, the polymorph thereof, the solvate thereof, the metabolite thereof, the isotopic derivative thereof, or the prodrug thereof according to claim 1 or 2, wherein the compound represented by formula I is any one of the following compounds:

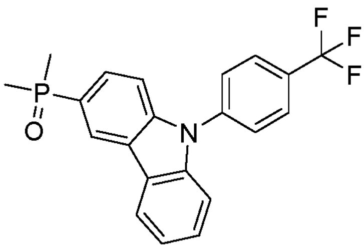 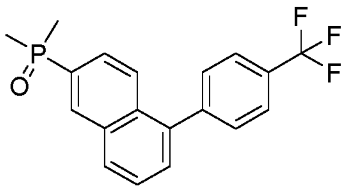 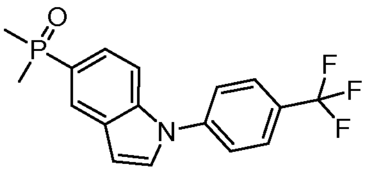

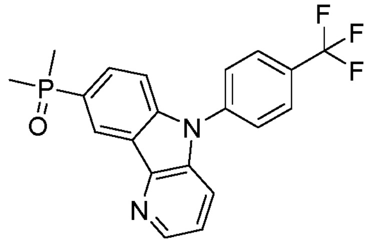
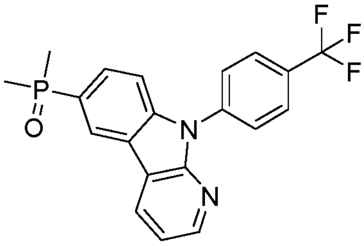
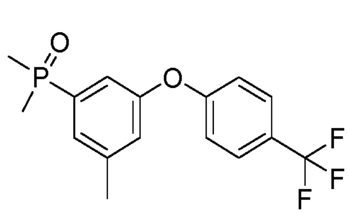

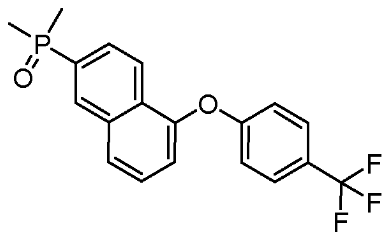
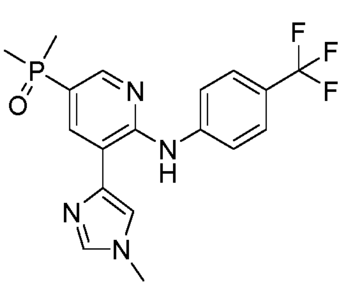
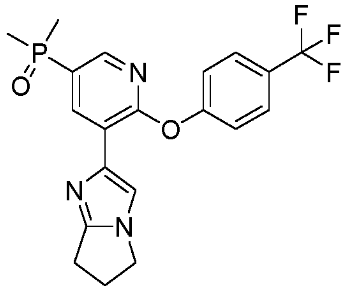

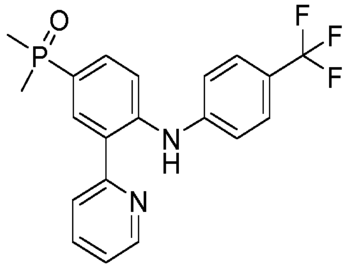
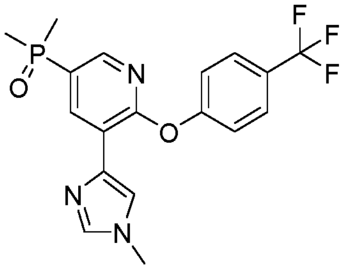
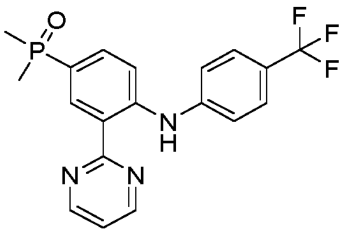

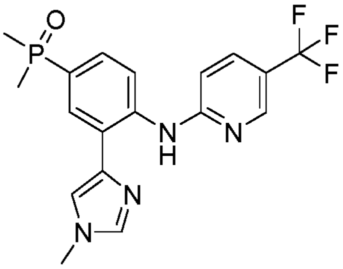
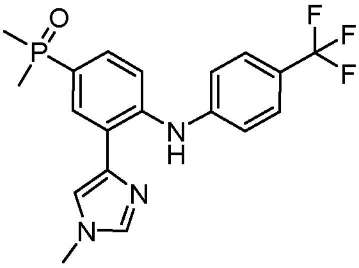
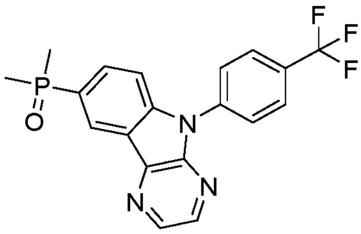

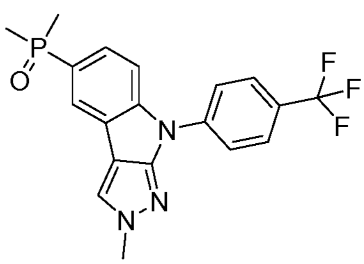
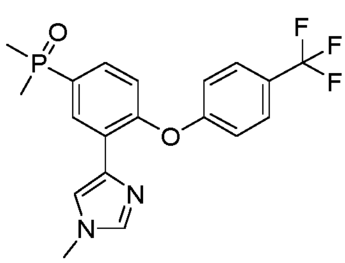
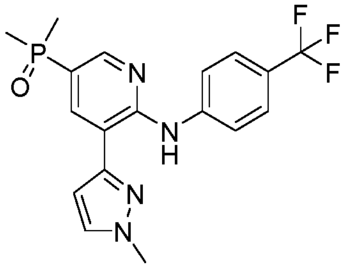

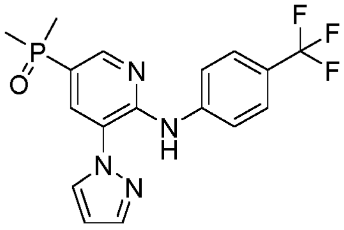

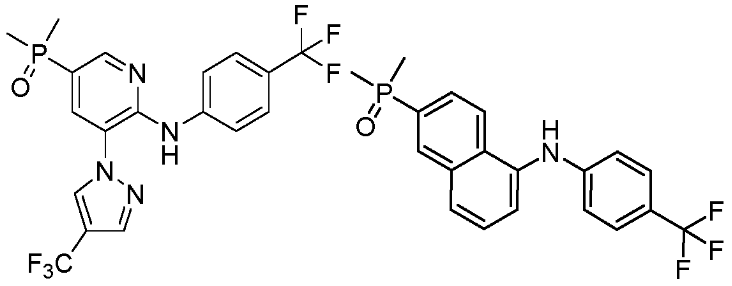

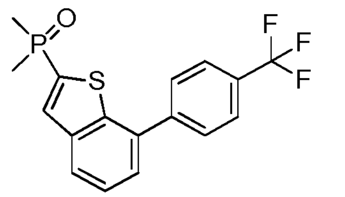

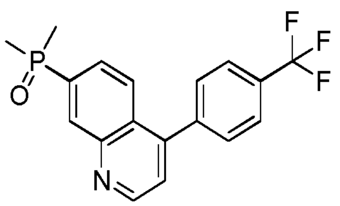

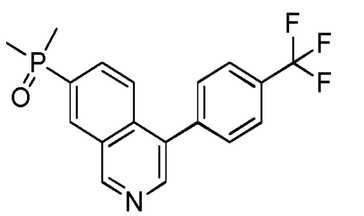

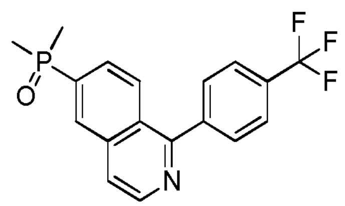

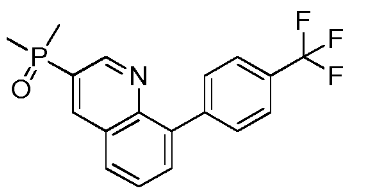

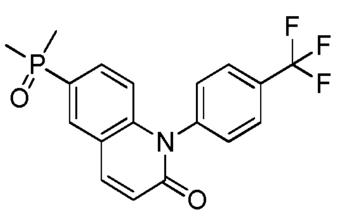

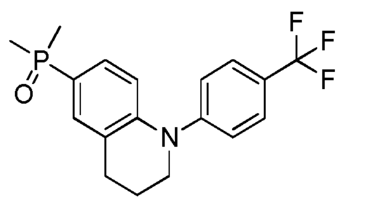

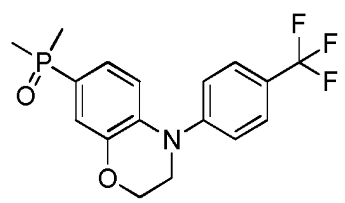

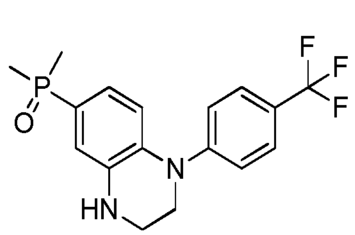

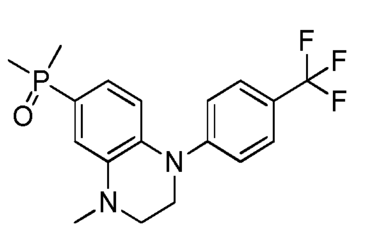

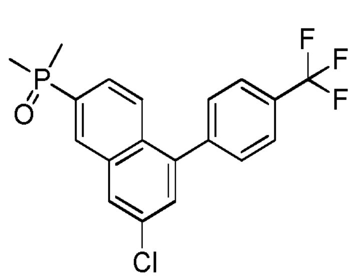

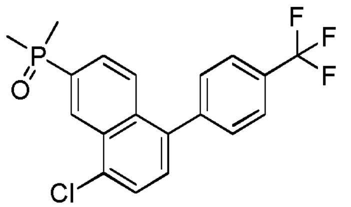

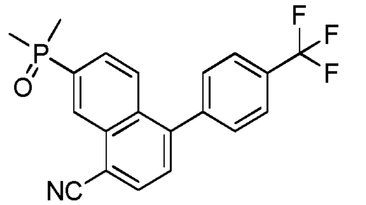

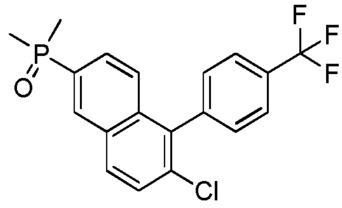

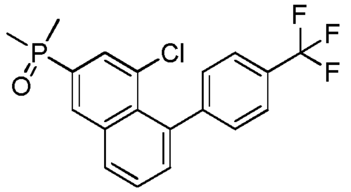

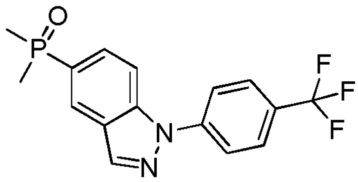

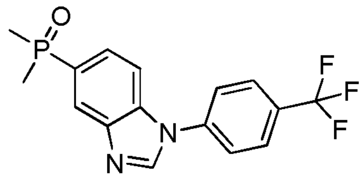

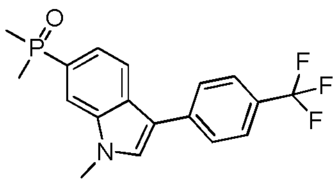

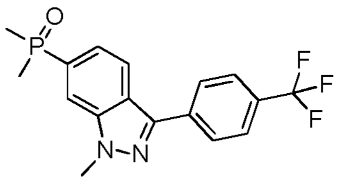

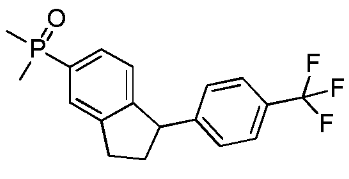

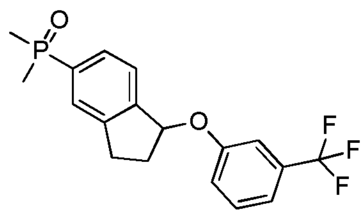

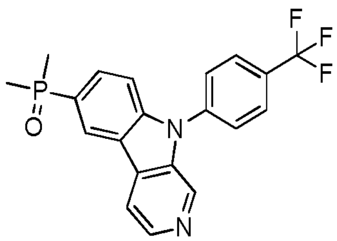

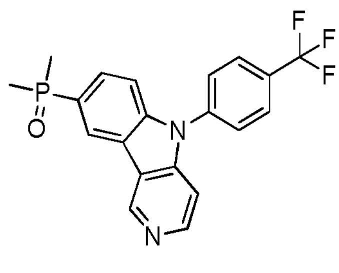

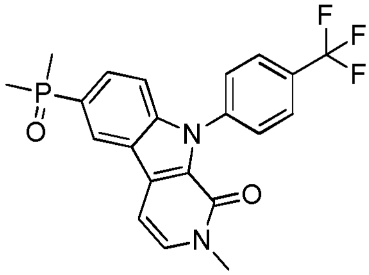

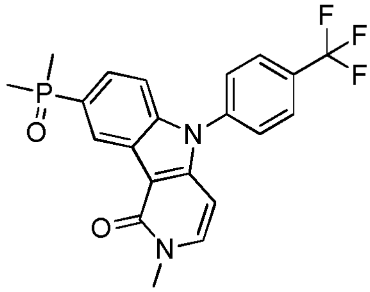

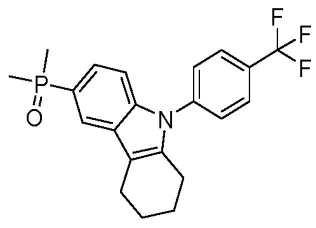
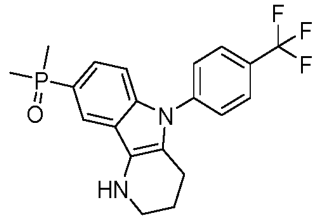
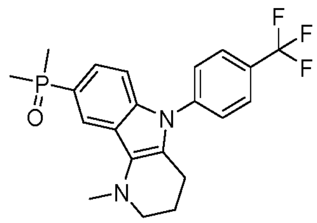
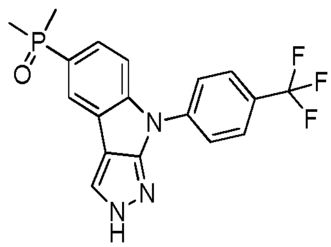
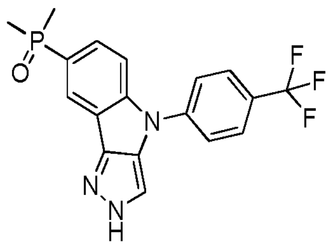
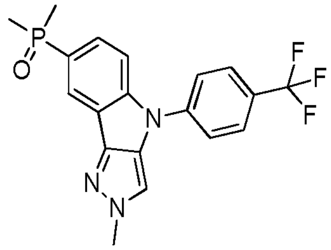
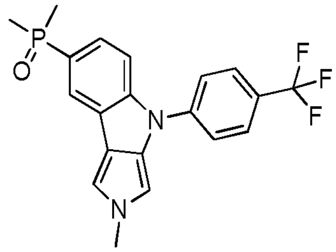
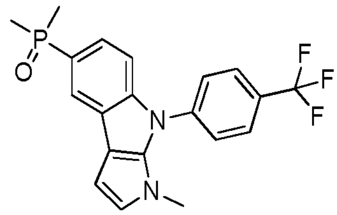
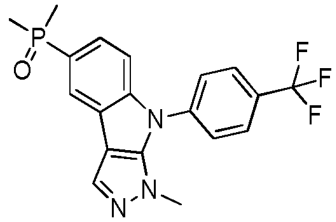
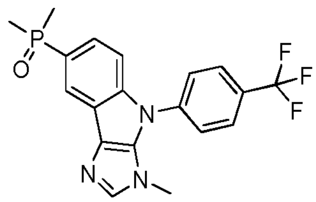
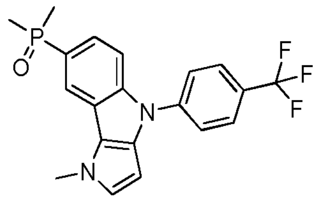
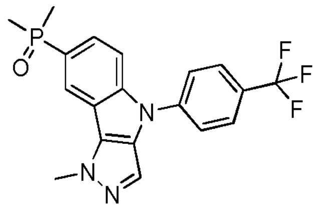
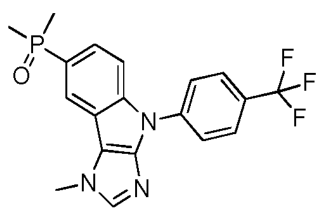
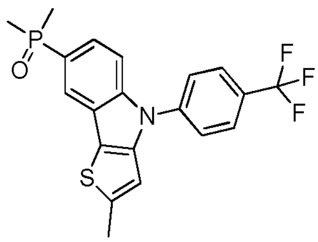
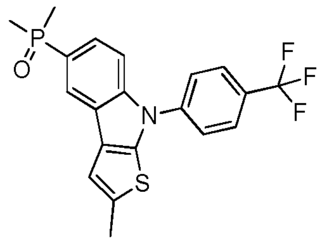
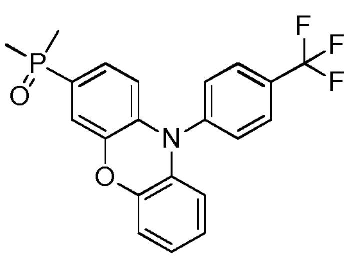
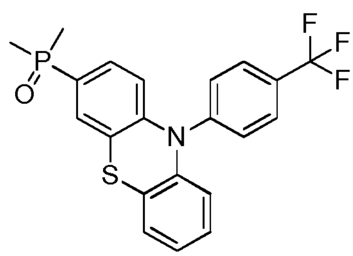

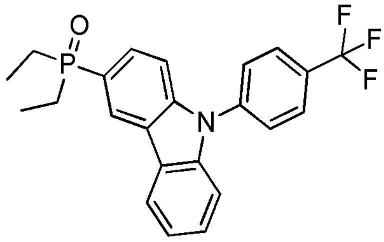
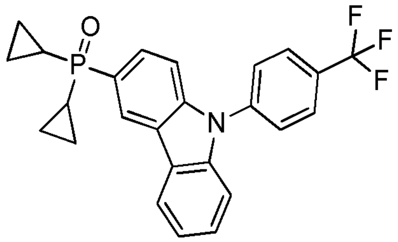
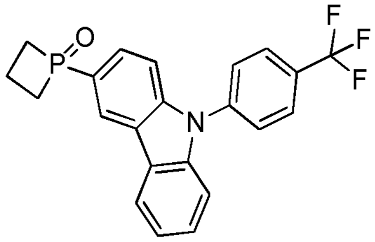

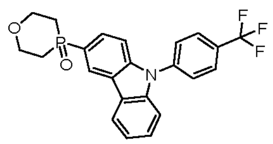
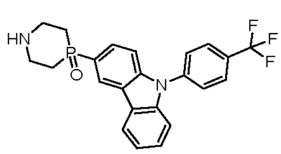
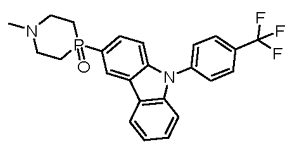
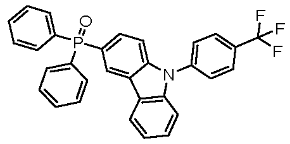

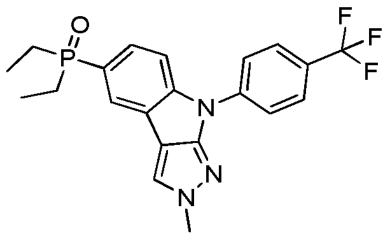
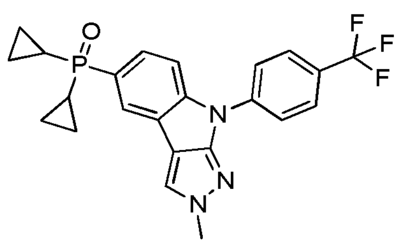
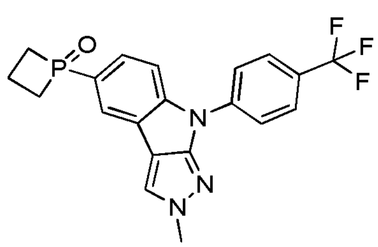

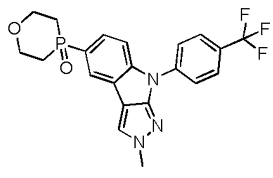
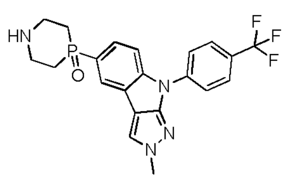
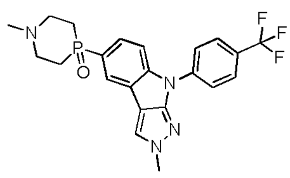
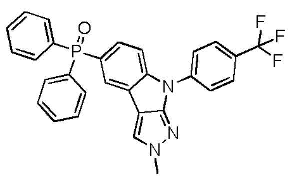

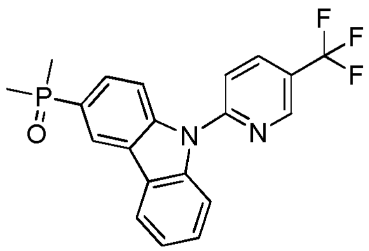
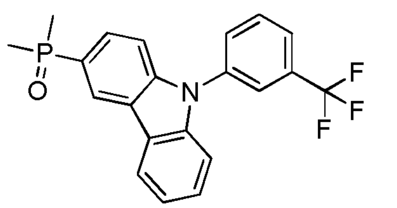
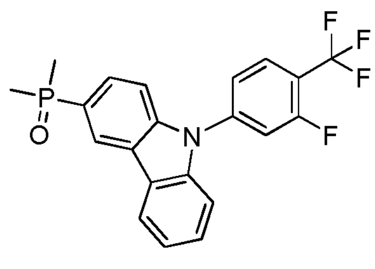

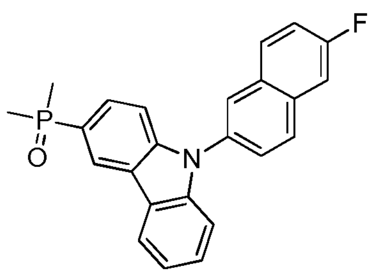
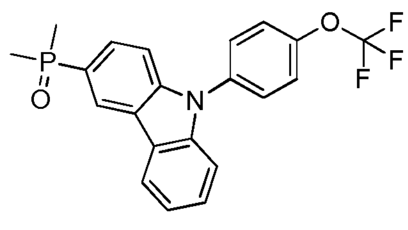
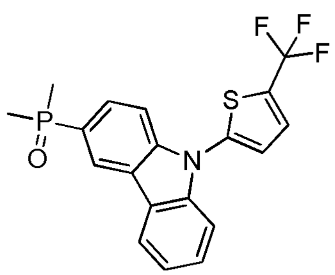

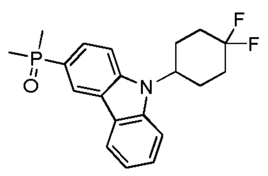
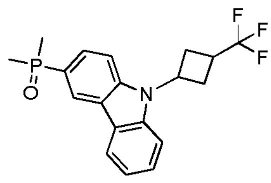
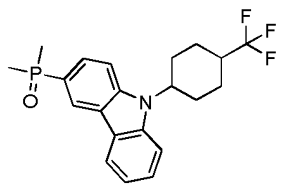
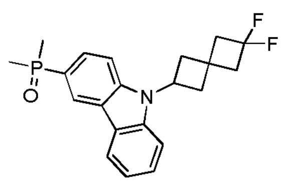

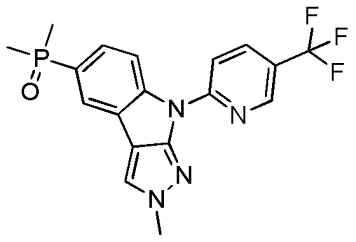

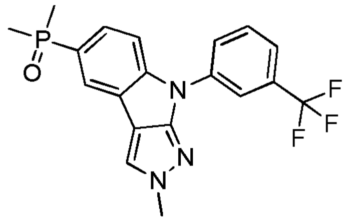

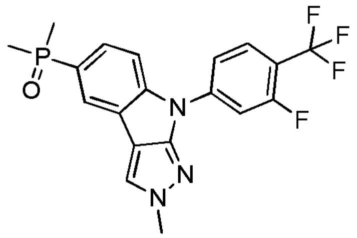

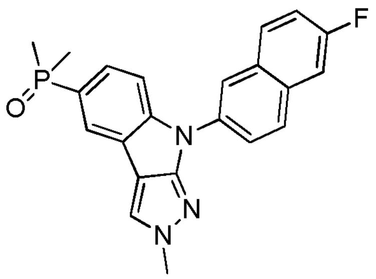

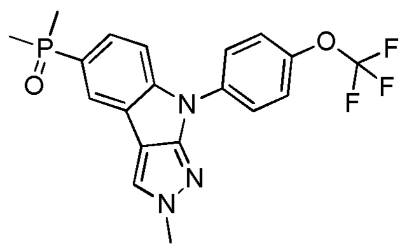

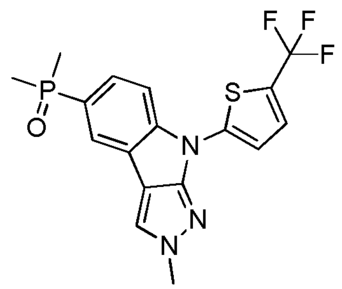

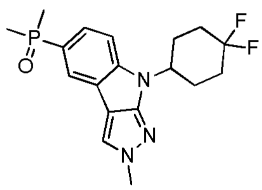

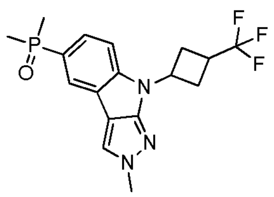

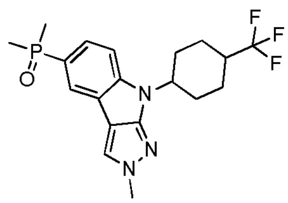

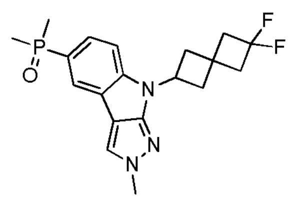

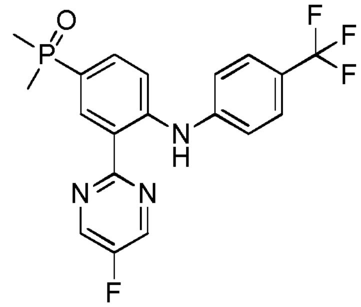

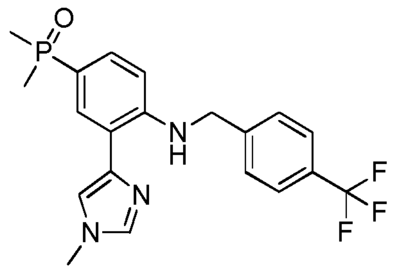

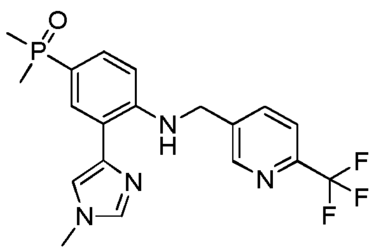

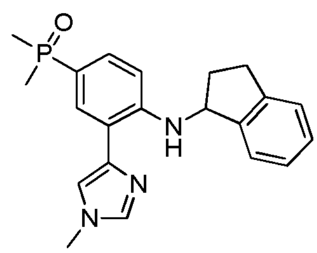

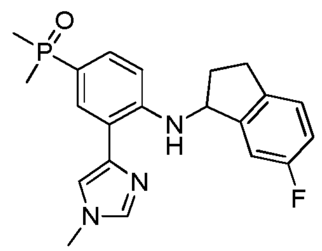

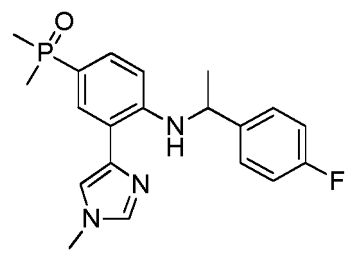

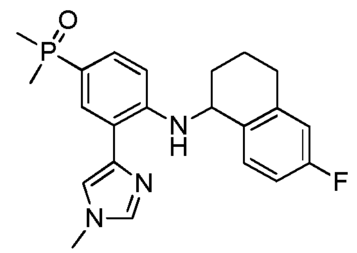

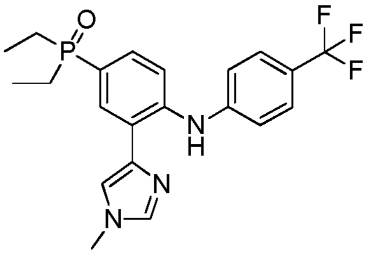
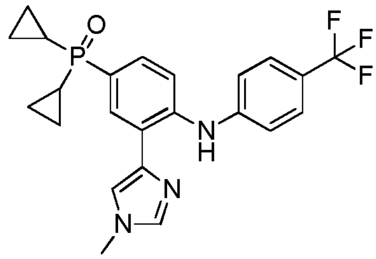
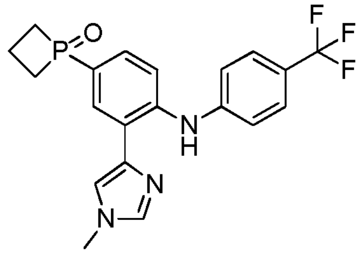

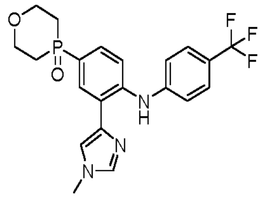
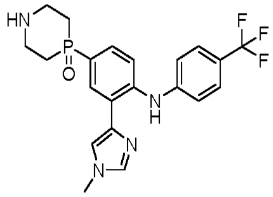
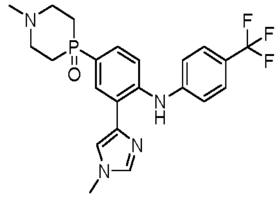
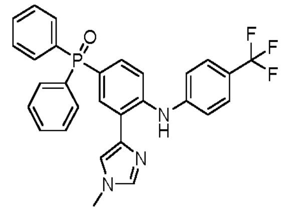

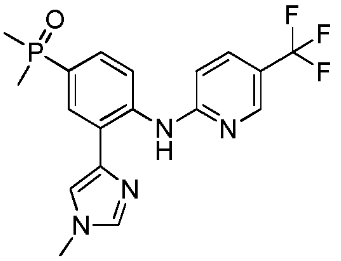
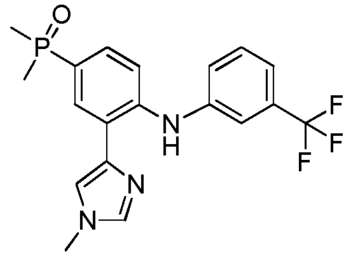
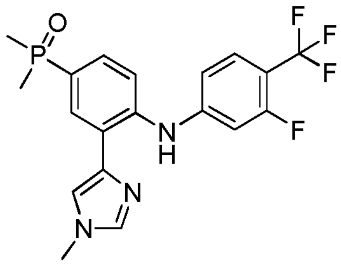

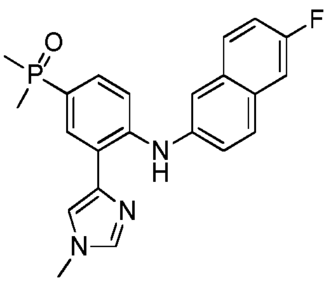
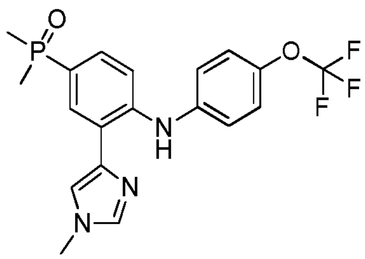
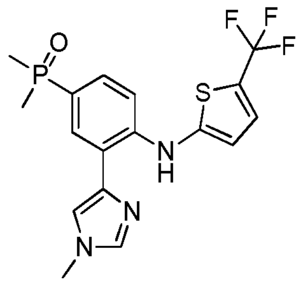

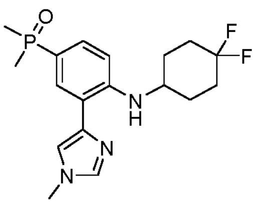
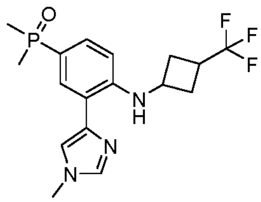
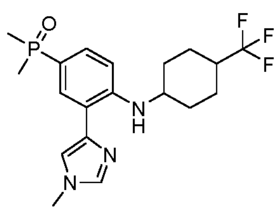
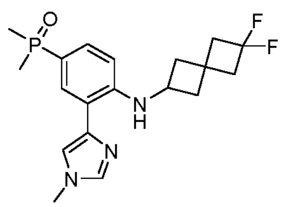

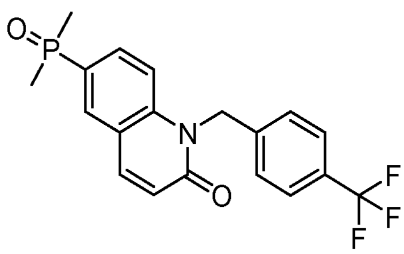
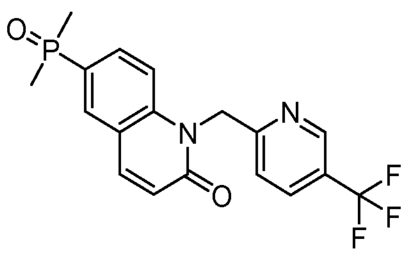

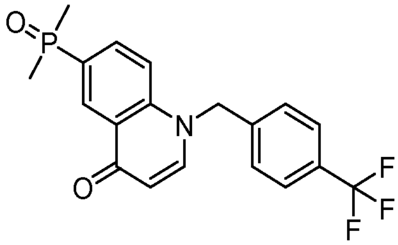
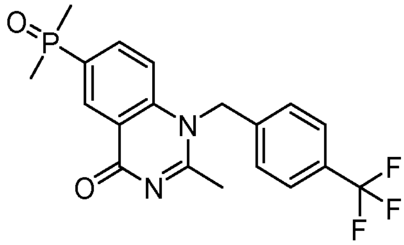
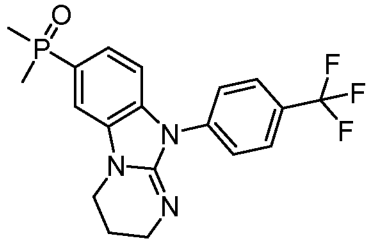

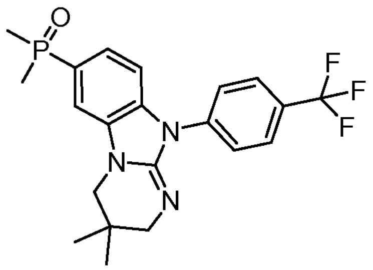
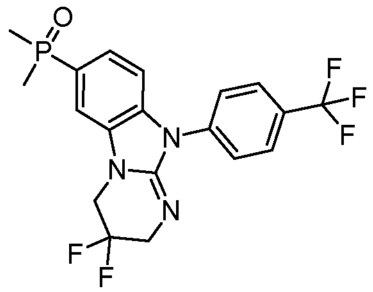
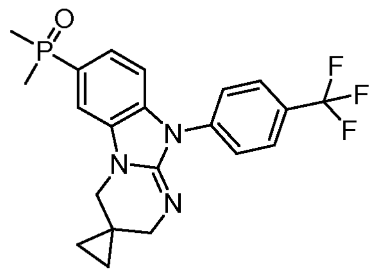

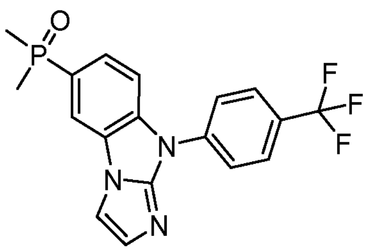
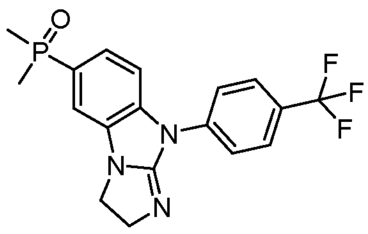
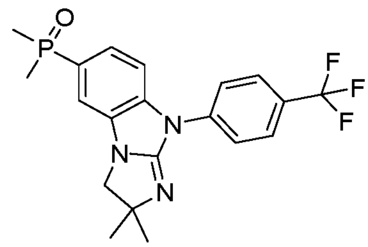

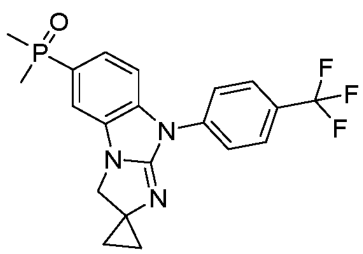

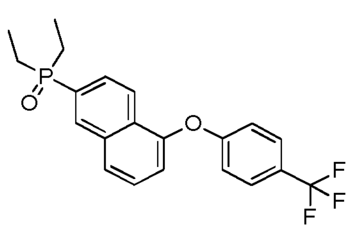
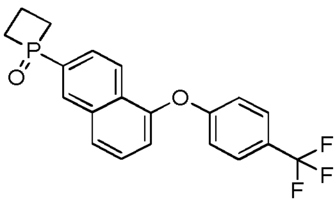
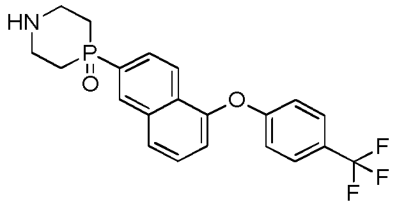

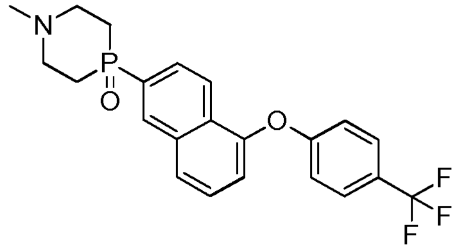
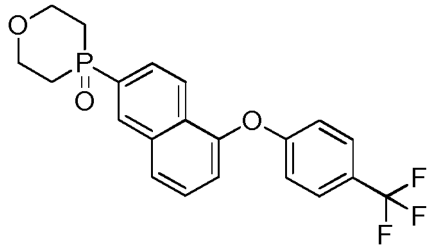

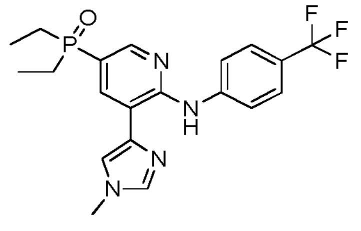
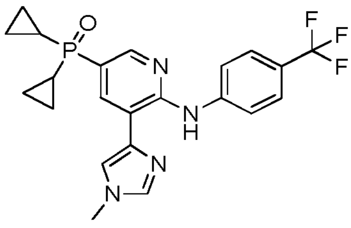
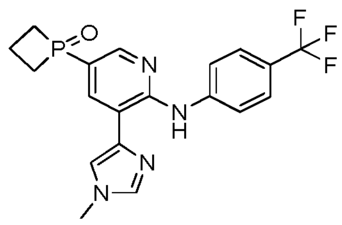

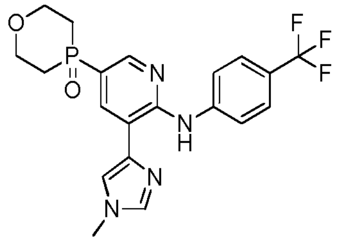
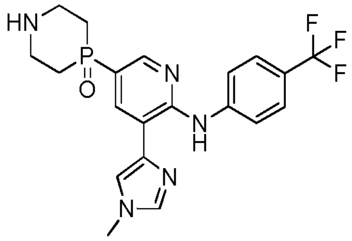
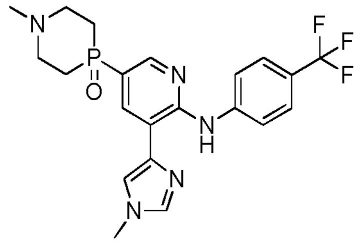

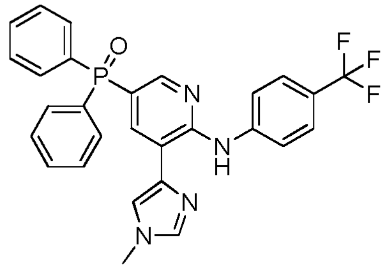
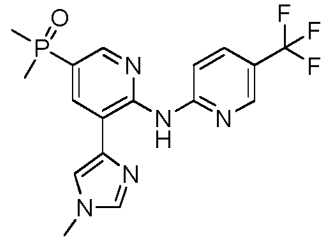
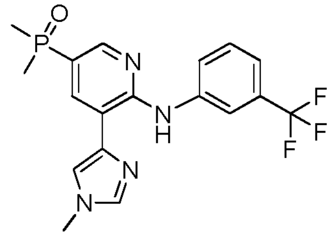

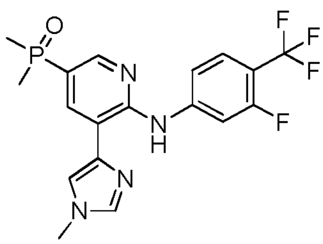
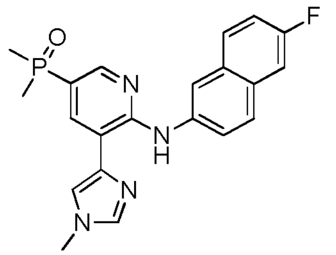
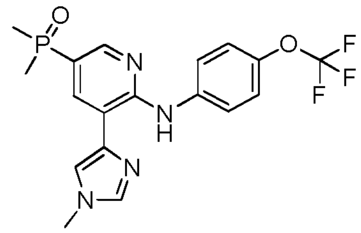

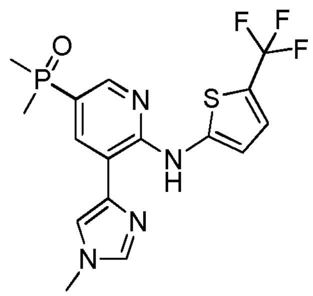
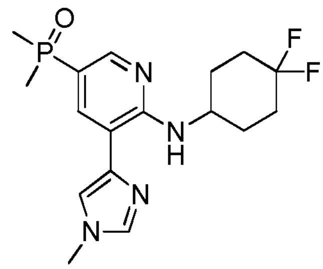
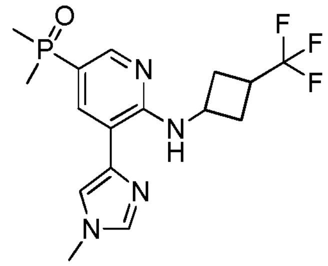

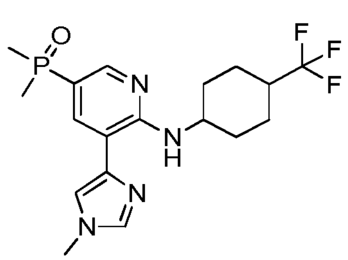
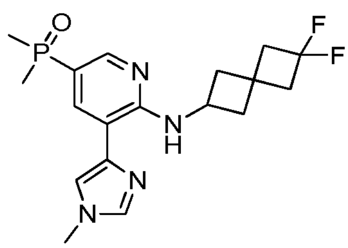

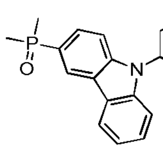
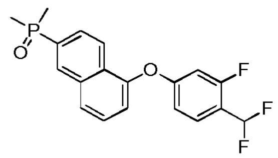
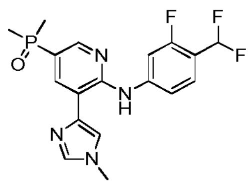
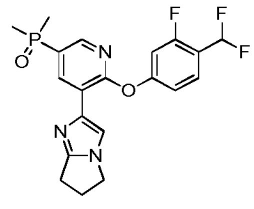

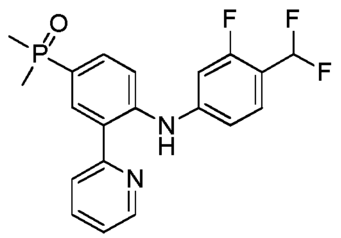
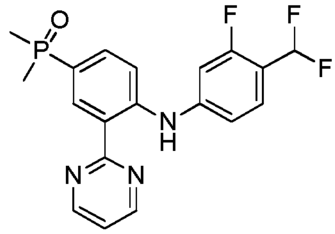
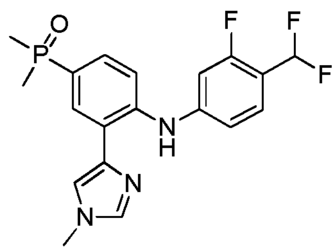

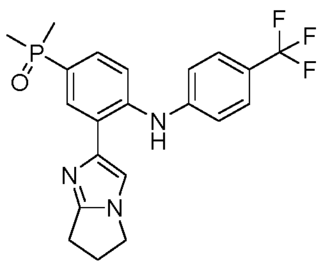
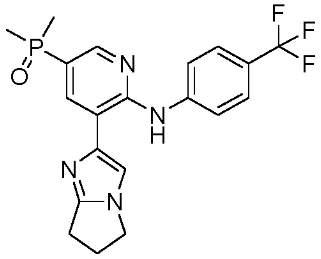
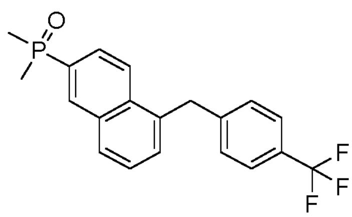

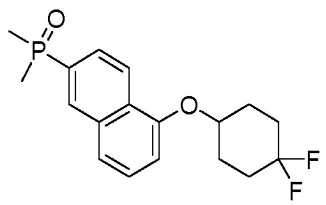
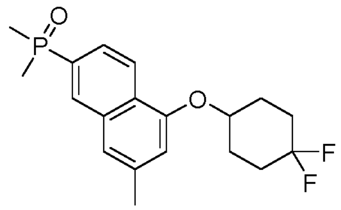
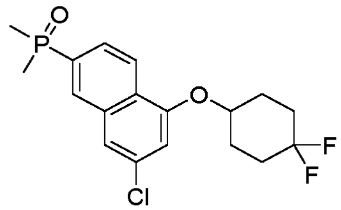

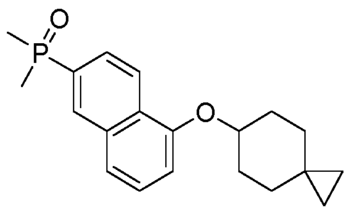
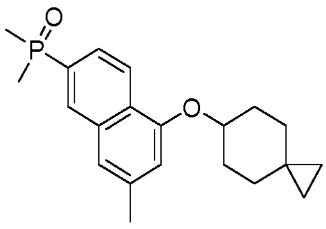
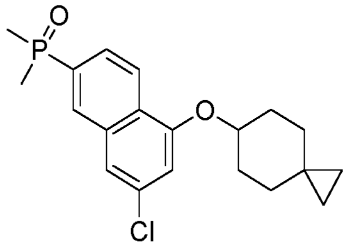

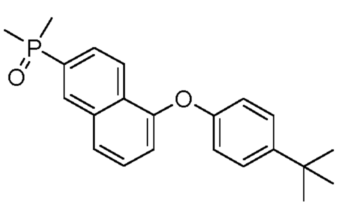
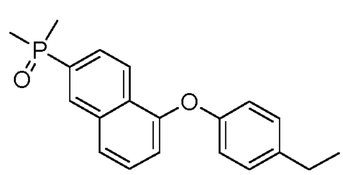
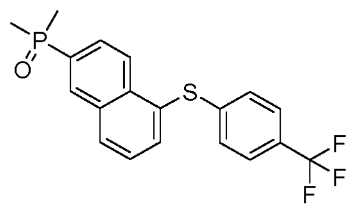

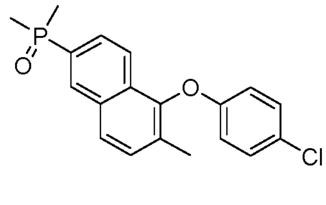
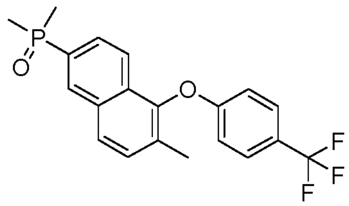
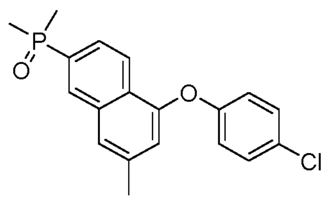
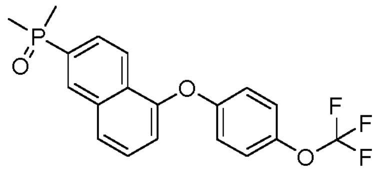
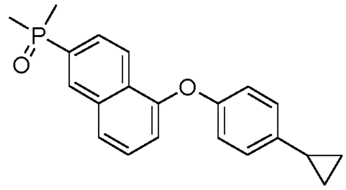
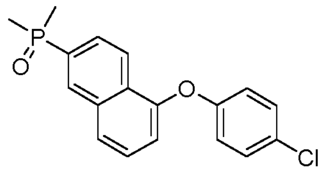
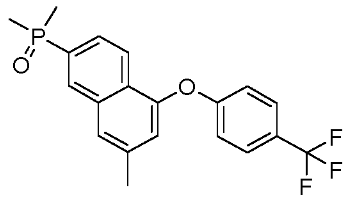
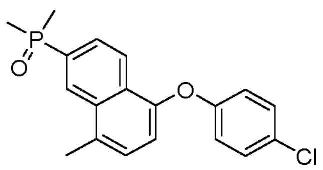
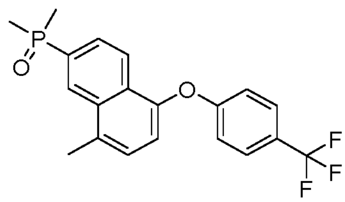
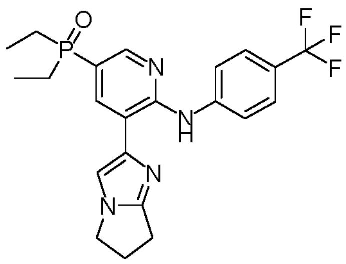
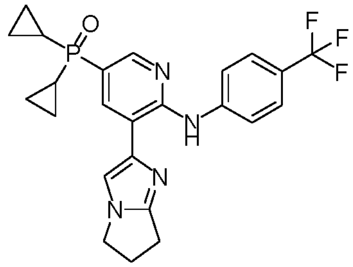
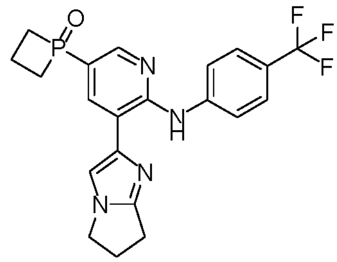
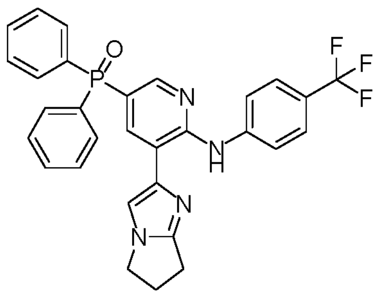
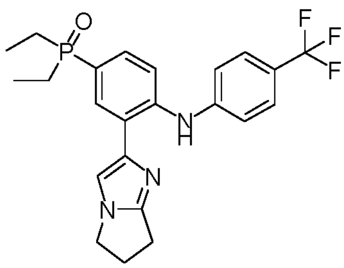
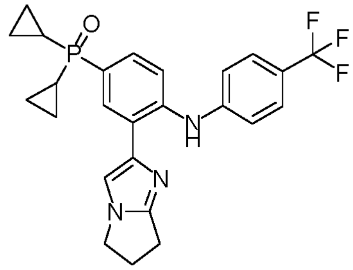
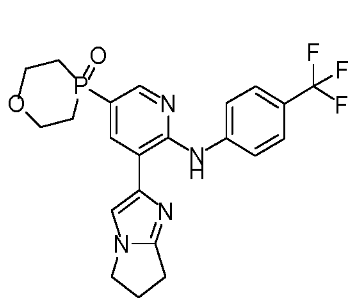
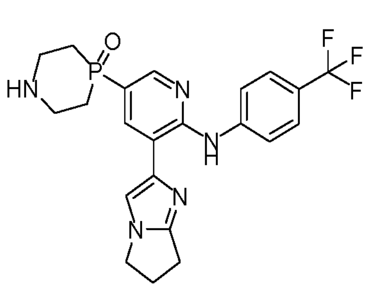
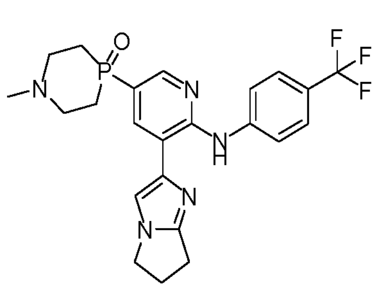

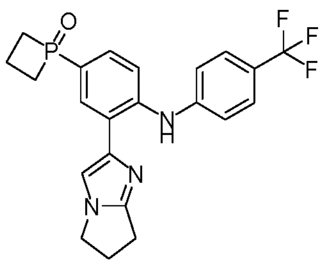

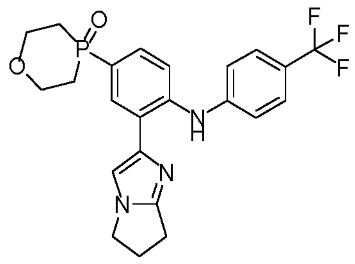

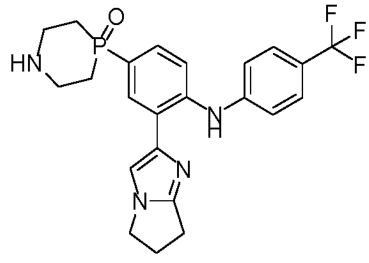

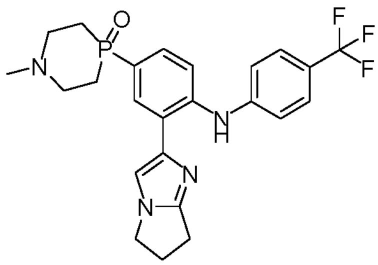

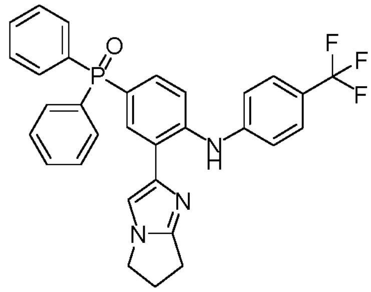

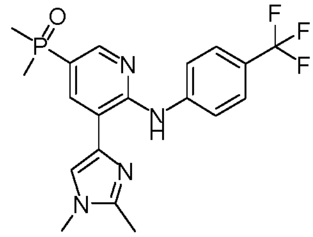

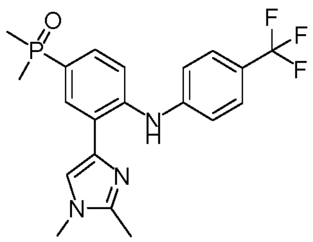

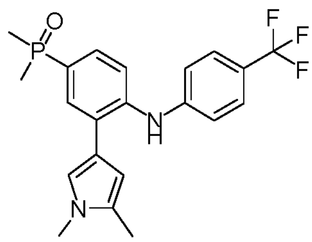

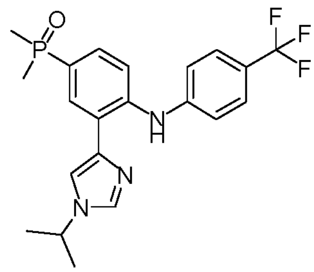

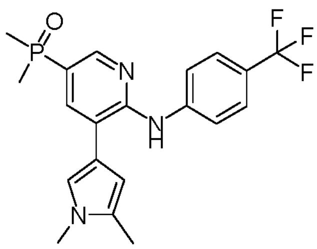

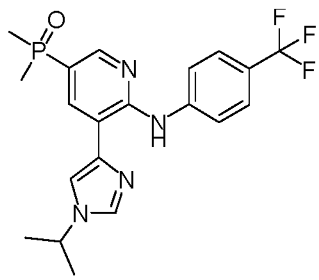

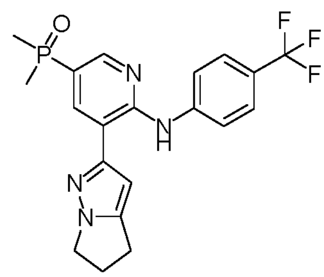

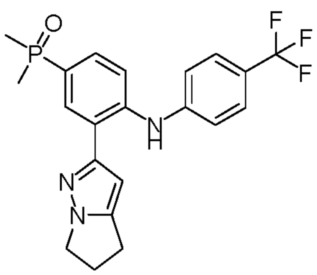

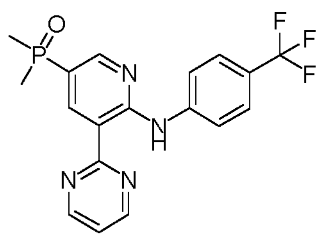

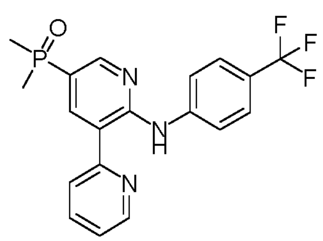

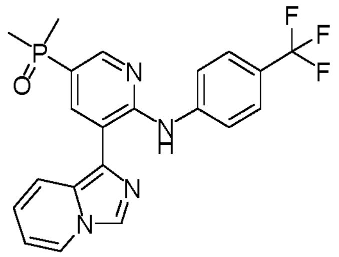
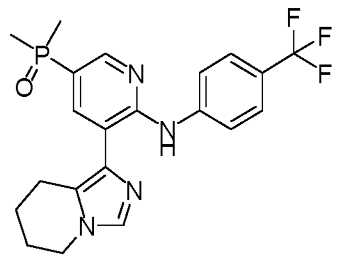
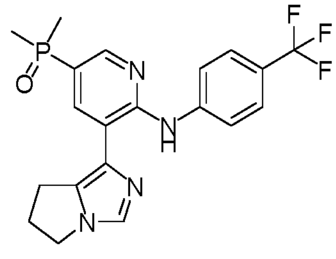
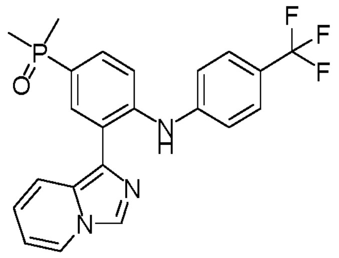
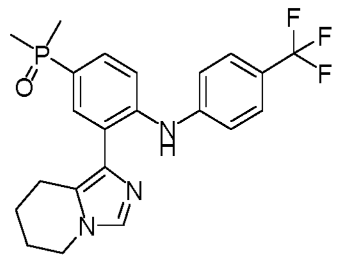
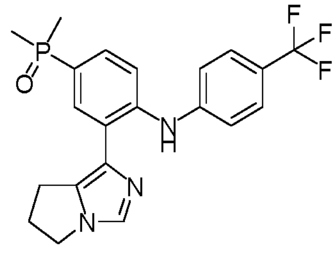
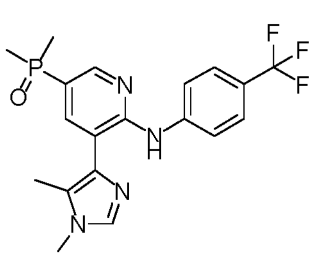
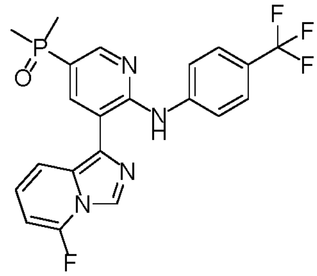
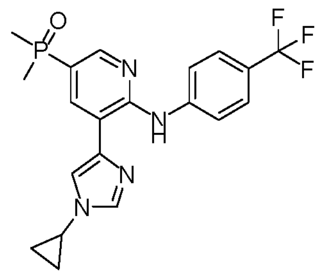
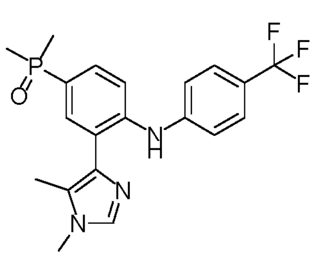
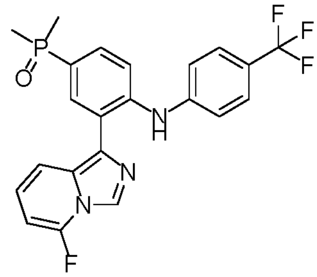
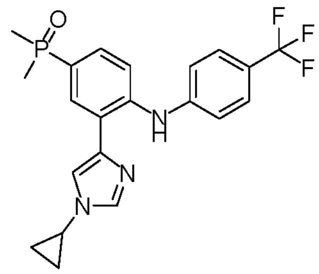
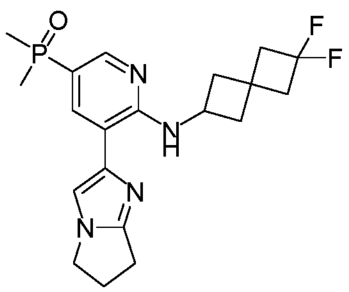
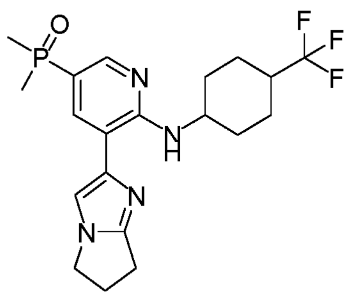
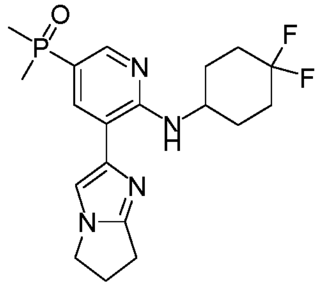

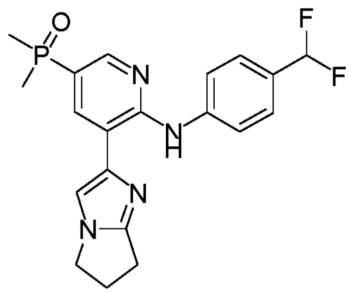
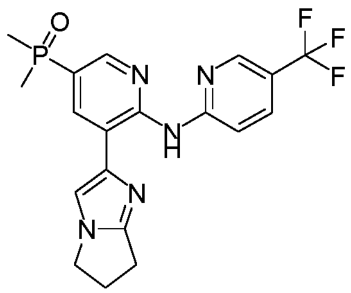
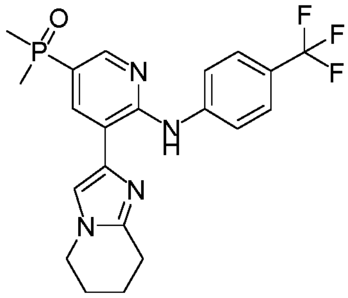

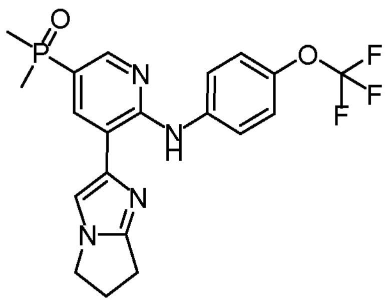
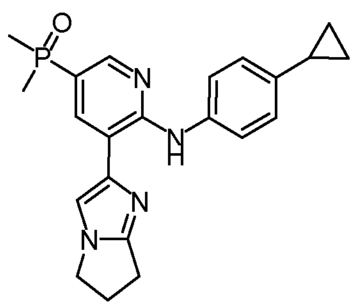
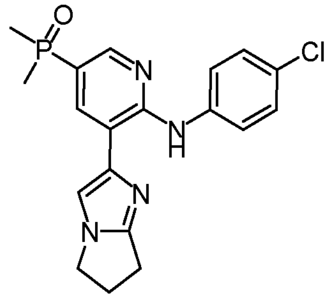

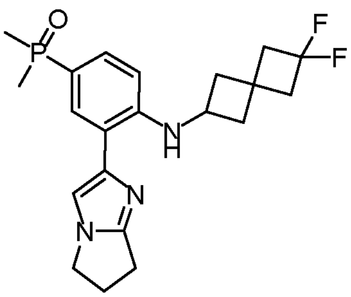
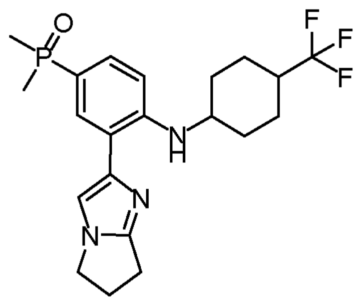
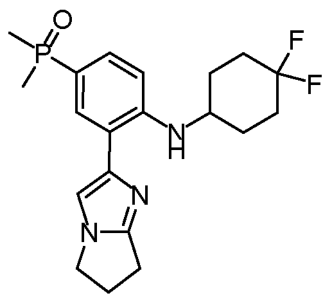

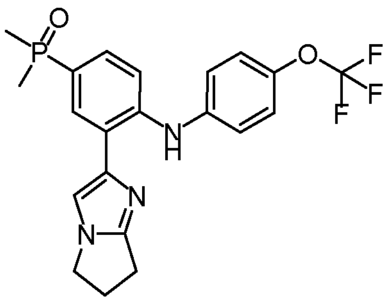
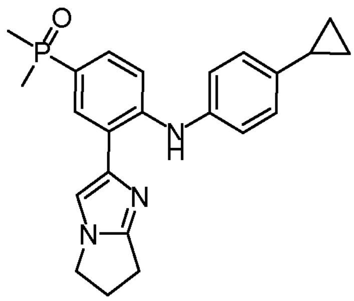
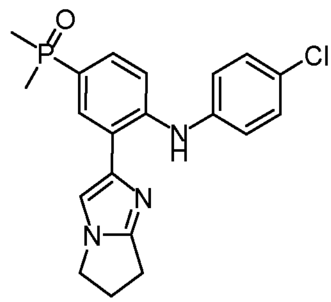

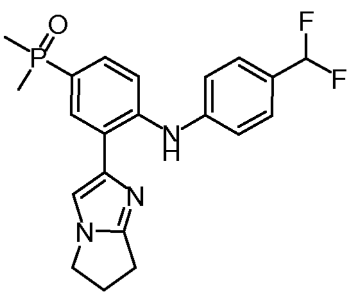
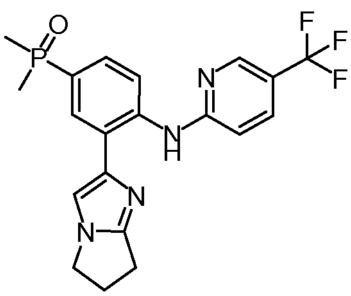
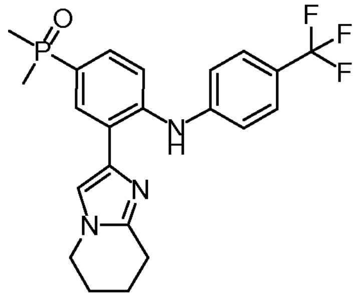

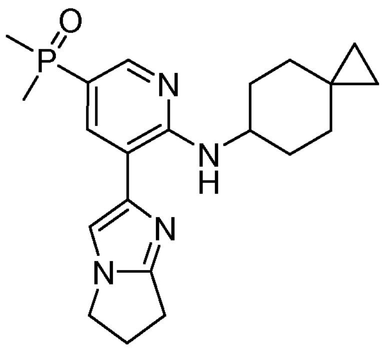
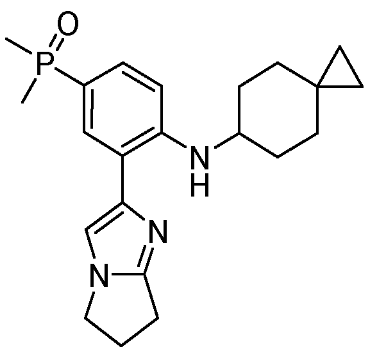
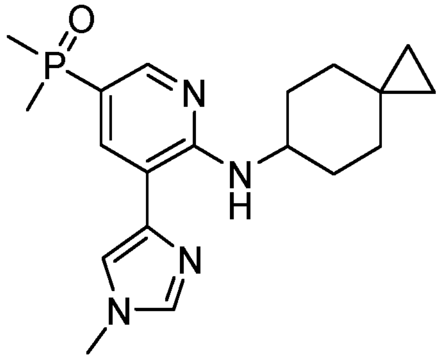
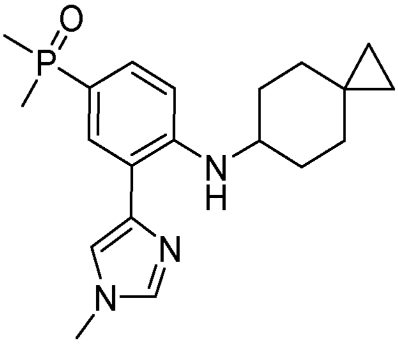
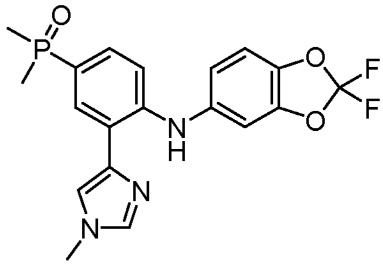
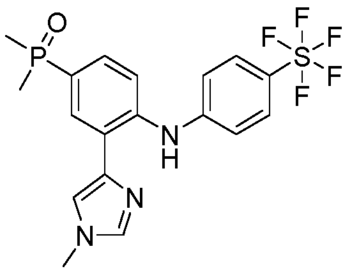
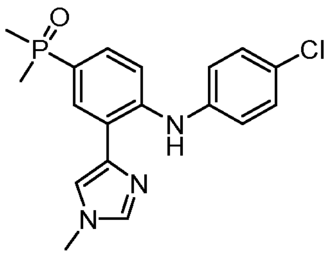
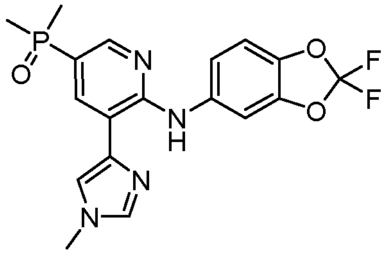
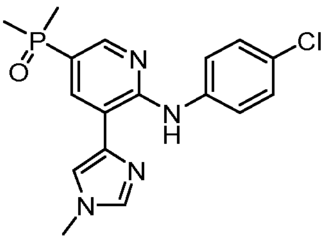
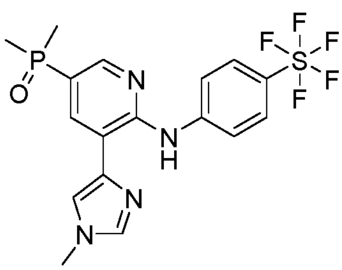
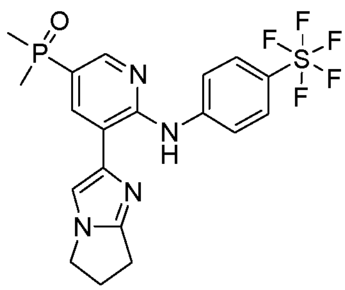
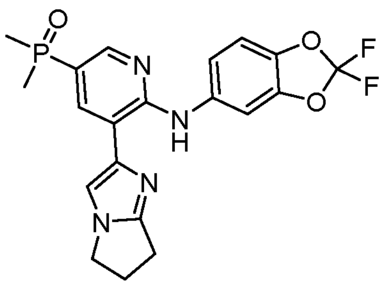

**18.** A pharmaceutical composition, comprising:

(1) the compound represented by formula I, the pharmaceutically acceptable salt thereof, the ester thereof, the stereoisomer thereof, the tautomer thereof, the polymorph thereof, the solvate thereof, the metabolite thereof, the isotopic derivative thereof, or the prodrug thereof according to any one of claims 1-17, and
(2) a pharmaceutically acceptable auxiliary material.

**19.** Use of the compound represented by formula I, the pharmaceutically acceptable salt thereof, the ester thereof, the stereoisomer thereof, the tautomer thereof, the polymorph thereof, the solvate thereof, the metabolite thereof, the isotopic derivative thereof, or the prodrug thereof according to any one of claims 1-17, or the pharmaceutical composition according to claim 18 in preparing a medicament, wherein the medicament is used for treating diseases and disorders mediated by TEAD, such as cancer (e.g. solid tumors and leukemia), organ regeneration, wound healing, and fibrosis.

## INTERNATIONAL SEARCH REPORT

International application No.
**PCT/CN2023/127202**

**A. CLASSIFICATION OF SUBJECT MATTER**

C07F 9/572(2006.01)i; C07F 9/6506(2006.01)i; C07F 9/6584(2006.01)i; A61K 31/66(2006.01)i; A61K 31/505(2006.01)i; A61P 35/00(2006.01)i

According to International Patent Classification (IPC) or to both national classification and IPC

**B. FIELDS SEARCHED**

Minimum documentation searched (classification system followed by classification symbols)

IPC:C07F 9/-; A61K 31/-; A61P 35/-

Documentation searched other than minimum documentation to the extent that such documents are included in the fields searched

Electronic data base consulted during the international search (name of data base and, where practicable, search terms used)

CNABS; CNTXT; VEN; WOTXT; USTXT; EPTXT; GBTXT; CATXT; CNKI; 万方, WANFANG; STN; WEB OF SCIENCE: 转录增强蛋白域; Hippo信号通路; 癌症; 肿瘤; 实体瘤; 白血病; 器官再生; 伤口愈合; 纤维化; 张贵平; 李家鹏; 王奎锋; 段霜霜; 郑计岳; 童晨骅; 王翔; 谢胜武; 刘涛; 勤浩医药（苏州）有限公司; TEAD; YAP/TAZ; CANCER+; TUMOR+; TUMOUR+; LUKAEMIA+; ORGAN REGENERAT+; HEAL+; FIBROSIS+; 式I, I-1, I-2, I-3, I-4, I-5, I-6, I-7结构; 具体化合物结构

**C. DOCUMENTS CONSIDERED TO BE RELEVANT**

| Category* | Citation of document, with indication, where appropriate, of the relevant passages | Relevant to claim No. |
|---|---|---|
| PX | WO 2023204822 A1 (VIVACE THERAPEUTICS, INC.) 26 October 2023 (2023-10-26)<br>description, paragraphs 254-256, 276-279, 299-307, and 337-340 | 1-19 |
| X | CN 102105150 A (ARIAD PHARMACEUTICALS INC.) 22 June 2011 (2011-06-22)<br>description, page 173, compound of sequence 12, page 177, compound of sequence 10, and pages 193, compounds of sequences 13 and 21 in table, and paragraphs 600-602 and 612 | 1-19 |
| X | WO 2019085916 A1 (BEIJING TIDE PHARMACEUTICAL CO., LTD.) 09 May 2019 (2019-05-09)<br>description, page 9, and page 16, paragraph 1 | 1-4, 6, 8, 14-15, 18 |
| X | Aline Marian et al. "Diethyl (iodoethynyl)phosphonate and (iodoethynyl)diphenylphosphane oxide: crystal structures and some cycloaddition reactions"<br>*Zeitschrift für Naturforschung,* Vol. 75, No. 6-7, 30 June 2020 (2020-06-30), 529-536<br>ISSN: 0932-0776,<br>page 531, right-hand column, Schema 2 | 1-6, 14-15 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **24.01月2024（24.01.2024）** | **31 January 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/127202**

| C. | DOCUMENTS CONSIDERED TO BE RELEVANT | |
|---|---|---|
| Category* | Citation of document, with indication, where appropriate, of the relevant passages | Relevant to claim No. |
| A | CN 111039941 A (SHANGHAI PHARMACEUTICALS HOLDING CO., LTD.) 21 April 2020 (2020-04-21) claims 1-21 | 1-19 |
| A | CN 112442011 A (RUNJIA (SUZHOU) PHARMACEUTICAL TECHNOLOGY CO., LTD.) 05 March 2021 (2021-03-05) claims 1-13 | 1-19 |
| A | CN 114502564 A (BETTA PHARMACEUTICALS CO., LTD.) 13 May 2022 (2022-05-13) claim 1 | 1-19 |
| A | WO 2022159986 A1 (IKENA ONCOLOGY, INC.) 28 July 2022 (2022-07-28) claims 1-21 | 1-19 |

Form PCT/ISA/210 (second sheet) (July 2022)

**INTERNATIONAL SEARCH REPORT**
**Information on patent family members**

International application No.
**PCT/CN2023/127202**

| Patent document cited in search report | Publication date (day/month/year) | Patent family member(s) | Publication date (day/month/year) |
|---|---|---|---|
| WO 2023204822 A1 | 26 October 2023 | None | |
| CN 102105150 A | 22 June 2011 | HUS 1900029 I1 | 28 April 2020 |
| | | LTPA 2019510 I1 | 10 June 2019 |
| | | LTC 2300013 I2 | 11 April 2022 |
| | | NO 2300013 T3 | 03 February 2018 |
| | | KR 20160132127 A | 16 November 2016 |
| | | KR 101860057 B1 | 21 May 2018 |
| | | US 2020317705 A1 | 08 October 2020 |
| | | JP 2022116057 A | 09 August 2022 |
| | | US 2012202776 A1 | 09 August 2012 |
| | | US 9012462 B2 | 21 April 2015 |
| | | CY 1119534 T1 | 04 April 2018 |
| | | IL 208716 A0 | 30 December 2010 |
| | | IL 208716 B | 28 February 2018 |
| | | US 2020048288 A1 | 13 February 2020 |
| | | US 2015225436 A1 | 13 August 2015 |
| | | EP 2300013 A1 | 30 March 2011 |
| | | EP 2300013 A4 | 12 September 2012 |
| | | EP 2300013 B1 | 06 September 2017 |
| | | CA 2723961 A1 | 26 November 2009 |
| | | CA 2723961 C | 21 March 2017 |
| | | IL 257083 A | 29 March 2018 |
| | | IL 257083 B | 29 August 2019 |
| | | MX 353308 B | 08 January 2018 |
| | | JP 2017186345 A | 12 October 2017 |
| | | JP 6271064 B2 | 31 January 2018 |
| | | BRPI 0908637 A2 | 23 October 2018 |
| | | BRPI 0908637 B1 | 17 November 2020 |
| | | BRPI 0908637 B8 | 25 May 2021 |
| | | LUC 00120 I1 | 24 May 2019 |
| | | LUC 00120 I2 | 27 December 2019 |
| | | NO 2019024 I1 | 20 May 2019 |
| | | HUE 035029 T2 | 28 March 2018 |
| | | JP 2020125308 A | 20 August 2020 |
| | | SI 2300013 T1 | 30 March 2018 |
| | | WO 2009143389 A1 | 26 November 2009 |
| | | EP 3210609 A1 | 30 August 2017 |
| | | KR 20180014882 A | 09 February 2018 |
| | | KR 101956261 B1 | 08 March 2019 |
| | | HRP 20171534 T1 | 26 January 2018 |
| | | HRP 20171534 T8 | 24 August 2018 |
| | | ES 2645689 T3 | 07 December 2017 |
| | | US 2017218000 A1 | 03 August 2017 |
| | | LT 2300013 T | 27 December 2017 |
| | | EA 201071339 A1 | 30 June 2011 |
| | | EA 029131 B1 | 28 February 2018 |
| | | AU 2009248923 A1 | 26 November 2009 |
| | | AU 2009248923 B2 | 29 January 2015 |
| | | PT 2300013 T | 31 October 2017 |
| | | NL 300990 I1 | 29 May 2019 |

Form PCT/ISA/210 (patent family annex) (July 2022)

**INTERNATIONAL SEARCH REPORT**
**Information on patent family members**

International application No.
**PCT/CN2023/127202**

| Patent document cited in search report | | | Publication date (day/month/year) | Patent family member(s) | | | Publication date (day/month/year) |
|---|---|---|---|---|---|---|---|
| | | | | NL | 300990 | I2 | 11 July 2019 |
| | | | | JP | 2018065864 | A | 26 April 2018 |
| | | | | JP | 6483233 | B2 | 13 March 2019 |
| | | | | FR | 19C1033 | I1 | 21 June 2019 |
| | | | | FR | 19C1033 | I2 | 31 January 2020 |
| | | | | JP | 2019094344 | A | 20 June 2019 |
| | | | | JP | 6690032 | B2 | 28 April 2020 |
| | | | | KR | 20110010801 | A | 07 February 2011 |
| | | | | KR | 101781605 | B1 | 25 September 2017 |
| | | | | DK | 2300013 | T3 | 04 December 2017 |
| | | | | HK | 1158497 | A1 | 20 July 2012 |
| | | | | PL | 2300013 | T3 | 30 May 2018 |
| | | | | MX | 2010012703 | A | 21 December 2010 |
| | | | | JP | 2015163621 | A | 10 September 2015 |
| | | | | JP | 6190415 | B2 | 30 August 2017 |
| | | | | JP | 2011523646 | A | 18 August 2011 |
| WO | 2019085916 | A1 | 09 May 2019 | JP | 2021501184 | A | 14 January 2021 |
| | | | | JP | 7207634 | B2 | 18 January 2023 |
| | | | | KR | 20200081381 | A | 07 July 2020 |
| | | | | AU | 2018361276 | A1 | 14 May 2020 |
| | | | | AU | 2018361276 | B2 | 24 November 2022 |
| | | | | US | 2021179648 | A1 | 17 June 2021 |
| | | | | US | 11414444 | B2 | 16 August 2022 |
| | | | | CA | 3076680 | A1 | 09 May 2019 |
| | | | | EP | 3712144 | A1 | 23 September 2020 |
| | | | | EP | 3712144 | A4 | 14 April 2021 |
| | | | | US | 2022389039 | A1 | 08 December 2022 |
| CN | 111039941 | A | 21 April 2020 | None | | | |
| CN | 112442011 | A | 05 March 2021 | None | | | |
| CN | 114502564 | A | 13 May 2022 | WO | 2021057882 | A1 | 01 April 2021 |
| | | | | US | 2022402948 | A1 | 22 December 2022 |
| WO | 2022159986 | A1 | 28 July 2022 | AU | 2022210800 | A1 | 10 August 2023 |
| | | | | CA | 3205726 | A1 | 28 July 2022 |
| | | | | KR | 20230149885 | A | 27 October 2023 |
| | | | | BR | 112023014751 | A2 | 03 October 2023 |
| | | | | IL | 304492 | A | 01 September 2023 |
| | | | | EP | 4281073 | A1 | 29 November 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202211328612
- CN 202310331481
- CN 202311368477
- CN 202311368432
- CN 202311368532
- WO 2013103738 A1
- WO 2020214734 A1
- WO 2019040380 A1


### Non-patent literature cited in the description


- **P. HEINRICH STAHL** ; **CAMILLE G. WERMUTH**. Handbook of Pharmaceutical Salts: Properties, Selection, and Use. 2011
- **PAUL J SHESKEY** ; **BRUNO C HANCOCK** ; **GARY P MOSS** ; **DAVID J GOLDFARB**. Handbook of Pharmaceutical Excipients. 2020
- *CHEMICAL ABSTRACTS*, 2290608-13-6
- *CHEMICAL ABSTRACTS*, 2821763-12-4